# EUROPEAN PATENT SPECIFICATION

(11) **EP 2 666 859 B1**
(45) Date of publication and mention of the grant of the patent: **02.01.2019**
(21) Application number: 13172958.4
(22) Date of filing: 30.03.2007
(51) Int. Cl.: C12N 15/113, C07H 21/00, A61K 31/712

(54) **PHARMACEUTICAL COMPOSITION COMPRISING ANTI-MIRNA ANTISENSE OLIGONUCLEOTIDES**
PHARMAZEUTISCHE ZUSAMMENSETZUNGEN MIT ANTI-MIRNA-ANTISENSE-OLIGONUKLEOTIDEN
COMPOSITIONS PHARMACEUTIQUES COMPRENANT DES OLIGONUCLÉOTIDES ANTISENS ANTI-MIRNA

(30) Priority: 03.04.2006 US 788995 P; 03.04.2006 DK 200600478; 01.05.2006 DK 200600615; 01.05.2006 US 796813 P; 18.08.2006 US 838710 P; 30.10.2006 DK 200601401
(43) Date of publication of application: 27.11.2013
(62) Divisional of application: 10158023.1
(73) Proprietor: Roche Innovation Center Copenhagen A/S, 2970 Hørsholm (DK)
(72) Inventor: Elmén, Joacim, 120 67 Stockholm (SE); Kauppinen, Sakari, 2765 Smoerum (DK); Kearney, Phil, Picton New South Wales 2571 (AU)
(74) Representative: D Young & Co LLP

(56) References cited:
- WO-A2-2004/046160
- WO-A2-2005/013901
- WO-A2-2007/112753
- CHAN J A ET AL: "MicroRNA-21 is an antiapoptotic factor in human glioblastoma cells", CANCER RESEARCH, vol. 65, no. 14, 15 July 2005 (2005-07-15) , pages 6029-6033, XP002457479, ISSN: 0008-5472, DOI: 10.1158/0008-5472.CAN-05-0137
- ELAYADI A ET AL: "Implications of High-Affinity Hybridization by locked Nucleic Acid Oligomers for Inhibition of human Telomerase", BIOCHEMISTRY, vol. 41, no. 31, 2002, pages 9973-9981, XP002990849, ISSN: 0006-2960, DOI: 10.1021/BI025907J
- LECELLIER CHARLES-HENRI ET AL: "A cellular MicroRNA mediates antiviral defense in human cells", SCIENCE, vol. 308, no. 5721, 22 April 2005 (2005-04-22), pages 557-560, XP002609815, ISSN: 0036-8075
- OROM ET AL: "LNA-modified oligonucleotides mediate specific inhibition of microRNA function", GENE, vol. 372, 10 May 2006 (2006-05-10), pages 137-141, XP005401958, ISSN: 0378-1119, DOI: 10.1016/J.GENE.2005.12.031
- JEPSEN J S ET AL: "LNA-ANTISENSE RIVALS SIRNA FOR GENE SILENCING", CURRENT OPINION IN DRUG DISCOVERY AND DEVELOPMENT, CURRENT DRUGS, LONDON, GB, vol. 7, no. 2, 1 January 2004 (2004-01-01) , pages 188-194, XP009083873, ISSN: 1367-6733
- Sakari Kauppinen: "Novel antisense drugs for micro-RNA therapeutics", European pharmaceutical review, 20 July 2006 (2006-07-20), XP055218913, Retrieved from the Internet: URL:http://www.europeanpharmaceuticalrevie w.com/2647/european-pharmaceutical-review- magazine/past-issues/novel-antisense-drugs -for-micro-rna-therapeutics/ [retrieved on 2015-10-07]
- Elmen, J. et al. : 'LNA-antimiRs: promising candidates for therapeutic intervention of disease-related microRNAs.', Poster presented at the 71st Symposium on Quantitative Biology: Regulatory RNAs, Cold Spring Harbor, NY, USA, May 31 - June 5, 2006

### SEQUENCE LISTING

<110> Elmen, Joacim Kearney, Phil Kauppinen, Sakari
<120> PHARMACEUTICAL COMPOSITION
<130> 2763.0140006
<140> 12/295,960
   <141> 2009-08-20
<150> PCT/DK2007/000168
   <151> 2007-03-30
<150> PA 2006 00478
   <151> 2006-04-03
<150> US 60/788,995
   <151> 2006-04-03
<150> PA 2006 00615
   <151> 2006-05-01
<150> US 60/796,813
   <151> 2006-05-01
<150> US 60/838,710
   <151> 2006-08-18
<150> PA 2006 01401
   <151> 2006-10-30
<160> 110
<170> PatentIn version 3.3
<210> 1
   <211> 23
   <212> RNA
   <213> Homo sapiens
<400> 1
   uggaguguga caaugguguu ugu 23
<210> 2
   <211> 23
   <212> RNA
   <213> Homo sapiens
<400> 2
   ugugcaaauc caugcaaaac uga 23
<210> 3
   <211> 22
   <212> RNA
   <213> Homo sapiens
<400> 3
   uuaaugcuaa ucgugauagg gg 22
<210> 4
   <211> 22
   <212> RNA
   <213> Homo sapiens
<400> 4
   uagcuuauca gacugauguu ga 22
<210> 5
   <211> 22
   <212> RNA
   <213> Homo sapiens
<400> 5
   uuuguucguu cggcucgcgu ga 22
<210> 6
   <211> 6
   <212> DNA
   <213> Artificial Sequence
<220>
   <223> Synthetic oligonucleotide
<400> 6
   tttgca 6
<210> 7
   <211> 6
   <212> DNA
   <213> Artificial Sequence
<220>
   <223> Synthetic oligonucleotide
<400> 7
   acactc 6
<210> 8
   <211> 6
   <212> DNA
   <213> Artificial Sequence
<220>
   <223> Synthetic oligonucleotide
<400> 8
   agcatt 6
<210> 9
   <211> 6
   <212> DNA
   <213> Artificial Sequence
<220>
   <223> Synthetic oligonucleotide
<400> 9
   cgaaca 6
<210> 10
   <211> 6
   <212> DNA
   <213> Artificial Sequence
<220>
   <223> Synthetic oligonucleotide
<400> 10
   ataagc 6
<210> 11
   <211> 23
   <212> DNA
   <213> Artificial Sequence
<220>
   <223> Synthetic oligonucleotide
<220>
   <221> misc_feature
   <222> 1, 2, 3, 4, 20, 21, 22, 23
   <223> LNA modified nucleotide
<400> 11
   acaaacacca ttgtcacact cca 23
<210> 12
   <211> 23
   <212> DNA
   <213> Artificial Sequence
<220>
   <223> Synthetic oligonucleotide
<220>
   <221> misc_feature
   <222> 7, 8, 9, 10, 11, 12, 13, 14
   <223> LNA modified nucleotide
<400> 12
   acaaacacca ttgtcacact cca 23
<210> 13
   <211> 23
   <212> DNA
   <213> Artificial Sequence
<220>
   <223> Synthetic oligonucleotide
<220>
   <221> misc_feature
   <222> 3, 6, 9, 12, 15, 18, 21
   <223> LNA modified nucleotide
<400> 13
   acaaacacca ttgtcacact cca 23
<210> 14
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<400> 14
   ccattgtcac actcc 15
<210> 15
   <211> 15
   <212> DNA
   <213> Artificial Sequence
<220>
   <223> Synthetic oligonucleotide
<220>
   <221> misc_feature
   <222> 1, 3, 5, 6, 7, 10, 11, 12, 14, 15
   <223> LNA modified nucleotide
<400> 15
   ccattgtcac actcc 15
<210> 16
   <211> 13
   <212> DNA
   <213> Artificial Sequence
<220>
   <223> Synthetic oligonucleotide
<220>
   <221> misc_feature
   <222> 1, 2, 3, 4, 5, 7, 8, 9, 10, 12, 13
   <223> LNA modified nucleotide
<400> 16
   attgtcacac tcc 13
<210> 17
   <211> 11
   <212> DNA
   <213> Artificial Sequence
<220>
   <223> Synthetic oligonucleotide
<220>
   <221> misc_feature
   <222> 1, 2, 3, 5, 6, 7, 8, 10, 11
   <223> LNA modified nucleotide
<400> 17
   tgtcacactc c 11
<210> 18
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-OME-RNA units
<400> 18
   ccattgtcac actcc 15
<210> 19
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-fluoro DNA units
<400> 19
   ccattgtcac actcc 15
<210> 20
   <211> 23
   <212> DNA
   <213> Artificial Sequence
<220>
   <223> Synthetic oligonucleotide
<220>
   <221> misc_feature
   <222> 1, 2, 3, 4, 20, 21, 22, 23
   <223> LNA modified nucleotide
<400> 20
   tcagttttgc atggatttgc aca 23
<210> 21
   <211> 23
   <212> DNA
   <213> Artificial Sequence
<220>
   <223> Synthetic oligonucleotide
<220>
   <221> misc_feature
   <222> 7, 8, 9, 10, 11, 12, 13, 14
   <223> LNA modified nucleotide
<400> 21
   tcagttttgc atggatttgc aca 23
<210> 22
   <211> 23
   <212> DNA
   <213> Artificial Sequence
<220>
   <223> Synthetic oligonucleotide
<220>
   <221> misc_feature
   <222> 3, 6, 9, 12, 15, 18, 21
   <223> LNA modified nucleotide
<400> 22
   tcagttttgc atggatttgc aca 23
<210> 23
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<400> 23
   tgcatggatt tgcac 15
<210> 24
   <211> 15
   <212> DNA
   <213> Artificial Sequence
<220>
   <223> Synthetic oligonucleotide
<220>
   <221> misc_feature
   <222> 1, 3, 5, 6, 7, 10, 11, 12, 14, 15
   <223> LNA modified nucleotide
<400> 24
   tgcatggatt tgcac 15
<210> 25
   <211> 13
   <212> DNA
   <213> Artificial Sequence
<220>
   <223> Synthetic oligonucleotide
<220>
   <221> misc_feature
   <222> 1, 3, 4, 5, 7, 8, 9, 10, 12, 13
   <223> LNA modified nucleotide
<400> 25
   catggatttg cac 13
<210> 26
   <211> 11
   <212> DNA
   <213> Artificial Sequence
<220>
   <223> Synthetic oligonucleotide
<220>
   <221> misc_feature
   <222> 1, 2, 3, 5, 6, 7, 8 , 10, 11
   <223> LNA modified nucleotide
<400> 26
   tggatttgca c 11
<210> 27
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-OME-RNA units
<400> 27
   tgcatggatt tgcac 15
<210> 28
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-fluoro DNA units
<400> 28
   tgcatggatt tgcac 15
<210> 29
   <211> 22
   <212> DNA
   <213> Artificial Sequence
<220>
   <223> Synthetic oligonucleotide
<220>
   <221> misc_feature
   <222> 1, 2, 3, 4, 20, 21, 22, 23
   <223> LNA modified nucleotide
<400> 29
   cccctatcac gattagcatt aa 22
<210> 30
   <211> 22
   <212> DNA
   <213> Artificial Sequence
<220>
   <223> Synthetic oligonucleotide
<220>
   <221> misc_feature
   <222> 7, 8, 9, 10, 11, 12, 13, 14
   <223> LNA modified nucleotide
<400> 30
   cccctatcac gattagcatt aa 22
<210> 31
   <211> 22
   <212> DNA
   <213> Artificial Sequence
<220>
   <223> Synthetic oligonucleotide
<220>
   <221> misc_feature
   <222> 2, 5, 8, 11, 14, 17, 20
   <223> LNA modified nucleotide
<400> 31
   cccctatcac gattagcatt aa 22
<210> 32
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<400> 32
   tcacgattag catta 15
<210> 33
   <211> 15
   <212> DNA
   <213> Artificial Sequence
<220>
   <223> Synthetic oligonucleotide
<220>
   <221> misc_feature
   <222> 1, 3, 5, 6, 7, 10, 11, 12, 14, 15
   <223> LNA modified nucleotide
<400> 33
   tcacgattag catta 15
<210> 34
   <211> 13
   <212> DNA
   <213> Artificial Sequence
<220>
   <223> Synthetic oligonucleotide
<220>
   <221> misc_feature
   <222> 1, 2, 3, 4, 5, 7, 8, 9, 10, 12, 13
   <223> LNA modified nucleotide
<400> 34
   acgattagca tta 13
<210> 35
   <211> 11
   <212> DNA
   <213> Artificial Sequence
<220>
   <223> Synthetic oligonucleotide
<220>
   <221> misc_feature
   <222> 1, 2, 3, 5, 6, 7, 9, 10, 11
   <223> LNA modified nucleotide
<400> 35
   gattagcatt a 11
<210> 36
   <211> 15
   <212> DNA
   <213> Artificial Sequence
<220>
   <223> Synthetic oligonucleotide
<220>
   <221> misc_feature
   <222> 1, 3, 6, 7, 8, 10, 12, 14, 15
   <223> LNA modified nucleotide
<220>
   <221> misc_feature
   <222> 2, 4, 5, 9, 11, 13
   <223> 2-prime-OME RNA units
<400> 36
   tcacgattag catta 15
<210> 37
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-fluoro DNA units
<400> 37
   tcacgattag catta 15
<210> 38
   <211> 22
   <212> DNA
   <213> Artificial Sequence
<220>
   <223> Synthetic oligonucleotide
<220>
   <221> misc_feature
   <222> 1, 2, 3, 4, 20, 21, 22, 23
   <223> LNA modified nucleotide
<400> 38
   tcaacatcag tctgataagc ta 22
<210> 39
   <211> 22
   <212> DNA
   <213> Artificial Sequence
<220>
   <223> Synthetic oligonucleotide
<220>
   <221> misc_feature
   <222> 7, 8, 9, 10, 11, 12, 13, 14
   <223> LNA modified nucleotide
<400> 39
   tcaacatcag tctgataagc ta 22
<210> 40
   <211> 22
   <212> DNA
   <213> Artificial Sequence
<220>
   <223> Synthetic oligonucleotide
<220>
   <221> misc_feature
   <222> 3, 6, 9, 12, 15, 18, 19, 21
   <223> LNA modified nucleotide
<400> 40
   tcatcatcag tctgataagc tt 22
<210> 41
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<400> 41
   tcagtctgat aagct 15
<210> 42
   <211> 15
   <212> DNA
   <213> Artificial Sequence
<220>
   <223> Synthetic oligonucleotide
<220>
   <221> misc_feature
   <222> 1, 3, 5, 6, 7, 10, 11, 12, 14, 15
   <223> LNA modified nucleotide
<400> 42
   tcagtctgat aagct 15
<210> 43
   <211> 13
   <212> DNA
   <213> Artificial Sequence
<220>
   <223> Synthetic oligonucleotide
<220>
   <221> misc_feature
   <222> 1, 2, 3, 4, 5, 7, 8, 9, 10, 12, 13
   <223> LNA modified nucleotide
<400> 43
   agtctgataa gct 13
<210> 44
   <211> 11
   <212> DNA
   <213> Artificial Sequence
<220>
   <223> Synthetic oligonucleotide
<220>
   <221> misc_feature
   <222> 1, 2, 3, 5, 7, 8, 9, 10, 11
   <223> LNA modified nucleotide
<400> 44
   tctgataagc t 15
<210> 45
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-OME RNA units
<400> 45
   tcagtctgat aagct 15
<210> 46
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-fluoro DNA units
<400> 46
   tcagtctgat aagct 15
<210> 47
   <211> 22
   <212> DNA
   <213> Artificial Sequence
<220>
   <223> Synthetic oligonucleotide
<220>
   <221> misc_feature
   <222> 1, 2, 3, 4, 19, 20, 21, 22
   <223> LNA modified nucleotide
<400> 47
   tctcgcgtgc cgttcgttct tt 22
<210> 48
   <211> 22
   <212> DNA
   <213> Artificial Sequence
<220>
   <223> Synthetic oligonucleotide
<220>
   <221> misc_feature
   <222> 7, 8, 9, 10, 11, 12, 13, 14
   <223> LNA modified nucleotide
<400> 48
   tctcgcgtgc cgttcgttct tt 22
<210> 49
   <211> 22
   <212> DNA
   <213> Artificial Sequence
<220>
   <223> Synthetic oligonucleotide
<220>
   <221> misc_feature
   <222> 3, 6, 9, 12, 15, 18, 21
   <223> LNA modified nucleotide
<400> 49
   tctcgcgtgc cgttcgttct tt 22
<210> 50
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<400> 50
   gtgccgttcg ttctt 15
<210> 51
   <211> 15
   <212> DNA
   <213> Artificial Sequence
<220>
   <223> Synthetic oligonucleotide
<220>
   <221> misc_feature
   <222> 1, 3, 5, 6, 7, 10, 11, 12, 14, 15
   <223> LNA modified nucleotide
<400> 51
   gtgccgttcg ttctt 15
<210> 52
   <211> 13
   <212> DNA
   <213> Artificial Sequence
<220>
   <223> Synthetic oligonucleotide
<220>
   <221> misc_feature
   <222> 1, 2, 3, 4, 5, 7, 9, 10, 11, 12, 13
   <223> LNA modified nucleotide
<400> 52
   gccgttcgtt ctt 13
<210> 53
   <211> 11
   <212> DNA
   <213> Artificial Sequence
<220>
   <223> Synthetic oligonucleotide
<220>
   <221> misc_feature
   <222> 1, 2, 3, 4, 6, 7, 8, 9, 10, 11
   <223> LNA modified nucleotide
<400> 53
   cgttcgttct t 11
<210> 54
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-OME RNA units
<400> 54
   gtgccgttcg ttctt 15
<210> 55
   <211> 15
   <212> DNA
   <213> Artificial Sequence
<220>
   <223> Synthetic oligonucleotide
<220>
   <221> misc_feature
   <222> 1, 3, 6, 7, 10, 12, 14, 15
   <223> LNA modified nucleotide
<220>
   <221> misc_feature
   <222> 2, 4, 5, 8, 9, 11, 13
   <223> 2-prime-fluoro RNA units
<400> 55
   gtgccgttcg ttctt 15
<210> 56
   <211> 16
   <212> DNA
   <213> Artificial Sequence
<220>
   <223> Synthetic oligonucleotide
<220>
   <221> misc_feature
   <222> (1)...(16)
   <223> phosphorothioate linkage
<220>
   <221> misc_feature
   <222> 2, 8, 14
   <223> LNA modified, 5-prime methylated cytosine
<220>
   <221> misc_feature
   <222> 5, 11
   <223> LNA modified nucleotide
<400> 56
   ccattgtcac actcca 16
<210> 57
   <211> 16
   <212> DNA
   <213> Artificial Sequence
<220>
   <223> Synthetic oligonucleotide
<220>
   <221> misc_feature
   <222> (1)...(16)
   <223> phosphorothioate linkage
<220>
   <221> misc_feature
   <222> 3, 6, 9
   <223> LNA modified nucleotide
<220>
   <221> misc_feature
   <222> 12, 15
   <223> LNA modified, 5-prime methylated cytosine
<400> 57
   ccattgtcac actcca 16
<210> 58
   <211> 16
   <212> DNA
   <213> Artificial Sequence
<220>
   <223> Synthetic oligonucleotide
<220>
   <221> misc_feature
   <222> (1)...(16)
   <223> phosphorothioate linkage
<220>
   <221> misc_feature
   <222> 1, 2, 14, 15
   <223> LNA modified, 5-prime methylated cytosine
<220>
   <221> misc_feature
   <222> 3, 13
   <223> LNA modified nucleotide
<400> 58
   ccattgtcac actcca 16
<210> 59
   <211> 15
   <212> DNA
   <213> Artificial Sequence
<220>
   <223> Synthetic oligonucleotide
<220>
   <221> misc_feature
   <222> (1)...(15)
   <223> phosphorothioate linkage
<220>
   <221> misc_feature
   <222> 1, 10, 12, 14, 15
   <223> LNA modified, 5-prime methylated cytosine
<220>
   <221> misc_feature
   <222> 3, 6, 7
   <223> LNA modified nucleotide
<400> 59
   ccattgtcac actcc 15
<210> 60
   <211> 15
   <212> DNA
   <213> Artificial Sequence
<220>
   <223> Synthetic oligonucleotide
<220>
   <221> misc_feature
   <222> (1)...(15)
   <223> phosphorothioate linkage
<220>
   <221> misc_feature
   <222> 1, 6, 10, 12, 15
   <223> LNA modified, 5-prime methylated cytosine
<220>
   <221> misc_feature
   <222> 3, 7, 14
   <223> LNA modified nucleotide
<400> 60
   ccattctgac cctac 15
<210> 61
   <211> 16
   <212> DNA
   <213> Artificial Sequence
<220>
   <223> Synthetic oligonucleotide
<220>
   <221> misc_feature
   <222> (1)...(16)
   <223> phosphorothioate linkage
<220>
   <221> misc_feature
   <222> 3, 6, 9, 12
   <223> LNA modified nucleotide
<220>
   <221> misc_feature
   <222> 15
   <223> LNA modified, 5-prime methylated cytosine
<400> 61
   ccattgtctc aatcca 16
<210> 62
   <211> 13
   <212> DNA
   <213> Artificial Sequence
<220>
   <223> Synthetic oligonucleotide
<220>
   <221> misc_feature
   <222> (1)...(13)
   <223> phosphorothioate linkage
<220>
   <221> misc_feature
   <222> 1, 4, 5
   <223> LNA modified nucleotide
<220>
   <221> misc_feature
   <222> 8, 10, 12, 13
   <223> LNA modified, 5-prime methylated cytosine
<220>
<400> 62
   attgtcacac tcc 13
<210> 63
   <211> 15
   <212> DNA
   <213> Artificial Sequence
<220>
   <223> Synthetic oligonucleotide
<220>
   <221> misc_feature
   <222> (1)..(15)
   <223> Phosphorothioate linkage
<220>
   <221> misc_feature
   <222> 1, 6, 10, 12, 15
   <223> LNA modified, 5-prime-methylated cytosine
<220>
   <221> misc_feature
   <222> 3, 7, 14
   <223> LNA modified nucleotide
<400> 63
   ccattctgac cctac 15
<210> 64
   <211> 22
   <212> RNA
   <213> Homo sapiens
<400> 64
   uagcuuauca gacugauguu ga 22
<210> 65
   <211> 16
   <212> DNA
   <213> Artificial Sequence
<220>
   <223> Synthetic oligonucleotide
<220>
   <221> misc_feature
   <222> 1, 2, 3, 13, 14, 15
   <223> LNA modified nucleotide
<400> 65
   tcagtctgat aagcta 16
<210> 66
   <211> 16
   <212> DNA
   <213> Artificial Sequence
<220>
   <223> Synthetic oligonucleotide
<220>
   <221> misc_feature
   <222> 1, 2, 3, 13, 14, 15
   <223> LNA modified nucleotide
<400> 66
   tccgtcttag aagata 16
<210> 67
   <211> 15
   <212> DNA
   <213> Artificial Sequence
<220>
   <223> Synthetic oligonucleotide
<220>
   <221> misc_feature
   <222> 1, 3, 6, 7, 10 12, 14, 15
   <223> LNA modified nucleotide
<400> 67
   tctgtcagat acgat 15
<210> 68
   <211> 15
   <212> DNA
   <213> Artificial Sequence
<220>
   <223> Synthetic oligonucleotide
<220>
   <221> misc_feature
   <222> 1, 3, 6, 7, 10 12, 14, 15
   <223> LNA modified nucleotide
<400> 68
   tcagtctgat aagct 15
<210> 69
   <211> 15
   <212> DNA
   <213> Artificial Sequence
<220>
   <223> Synthetic oligonucleotide
<220>
   <221> misc_feature
   <222> 1, 3, 4, 6, 7, 8, 10 12, 14, 15
   <223> LNA modified nucleotide
<400> 69
   tcagtctgat aagct 15
<210> 70
   <211> 7
   <212> DNA
   <213> Artificial Sequence
<220>
   <223> Synthetic oligonucleotide
<400> 70
   atttgca 7
<210> 71
   <211> 8
   <212> DNA
   <213> Artificial Sequence
<220>
   <223> Synthetic oligonucleotide
<400> 71
   gatttgca 8
<210> 72
   <211> 9
   <212> DNA
   <213> Artificial Sequence
<220>
   <223> Synthetic oligonucleotide
<400> 72
   ggatttgca 9
<210> 73
   <211> 7
   <212> DNA
   <213> Artificial Sequence
<220>
   <223> Synthetic oligonucleotide
<400> 73
   cacactc 7
<210> 74
   <211> 8
   <212> DNA
   <213> Artificial Sequence
<220>
   <223> Synthetic oligonucleotide
<400> 74
   tcacactc 8
<210> 75
   <211> 9
   <212> DNA
   <213> Artificial Sequence
<220>
   <223> Synthetic oligonucleotide
<400> 75
   gtcacactc 9
<210> 76
   <211> 7
   <212> DNA
   <213> Artificial Sequence
<220>
   <223> Synthetic oligonucleotide
<400> 76
   tagcatt 7
<210> 77
   <211> 8
   <212> DNA
   <213> Artificial Sequence
<220>
   <223> Synthetic oligonucleotide
<400> 77
   ttagcatt 8
<210> 78
   <211> 9
   <212> DNA
   <213> Artificial Sequence
<220>
   <223> Synthetic oligonucleotide
<400> 78
   attagcatt 9
<210> 79
   <211> 7
   <212> DNA
   <213> Artificial Sequence
<220>
   <223> Synthetic oligonucleotide
<400> 79
   acgaaca 7
<210> 80
   <211> 8
   <212> DNA
   <213> Artificial Sequence
<220>
   <223> Synthetic oligonucleotide
<400> 80
   aacgaaca 8
<210> 81
   <211> 9
   <212> DNA
   <213> Artificial Sequence
<220>
   <223> Synthetic oligonucleotide
<400> 81
   gaacgaaca 9
<210> 82
   <211> 16
   <212> DNA
   <213> Artificial Sequence
<220>
   <223> Synthetic oligonucleotide
<220>
   <221> misc_feature
   <222> 3, 15
   <223> LNA modified, 5-prime methylated cytosine
<220>
   <221> misc_feature
   <222> 6, 9, 12
   <223> LNA modified nucleotide
<400> 82
   tgcatggatt tgcaca 16
<210> 83
   <211> 15
   <212> DNA
   <213> Artificial Sequence
<220>
   <223> Synthetic oligonucleotide
<220>
   <221> misc_feature
   <222> 3, 15
   <223> LNA modified, 5-prime methylated cytosine
<220>
   <221> misc_feature
   <222> 6, 9, 12
   <223> LNA modified nucleotide
<400> 83
   tgcatggatt tgcac 15
<210> 84
   <211> 13
   <212> DNA
   <213> Artificial Sequence
<220>
   <223> Synthetic oligonucleotide
<220>
   <221> misc_feature
   <222> 1, 13
   <223> LNA modified, 5-prime methylated cytosine
<220>
   <221> misc_feature
   <222> 4, 7, 10
   <223> LNA modified nucleotide
<400> 84
   catggatttg cac 13
<210> 85
   <211> 15
   <212> DNA
   <213> Artificial Sequence
<220>
   <223> Synthetic oligonucleotide
<220>
   <221> misc_feature
   <222> 2, 4, 6, 8, 10, 12, 14
   <223> LNA modified nucleotide
<400> 85
   tgcatggatt tgcac 15
<210> 86
   <211> 13
   <212> DNA
   <213> Artificial Sequence
<220>
   <223> Synthetic oligonucleotide
<220>
   <221> misc_feature
   <222> 2, 4, 6, 8, 10, 12
   <223> LNA modified nucleotide
<400> 86
   catggatttg cac 13
<210> 87
   <211> 13
   <212> DNA
   <213> Artificial Sequence
<220>
   <223> Synthetic oligonucleotide
<220>
   <221> misc_feature
   <222> 1, 13
   <223> LNA modified, 5-prime methylated cytosine
<220>
   <221> misc_feature
   <222> 4, 5, 8, 10, 12
   <223> LNA modified nucleotide
<400> 87
   catggatttg cac 13
<210> 88
   <211> 15
   <212> DNA
   <213> Artificial Sequence
<220>
   <223> Synthetic oligonucleotide
<220>
   <221> misc_feature
   <222> 3, 15
   <223> LNA modified, 5-prime methylated cytosine
<220>
   <221> misc_feature
   <222> 1, 6, 7, 10, 12, 14
   <223> LNA modified nucleotide
<400> 88
   tgcatggatt tgcac 15
<210> 89
   <211> 16
   <212> DNA
   <213> Artificial Sequence
<220>
   <223> Synthetic oligonucleotide
<220>
   <221> misc_feature
   <222> 3, 15
   <223> LNA modified, 5-prime methylated cytosine
<220>
   <221> misc_feature
   <222> 1, 5, 7, 9, 10, 12, 14
   <223> LNA modified nucleotide
<400> 89
   tgcatggatt tgcaca 16
<210> 90
   <211> 16
   <212> DNA
   <213> Artificial Sequence
<220>
   <223> Synthetic oligonucleotide
<220>
   <221> misc_feature
   <222> 2, 8, 14
   <223> LNA modified 5-prime methylated cytosine
<220>
   <221> misc_feature
   <222> 5, 11
   <223> LNA modified nucleotide
<400> 90
   ccattgtcac actcca 16
<210> 91
   <211> 16
   <212> DNA
   <213> Artificial Sequence
<220>
   <223> Synthetic oligonucleotide
<220>
   <221> misc_feature
   <222> 2, 14
   <223> LNA modified, 5-prime methylated cytosine
<220>
   <221> misc_feature
   <222> 5, 8, 11
   <223> LNA modified nucleotide
<400> 91
   ccattgtaac tctcca 16
<210> 92
   <211> 16
   <212> DNA
   <213> Artificial Sequence
<220>
   <223> Synthetic oligonucleotide
<220>
   <221> misc_feature
   <222> 12, 15
   <223> LNA modified, 5-prime methylated cytosine
<220>
   <221> misc_feature
   <222> 3, 6, 9
   <223> LNA modified nucleotide
<400> 92
   ccattgtcac actcca 16
<210> 93
   <211> 15
   <212> DNA
   <213> Artificial Sequence
<220>
   <223> Synthetic oligonucleotide
<220>
   <221> misc_feature
   <222> 2, 8, 14
   <223> LNA modified, 5-prime methylated cytosine
<220>
   <221> misc_feature
   <222> 5, 11
   <223> LNA modified nucleotide
<400> 93
   ccattgtcac actcc 15
<210> 94
   <211> 13
   <212> DNA
   <213> Artificial Sequence
<220>
   <223> Synthetic oligonucleotide
<220>
   <221> misc_feature
   <222> 6, 12
   <223> LNA modified, 5-prime methylated cytosine
<220>
   <221> misc_feature
   <222> 3, 9
   <223> LNA modified nucleotide
<400> 94
   attgtcacac tcc 13
<210> 95
   <211> 15
   <212> DNA
   <213> Artificial Sequence
<220>
   <223> Synthetic oligonucleotide
<220>
   <221> misc_feature
   <222> 12, 15
   <223> LNA modified, 5-prime methylated cytosine
<220>
   <221> misc_feature
   <222> 3, 6, 9
   <223> LNA modified nucleotide
<400> 95
   ccattgtcac actcc 15
<210> 96
   <211> 13
   <212> DNA
   <213> Artificial Sequence
<220>
   <223> Synthetic oligonucleotide
<220>
   <221> misc_feature
   <222> 10, 13
   <223> LNA modified, 5-prime methylated cytosine
<220>
   <221> misc_feature
   <222> 1, 4, 7
   <223> LNA modified nucleotide
<400> 96
   attgtcacac tcc 13
<210> 97
   <211> 13
   <212> DNA
   <213> Artificial Sequence
<220>
   <223> Synthetic oligonucleotide
<220>
   <221> misc_feature
   <222> 6, 8, 10, 12
   <223> LNA modified, 5-prime methylated cytosine
<220>
   <221> misc_feature
   <222> 2, 4
   <223> LNA modified nucleotide
<400> 97
   attgtcacac tcc 13
<210> 98
   <211> 13
   <212> DNA
   <213> Artificial Sequence
<220>
   <223> Synthetic oligonucleotide
<220>
   <221> misc_feature
   <222> 8, 10, 12, 13
   <223> LNA modified, 5-prime methylated cytosine
<220>
   <221> misc_feature
   <222> 1, 4, 5
   <223> LNA modified nucleotide
<400> 98
   attgtcacac tcc 13
<210> 99
   <211> 15
   <212> DNA
   <213> Artificial Sequence
<220>
   <223> Synthetic oligonucleotide
<220>
   <221> misc_feature
   <222> 1, 10, 12, 14, 15
   <223> LNA modified, 5-prime methylated cytosine
<220>
   <221> misc_feature
   <222> 3, 6, 7
   <223> LNA modified nucleotide
<400> 99
   ccattgtcac actcc 15
<210> 100
   <211> 16
   <212> DNA
   <213> Artificial Sequence
<220>
   <223> Synthetic oligonucleotide
<220>
   <221> misc_feature
   <222> 1, 15
   <223> LNA modified, 5-prime methylated cytosine
<220>
   <221> misc_feature
   <222> 4, 7, 11
   <223> LNA modified nucleotide
<400> 100
   ccattgtcac actcca 16
<210> 101
   <211> 16
   <212> DNA
   <213> Artificial Sequence
<220>
   <223> Synthetic oligonucleotide
<220>
   <221> misc_feature
   <222> 1, 2, 14, 15
   <223> LNA modified, 5-prime methylated cytosine
<220>
   <221> misc_feature
   <222> 3, 13
   <223> LNA modified nucleotide
<400> 101
   ccattgtcac actcca 16
<210> 102
   <211> 16
   <212> DNA
   <213> Artificial Sequence
<220>
   <223> Synthetic oligonucleotide
<220>
   <221> misc_feature
   <222> 2, 11
   <223> LNA modified, 5-prime methylated cytosine
<220>
   <221> misc_feature
   <222> 5, 8, 14
   <223> LNA modified nucleotide
<400> 102
   tcacgattag cattaa 16
<210> 103
   <211> 16
   <212> DNA
   <213> Artificial Sequence
<220>
   <223> Synthetic oligonucleotide
<220>
   <221> misc_feature
   <222> 5
   <223> LNA modified, 5-prime methylated cytosine
<220>
   <221> misc_feature
   <222> 2, 8, 11, 14
   <223> LNA modified nucleotide
<400> 103
   atcacgatta gcatta 16
<210> 104
   <211> 16
   <212> DNA
   <213> Artificial Sequence
<220>
   <223> Synthetic oligonucleotide
<220>
   <221> misc_feature
   <222> 11
   <223> LNA modified, 5-prime methylated cytosine
<220>
   <221> misc_feature
   <222> 1, 3, 5, 7, 9, 13, 15
   <223> LNA modified nucleotide
<400> 104
   tcacgattag cattaa 16
<210> 105
   <211> 16
   <212> DNA
   <213> Artificial Sequence
<220>
   <223> Synthetic oligonucleotide
<220>
   <221> misc_feature
   <222> 12
   <223> LNA modified, 5-prime methylated cytosine
<220>
   <221> misc_feature
   <222> 1, 4, 6, 8, 10, 14
   <223> LNA modified nucleotide
<400> 105
   atcacgatta gcatta 16
<210> 106
   <211> 15
   <212> DNA
   <213> Artificial Sequence
<220>
   <223> Synthetic oligonucleotide
<220>
   <221> misc_feature
   <222> 5
   <223> LNA modified, 5-prime methylated cytosine
<220>
   <221> misc_feature
   <222> 2, 8, 11, 14
   <223> LNA modified nucleotide
<400> 106
   gagccgaacg aacaa 15
<210> 107
   <211> 13
   <212> DNA
   <213> Artificial Sequence
<220>
   <223> Synthetic oligonucleotide
<220>
   <221> misc_feature
   <222> 3
   <223> LNA modified, 5-prime methylated cytosine
<220>
   <221> misc_feature
   <222> 6, 9, 12
   <223> LNA modified nucleotide
<400> 107
   gccgaacgaa caa 13
<210> 108
   <211> 15
   <212> DNA
   <213> Artificial Sequence
<220>
   <223> Synthetic oligonucleotide
<220>
   <221> misc_feature
   <222> 5, 9, 13
   <223> LNA modified, 5-prime methylated cytosine
<220>
   <221> misc_feature
   <222> 1, 3, 7, 11, 15
   <223> LNA modified nucleotide
<400> 108
   gagccgaacg aacaa 15
<210> 109
   <211> 13
   <212> DNA
   <213> Artificial Sequence
<220>
   <223> Synthetic oligonucleotide
<220>
   <221> misc_feature
   <222> 3, 7, 11
   <223> LNA modified, 5-prime methylated cytosine
<220>
   <221> misc_feature
   <222> 1, 5, 9, 13
   <223> LNA modified nucleotide
<400> 109
   gccgaacgaa caa 13
<210> 110
   <211> 15
   <212> DNA
   <213> Artificial Sequence
<220>
   <223> Synthetically generated oligonucleotide
<400> 110
   ccattgtcac actcc 15

## Description

### FIELD OF THE INVENTION

The present invention concerns pharmaceutical composiions comprising LNA-containing single stranded oligonucleotides capable of inhibiting disease-inducing microRNAs.

### BACKGROUND OF THE INVENTION

### MicroRNAs - novel regulators of gene expression

MicroRNAs (miRNAs) are an abundant class of short endogenous RNAs that act as post-transcriptional regulators of gene expression by base-pairing with their target mRNAs. The mature miRNAs are processed sequentially from longer hairpin transcripts by the RNAse III ribonucleases Drosha (Lee et al. 2003) and Dicer (Hutvagner et al. 2001, Ketting et al. 2001). To date more than 3400 miRNAs have been annotated in vertebrates, invertebrates and plants according to the miRBase microRNA database release 7.1 in October 2005 (Griffith-Jones 2004, Griffith-Jones et al. 2006), and many miRNAs that correspond to putative genes have also been identified.

Most animal miRNAs recognize their target sites located in 3'-UTRs by incomplete base-pairing, resulting in translational repression of the target genes (Bartel 2004). An increasing body of research shows that animal miRNAs play fundamental biological roles in cell growth and apoptosis (Brennecke et al. 2003), hematopoietic lineage differentiation (Chen et al. 2004), life-span regulation (Boehm and Slack 2005), photoreceptor differentiation (Li and Carthew 2005), homeobox gene regulation (Yekta et al. 2004, Hornstein et al. 2005), neuronal asymmetry (Johnston et al. 2004), insulin secretion (Poy et al. 2004), brain morphogenesis (Giraldez et al. 2005), muscle proliferation and differentiation (Chen, Mandel et al. 2005, Kwon et al. 2005, Sokol and Ambros 2005), cardiogenesis (Zhao et al. 2005) and late embryonic development in vertebrates (Wienholds et al. 2005).

### MicroRNAs in human diseases

miRNAs are involved in a wide variety of human diseases. One is spinal muscular atrophy (SMA), a paediatric neurodegenerative disease caused by reduced protein levels or loss-of-function mutations of the survival of motor neurons (SMN) gene (Paushkin et al. 2002). A mutation in the target site of miR-189 in the human SLITRK1 gene was recently shown to be associated with Tourette's syndrome (Abelson et al. 2005), while another recent study reported that the hepatitis C virus (HCV) RNA genome interacts with a host-cell microRNA, the liver-specific miR-122a, to facilitate its replication in the host (Jopling et al. 2005). Other diseases in which miRNAs or their processing machinery have been implicated, include frag-ile X mental retardation (FXMR) caused by absence of the fragile X mental retardation protein (FMRP) (Nelson et al. 2003, Jin et al. 2004) and DiGeorge syndrome (Landthaler et al. 2004).

In addition, perturbed miRNA expression patterns have been reported in many human cancers. For example, the human miRNA genes miR15a and miR16-1 are deleted or down-regulated in the majority of B-cell chronic lymphocytic leukemia (CLL) cases, where a unique signature of 13 miRNA genes was recently shown to associate with prognosis and progression (Calin et al. 2002, Calin et al. 2005). The role of miRNAs in cancer is further supported by the fact that more than 50% of the human miRNA genes are located in cancer-associated genomic regions or at fragile sites (Calin et al. 2004). Recently, systematic expression analysis of a diversity of human cancers revealed a general down-regulation of miRNAs in tumors compared to normal tissues (Lu et al. 2005). Interestingly, miRNA-based classification of poorly differentiated tumors was successful, whereas mRNA profiles were highly inaccurate when applied to the same samples. miRNAs have also been shown to be deregulated in breast cancer (Iorio et al. 2005), lung cancer (Johnson et al. 2005) and colon cancer (Michael et al. 2004), while the miR-17-92 cluster, which is amplified in human B-cell lymphomas and miR-155 which is upregulated in Burkitt's lymphoma have been reported as the first human miRNA oncogenes (Eis et al. 2005, He et al. 2005). Thus, human miRNAs would not only be highly useful as biomarkers for future cancer diagnostics, but are rapidly emerging as attractive targets for disease intervention by oligonucleotide technologies.

### Inhibition of microRNAs using single stranded oligonucleotides

Several oligonucleotide approaches have been reported for inhibition of miRNAs.

WO03/029459 (Tuschl) claims oligonucleotides which encode microRNAs and their complements of between 18 - 25 nucleotides in length which may comprise nucleotide analogues. LNA is suggested as a possible nucleotide analogue, although no LNA containing olginucleotides are disclosed. Tuschl claims that miRNA oligonucleotides may be used in therapy.

US2005/0182005 discloses a 24mer 2'OMe RNA oligoribonucleotide complementary to the longest form of miR 21 which was found to reduce miR 21 induced repression, whereas an equivalent DNA containing oligonucleotide did not. The term 2'OMe-RNA refers to an RNA analogue where there is a substitution to methyl at the 2' position (2'OMethyl).

US2005/0227934 (Tuschl) refers to antimir molecules with upto 50% DNA residues. It also reports that antimirs containing 2' OMe RNA were used against pancreatic microRNAs but it appears that no actual oligonucleotide structures are disclosed.

US20050261218 (ISIS) claims an oligomeric compound comprising a first region and a second region, wherein at least one region comprises a modification and a portion of the oligomeric compound is targeted to a small non-coding RNA target nucleic acid, wherein the small non-coding RNA target nucleic acid is a miRNA. Oligomeric compounds of between 17 and 25 nucleotides in length are claimed. The examples refer to entirely 2' OMe PS compounds, 21mers and 20mer and 2'OMe gapmer oligonucleotides targeted against a range of pre-miRNA and mature miRNA targets.

Boutla et al. 2003 (Nucleic Acids Research 31: 4973-4980) describe the use of DNA antisense oligonucleotides complementary to 11 different miRNAs in Drosophila as well as their use to inactivate the miRNAs by injecting the DNA oligonucleotides into fly embryos. Of the 11 DNA antisense oligonucleotides, only 4 constructs showed severe interference with normal development, while the remaining 7 oligonucleotides didn't show any phenotypes presumably due to their inability to inhibit the miRNA in question.

An alternative approach to this has been reported by Hutvagner et al. (2004) and Leaman et al. (2005), in which 2'-O-methyl antisense oligonucleotides, complementary to the mature miRNA could be used as potent and irreversible inhibitors of short interfering RNA (siRNA) and miRNA function in vitro and in vivo in Drosophila and C. elegans, thereby inducing a loss-of-function phenotype. A drawback of this method is the need of high 2'-O-methyl oligonucleotide concentrations (100 micromolar) in transfection and injection experiments, which may be toxic to the animal. This method was recently applied to mice studies, by conjugating 2'-O-methyl antisense oligonucleotides complementary to four different miRNAs with cholesterol for silencing miRNAs in vivo (Krützfedt et al. 2005). These so-called antagomirs were administered to mice by intravenous injections. Although these experiments resulted in effective silencing of endogenous miRNAs in vivo, which was found to be specific, efficient and long-lasting, a major drawback was the need of high dosage (80 mg/kg) of 2'-O-Me antagomir for efficient silencing.

Inhibition of microRNAs using LNA-modified oligonucleotides have previously been described by Chan et al. Cancer Research 2005, 65 (14) 6029-6033, Lecellier et al. Science 2005, 308, 557-560, Naguibneva et al. Nature Cell Biology 2006 8 (3), 278-84 and Ørum et al. Gene 2006, (Available online 24 February 2006). In all cases, the LNA-modified anti-mir oligonucleotides were complementary to the entire mature microRNA, i.e. 20-23 nucleotides in length, which hampers efficient in vivo uptake and wide biodistribution of the molecules.

Naguibneva (Naguibneva et al. Nature Cell Biology 2006 8 describes the use of mixmer DNA-LNA-DNA antisense oligonucleotide anti-mir to inhibit microRNA miR-181 function in vitro, in which a block of 8 LNA nucleotides is located at the center of the molecule flanked by 6 DNA nucleotides at the 5' end, and 9 DNA nucleotides at the 3' end, respectively. A major drawback of this antisense design is low in vivo stability due to low nuclease resistance of the flanking DNA ends.

While Chan et al. (Chan et al. Cancer Research 2005, 65 (14) 6029-6033), and Ørum et al. (Ørum et al. Gene 2006, (Available online 24 February 2006) do not disclose the design of the LNA-modified anti-mir molecules used in their study, Lecellier et al. (Lecellier et al. Science 2005, 308, 557-560) describes the use of gapmer LNA-DNA-LNA antisense oligonucleotide anti-mir to inhibit microRNA function, in which a block of 4 LNA nucleotides is located both at the 5' end, and at the 3' end, respectively, with a window of 13 DNA nucleotides at the center of the molecule. A major drawback of this antisense design is low in vivo uptake, as well as low in vivo stability due to the 13 nucleotide DNA stretch in the anti-mir oligonucleotide.
WO 2005/013901 discloses oligomeric compounds and compositions for use in modulation of small non-coding RNAs. Elmen et al., poster at the 71st Symposium on Quantitative Biology - Regulatory RNAs, Cold Spring Harbor, NY., United States, May 31-June 5, 2006, refers to LNA-antimiRs: Promising candidates for therapeutic intervention of disease-related microRNAs. Czech, New England Journal of Medicine 2006, vol 354, pp1194-1195 refers to microRNAs as therapeutic targets.

Thus, there is a need in the field for improved oligonucleotides capable of inhibiting microRNAs.

### SUMMARY OF THE INVENTION

The present invention is based upon the discovery that the use of short oligonucleotides designed to bind with high affinity to miRNA targets are highly effective in alleviating the repression of mRNA by microRNAs in vivo.

Whilst not wishing to be bound to any specific theory, the evidence disclosed herein indicates that the highly efficient targeting of miRNAs in vivo is achieved by designing oligonucleotides with the aim of forming a highly stable duplex with the miRNA target in vivo. This is achieved by the use of high affinity nucleotide analogues such as at least one LNA units and suitably further high affinity nucleotide analogues, such as LNA, 2'-MOE RNA or 2'-Fluoro nucleotide analogues, in a short, such as 10-17 or 10 - 16 nucleobase oligonucleotides. In one aspect the aim is to generate an oligonucleotide of a length which is unlikely to form a siRNA complex (i.e. a short oligonucleotide), and with sufficient loading of high affinity nucleotide analogues that the oligonucleotide sticks almost permenantly to its miRNA target, effectively forming a stable and non-functional duplex with the miRNA target. We have found that such designs are considerably more effective than the prior art oligonucleotides, particularly gapmer and blockmer designs and oligonucleotides which have a long length, e.g. 20 - 23mers. The term 2'fluor-DNA refers to an DNA analogue where the is a substitution to fluor at the 2' position (2'F).

In a first aspect the invention provides a single stranded oligonucleotide, 8-17 nucleotides in length, capable of reducing the effective amount of a mature human microRNA in a cell, wherein at least one of the nucleobase units of the oligonucleotide is a LNA nucleotide unit, and wherein all the internucleoside linkages are phosphorothioate linkages, wherein the oligonucleotide is complementary to the mature human microRNA sequence, wherein the single stranded oligonucleotide comprises a region of contiguous nucleobase sequence which is 100% complementary to the human microRNA seed region, and wherein the oligonucleotide comprises at least one LNA nucleotide unit in a position which is within the region complementary to the miRNA seed region, further wherein:
(a) nucleobase units 1 to 6 (inclusive) of the single stranded oligonucleotide as measured from the 3' end of the single stranded oligonucleotide are complementary to the microRNA seed region sequence; or
(b) nucleobase units 1 to 7 (inclusive) of the single stranded oligonucleotide as measured from the 3' end of the single stranded oligonucleotide are complementary to the microRNA seed region sequence; or
(c) nucleobase units 2 to 7 (inclusive) of the single stranded oligonucleotide as measured from the 3' end of the single stranded oligonucleotide are complementary to the microRNA seed region sequence.

In a second aspect the invention provides a single stranded oligonucleotide, 8-17 nucleotides in length, capable of reducing the effective amount of a mature human microRNA in a cell, wherein at least one of the nucleobase units of the oligonucleotide is a LNA nucleotide unit, and wherein all the internucleoside linkages are phosphorothioate linkages, wherein the oligonucleotide is complementary to the mature human microRNA sequence, wherein the single stranded oligonucleotide comprises a region of contiguous nucleobase sequence which is 100% complementary to the human microRNA seed region, and wherein the oligonucleotide comprises at least one LNA nucleotide unit in a position which is within the region complementary to the miRNA seed region, wherein the oligonucleotide is for use as a medicament, further wherein:
(a) nucleobase units 1 to 6 (inclusive) of the single stranded oligonucleotide as measured from the 3' end of the single stranded oligonucleotide are complementary to the microRNA seed region sequence; or
(b) nucleobase units 1 to 7 (inclusive) of the single stranded oligonucleotide as measured from the 3' end of the single stranded oligonucleotide are complementary to the microRNA seed region sequence; or
(c) nucleobase units 2 to 7 (inclusive) of the single stranded oligonucleotide as measured from the 3' end of the single stranded oligonucleotide are complementary to the microRNA seed region sequence.

In a third aspect the invention provides for a pharmaceutical composition comprising the single stranded oligonucleotide of the invention and a pharmaceutically acceptable diluent, carrier, or adjuvant.

Described herein is a pharmaceutical composition comprising a single stranded oligonucleotide having a length of between 8 and 17, such as10 and 17, such as 8 - 16 or 10 - 16 nucleobase units, a pharmaceutically acceptable diluent, carrier, or adjuvant, wherein at least one of the nucleobase units of the single stranded oligonucleotide is a high affinity nucleotide analohue, such as a Locked Nucleic Acid (LNA) nucleobase unit, and wherein the single stranded oligonucleotide is complementary to a human microRNA sequence.

The high affinity nucleotide analogues are nucleotide analogues which result in oligonucleotide which has a higher thermal duplex stability with a complementary RNA nucleotide than the binding affinity of an equivalent DNA nucleotide. This is typically determined by measuring the Tₘ.

We have not identified any significant off-target effects when using these short, high affinity oligonucleotides targeted against specific miRNAs. Indeed, the evidence provided herein indicates the effects on mRNA expression are either due to the presence of a complementary sequence to the targeted miRNA (primary mRNA targets) within the mRNA or secondary effects on mRNAs which are regulated by primary mRNA targets (secondary mRNA targets). No toxicity effects were identified indicating no significant detrimental off-target effects.

Described herein is a pharmaceutical composition comprising a single stranded oligonucleotide having a length of between 8 and 17 nucleobase units, such as between 10 and 17 nucleobase units, such as between 10 and 16 nucleobase units, and a pharmaceutically acceptable diluent, carrier, or adjuvant, wherein at least one of the nucleobase units of the single stranded oligonucleotide is a Locked Nucleic Acid (LNA) nucleobase unit, and wherein the single stranded oligonucleotide is complementary to a human microRNA sequence.

Described herein is the use of an oligonucleotide according to the invention, such as those which may form part of the pharmaceutical composition, for the manufacture of a medicament for the treatment of a disease or medical disorder associated with the presence or over-expression (upregulation) of the microRNA.

Described herein is a method for the treatment of a disease or medical disorder associated with the presence or over-expression of the microRNA, comprising the step of administering a composition (such as the pharmaceutical composition) according to the invention to a person in need of treatment.

Described herein is a method for reducing the effective amount of a miRNA in a cell or an organism, comprising administering a composition (such as the pharmaceutical composition) according to the invention or a single stranded oligonucleotide according to the invention to the cell or the organism. Reducing the effective amount in this context refers to the reduction of functional miRNA present in the cell or organism. It is recognised that the preferred oligonucleotides according to the invention may not always significantly reduce the actual amount of miRNA in the cell or organism as they typically form very stable duplexes with their miRNA targets.

Described herein is a method for de-repression of a target mRNA of a miRNA in a cell or an organism, comprising administering a composition (such as the pharmaceutical composition) or a single stranded oligonucleotide according to the invention to the cell or the organism.

Described herein is the use of a single stranded oligonucleotide of between 8 - 16 such as 10 - 16 nucleobases in length, for the manufacture of a medicament for the treatment of a disease or medical disorder associated with the presence or over-expression of the microRNA.

Described herein is a method for the treatment of a disease or medical disorder associated with the presence or over-expression of the microRNA, comprising the step of administering a composition (such as the pharmaceutical composition) comprising a single stranded oligonucleotide of between between 8 - 16 such as between 10 - 16 nucleobases in length to a person in need of treatment.

Described herein is a method for reducing the effective amount of a miRNA target (i.e. 'available' miRNA) in a cell or an organism, comprising administering a composition (such as the pharmaceutical composition) comprising a single stranded oligonucleotide of between 8 - 16 such as between 10 - 16 nucleobases to the cell or the organism.

Described herein is a method for de-repression of a target mRNA of a miRNA in a cell or an organism, comprising a single stranded oligonucleotide of between 8
- 16 such as between 10 - 16 nucleobases or (or a composition comprising said oligonucleotide) to the cell or the organism.

Described herein is a method for the synthesis of a single stranded oligonucleotide targeted against a human microRNA, such as a single stranded oligonucleotide described herein, said method comprising the steps of:
a. Optionally selecting a first nucleobase, counting from the 3' end, which is a nucleotide analogue, such as an LNA nucleobase.
b. Optionally selecting a second nucleobase, counting from the 3' end, which is an nucleotide analogue, such as an LNA nucleobase.
c. Selecting a region of the single stranded oligonucleotide which corresponds to the miRNA seed region, wherein said region is as defined herein.
d. Optionally selecting a seventh and eight nucleobase is as defined herein.
e. Optionally selecting a 5' region of the single stranded oligonucleotide is as defined herein.
f. Optionally selecting a 5' terminal of the single stranded oligonucleotide is as defined herein.

Wherein the synthesis is performed by sequential synthesis of the regions defined in steps a - f, wherein said synthesis may be performed in either the 3'-5' (a to f) or 5' - 3' (f to a)direction, and wherein said single stranded oligonucleotide is complementary to a sequence of the miRNA target.

In one embodiment the oligonucleotide of the invention is designed not to be recruited by RISC or to mediate RISC directed cleavage of the miRNA target. It has been considered that by using long oligonucleotides, e.g. 21 or 22mers, particularly RNA oligonucleotides, or RNA 'analogue' oligonucleotide which are complementary to the miRNA target, the oligonucleotide can compete against the target mRNA in terms of RISC complex association, and thereby alleviate miRNA repression of miRNA target mRNAs via the introduction of an oligonucleotide which competes as a substrate for the miRNA.

However, the present invention seeks to prevent such undesirable target mRNA cleavage or translational inhibition by providing oligonucleotides capable of complementary, and apparently in some cases almost irreversible binding to the mature microRNA. This appears to result in a form of protection against degredation or cleavage (e.g. by RISC or RNAseH or other endo or exo-nucleases), which may not result in substantial or even significant reduction of the miRNA (e.g. as detected by northern blot using LNA probes) within a cell, but ensures that the effective amount of the miRNA, as measured by de-respression analysis is reduced considerably. Therefore, in one aspect, the invention provides oligonucleotides which are purposefully designed not to be compabible with the RISC complex, but to remove miRNA by titration by the oligonucleotide. Although not wishing to be bound to a specific theory of why the oligonucleotides of the present invention are so effective, in analagy with the RNA based oligonucleotides (or complete 2'OMe oliugonucleotides), it appears that the oligonucleotides according to the present invention work through non-competitive inhibition of miRNA function as they effectively remove the available miRNA from the cytoplasm, where as the prior art oligonucleotides provide an alterntive miRNA substrate, which may act as a competitor inhibitor, the effectiveness of which would be far more dependant upon the concentration of the oligonucoleotide in the cytoplasm, as well as the concentration of the target mRNA and miRNA.

Again, whilst not wishing to be bound to any specific theory, one further possibility that may exist with the use of oligonucleotides of approximately similar length to the miRNA targets, is that the oligonucleotides could form a siRNA like duplex with the miRNA target, a situation which would reduce the effectiveness of the oligonucleotide. It is also possible that the oligonucleotides themselves could be used as the guiding strand within the RISC complex, thereby generating the possibility of RISC directed degredation of non-specific targets which just happen to have sufficient complementarity to the oligonucleotide guide.

By selecting short oligonucleotides for targeting miRNA sequences, such problems are avoided.

Short oligonucleotides which incorporate LNA are known from the reagents area, such as the LNA (see for example WO2005/098029 and WO 2006/069584). However the molecules designed for diagnostic or reagent use are very different in design than those for pharmaceutical use. For example, the terminal nucleobases of the reagent oligos are typically not LNA, but DNA, and the internucleoside linkages are typically other than phosphorothioate, the preferred linkage for use in the oligonucleotides of the present invention. The invention therefore provides for a novel class of oligonucleotide per se.

The invention further provides for a (single stranded) oligonucleotide as described in the conext of the pharmaceutical composition of the invention, wherein said oligonucleotide comprises either
i) at least one 3' terminal LNA unit, and/or
ii) at least one 5' teriminal LNA unit.

The oligonucleotide is fully phosphorothiolated. As referred to herein, other preferred aspects of the oligonucleotide according to the invention is that the second 3' nucleobase, and/or the 9^{th} and 10^{th} (from the 3' end), may also be LNA.

The inventors have found that other methods of avoiding RNA cleavage (such as exonuclease degredation in blood serum, or RISC associated cleavage of the oligonucleotide according to the invention are possible, and as such the invention also provides for a single stranded oligonucleotide which comprises of either:
a. an LNA unit at position 1 and 2 counting from the 3' end and/or
b. an LNA unit at position 9 and/or 10, also counting from the 3' end, and/or
c. either one or two 5' LNA units.

Whislt the benfits of these other aspects may be seen with longer oligonucleotides, such as nucleotide of up to 26 nucleobase units in length, it is considered these features may also be used with the shorter oligonucleotides referred to herein, such as the oligonucleotides of between 10 - 17 or 10 - 16 nucleobases described herein. It is highly preferably that the olifonucleotides comprise high affinity nucleotide analogues, such as those referred to herein, most preferably LNA units.

The inventors have therefore surprisingly found that carefully designed single stranded oligonucleotides comprising locked nucleic acid (LNA) units in a particular order show significant silencing of microRNAs, resulting in reduced microRNA levels. It was found that tight binding of said oligonucleotides to the so-called seed sequence, nucleotides 2 to 8 or 2 - 7, counting from the 5' end, of the target microRNAs was important. Nucleotide 1 of the target microRNAs is a non-pairing base and is most likely hidden in a binding pocket in the Ago 2 protein. Whislt not wishing to be bound to a specific theory, the present inventors consider that by selecting the seed region sequences, particularly with oligonculeoitdes that comprise LNA, preferably LNA units in the region which is complementary to the seed region, the duplex between miRNA and oligonucleotide is particularly effective in targeting miRNAs, avoiding off target effects, and possibly providing a further feature which prevents RISC directed miRNA function.

The inventors have surprisingly found that microRNA silencing is even more enhanced when LNA-modified single stranded oligonucleotides do not contain a nucleotide at the 3' end corresponding to this non-paired nucleotide 1. It was further found that two LNA units in the 3' end of the oligonucleotides according to the present invention made said oligonucleotides highly nuclease resistant.

It was further found that the oligonucleotides of the invention which have at least one nucleotide analogue, such as an LNA nucleotide in the positions corresponding to positions 10 and 11, counting from the 5' end, of the target microRNA may prevent cleavage of the oligonucleotides of the invention

Accordingly, described herein is an oligonucleotide having a length of from 12 to 26 nucleotides, wherein
i) the first nucleotide, counting from the 3' end, is a locked nucleic acid (LNA) unit;
ii) the second nucleotide, counting from the 3' end, is an LNA unit; and
iii) the ninth and/or the tenth nucleotide, counting from the 3' end, is an LNA unit.

The invention further provides for the oligonucleotides as defined herein for use as a medicament.

The invention further relates to compositions comprising the oligonucleotides defined herein and a pharmaceutically acceptable carrier.

As mentioned above, microRNAs are related to a number of diseases. Hence, described herein is the use of an oligonucleotide as defined herein for the manufacture of a medicament for the treatment of a disease associated with the expression of microRNAs selected from the group consisting of spinal muscular atrophy, Tourette's syndrome, hepatitis C virus, fragile X mental retardation, DiGeorge syndrome and cancer, such as chronic lymphocytic leukemia, breast cancer, lung cancer and colon cancer, in particular cancer.

Described herein is a method to reduce the levels of target microRNA by contacting the target microRNA to an oligonucleotide as defined herein, wherein the oligonucleotide
1. is complementary to the target microRNA
2. does not contain a nucleotide at the 3' end that corresponds to the first 5' end nucleotide of the target microRNA.

Described herein is an oligonucleotide comprising a nucleobase sequence selected from the group consisting of SEQ IDs NO 1 -534, SEQ ID NOs 539-544, SEQ ID NOs 549 - 554, SEQ ID NOs 559-564, SEQ ID NOs 569-574 and SEQ ID NOs 594 - 598, and SEQ ID NOs 579 - 584, or a pharmaceutical composition comprising said oligonucleotide. The oligonucleotide has a nucleobase seqeunce of between 8 - 17 nucleobases, such as 8, 9, 10, 11, 12, 13, 14, 15, 16 or 17 nucleobases.

### BRIEF DESCRIPTION OF THE DRAWINGS

**Fig.** 1. The effect of treatment with different LNA anti-miR oligonucleotides on target nucleic acid expression in the miR-122a expressing cell line Huh-7. Shown are amounts of miR-122a (arbitrary units) derived from miR-122a specific qRT-PCR as compared to untreated cells (mock). The LNA anti-miR oligonuclelotides were used at two concentrations, 1 and 100 nM, respectively. Included is also a mismatch control (SPC3350) to SPC3349 (also referred to herein as SPC3549).
**Fig. 2****.** Assessment of LNA anti-miR-122a knock-down dose-response for SPC3548 and SPC3549 in comparison with SPC3372 in vivo in mice livers using miR-122a real-time RT-PCR.
**Fig. 2b** miR-122 levels in the mouse liver after treatment with different LNA-antimiRs. The LNA-antimiR molecules SPC3372 and SPC3649 were administered into normal mice by three i.p. injections on every second day over a six-day-period at indicated doses and sacrificed 48 hours after last dose. Total RNA was extracted from the mice livers and miR-122 was measured by miR-122 specific qPCR.
**Fig. 3****.** Assessment of plasma cholesterol levels in LNA-antimiR-122a treated mice compared to the control mice that received saline.
**Fig. 4a**. Assessment of relative Bckdk mRNA levels in LNA antimiR-122a treated mice in comparison with saline control mice using real-time quantitative RT-PCR.
**Fig. 4b**. Assessment of relative aldolase A mRNA levels in LNA antimiR-122a treated mice in comparison with saline control mice using real-time quantitative RT-PCR.
**Fig. 4c**. Assessment of GAPDH mRNA levels in LNA antimiR-122a treated mice (animals 4-30) in comparison with saline control mice (animals 1-3) using real-time quantitative RT-PCR.
**Fig. 5**. Assessment of LNA-antimiR™ -122a knock-down dose-response in vivo in mice livers using miR-122a real-time RT-PCR. Six groups of animals (5 mice per group) were treated in the following manner. Group 1 animals were injected with 0.2ml saline by i.v. on 3 successive days, Group 2 received 2.5mg/kg SPC3372, Group 3 received 6.25 mg/kg, Group 4 received 12.5 mg/kg and Group 5 received 25 mg/kg, while Group 6 received 25 mg/kg SPC 3373 (mismatch LNA-antimiR™ oligonucleotide), all in the same manner. The experiment was repeated (therefore n = 10) and the combined results are shown.
**Fig. 6****.** Northern blot comparing SPC3649 with SPC3372. Total RNA from one mouse in each group were subjected to miR-122 specific northern blot. Mature miR-122 and the duplex (blocked microRNA) formed between the LNA-antimiR and miR-122 is indicated.
**Fig. 7****.** Mice were treated with 25 mg/kg/day LNA-antimiR or saline for three consecutive days and sacrificed 1, 2 or 3 weeks after last dose. Included are also the values from the animals sacrificed 24 hours after last dose (example 11 "old design"). miR-122 levels were assessed by qPCR and normalized to the mean of the saline group at each individual time point. Included are also the values from the animals sacrificed 24 hours after last dose (shown mean and SD, n=7, 24h n=10). Sacrifice day 9, 16 or 23 corresponds to sacrifice 1, 2 or 3 weeks after last dose.).
**Fig. 8****.** Mice were treated with 25 mg/kg/day LNA-antimiR or saline for three consecutive days and sacrificed 1, 2 or 3 weeks after last dose. Included are also the values from the animals sacrificed 24 hours after last dose (example 11 "old design"). Plasma cholesterol was measured and normalized to the saline group at each time point (shown mean and SD, n=7, 24h n=10).

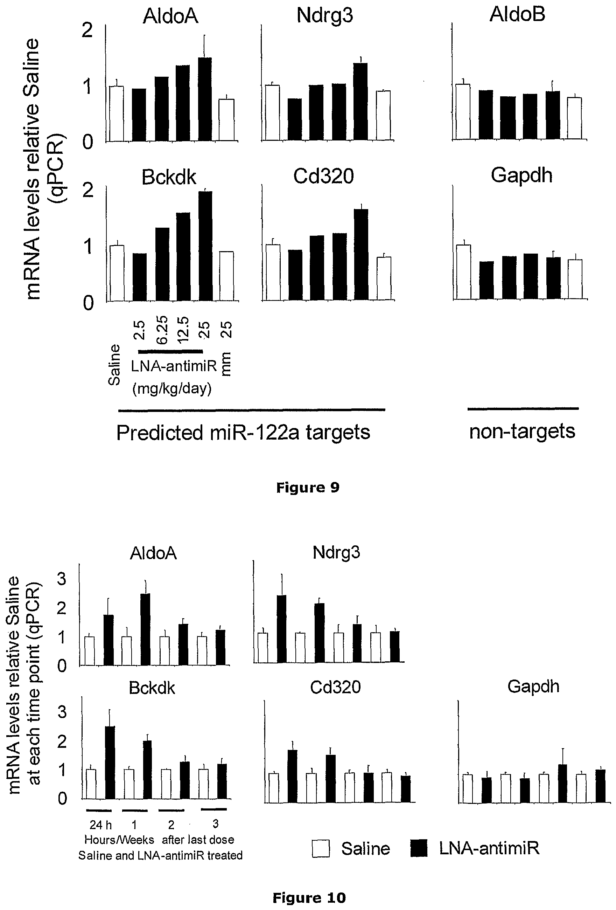

**Fig. 9****.** Dose dependent miR-122a target mRNA induction by SPC3372 inhibition of miR-122a. Mice were treated with different SPC3372 doses for three consecutive days, as described above and sacrificed 24 hours after last dose. Total RNA extracted from liver was subjected to qPCR. Genes with predicted miR-122 target site and observed to be upregulated by microarray analysis were investigated for dose-dependent induction by increasing SPC3372 doses using qPCR. Total liver RNA from 2 to 3 mice per group sacrificed 24 hours after last dose were subjected to qPCR for the indicated genes. Shown in figure 9 is mRNA levels relative to Saline group, n=2-3 (2.5 - 12.5 mg/kg/day: n=2, no SD). Shown is also the mismatch control (mm, SPC3373)
**Fig. 10****.** Transient induction of miR-122a target mRNAs following SPC3372 treatment. NMRI female mice were treated with 25 mg/kg/day SPC3372 along with saline control for three consecutive days and sacrificed 1, 2 or 3 weeks after last dose, respectively. RNA was extracted from livers and mRNA levels of predicted miR-122a target mRNAs, selected by microarray data were investigated by qPCR. Three animals from each group were analysed.
**Fig. 11****.** Induction of Vldlr in liver by SPC3372 treatment. The same liver RNA samples as in previous example (fig. 10) were investigated for Vldlr induction.
**Fig. 12****.** Stability of miR-122a/ SPC3372 duplex in mouse plasma. Stability of SPC3372 and SPC3372/miR-122a duplex were tested in mouse plasma at 37°C over 96 hours. Shown in figure 12 is a SYBR-Gold stained PAGE.
**Fig. 13****.** Sequestering of mature miR-122a by SPC3372 leads to duplex formation. Shown in figure 13 is a membrane probed with a miR-122a specific probe (upper panel) and re-probed with a Let-7 specific probe (lower panel). With the miR-122 probe, two bands could be detected, one corresponding to mature miR-122 and one corresponding to a duplex between SPC3372 and miR-122.
**Fig. 14****.** miR-122a sequestering by SPC3372 along with SPC3372 distribution assessed by in situ hybridization of liver sections. Liver cryo-sections from treated animals were
**Fig. 15****.** Liver gene expression in miR-122 LNA-antimiR treated mice.
   Saline and LNA-antimiR treated mice were compared by genome-wide expression profiling using Affymetrix Mouse Genome 430 2.0 arrays. (a,1) Shown is number of probes displaying differentially expression in liver samples of LNA-antimiR-122 treated and saline treated mice 24 hours post treatment. (b,2) The occurrence of miR-122 seed sequence in differentially expressed genes. The plot shows the percentage of transcripts with at least one miR-122 seed recognition sequence in their 3' UTR. Random: Random sequences were generated and searched for miR-122 seed recognition sequences.
   Temporal liver gene expression profiles in LNA-antimiR treated mice. Mice were treated with 25 mg/kg/day LNA-antimiR or saline for three consecutive days and sacrificed 1, 2 or 3 weeks after last dose. Included are also the values from the animals sacrificed 24 hours after last dose. (c,3) RNA samples from different time points were also subjected to expression profiling. Hierarchical cluster analysis of expression profiles of genes identified as differentially expressed between LNA-antimiR and saline treated mice 24 hours, one week or three weeks post treatment. (d,4) Expression profiles of genes identified as differentially expressed between LNA-antimiR and saline treated mice 24 hours post treatment were followed over time. The expression ratios of up- and down-regulated genes in LNA-antimiR treated mice approach 1 over the time-course, indicating a reversible effect of the LNA-antimiR treatment.

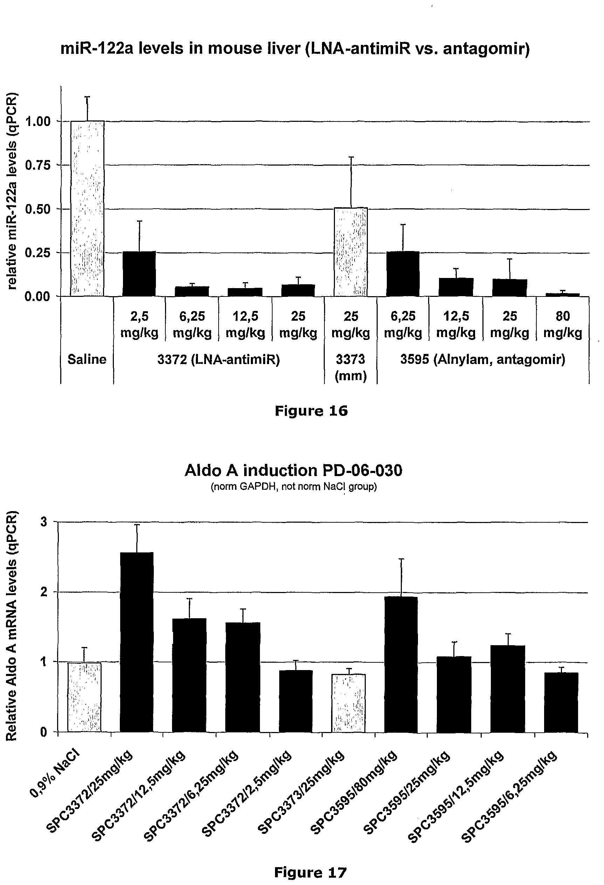

**Fig. 16****.** The effect of treatment with SPC3372 and 3595 on miR-122 levels in mice livers.
**Fig. 17****.** The effect of treatment with SPC3372 and 3595 on Aldolase A levels in mice livers.

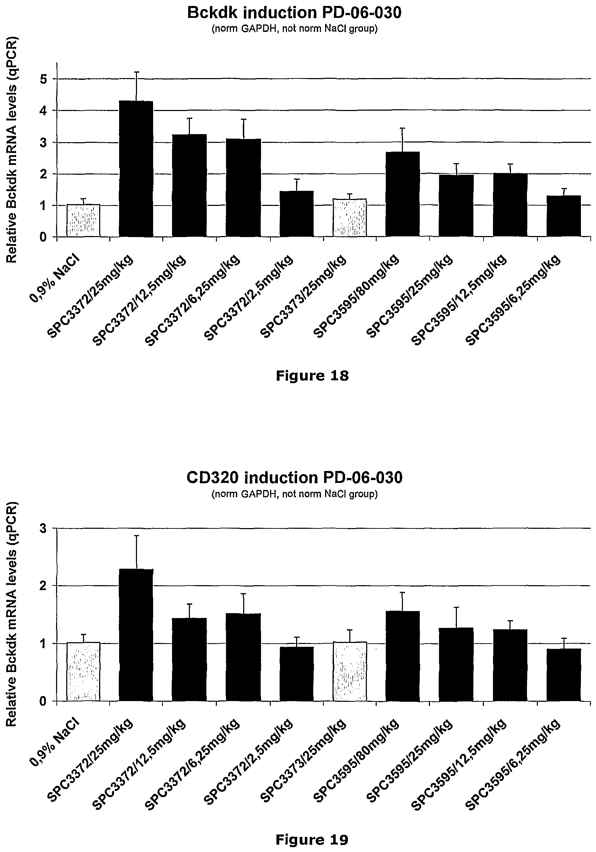

**Fig. 18****.** The effect of treatment with SPC3372 and 3595 on Bckdk levels in mice livers.
**Fig. 19****.** The effect of treatment with SPC3372 and 3595 on CD320 levels in mice livers.

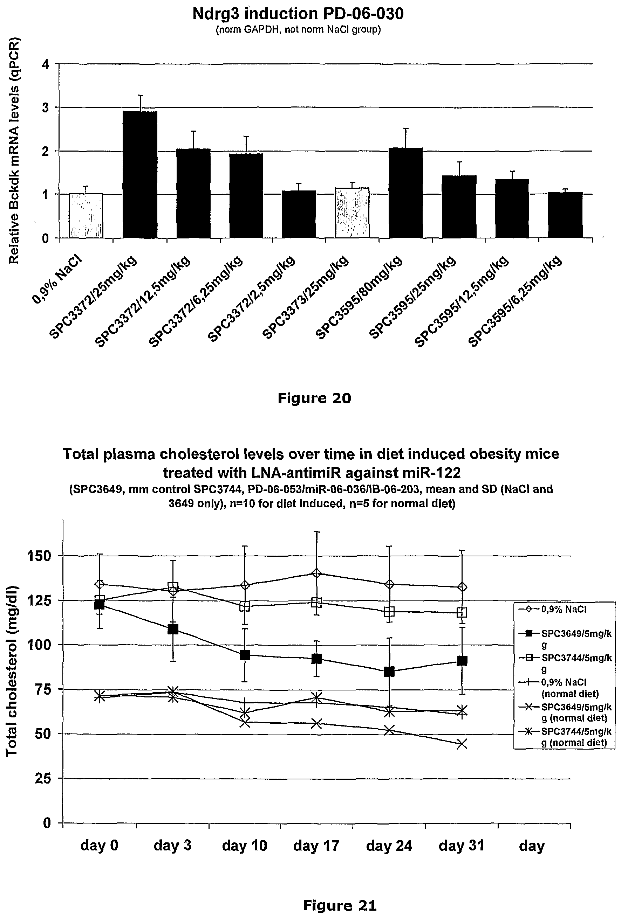

**Fig. 20****.** The effect of treatment with SPC3372 and 3595 on Ndrg3 levels in mice livers.
**Fig.21****.** The effect of long-term treatment with SPC3649 on total plasma cholesterol in hypercholesterolemic and normal mice. Weekly samples of blood plasma were obtained from the SPC3649 treated and saline control mice once weekly followed by assessment of total plasma cholesterol. The mice were treated with 5 mg/kg SPC3649, SPC3744 or saline twice weekly. Normal mice given were treated in parallel.

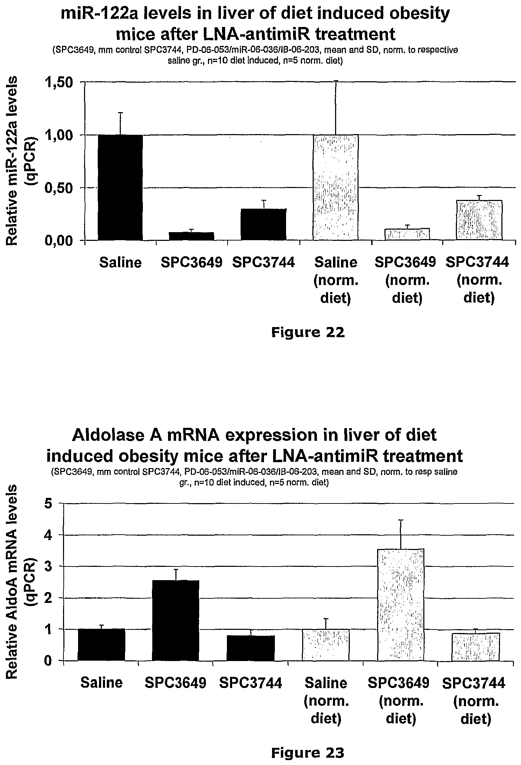

**Fig.22****.** The effect of long-term treatment with SPC3649 on miR-122 levels in hypercholesterolemic and normal mice.
**Fig. 23****.** The effect of long-term treatment with SPC3649 on Aldolase A levels in hypercholesterolemic and normal mice.

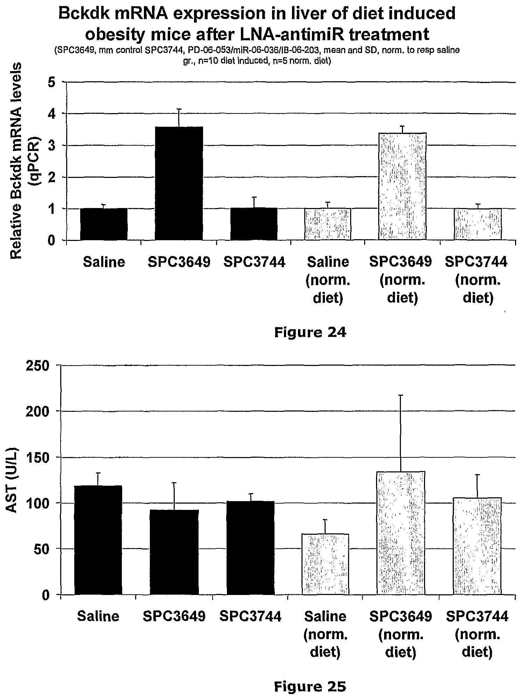

**Fig. 24****.** The effect of long-term treatment with SPC3649 on Bckdk levels in hypercholesterolemic and normal mice.
**Fig. 25****.** The effect of long-term treatment with SPC3649 on AST levels in hypercholesterolemic and normal mice.

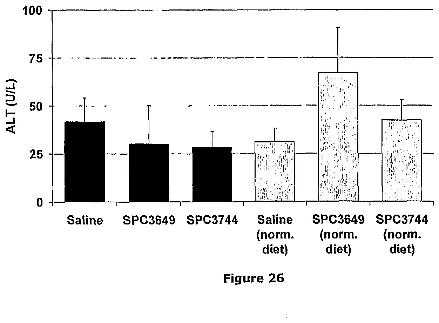

**Fig. 26****.** The effect of long-term treatment with SPC3649 on ALT levels in hypercholesterolemic and normal mice.

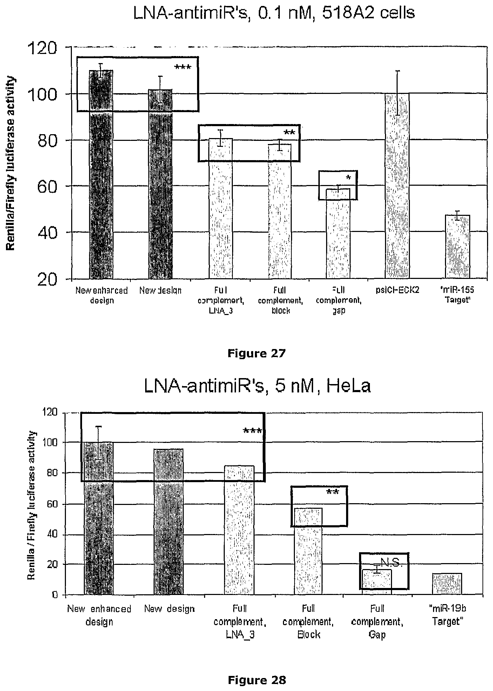

**Fig. 27****.** Functional de-repression of renilla luciferase with miR-155 target by miR-155 blocking oligonucleotides in an endogenously miR-155 expressing cell line, 518A2. "psiCheck2" is the plasmid without miR-155 target, i.e. full expression and "miR-155 target" is the corresponding plasmid with miR-155 target but not co-trasfected with oligo blocking miR-155 and hence represent fully miR-155 repressed renilla luciferace expression.
**Fig. 28****.** Functional de-repression of renilla luciferase with miR-19b target by miR-19b blocking oligonucleotides in an endogenously miR-19b expressing cell line, HeLa. "miR-19b target" is the plasmid with miR-19b target but not co-trasfected with oligo blocking miR-19b and hence represent fully miR-19b repressed renilla luciferace expression.

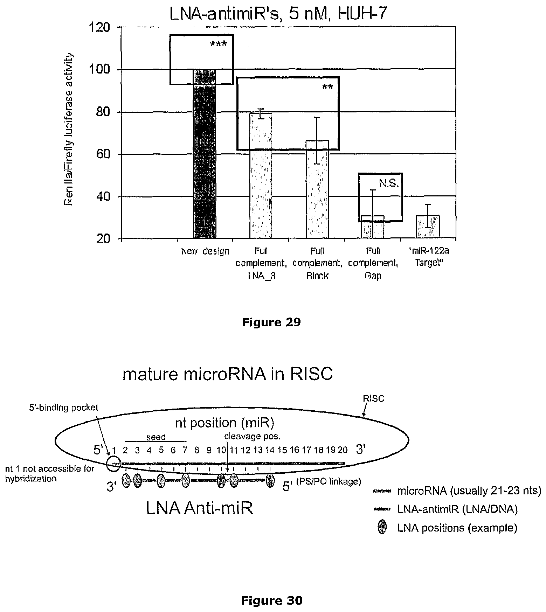

**Fig. 29****.** Functional de-repression of renilla luciferase with miR-122 target by miR-122 blocking oligonucleotides in an endogenously miR-122 expressing cell line, Huh-7. "miR-122 target" is the corresponding plasmid with miR-122 target but not co-trasfected with oligo blocking miR-122 and hence represent fully miR-122 repressed renilla luciferace expression.
**Fig. 30****.** Diagram illustrating the alignment of an oligonucleotide according to the invention and a microRNA target.

### DETAILED DESCRIPTION OF THE INVENTION

The invention provides pharmaceutical compositions according to the claims comprising short single stranded oligonucleotides, of length of between 8 and 17 such as between 10 and 17 nucleobases which are complementary to human microRNAs. The short oligonucleotides are
particularly effective at alleviating miRNA repression in vivo. It is found that the incorporation of high affinity nucleotide analogues into the oligonucleotides results in highly effective anti-microRNA molecules which appear to function via the formation of almost irreversible duplexes with the miRNA target, rather than RNA cleavage based mechanisms, such as mechanisms associated with RNaseH or RISC.

The single stranded oligonucleotide according to the invention comprises a region of contiguous nucleobase sequence which is 100% complementary to the human microRNA seed region.

The single stranded oligonucleotide according to the invention is complementary to the mature human microRNA sequence.

In one embodiment the single stranded oligonucleotide according to the invention is complementary to a microRNA sequence, such as a microRNA sequence selected from the group consisting of: hsa-let-7a, hsa-let-7b, hsa-let-7c, hsa-let-7d, hsa-let-7e, hsa-let-7f, hsa-miR-15a, hsa-miR-16, hsa-miR-17-5p, hsa-miR-17-3p, hsa-miR-18a, hsa-miR-19a, hsa-miR-19b, hsa-miR-20a, hsa-miR-21, hsa-miR-22, hsa-miR-23a, hsa-miR-189, hsa-miR-24, hsa-miR-25, hsa-miR-26a, hsa-miR-26b, hsa-miR-27a, hsa-miR-28, hsa-miR-29a, hsa-miR-30a-5p, hsa-miR-30a-3p, hsa-miR-31, hsa-miR-32, hsa-miR-33, hsa-miR-92, hsa-miR-93, hsa-miR-95, hsa-miR-96, hsa-miR-98, hsa-miR-99a, hsa-miR-100, hsa-miR-101, hsa-miR-29b, hsa-miR-103, hsa-miR-105, hsa-miR-106a, hsa-miR-107, hsa-miR-192, hsa-miR-196a, hsa-miR-197, hsa-miR-198, hsa-miR-199a, hsa-miR-199a*, hsa-miR-208, hsa-miR-129, hsa-miR-148a, hsa-miR-30c, hsa-miR-30d, hsa-miR-139, hsa-miR-147, hsa-miR-7, hsa-miR-10a, hsa-miR-10b, hsa-miR-34a, hsa-miR-181a, hsa-miR-181b, hsa-miR-181c, hsa-miR-182, hsa-miR-182*, hsa-miR-183, hsa-miR-187, hsa-miR-199b, hsa-miR-203, hsa-miR-204, hsa-miR-205, hsa-miR-210, hsa-miR-211, hsa-miR-212, hsa-miR-181a*, hsa-miR-214, hsa-miR-215, hsa-miR-216, hsa-miR-217, hsa-miR-218, hsa-miR-219, hsa-miR-220, hsa-miR-221, hsa-miR-222, hsa-miR-223, hsa-miR-224, hsa-miR-200b, hsa-let-7g, hsa-let-7i, hsa-miR-1, hsa-miR-15b, hsa-miR-23b, hsa-miR-27b, hsa-miR-30b, hsa-miR-122a, hsa-miR-124a, hsa-miR-125b, hsa-miR-128a, hsa-miR-130a, hsa-miR-132, hsa-miR-133a, hsa-miR-135a, hsa-miR-137, hsa-miR-138, hsa-miR-140, hsa-miR-141, hsa-miR-142-5p, hsa-miR-142-3p, hsa-miR-143, hsa-miR-144, hsa-miR-145, hsa-miR-152, hsa-miR-153, hsa-miR-191, hsa-miR-9, hsa-miR-9*, hsa-miR-125a, hsa-miR-126*, hsa-miR-126, hsa-miR-127, hsa-miR-134, hsa-miR-136, hsa-miR-146a, hsa-miR-149, hsa-miR-150, hsa-miR-154, hsa-miR-154*, hsa-miR-184, hsa-miR-185, hsa-miR-186, hsa-miR-188, hsa-miR-190, hsa-miR-193a, hsa-miR-194, hsa-miR-195, hsa-miR-206, hsa-miR-320, hsa-miR-200c, hsa-miR-155, hsa-miR-128b, hsa-miR-106b, hsa-miR-29c, hsa-miR-200a, hsa-miR-302a*, hsa-miR-302a, hsa-miR-34b, hsa-miR-34c, hsa-miR-299-3p, hsa-miR-301, hsa-miR-99b, hsa-miR-296, hsa-miR-130b, hsa-miR-30e-5p, hsa-miR-30e-3p, hsa-miR-361, hsa-miR-362, hsa-miR-363, hsa-miR-365, hsa-miR-302b*, hsa-miR-302b, hsa-miR-302c*, hsa-miR-302c, hsa-miR-302d, hsa-miR-367, hsa-miR-368, hsa-miR-369-3p, hsa-miR-370, hsa-miR-371, hsa-miR-372, hsa-miR-373*, hsa-miR-373, hsa-miR-374, hsa-miR-375, hsa-miR-376a, hsa-miR-377, hsa-miR-378, hsa-miR-422b, hsa-miR-379, hsa-miR-380-5p, hsa-miR-380-3p, hsa-miR-381, hsa-miR-382, hsa-miR-383, hsa-miR-340, hsa-miR-330, hsa-miR-328, hsa-miR-342, hsa-miR-337, hsa-miR-323, hsa-miR-326, hsa-miR-151, hsa-miR-135b, hsa-miR-148b, hsa-miR-331, hsa-miR-324-5p, hsa-miR-324-3p, hsa-miR-338, hsa-miR-339, hsa-miR-335, hsa-miR-133b, hsa-miR-325, hsa-miR-345, hsa-miR-346, ebv-miR-BHRF1-1, ebv-miR-BHRF1-2*, ebv-miR-BHRF1-2, ebv-miR-BHRF1-3, ebv-miR-BART1-5p, ebv-miR-BART2, hsa-miR-384, hsa-miR-196b, hsa-miR-422a, hsa-miR-423, hsa-miR-424, hsa-miR-425-3p, hsa-miR-18b, hsa-miR-20b, hsa-miR-448, hsa-miR-429, hsa-miR-449, hsa-miR-450, hcmv-miR-UL22A, hcmv-miR-UL22A*, hcmv-miR-UL36, hcmv-miR-UL112, hcmv-miR-UL148D, hcmv-miR-US5-1, hcmv-miR-US5-2, hcmv-miR-US25-1, hcmv-miR-US25-2-5p, hcmv-miR-US25-2-3p, hcmv-miR-US33, hsa-miR-191*, hsa-miR-200a*, hsa-miR-369-5p, hsa-miR-431, hsa-miR-433, hsa-miR-329, hsa-miR-453, hsa-miR-451, hsa-miR-452, hsa-miR-452*, hsa-miR-409-5p, hsa-miR-409-3p, hsa-miR-412, hsa-miR-410, hsa-miR-376b, hsa-miR-483, hsa-miR-484, hsa-miR-485-5p, hsa-miR-485-3p, hsa-miR-486, hsa-miR-487a, kshv-miR-K12-10a, kshv-miR-K12-10b, kshv-miR-K12-11, kshv-miR-K12-1, kshv-miR-K12-2, kshv-miR-K12-9*, kshv-miR-K12-9, kshv-miR-K12-8, kshv-miR-K12-7, kshv-miR-K12-6-5p, kshv-miR-K12-6-3p, kshv-miR-K12-5, kshv-miR-K12-4-5p, kshv-miR-K12-4-3p, kshv-miR-K12-3, kshv-miR-K12-3*, hsa-miR-488, hsa-miR-489, hsa-miR-490, hsa-miR-491, hsa-miR-511, hsa-miR-146b, hsa-miR-202*, hsa-miR-202, hsa-miR-492, hsa-miR-493-5p, hsa-miR-432, hsa-miR-432*, hsa-miR-494, hsa-miR-495, hsa-miR-496, hsa-miR-193b, hsa-miR-497, hsa-miR-181d, hsa-miR-512-5p, hsa-miR-512-3p, hsa-miR-498, hsa-miR-520e, hsa-miR-515-5p, hsa-miR-515-3p, hsa-miR-519e*, hsa-miR-519e, hsa-miR-520f, hsa-miR-526c, hsa-miR-519c, hsa-miR-520a*, hsa-miR-520a, hsa-miR-526b, hsa-miR-526b*, hsa-miR-519b, hsa-miR-525, hsa-miR-525*, hsa-miR-523, hsa-miR-518f*, hsa-miR-518f, hsa-miR-520b, hsa-miR-518b, hsa-miR-526a, hsa-miR-520c, hsa-miR-518c*, hsa-miR-518c, hsa-miR-524*, hsa-miR-524, hsa-miR-517*, hsa-miR-517a, hsa-miR-519d, hsa-miR-521, hsa-miR-520d*, hsa-miR-520d, hsa-miR-517b, hsa-miR-520g, hsa-miR-516-5p, hsa-miR-516-3p, hsa-miR-518e, hsa-miR-527, hsa-miR-518a, hsa-miR-518d, hsa-miR-517c, hsa-miR-520h, hsa-miR-522, hsa-miR-519a, hsa-miR-499, hsa-miR-500, hsa-miR-501, hsa-miR-502, hsa-miR-503, hsa-miR-504, hsa-miR-505, hsa-miR-513, hsa-miR-506, hsa-miR-507, hsa-miR-508, hsa-miR-509, hsa-miR-510, hsa-miR-514, hsa-miR-532, hsa-miR-299-5p, hsa-miR-18a*, hsa-miR-455, hsa-miR-493-3p, hsa-miR-539, hsa-miR-544, hsa-miR-545, hsa-miR-487b, hsa-miR-551a, hsa-miR-552, hsa-miR-553, hsa-miR-554, hsa-miR-92b, hsa-miR-555, hsa-miR-556, hsa-miR-557, hsa-miR-558, hsa-miR-559, hsa-miR-560, hsa-miR-561, hsa-miR-562, hsa-miR-563, hsa-miR-564, hsa-miR-565, hsa-miR-566, hsa-miR-567, hsa-miR-568, hsa-miR-551b, hsa-miR-569, hsa-miR-570, hsa-miR-571, hsa-miR-572, hsa-miR-573, hsa-miR-574, hsa-miR-575, hsa-miR-576, hsa-miR-577, hsa-miR-578, hsa-miR-579, hsa-miR-580, hsa-miR-581, hsa-miR-582, hsa-miR-583, hsa-miR-584, hsa-miR-585, hsa-miR-548a, hsa-miR-586, hsa-miR-587, hsa-miR-548b, hsa-miR-588, hsa-miR-589, hsa-miR-550, hsa-miR-590, hsa-miR-591, hsa-miR-592, hsa-miR-593, hsa-miR-595, hsa-miR-596, hsa-miR-597, hsa-miR-598, hsa-miR-599, hsa-miR-600, hsa-miR-601, hsa-miR-602, hsa-miR-603, hsa-miR-604, hsa-miR-605, hsa-miR-606, hsa-miR-607, hsa-miR-608, hsa-miR-609, hsa-miR-610, hsa-miR-611, hsa-miR-612, hsa-miR-613, hsa-miR-614, hsa-miR-615, hsa-miR-616, hsa-miR-548c, hsa-miR-617 hsa-miR-618, hsa-miR-619, hsa-miR-620, hsa-miR-621, hsa-miR-622, hsa-miR-623, hsa-miR-624, hsa-miR-625, hsa-miR-626, hsa-miR-627, hsa-miR-628, hsa-miR-629, hsa-miR-630, hsa-miR-631, hsa-miR-33b, hsa-miR-632, hsa-miR-633, hsa-miR-634, hsa-miR-635, hsa-miR-636, hsa-miR-637, hsa-miR-638, hsa-miR-639, hsa-miR-640, hsa-miR-641, hsa-miR-642, hsa-miR-643, hsa-miR-644, hsa-miR-645, hsa-miR-646, hsa-miR-647, hsa-miR-648, hsa-miR-649, hsa-miR-650, hsa-miR-651, hsa-miR-652, hsa-miR-548d, hsa-miR-661, hsa-miR-662, hsa-miR-663, hsa-miR-449b, hsa-miR-653, hsa-miR-411, hsa-miR-654, hsa-miR-655, hsa-miR-656, hsa-miR-549, hsa-miR-657, hsa-miR-658, hsa-miR-659, hsa-miR-660, hsa-miR-421, hsa-miR-542-5p, hcmv-miR-US4, hcmv-miR-UL70-5p, hcmv-miR-UL70-3p, hsa-miR-363*, hsa-miR-376a*, hsa-miR-542-3p, ebv-miR-BART1-3p, hsa-miR-425-5p, ebv-miR-BART3-5p, ebv-miR-BART3-3p, ebv-miR-BART4, ebv-miR-BART5, ebv-miR-BART6-5p, ebv-miR-BART6-3p, ebv-miR-BART7, ebv-miR-BART8-5p, ebv-miR-BART8-3p, ebv-miR-BART9, ebv-miR-BART10, ebv-miR-BART11-5p, ebv-miR-BART11-3p, ebv-miR-BART12, ebv-miR-BART13, ebv-miR-BART14-5p, ebv-miR-BART14-3p, kshv-miR-K12-12, ebv-miR-BART15, ebv-miR-BART16, ebv-miR-BART17-5p, ebv-miR-BART17-3p, ebv-miR-BART18, ebv-miR-BART19, ebv-miR-BART20-5p, ebv-miR-BART20-3p, hsv1-miR-H1, hsa-miR-758, hsa-miR-671, hsa-miR-668, hsa-miR-767-5p, hsa-miR-767-3p, hsa-miR-454-5p, hsa-miR-454-3p, hsa-miR-769-5p, hsa-miR-769-3p, hsa-miR-766, hsa-miR-765, hsa-miR-768-5p, hsa-miR-768-3p, hsa-miR-770-5p, hsa-miR-802, hsa-miR-801, hsa-miR-675.

In one embodiment the single stranded oligonucleotide according to the invention is complementary to a microRNA sequence, such as a microRNA sequence selected from the group consisting of: hsa-let-7a, hsa-let-7b, hsa-let-7c, hsa-let-7d, hsa-let-7e, hsa-let-7f, hsa-miR-15a, hsa-miR-16, hsa-miR-17-5p, hsa-miR-17-3p, hsa-miR-18a, hsa-miR-19a, hsa-miR-20a, hsa-miR-22, hsa-miR-23a, hsa-miR-189, hsa-miR-24, hsa-miR-25, hsa-miR-26a, hsa-miR-26b, hsa-miR-27a, hsa-miR-28, hsa-miR-29a, hsa-miR-30a-5p, hsa-miR-30a-3p, hsa-miR-31, hsa-miR-32, hsa-miR-33, hsa-miR-92, hsa-miR-93, hsa-miR-95, hsa-miR-96, hsa-miR-98, hsa-miR-99a, hsa-miR-100, hsa-miR-101, hsa-miR-29b, hsa-miR-103, hsa-miR-105, hsa-miR-106a, hsa-miR-107, hsa-miR-192, hsa-miR-196a, hsa-miR-197, hsa-miR-198, hsa-miR-199a, hsa-miR-199a*, hsa-miR-208, hsa-miR-129, hsa-miR-148a, hsa-miR-30c, hsa-miR-30d, hsa-miR-139, hsa-miR-147, hsa-miR-7, hsa-miR-10a, hsa-miR-10b, hsa-miR-34a, hsa-miR-181a, hsa-miR-181b, hsa-miR-181c, hsa-miR-182, hsa-miR-182*, hsa-miR-183, hsa-miR-187, hsa-miR-199b, hsa-miR-203, hsa-miR-204, hsa-miR-205, hsa-miR-210, hsa-miR-211, hsa-miR-212, hsa-miR-181a*, hsa-miR-214, hsa-miR-215, hsa-miR-216, hsa-miR-217, hsa-miR-218, hsa-miR-219, hsa-miR-220, hsa-miR-221, hsa-miR-222, hsa-miR-223, hsa-miR-224, hsa-miR-200b, hsa-let-7g, hsa-let-7i, hsa-miR-1, hsa-miR-15b, hsa-miR-23b, hsa-miR-27b, hsa-miR-30b, hsa-miR-124a, hsa-miR-125b, hsa-miR-128a, hsa-miR-130a, hsa-miR-132, hsa-miR-133a, hsa-miR-135a, hsa-miR-137, hsa-miR-138, hsa-miR-140, hsa-miR-141, hsa-miR-142-5p, hsa-miR-142-3p, hsa-miR-143, hsa-miR-144, hsa-miR-145, hsa-miR-152, hsa-miR-153, hsa-miR-191, hsa-miR-9, hsa-miR-9*, hsa-miR-125a, hsa-miR-126*, hsa-miR-126, hsa-miR-127, hsa-miR-134, hsa-miR-136, hsa-miR-146a, hsa-miR-149, hsa-miR-150, hsa-miR-154, hsa-miR-154*, hsa-miR-184, hsa-miR-185, hsa-miR-186, hsa-miR-188, hsa-miR-190, hsa-miR-193a, hsa-miR-194, hsa-miR-195, hsa-miR-206, hsa-miR-320, hsa-miR-200c, hsa-miR-128b, hsa-miR-106b, hsa-miR-29c, hsa-miR-200a, hsa-miR-302a*, hsa-miR-302a, hsa-miR-34b, hsa-miR-34c, hsa-miR-299-3p, hsa-miR-301, hsa-miR-99b, hsa-miR-296, hsa-miR-130b, hsa-miR-30e-5p, hsa-miR-30e-3p, hsa-miR-361, hsa-miR-362, hsa-miR-363, hsa-miR-365, hsa-miR-302b*, hsa-miR-302b, hsa-miR-302c*, hsa-miR-302c, hsa-miR-302d, hsa-miR-367, hsa-miR-368, hsa-miR-369-3p, hsa-miR-370, hsa-miR-371, hsa-miR-372, hsa-miR-373*, hsa-miR-373, hsa-miR-374, hsa-miR-376a, hsa-miR-377, hsa-miR-378, hsa-miR-422b, hsa-miR-379, hsa-miR-380-5p, hsa-miR-380-3p, hsa-miR-381, hsa-miR-382, hsa-miR-383, hsa-miR-340, hsa-miR-330, hsa-miR-328, hsa-miR-342, hsa-miR-337, hsa-miR-323, hsa-miR-326, hsa-miR-151, hsa-miR-135b, hsa-miR-148b, hsa-miR-331, hsa-miR-324-5p, hsa-miR-324-3p, hsa-miR-338, hsa-miR-339, hsa-miR-335, hsa-miR-133b, hsa-miR-325, hsa-miR-345, hsa-miR-346, ebv-miR-BHRF1-1, ebv-miR-BHRF1-2*, ebv-miR-BHRF1-2, ebv-miR-BHRF1-3, ebv-miR-BART1-5p, ebv-miR-BART2, hsa-miR-384, hsa-miR-196b, hsa-miR-422a, hsa-miR-423, hsa-miR-424, hsa-miR-425-3p, hsa-miR-18b, hsa-miR-20b, hsa-miR-448, hsa-miR-429, hsa-miR-449, hsa-miR-450, hcmv-miR-UL22A, hcmv-miR-UL22A*, hcmv-miR-UL36, hcmv-miR-UL112, hcmv-miR-UL148D, hcmv-miR-US5-1, hcmv-miR-US5-2, hcmv-miR-US25-1, hcmv-miR-US25-2-5p, hcmv-miR-US25-2-3p, hcmv-miR-US33, hsa-miR-191*, hsa-miR-200a*, hsa-miR-369-5p, hsa-miR-431, hsa-miR-433, hsa-miR-329, hsa-miR-453, hsa-miR-451, hsa-miR-452, hsa-miR-452*, hsa-miR-409-5p, hsa-miR-409-3p, hsa-miR-412, hsa-miR-410, hsa-miR-376b, hsa-miR-483, hsa-miR-484, hsa-miR-485-5p, hsa-miR-485-3p, hsa-miR-486, hsa-miR-487a, kshv-miR-K12-10a, kshv-miR-K12-10b, kshv-miR-K12-11, kshv-miR-K12-1, kshv-miR-K12-2, kshv-miR-K12-9*, kshv-miR-K12-9, kshv-miR-K12-8, kshv-miR-K12-7, kshv-miR-K12-6-5p, kshv-miR-K12-6-3p, kshv-miR-K12-5, kshv-miR-K12-4-5p, kshv-miR-K12-4-3p, kshv-miR-K12-3, kshv-miR-K12-3*, hsa-miR-488, hsa-miR-489, hsa-miR-490, hsa-miR-491, hsa-miR-511, hsa-miR-146b, hsa-miR-202*, hsa-miR-202, hsa-miR-492, hsa-miR-493-5p, hsa-miR-432, hsa-miR-432*, hsa-miR-494, hsa-miR-495, hsa-miR-496, hsa-miR-193b, hsa-miR-497, hsa-miR-181d, hsa-miR-512-5p, hsa-miR-512-3p, hsa-miR-498, hsa-miR-520e, hsa-miR-515-5p, hsa-miR-515-3p, hsa-miR-519e*, hsa-miR-519e, hsa-miR-520f, hsa-miR-526c, hsa-miR-519c, hsa-miR-520a*, hsa-miR-520a, hsa-miR-526b, hsa-miR-526b*, hsa-miR-519b, hsa-miR-525, hsa-miR-525*, hsa-miR-523, hsa-miR-518f*, hsa-miR-518f, hsa-miR-520b, hsa-miR-518b, hsa-miR-526a, hsa-miR-520c, hsa-miR-518c*, hsa-miR-518c, hsa-miR-524*, hsa-miR-524, hsa-miR-517*, hsa-miR-517a, hsa-miR-519d, hsa-miR-521, hsa-miR-520d*, hsa-miR-520d, hsa-miR-517b, hsa-miR-520g, hsa-miR-516-5p, hsa-miR-516-3p, hsa-miR-518e, hsa-miR-527, hsa-miR-518a, hsa-miR-518d, hsa-miR-517c, hsa-miR-520h, hsa-miR-522, hsa-miR-519a, hsa-miR-499, hsa-miR-500, hsa-miR-501, hsa-miR-502, hsa-miR-503, hsa-miR-504, hsa-miR-505, hsa-miR-513, hsa-miR-506, hsa-miR-507, hsa-miR-508, hsa-miR-509, hsa-miR-510, hsa-miR-514, hsa-miR-532, hsa-miR-299-5p, hsa-miR-18a*, hsa-miR-455, hsa-miR-493-3p, hsa-miR-539, hsa-miR-544, hsa-miR-545, hsa-miR-487b, hsa-miR-551a, hsa-miR-552, hsa-miR-553, hsa-miR-554, hsa-miR-92b, hsa-miR-555, hsa-miR-556, hsa-miR-557, hsa-miR-558, hsa-miR-559, hsa-miR-560, hsa-miR-561, hsa-miR-562, hsa-miR-563, hsa-miR-564, hsa-miR-565, hsa-miR-566, hsa-miR-567, hsa-miR-568, hsa-miR-551b, hsa-miR-569, hsa-miR-570, hsa-miR-571, hsa-miR-572, hsa-miR-573, hsa-miR-574, hsa-miR-575, hsa-miR-576, hsa-miR-577, hsa-miR-578, hsa-miR-579, hsa-miR-580, hsa-miR-581, hsa-miR-582, hsa-miR-583, hsa-miR-584, hsa-miR-585, hsa-miR-548a, hsa-miR-586, hsa-miR-587, hsa-miR-548b, hsa-miR-588, hsa-miR-589, hsa-miR-550, hsa-miR-590, hsa-miR-591, hsa-miR-592, hsa-miR-593, hsa-miR-595, hsa-miR-596, hsa-miR-597, hsa-miR-598, hsa-miR-599, hsa-miR-600, hsa-miR-601, hsa-miR-602, hsa-miR-603, hsa-miR-604, hsa-miR-605, hsa-miR-606, hsa-miR-607, hsa-miR-608, hsa-miR-609, hsa-miR-610, hsa-miR-611, hsa-miR-612, hsa-miR-613, hsa-miR-614, hsa-miR-615, hsa-miR-616, hsa-miR-548c, hsa-miR-617, hsa-miR-618, hsa-miR-619, hsa-miR-620, hsa-miR-621, hsa-miR-622, hsa-miR-623, hsa-miR-624, hsa-miR-625, hsa-miR-626, hsa-miR-627, hsa-miR-628, hsa-miR-629, hsa-miR-630, hsa-miR-631, hsa-miR-33b, hsa-miR-632, hsa-miR-633, hsa-miR-634, hsa-miR-635, hsa-miR-636, hsa-miR-637, hsa-miR-638, hsa-miR-639, hsa-miR-640, hsa-miR-641, hsa-miR-642, hsa-miR-643, hsa-miR-644, hsa-miR-645, hsa-miR-646, hsa-miR-647, hsa-miR-648, hsa-miR-649, hsa-miR-650, hsa-miR-651, hsa-miR-652, hsa-miR-548d, hsa-miR-661, hsa-miR-662, hsa-miR-663, hsa-miR-449b, hsa-miR-653, hsa-miR-411, hsa-miR-654, hsa-miR-655, hsa-miR-656, hsa-miR-549, hsa-miR-657, hsa-miR-658, hsa-miR-659, hsa-miR-660, hsa-miR-421, hsa-miR-542-5p, hcmv-miR-US4, hcmv-miR-UL70-5p, hcmv-miR-UL70-3p, hsa-miR-363*, hsa-miR-376a*, hsa-miR-542-3p, ebv-miR-BART1-3p, hsa-miR-425-5p, ebv-miR-BART3-5p, ebv-miR-BART3-3p, ebv-miR-BART4, ebv-miR-BART5, ebv-miR-BART6-5p, ebv-miR-BART6-3p, ebv-miR-BART7, ebv-miR-BART8-5p, ebv-miR-BART8-3p, ebv-miR-BART9, ebv-miR-BART10, ebv-miR-BART11-5p, ebv-miR-BART11-3p, ebv-miR-BART12, ebv-miR-BART13, ebv-miR-BART14-5p, ebv-miR-BART14-3p, kshv-miR-K12-12, ebv-miR-BART15, ebv-miR-BART16, ebv-miR-BART17-5p, ebv-miR-BART17-3p, ebv-miR-BART18, ebv-miR-BART19, ebv-miR-BART20-5p, ebv-miR-BART20-3p, hsv1-miR-H1, hsa-miR-758, hsa-miR-671, hsa-miR-668, hsa-miR-767-5p, hsa-miR-767-3p, hsa-miR-454-5p, hsa-miR-454-3p, hsa-miR-769-5p, hsa-miR-769-3p, hsa-miR-766, hsa-miR-765, hsa-miR-768-5p, hsa-miR-768-3p, hsa-miR-770-5p, hsa-miR-802, hsa-miR-801, hsa-miR-675

Preferred single stranded oligonucleotide according to the invention are complementary to a microRNA sequence selected from the group consisting of has-miR19b, hsa-miR21, hsa-miR 122, hsa-miR 142 a7b, hsa-miR 155, hsa-miR 375.

Preferred single stranded oligonucleotide according to the invention are complementary to a microRNA sequence selected from the group consisting of hsa-miR196b and has-181a.

In one embodiment, the oligonucleotide according to the invention does not comprise a nucleobase at the 3' end that corresponds to the first 5' end nucleotide of the target microRNA.

In one embodiment, the first nucleobase of the single stranded oligonucleotide according to the invention, counting from the 3' end, is a nucleotide analogue, such as an LNA unit.

In one embodiment, the second nucleobase of the single stranded oligonucleotide according to the invention, counting from the 3' end, is a nucleotide analogue, such as an LNA unit.

In one embodiment, the ninth and/or the tenth nucleotide of the single stranded oligonucleotide according to the invention, counting from the 3' end, is a nucleotide analogue, such as an LNA unit.

In one embodiment, the ninth nucleobase of the single stranded oligonucleotide according to the invention, counting from the 3' end is a nucleotide analogue, such as an LNA unit.

In one embodiment, the tenth nucleobase of the single stranded oligonucleotide according to the invention, counting from the 3' end is a nucleotide analogue, such as an LNA unit.

In one embodiment, both the ninth and the tenth nucleobase of the single stranded oligonucleotide according to the invention, calculated from the 3' end is a nucleotide analogue, such as an LNA unit.

In one embodiment, the single stranded oligonucleotide according to the invention does not comprise a region of more than 5 consecutive DNA nucleotide units. In one embodiment, the single stranded oligonucleotide according to the invention does not comprise a region of more than 6 consecutive DNA nucleotide units. In one embodiment, the single stranded oligonucleotide according to the invention does not comprise a region of more than 7 consecutive DNA nucleotide units. In one embodiment, the single stranded oligonucleotide according to the invention does not comprise a region of more than 8 consecutive DNA nucleotide units. In one embodiment, the single stranded oligonucleotide according to the invention does not comprise a region of more than 3 consecutive DNA nucleotide units. In one embodiment, the single stranded oligonucleotide according to the invention does not comprise a region of more than 2 consecutive DNA nucleotide units.

In one embodiment, the single stranded oligonucleotide comprises at least region consisting of at least two consecutive nucleotide analogue units, such as at least two consecutive LNA units.

In one embodiment, the single stranded oligonucleotide comprises at least region consisting of at least three consecutive nucleotide analogue units, such as at least three consecutive LNA units.

In one embodiment, the single stranded oligonucleotide of the invention does not comprise a region of more than 7 consecutive nucleotide analogue units, such as LNA units. In one embodiment, the single stranded oligonucleotide of the invention does not comprise a region of more than 6consecutive nucleotide analogue units, such as LNA units. In one embodiment, the single stranded oligonucleotide of the invention does not comprise a region of more than 5 consecutive nucleotide analogue units, such as LNA units. In one embodiment, the single stranded oligonucleotide of the invention does not comprise a region of more than 4 consecutive nucleotide analogue units, such as LNA units. In one embodiment, the single stranded oligonucleotide of the invention does not comprise a region of more than 3 consecutive nucleotide analogue units, such as LNA units. In one embodiment, the single stranded oligonucleotide of the invention does not comprise a region of more than 2 consecutive nucleotide analogue units, such as LNA units.

In one embodiment, the first or second 3' nucleobase of the single stranded oligonucleotide corresponds to the second 5' nucleotide of the microRNA sequence.

In one embodiment, nucleobase units 1 to 6 (inclusive) of the single stranded oligonucleotide as measured from the 3' end of the single stranded oligonucleotide are complementary to the microRNA seed region sequence.

In one embodiment, nucleobase units 1 to 7 (inclusive) of the single stranded oligonucleotide as measured from the 3' end of the single stranded oligonucleotide are complementary to the microRNA seed region sequence.

In one embodiment, nucleobase units 2 to 7 (inclusive) of the single stranded oligonucleotide as measured from the 3' end of the single stranded oligonucleotide are complementary to the microRNA seed region sequence.

The single stranded oligonucleotide comprises at least one LNA unit, in a position which is within the region complementary to the miRNA seed region. The single stranded oligonucleotide may, in one embodiment comprise at between one and 6 or between 1 and 7 nucleotide analogue units, such as between 1 and 6 and 1 and 7 LNA units, in a position which is within the region complementary to the miRNA seed region.

In one embodiment, the nucleobase sequence of the single stranded oligonucleotide which is complementary to the sequence of the microRNA seed region, is selected from the group consisting of (X)Xxxxxx, (X)xXxxxx, (X)xxXxxx, (X)xxxXxx, (X)xxxxXx and (X)xxxxxX, as read in a 3' - 5'direction, wherein "X" denotes a nucleotide analogue, (X) denotes an optional nucleotide analogue, such as an LNA unit, and "x" denotes a DNA or RNA nucleotide unit.

In one embodiment, the single stranded oligonucleotide comprises at least two nucleotide analogue units, such as at least two LNA units, in positions which are complementary to the miRNA seed region.

In one embodiment, the nucleobase sequence of the single stranded oligonucleotide which is complementary to the sequence of the microRNA seed region, is selected from the group consisting of (X)XXxxxx, (X)XxXxxx, (X)XxxXxx, (X)XxxxXx, (X)XxxxxX, (X)xXXxxx, (X)xXxXxx, (X)xXxxXx, (X)xXxxxX, (X)xxXXxx, (X)xxXxXx, (X)xxXxxX, (X)xxxXXx, (X)xxxXxX and (X)xxxxXX, wherein "X" denotes a nucleotide analogue, such as an LNA unit, (X) denotes an optional nucleotide analogue, such as an LNA unit, and "x" denotes a DNA or RNA nucleotide unit.

In one embodiment, the single stranded oligonucleotide comprises at least three nucleotide analogue units, such as at least three LNA units, in positions which are complementary to the miRNA seed region.

In one embodiment, the nucleobase sequence of the single stranded oligonucleotide which is complementary to the sequence of the microRNA seed region, is selected from the group consisting of (X)XXXxxx, (X)xXXXxx, (X)xxXXXx, (X)xxxXXX, (X)XXxXxx, (X)XXxxXx, (X)XXxxxX, (X)xXXxXx, (X)xXXxxX, (X)xxXXxX, (X)XxXXxx, (X)XxxXXx, (X)XxxxXX, (X)xXxXXx, (X)xXxxXX, (X)xxXxXX, (X)xXxXxX and (X)XxXxXx, wherein "X" denotes a nucleotide analogue, such as an LNA unit, (X) denotes an optional nucleotide analogue, such as an LNA unit, and "x" denotes a DNA or RNA nucleotide unit.

In one embodiment, the single stranded oligonucleotide comprises at least four nucleotide analogue units, such as at least four LNA units, in positions which are complementary to the miRNA seed region.

In one embodiment the nucleobase sequence of the single stranded oligonucleotide which is complementary to the sequence of the microRNA seed region, is selected from the group consisting of (X)xxXXX, (X)xXxXXX, (X)xXXxXX, (X)xXXXxX, (X)xXXXXx, (X)XxxXXXX, (X)XxXxXX, (X)XxXXxX, (X)XxXXx, (X)XXxxXX, (X)XXxXxX, (X)XXxXXx, (X)XXXxxX, (X)XXXxXx, and (X)XXXXxx, wherein "X" denotes a nucleotide analogue, such as an LNA unit, (X) denotes an optional nucleotide analogue, such as an LNA unit, and "x" denotes a DNA or RNA nucleotide unit.

In one embodiment, the single stranded oligonucleotide comprises at least five nucleotide analogue units, such as at least five LNA units, in positions which are complementary to the miRNA seed region.

In one embodiment, the nucleobase sequence of the single stranded oligonucleotide which is complementary to the sequence of the microRNA seed region, is selected from the group consisting of (X)xXXXXX, (X)XxXXXX, (X)XXxXXX, (X)XXXxXX, (X)XXXXxX and (X)XXXXXx, wherein "X" denotes a nucleotide analogue, such as an LNA unit, (X) denotes an optional nucleotide analogue, such as an LNA unit, and "x" denotes a DNA or RNA nucleotide unit.

In one embodiment, the single stranded oligonucleotide comprises six or seven nucleotide analogue units, such as six or seven LNA units, in positions which are complementary to the miRNA seed region.

In one embodiment, the nucleobase sequence of the single stranded oligonucleotide which is complementary to the sequence of the microRNA seed region, is selected from the group consisting of XXXXXX, XxXXXXX, XXxXXXX, XXXxXXX, XXXXxXX, XXXXXxX and XXXXXXx, wherein "X" denotes a nucleotide analogue, such as an LNA unit, such as an LNA unit, and "x" denotes a DNA or RNA nucleotide unit.

In one embodiment, the two nucleobase motif at position 7 to 8, counting from the 3' end of the single stranded oligonucleotide is selected from the group consisting of xx, XX, xX and Xx, wherein "X" denotes a nucleotide analogue, such as an LNA unit, such as an LNA unit, and "x" denotes a DNA or RNA nucleotide unit.

In one embodiment, the two nucleobase motif at position 7 to 8, counting from the 3' end of the single stranded oligonucleotide is selected from the group consisting of XX, xX and Xx, wherein "X" denotes a nucleotide analogue, such as an LNA unit, such as an LNA unit, and "x" denotes a DNA or RNA nucleotide unit.

In one embodiment, the single stranded oligonucleotide comprises at least 12 nucleobases and wherein the two nucleobase motif at position 11 to 12, counting from the 3' end of the single stranded oligonucleotide is selected from the group consisting of xx, XX, xX and Xx, wherein "X" denotes a nucleotide analogue, such as an LNA unit, such as an LNA unit, and "x" denotes a DNA or RNA nucleotide unit.

In one embodiment, the single stranded oligonucleotide comprises at least 12 nucleobases and wherein the two nucleobase motif at position 11 to 12, counting from the 3' end of the single stranded oligonucleotide is selected from the group consisting of XX, xX and Xx, wherein "X" denotes a nucleotide analogue, such as an LNA unit, such as an LNA unit, and "x" denotes a DNA or RNA nucleotide unit.

In one embodiment, the single stranded oligonucleotide comprises at least 13 nucleobases and wherein the three nucleobase motif at position 11 to 13, counting from the 3' end, is selected from the group consisting of xxx, Xxx, xXx, xxX, XXx, XxX, xXX and XXX, wherein "X" denotes a nucleotide analogue, such as an LNA unit, such as an LNA unit, and "x" denotes a DNA or RNA nucleotide unit.

In one embodiment, the three nucleobase motif at position 11 to 13, counting from the 3' end of the single stranded oligonucleotide, is selected from the group consisting of Xxx, xXx, xxX, XXx, XxX, xXX and XXX, wherein "X" denotes a nucleotide analogue, such as an LNA unit, such as an LNA unit, and "x" denotes a DNA or RNA nucleotide unit.

In one embodiment, the single stranded oligonucleotide comprises at least 14 nucleobases and wherein the four nucleobase motif at positions 11 to 14, counting from the 3' end, is selected from the group consisting of xxxx, Xxxx, xXxx, xxXx, xxxX, XXxx, XxXx, XxxX, xXXx, xXxX, xxXX, XXXx, XxXX, xXXX, XXxX and XXXX wherein "X" denotes a nucleotide analogue, such as an LNA unit, such as an LNA unit, and "x" denotes a DNA or RNA nucleotide unit.

In one embodiment, the four nucleobase motif at position 11 to 14 of the single stranded oligonucleotide, counting from the 3' end, is selected from the group consisting of Xxxx, xXxx, xxXx, xxxX, XXxx, XxXx, XxxX, xXXx, xXxX, xxXX, XXXx, XxXX, xXXX, XXxX and XXXX, wherein "X" denotes a nucleotide analogue, such as an LNA unit, and "x" denotes a DNA or RNA nucleotide unit.

In one embodiment, the single stranded oligonucleotide comprises 15 nucleobases and the five nucleobase motif at position 11 to 15, counting from the 3' end, is selected from the group consisting of Xxxxx, xXxxx, xxXxx, xxxXx, xxxxX, XXxxx, XxXxx, XxxXx, XxxxX, xXXxx, xXxXx, xXxxX, xxXXx, xxXxX, xxxXX, XXXxx, XXxxX, XxxXX, xXXXx, xxXXX, XXxXX, XxXxX, XXXXx, XXXxX, XXxXX, XxXXXX, xXXXX, and XXXXX wherein "X" denotes a nucleotide analogue, such as an LNA unit, such as an LNA unit, and "x" denotes a DNA or RNA nucleotide unit.

In one embodiment, the single stranded oligonucleotide comprises 16 nucleobases and the six nucleobase motif at positions 11 to 16, counting from the 3' end, is selected from the group consisting of Xxxxxx, xXxxxx, xxXxxx, xxxXxx, xxxxXx, xxxxxX, XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX, xxxxXX, XXXxxx, XXxXxx, XXxxXx, XXxxxX, XxXXxx, XxXxXx, XxXxxX, XxxXXx, XxxXxX, XxxxXX, xXXXxx, xXXxXx, xXXxxX, xXxXXx, xXxXxX, xXxxXX, xxXXXx, xxXXxX, xxXxXX, xxxXXX, XXXXxx, XXXxxX, XXxxXX, XxxXXX, xxXXXX, xXxXXX, XxXxXX, XXxXxX, XXXxXx, xXXxXX, XxXXxX, XXxXXx, xXXXxX, XxXXXx, xXXXXx, xXXXXX, XxXXXX, XXxXXX, XXXxXX, XXXXxX, XXXXXx, and XXXXXX wherein "X" denotes a nucleotide analogue, such as an LNA unit, such as an LNA unit, and "x" denotes a DNA or RNA nucleotide unit.

In one embodiment, the six nucleobase motif at positions 11 to 16 of the single stranded oligonucleotide, counting from the 3' end, is xxXxxX, wherein "X" denotes a nucleotide analogue, such as an LNA unit, such as an LNA unit, and "x" denotes a DNA or RNA nucleotide unit.

In one embodiment, the three 5' most nucleobases, is selected from the group consisting of Xxx, xXx, xxX, XXx, XxX, xXX and XXX, wherein "X" denotes a nucleotide analogue, such as an LNA unit, such as an LNA unit, and "x" denotes a DNA or RNA nucleotide unit. In one embodiment, x" denotes a DNA unit.

In one embodiment, the single stranded oligonucleotide comprises a nucleotide analogue unit, such as an LNA unit, at the 5' end.

In one embodiment, the nucleotide analogue units, such as X, are independently selected form the group consisting of: 2'-O-alkyl-RNA unit, 2'-OMe-RNA unit, 2'-amino-DNA unit, 2'-fluoro-DNA unit, LNA unit, PNA unit, HNA unit, INA unit.

In one embodiment, all the nucleobases of the single stranded oligonucleotide of the invention are nucleotide analogue units.

In one embodiment, the nucleotide analogue units, such as X, are independently selected form the group consisting of: 2'-OMe-RNA units, 2'-fluoro-DNA units, and LNA units,

In one embodiment, the single stranded oligonucleotide comprises said at least one LNA analogue unit and at least one further nucleotide analogue unit other than LNA.

In one embodiment, the non-LNA nucleotide analogue unit or units are independently selected from 2'-OMe RNA units and 2'-fluoro DNA units.

In one embodiment, the single stranded oligonucleotide consists of at least one sequence XYX or YXY, wherein X is LNA and Y is either a 2'-OMe RNA unit and 2'-fluoro DNA unit.

In one embodiment, the sequence of nucleobases of the single stranded oligonucleotide consists of alternative X and Y units.

In one embodiment, the single stranded oligonucleotide comprises alternating LNA and DNA units (Xx) or (xX).

In one embodiment, the single stranded oligonucleotide comprises a motif of alternating LNA followed by 2 DNA units (Xxx), xXx or xxX.

In one embodiment, at least one of the DNA or non-LNA nucleotide analogue units are replaced with a LNA nucleobase in a position selected from the positions identified as LNA nucleobase units in any one of the embodiments referred to above.

In one embodiment,"X" donates an LNA unit.

In one embodiment, the single stranded oligonucleotide comprises at least 2 nucleotide analogue units, such as at least 3 nucleotide analogue units, such as at least 4 nucleotide analogue units, such as at least 5 nucleotide analogue units, such as at least 6 nucleotide analogue units, such as at least 7 nucleotide analogue units, such as at least 8 nucleotide analogue units, such as at least 9 nucleotide analogue units, such as at least 10 nucleotide analogue units.

In one embodiment, the single stranded oligonucleotide comprises at least 2 LNA units, such as at least 3 LNA units, such as at least 4 LNA units, such as at least 5 LNA units, such as at least 6 LNA units, such as at least 7 LNA units, such as at least 8 LNA units, such as at least 9 LNA units, such as at least 10 LNA units.

In one embodiment wherein at least one of the nucleotide analogues, such as LNA units, is either cytosine or guanine, such as between 1 - 10 of the of the nucleotide analogues, such as LNA units, is either cytosine or guanine, such as 2, 3, 4, 5, 6, 7, 8, or 9 of the of the nucleotide analogues, such as LNA units, is either cytosine or guanine.

In one embodiment at least two of the nucleotide analogues such as LNA units is either cytosine or guanine. In one embodiment at least three of the nucleotide analogues such as LNA units is either cytosine or guanine. In one embodiment at least four of the nucleotide analogues such as LNA units is either cytosine or guanine. In one embodiment at least five of the nucleotide analogues such as LNA units is either cytosine or guanine. In one embodiment at least six of the nucleotide analogues such as LNA units is either cytosine or guanine. In one embodiment at least seven of the nucleotide analogues such as LNA units is either cytosine or guanine. In one embodiment at least eight of the nucleotide analogues such as LNA units is either cytosine or guanine.

In a preferred embodiment the nucleotide analogues have a higher thermal duplex stability a complementary RNA nucleotide than the binding affinity of an equivalent DNA nucleotide to said complementary RNA nucleotide.

In one embodiment, the nucleotide analogues confer enhanced serum stability to the single stranded oligonucleotide.

In one embodiment, the single stranded oligonucleotide forms an A-helix conformation with a complementary single stranded RNA molecule.

A duplex between two RNA molecules typically exists in an A-form conformation, where as a duplex between two DNA molecules typically exits in a B-form conformation. A duplex between a DNA and RNA molecule typically exists in a intermediate conformation (A/B form). The use of nucleotide analogues, such as beta-D-oxy LNA can be used to promote a more A form like conformation. Standard circular dichromisms (CD) or NMR analysis is used to determine the form of duplexes between the oligonucleotides of the invention and complementary RNA molecules.

As recruitment by the RISC complex is thought to be dependant upon the specific structural conformation of the miRNA/mRNA target, the oligonucleotides according to the present invention may, in one embodiment form a A/B- form duplex with a complementary RNA molecule.

However, we have also determined that the use of nucleotide analogues which promote the A-form structure can also be effective, such as the alpha-L isomer of LNA.

In one embodiment, the single stranded oligonucleotide forms an A/B-form conformation with a complementary single stranded RNA molecule.

In one embodiment, the single stranded oligonucleotide forms an A-from conformation with a complementary single stranded RNA molecule.

In one embodiment, the single stranded oligonucleotide according to the invention does not mediate RNAseH based cleavage of a complementary single stranded RNA molecule. Typically a stretch of at least 5 (typically not effective ofr RNAse H recruitment), more preferably at least 6, more preferably at least 7 or 8 consecutive DNA nucleobases (or alternative nucleobases which can recruit RNAseH, such as alpha L-amino LNA) are required in order for an oligonucleotide to be effective in recruitment of RNAseH.

EP 1 222 309 provides in vitro methods for determining RNaseH activity, which may be used to determine the ability to recruit RNaseH. A compound is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothiote linkage groups between all nucleotides in the oligonucleotide, using the methodology provided by Example 91 - 95 of EP 1 222 309..

A compound is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphiothiote linkage groups between all nucleotides in the oligonucleotide, using the methodology provided by Example 91 - 95 of EP 1 222 309.

In a highly preferred embodiment, the single stranded oligonucleotide of the invention is capable of forming a duplex with a complementary single stranded RNA nucleic acid molecule (typically of about the same length of said single stranded oligonucleotide) with phosphodiester internucleoside linkages, wherein the duplex has a Tₘ of at least about 60°C, indeed it is preferred that the single stranded oligonucleotide is capable of forming a duplex with a complementary single stranded RNA nucleic acid molecule with phosphodiester internucleoside linkages, wherein the duplex has a Tₘ of between about 70°C to about 95°C, such as a Tₘ of between about 70°C to about 90°C, such as between about 70°C and about 85°C.

In one embodiment, the single stranded oligonucleotide is capable of forming a duplex with a complementary single stranded DNA nucleic acid molecule with phosphodiester internucleoside linkages, wherein the duplex has a Tₘ of between about 50°C to about 95°C, such as between about 50°C to about 90°C, such as at least about 55°C, such as at least about 60°C, or no more than about 95°C

The single stranded oligonucleotide may, in one embodiment have a length of between 14 -16 nucleobases, including 15 nucleobases.

In one embodiment, the LNA unit or units are independently selected from the group consisting of oxy-LNA, thio-LNA, and amino-LNA, in either of the D-β and L-α configurations or combinations thereof.

In one specific embodiment the LNA units may be an ENA nucleobase.

In one the embodiment the LNA units are beta D oxy-LNA.

In one embodiment the LNA units are in alpha-L amino LNA.

In a preferable embodiment, the single stranded oligonucleotide comprises between 3 and 17 LNA units.

All the internucleoside linkages are phosphorothioate linkages.

In one embodiment, pharmaceutical composition according to the invention comprises a carrier such as saline or buffered saline.

In one embodiment, the method for the synthesis of a single stranded oligonucleotide targeted against a human microRNA, is performed in the 3' to 5' direction a - f.

The method for the synthesis of the single stranded oligonucleotide according to the invention may be performed using standard solid phase oligonucleotide systhesis.

### Definitions

The term 'nucleobase' refers to nucleotides, such as DNA and RNA, and nucleotide analogues.

The term "oligonucleotide" (or simply "oligo") refers, in the context of the present invention, to a molecule formed by covalent linkage of two or more nucleobases. When used in the context of the oligonucleotide of the invention (also referred to the single stranded oligonucleotide), the term "oligonucleotide"has a length of between 8 - 17 nucleobases, such as between 8 - 16 nucleobases, such as between 12 - 15 nucleobases,

In such an embodiment, the oligonucleotide of the invention may have a length of 8, 9, 10, 11, 12, 13, 14, 15, 16, or 17 nucleobases.

It will be recognised that for shorter oligonucleotides it may be necessary to increase the proportion of (high affinity) nucleotide analogues, such as LNA. Therefore in one embodiment at least about 30% of the nucleobases are nucleotide analogues, such as at least about 33%, such as at least about 40%, or at least about 50% or at least about 60%, such as at least about 66%, such as at least about 70%, such as at least about 80%, or at least about 90%. It will also be apparent that the oligonucleotide may comprise of a nucleobase sequence which consists of only nucleotide analogue sequences.

Herein, the term "nitrogenous base" is intended to cover purines and pyrimidines, such as the DNA nucleobases A, C, T and G, the RNA nucleobases A, C, U and G, as well as non-DNA/RNA nucleobases, such as 5-methylcytosine (^{Me}C), isocytosine, pseudoisocytosine, 5-bromouracil, 5-propynyluracil, 5-propyny-6-fluoroluracil, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine, in particular ^{Me}C. It will be understood that the actual selection of the non-DNA/RNA nucleobase will depend on the corresponding (or matching) nucleotide present in the microRNA strand which the oligonucleotide is intended to target. For example, in case the corresponding nucleotide is G it will normally be necessary to select a non-DNA/RNA nucleobase which is capable of establishing hydrogen bonds to G. In this specific case, where the corresponding nucleotide is G, a typical example of a preferred non-DNA/RNA nucleobase is ^{Me}C.

The term "internucleoside linkage group" is intended to mean a group capable of covalently coupling together two nucleobases, such as between DNA units, between DNA units and nucleotide analogues, between two non-LNA units, between a non-LNA unit and an LNA unit, and between two LNA units, etc. Preferred examples include phosphate, phpshodiester groups and phosphorothioate groups.

The internucleoside linkage may be selected form the group consisting of: -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, -NR^{H}-CO-NR^{H}-, and/or the internucleoside linkage may be selected form the group consisting of: -O-CO-O-, -O-CO-NR^{H}-, -NR^{H}-CO-CH₂-, -O-CH₂-CO-NR^{H}-, -O-CH₂-CH₂-NR^{H}-, -CO-NR^{H}-CH₂-, -CH₂-NR^{H}-CO-, -O-CH₂-CH₂-S-, -S-CH₂-CH₂-O, -S-CH₂-CH₂-S-, -CH₂-SO₂-CH₂-, -CH₂-CO-NR^{H}-, -O-CH₂-CH₂-NR^{H}-CO-, -CH₂-NCH₃-O-CH₂-, where R^{H} is selected from hydrogen and C₁₋₄-alkyl. Suitably, in some embodiments, sulphur (S) containing internucleoside linkages as provided above may be preferred

The terms "corresponding to" and "corresponds to" as used in the context of oligonucleotides refers to the comparison between either a nucleobase sequence of the compound of the invention, and the reverse complement thereof, or in one embodiment between a nucleobase sequence and an equivalent (identical) nucleobase sequence which may for example comprise other nucleobases but retains the same base sequence, or complement thereof. Nucleotide analogues are compared directly to their equivalent or corresponding natural nucleotides. Sequences which form the reverse complement of a sequence are referred to as the complement sequence of the sequence.

When referring to the length of a nucleotide molecule as referred to herein, the length corresponds to the number of monomer units, i.e. nucleobases, irrespective as to whether those monomer units are nucleotides or nucleotide analogues. With respect to nucleobases, the terms monomer and unit are used interchangeably herein.

It should be uncerstood that when the term "about" is used in the context of specific values or ranges of values, the disclosure should be read as to include the specific value or range referred to.

Preferred DNA analogues includes DNA analogues where the 2'-H group is substituted with a substitution other than -OH (RNA) e.g. by substitution with -O-CH₃, -O-CH₂-CH₂-O-CH₃, - O-CH₂-CH₂-CH₂-NH₂, -O-CH₂-CH₂-CH₂-OH or -F.

Preferred RNA anlogues includes RNA anlogues which have been modified in its 2'-OH group, e.g. by substitution with a group other than -H (DNA), for example -O-CH₃, -O-CH₂-CH₂-O-CH₃, -O-CH₂-CH₂-CH₂-NH₂, -O-CH₂-CH₂-CH₂-OH or -F.

In one emdodiement the nucleotide analogue is "ENA".

When used in the present context, the terms "LNA unit", "LNA monomer", "LNA residue", "locked nucleic acid unit", "locked nucleic acid monomer" or "locked nucleic acid residue", refer to a bicyclic nucleoside analogue. LNA units are described in *inter alia* WO 99/14226, WO 00/56746, WO 00/56748, WO 01/25248, WO 02/28875, WO 03/006475 and WO 03/095467. The LNA unit may also be defined with respect to its chemical formula. Thus, an "LNA unit", as used herein, has the chemical structure shown in Scheme 1 below: 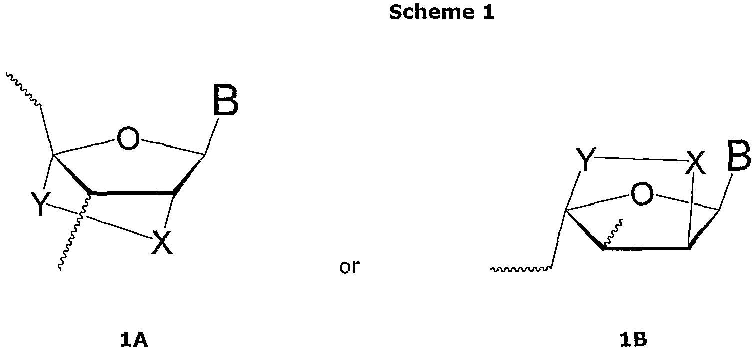 wherein
X is selected from the group consisting of O, S and NR^{H}, where R^{H} is H or C₁₋₄-alkyl;
Y is (-CH₂)ᵣ, where r is an integer of 1-4; and
B is a nitrogenous base.

When referring to substituting a DNA unit by its corresponding LNA unit in the context of the present invention, the term "corresponding LNA unit" is intended to mean that the DNA unit has been replaced by an LNA unit containing the same nitrogenous base as the DNA unit that it has replaced, e.g. the corresponding LNA unit of a DNA unit containing the nitrogenous base A also contains the nitrogenous base A. The exception is that when a DNA unit contains the base C, the corresponding LNA unit may contain the base C or the base ^{Me}C, preferably ^{Me}C.

Herein, the term "non-LNA unit" refers to a nucleoside different from an LNA-unit, i.e. the term "non-LNA unit" includes a DNA unit as well as an RNA unit. A preferred non-LNA unit is a DNA unit.

The terms "unit", "residue" and "monomer" are used interchangeably herein.

The term "at least one" encompasses an integer larger than or equal to 1, such as 1, 2, 3, 4, 5, 6, 7, 8, 9, 10, 11, 12, 13, 14, 15, 16, 17, 18, 19, 20 and so forth.

The terms "a" and "an" as used about a nucleotide, an agent, an LNA unit, etc., is intended to mean one or more. In particular, the expression "a component (such as a nucleotide, an agent, an LNA unit, or the like) selected from the group consisting of ..." is intended to mean that one or more of the cited components may be selected. Thus, expressions like "a component selected from the group consisting of A, B and C" is intended to include all combinations of A, B and C, i.e. A, B, C, A+B, A+C, B+C and A+B+C.

The term "thio-LNA unit" refers to an LNA unit in which X in Scheme 1 is S. A thio-LNA unit can be in both the beta-D form and in the alpha-L form. Generally, the beta-D form of the thio-LNA unit is preferred. The beta-D-form and alpha-L-form of a thio-LNA unit are shown in Scheme 3 as compounds **3A** and **3B,** respectively.

The term "amino-LNA unit" refers to an LNA unit in which X in Scheme 1 is NH or NR^{H}, where R^{H} is hydrogen or C₁₋₄-alkyl. An amino-LNA unit can be in both the beta-D form and in the alpha-L form. Generally, the beta-D form of the amino-LNA unit is preferred. The beta-D-form and alpha-L-form of an amino-LNA unit are shown in Scheme 4 as compounds **4A** and **4B,** respectively.

The term "oxy-LNA unit" refers to an LNA unit in which X in Scheme 1 is O. An Oxy-LNA unit can be in both the beta-D form and in the alpha-L form. Generally, the beta-D form of the oxy-LNA unit is preferred. The beta-D form and the alpha-L form of an oxy-LNA unit are shown in Scheme 5 as compounds **5A** and **5B,** respectively.

In the present context, the term "C₁₋₆-alkyl" is intended to mean a linear or branched saturated hydrocarbon chain wherein the longest chains has from one to six carbon atoms, such as methyl, ethyl, n-propyl, isopropyl, n-butyl, isobutyl, sec-butyl, tert-butyl, pentyl, isopentyl, neopentyl and hexyl. A branched hydrocarbon chain is intended to mean a C₁₋₆-alkyl substituted at any carbon with a hydrocarbon chain.

In the present context, the term "C₁₋₄-alkyl" is intended to mean a linear or branched saturated hydrocarbon chain wherein the longest chains has from one to four carbon atoms, such as methyl, ethyl, n-propyl, isopropyl, n-butyl, isobutyl, sec-butyl and tert-butyl. A branched hydrocarbon chain is intended to mean a C₁₋₄-alkyl substituted at any carbon with a hydrocarbon chain.

When used herein the term "C₁₋₆-alkoxy" is intended to mean C₁₋₆-alkyl-oxy, such as methoxy, ethoxy, n-propoxy, isopropoxy, n-butoxy, isobutoxy, sec-butoxy, tert-butoxy, pentoxy, isopentoxy, neopentoxy and hexoxy.

In the present context, the term "C₂₋₆-alkenyl" is intended to mean a linear or branched hydrocarbon group having from two to six carbon atoms and containing one or more double bonds. Illustrative examples of C₂₋₆-alkenyl groups include allyl, homo-allyl, vinyl, crotyl, butenyl, butadienyl, pentenyl, pentadienyl, hexenyl and hexadienyl. The position of the unsaturation (the double bond) may be at any position along the carbon chain.

In the present context the term "C₂₋₆-alkynyl" is intended to mean linear or branched hydrocarbon groups containing from two to six carbon atoms and containing one or more triple bonds. Illustrative examples of C₂₋₆-alkynyl groups include acetylene, propynyl, butynyl, pentynyl and hexynyl. The position of unsaturation (the triple bond) may be at any position along the carbon chain. More than one bond may be unsaturated such that the "C₂₋₆-alkynyl" is a di-yne or enedi-yne as is known to the person skilled in the art.

As used herein, "hybridisation" means hydrogen bonding, which may be Watson-Crick, Hoogsteen, reversed Hoogsteen hydrogen bonding, etc., between complementary nucleoside or nucleotide bases. The four nucleobases commonly found in DNA are G, A, T and C of which G pairs with C, and A pairs with T. In RNA T is replaced with uracil (U), which then pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA or RNA molecule, then the oligonucleotide and the DNA or RNA are considered to be complementary to each other at that position. The DNA or RNA strand are considered complementary to each other when a sufficient number of nucleotides in the oligonucleotide can form hydrogen bonds with corresponding nucleotides in the target DNA or RNA to enable the formation of a stable complex. To be stable *in vitro* or *in vivo* the sequence of an oligonucleotide need not be 100% complementary to its target microRNA. The terms "complementary" and "specifically hybridisable" thus imply that the oligonucleotide binds sufficiently strong and specific to the target molecule to provide the desired interference with the normal function of the target whilst leaving the function of non-target RNAs unaffected.

In a preferred example the oligonucleotide of the invention is 100% complementary to a human microRNA sequence, such as one of the microRNA sequences refered to herein.

The oligonucleotide of the invention comprises a contiguous sequence which is 100% complementary to the seed region of the human microRNA sequence.

MicroRNAs are short, non-coding RNAs derived from endogenous genes that act as post-transcriptional regulators of gene expression. They are processed from longer (ca 70-80 nt) hairpin-like precursors termed pre-miRNAs by the RNAse III enzyme Dicer. MicroRNAs assemble in ribonucleoprotein complexes termed miRNPs and recognize their target sites by antisense complementarity thereby mediating down-regulation of their target genes. Near-perfect or perfect complementarity between the miRNA and its target site results in target mRNA cleavage, whereas limited complementarity between the microRNA and the target site results in translational inhibition of the target gene.

The term "microRNA" or "miRNA", in the context of the present invention, means an RNA oligonucleotide consisting of between 18 to 25 nucleotides in length. In functional terms miRNAs are typically regulatory endogenous RNA molecules.

The terms "target microRNA" or "target miRNA" refer to a microRNA with a biological role in human disease, e.g. an upregulated, oncogenic miRNA or a tumor suppressor miRNA in cancer, thereby being a target for therapeutic intervention of the disease in question.

The terms "target gene" or "target mRNA" refer to regulatory mRNA targets of microRNAs, in which said "target gene" or "target mRNA" is regulated post-transcriptionally by the microRNA based on near-perfect or perfect complementarity between the miRNA and its target site resulting in target mRNA cleavage; or limited complementarity, often conferred to complementarity between the so-called seed sequence (nucleotides 2-7 of the miRNA) and the target site resulting in translational inhibition of the target mRNA.

In the context of the present invention the oligonucleotide is single stranded, this refers to the situation where the oligonucleotide is in the absence of a complementary oligonucleotide - i.e. it is not a double stranded oligonucleotide complex, such as an siRNA. In one embodiment, the composition according ot the invention does not comprise a further oligonucleotide which has a region of complementarity with the single stranded oligonucleotide of five or more consecutive nucleobases, such as eight or more, or 12 or more of more consecutive nucleobases. It is considered that the further oligonucleotide is not covalently linked to the single stranded oligonucleotide.

### Modification of nucleotides in positions 3 to 8, counting from the 3' end

In the following embodiments which refer to the modification of nucleotides in positions 3 to 8, counting from the 3' end, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit. "x" is preferably DNA or RNA, most preferably DNA.

In an interesting embodiment of the invention, the oligonucleotides of the invention are modified in positions 3 to 8, counting from the 3' end. The design of this sequence may be defined by the number of non-LNA units present or by the number of LNA units present. In a preferred embodiment of the former, at least one, such as one, of the nucleotides in positions three to eight, counting from the 3' end, is a non-LNA unit. In another embodiment, at least two, such as two, of the nucleotides in positions three to eight, counting from the 3' end, are non-LNA units. In yet another embodiment, at least three, such as three, of the nucleotides in positions three to eight, counting from the 3' end, are non-LNA units. In still another embodiment, at least four, such as four, of the nucleotides in positions three to eight, counting from the 3' end, are non-LNA units. In a further embodiment, at least five, such as five, of the nucleotides in positions three to eight, counting from the 3' end, are non-LNA units. In yet a further embodiment, all six nucleotides in positions three to eight, counting from the 3' end, are non-LNA units. In a preferred embodiment, said non-LNA unit is a DNA unit.

Alternatively defined, in a preferred embodiment, the oligonucleotide according to the invention comprises at least one LNA unit in positions three to eight, counting from the 3' end. In an embodiment thereof, the oligonucleotide according to the present invention comprises one LNA unit in positions three to eight, counting from the 3' end. The substitution pattern for the nucleotides in positions three to eight, counting from the 3' end, may be selected from the group consisting of Xxxxxx, xXxxxx, xxXxxx, xxxXxx, xxxxXx and xxxxxX, wherein "X" denotes an LNA unit and "x" denotes a non-LNA unit.

In another embodiment, the oligonucleotide according to the present invention comprises at least two LNA units in positions three to eight, counting from the 3' end. In an embodiment thereof, the oligonucleotide according to the present invention comprises two LNA units in positions three to eight, counting from the 3' end. The substitution pattern for the nucleotides in positions three to eight, counting from the 3' end, may be selected from the group consisting of XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX and xxxxXX, wherein "X" denotes an LNA unit and "x" denotes a non-LNA unit. In a preferred embodiment, the substitution pattern for the nucleotides in positions three to eight, counting from the 3' end, is selected from the group consisting of XxXxxx, XxxXxx, XxxxXx, XxxxxX, xXxXxx, xXxxXx, xXxxxX, xxXxXx, xxXxxX and xxxXxX, wherein "X" denotes an LNA unit and "x" denotes a non-LNA unit. In a more preferred embodiment, the substitution pattern for the nucleotides in positions three to eight, counting from the 3' end, is selected from the group consisting of xXxXxx, xXxxXx, xXxxxX, xxXxXx, xxXxxX and xxxXxX, wherein "X" denotes an LNA unit and "x" denotes a non-LNA unit. In an even more preferred embodiment, the substitution pattern for the nucleotides in positions three to eight, counting from the 3' end, is selected from the group consisting of xXxXxx, xXxxXx and xxXxXx, wherein "X" denotes an LNA unit and "x" denotes a non-LNA unit. In a most preferred embodiment, the substitution pattern for the nucleotides in positions three to eight, counting from the 3' end, is xXxXxx, wherein "X" denotes an LNA unit and "x" denotes a non-LNA unit.

In yet another embodiment, the oligonucleotide according to the present invention comprises at least three LNA units in positions three to eight, counting from the 3' end. In an embodiment thereof, the oligonucleotide according to the present invention comprises three LNA units in positions three to eight, counting from the 3' end. The substitution pattern for the nucleotides in positions three to eight, counting from the 3' end, may be selected from the group consisting of XXXxxx, xXXXxx, xxXXXx, xxxXXX, XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit. In a preferred embodiment, the substitution pattern for the nucleotides in positions three to eight, counting from the 3' end, is selected from the group consisting of XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit. In a more preferred embodiment, the substitution pattern for the nucleotides in positions three to eight, counting from the 3' end, is selected from the group consisting of xXXxXx, xXXxxX, xxXXxX, xXxXXx, xXxxXX, xxXxXX and xXxXxX, wherein "X" denotes an LNA unit and "x" denotes a non-LNA unit. In an even more preferred embodiment, the substitution pattern for the nucleotides in positions three to eight, counting from the 3' end, is xXxXxX or XxXxXx, wherein "X" denotes an LNA unit and "x" denotes a non-LNA unit. In a most preferred embodiment, the substitution pattern for the nucleotides in positions three to eight, counting from the 3' end, is xXxXxX, wherein "X" denotes an LNA unit and "x" denotes a non-LNA unit.

In a further embodiment, the oligonucleotide according to the present invention comprises at least four LNA units in positions three to eight, counting from the 3' end. In an embodiment thereof, the oligonucleotide according to the present invention comprises four LNA units in positions three to eight, counting from the 3' end. The substitution pattern for the nucleotides in positions three to eight, counting from the 3' end, may be selected from the group consisting of xxXXXX, xXxXXX, xXXxXX, xXXXxX, xXXXXx, XxxXXX, XxXxXX, XxXXxX, XxXXXx, XXxxXX, XXxXxX, XXxXXx, XXXxxX, XXXxXx and XXXXxx, wherein "X" denotes an LNA unit and "x" denotes a non-LNA unit.

In yet a further embodiment, the oligonucleotide according to the present invention comprises at least five LNA units in positions three to eight, counting from the 3' end. In an embodiment thereof, the oligonucleotide according to the present invention comprises five LNA units in positions three to eight, counting from the 3' end. The substitution pattern for the nucleotides in positions three to eight, counting from the 3' end, may be selected from the group consisting of xXXXXX, XxXXXX, XXxXXX, XXXxXX, XXXXxX and XXXXXx, wherein "X" denotes an LNA unit and "x" denotes a non-LNA unit.

Preferably, the oligonucleotide according to the present invention comprises one or two LNA units in positions three to eight, counting from the 3' end. This is considered advantageous for the stability of the A-helix formed by the oligo:microRNA duplex, a duplex resembling an RNA:RNA duplex in structure.

In a preferred embodiment, said non-LNA unit is a DNA unit.

### Variation of the length of the oligonucleotides

The length of the oligonucleotides of the invention need not match the length of the target microRNAs exactly. Accordingly, the length of the oligonucleotides of the invention may vary. Indeed it is considered advantageous to have short oligonucleotides, such as between 10 - 17 or 10 - 16 nucleobases.

The oligonucleotide according to the present invention has a length of from 8 to 17 nucleotides, such as a length of from 10 - 17, such as from 12 to 17 nucleotides, such as a length of 10, 11, 12, 13, 14, 15, 16 or 17 nucleotides, most preferably a length of from 10 to 16, such as between 12 to 16 nucleotides, such as a length of 10, 11, 12, 13, 14, 15 or 16 nucleotides.

### Modification of nucleotides from position 11, counting from the 3' end, to the 5' end

The substitution pattern for the nucleotides from position 11, counting from the 3' end, to the 5' end may include nucleotide analogue units (such as LNA) or it may not. In a preferred embodiment, the oligonucleotide according to the present invention comprises at least one nucleotide analogue unit (such as LNA), such as one nucleotide analogue unit, from position 11, counting from the 3' end, to the 5' end. In another preferred embodiment, the oligonucleotide according to the present invention comprises at least two nucleotide analogue units, such as LNA units, such as two nucleotide analogue units, from position 11, counting from the 3' end, to the 5' end.

In the following embodiments which refer to the modification of nucleotides in the nucleobases from psotion 11 to the 5' end of the oligonucleotide, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit. "x" is preferably DNA or RNA, most preferably DNA.

In one embodiment, the oligonucleotide according to the present invention has the following substitution pattern, which is repeated from nucleotide eleven, counting from the 3' end, to the 5' end: xXxX or XxXx, wherein "X" denotes an LNA unit and "x" denotes a non-LNA unit. In another embodiment, the oligonucleotide according to the present invention has the following substitution pattern, which is repeated from nucleotide eleven, counting from the 3' end, to the 5' end: XxxXxx, xXxxXx or xxXxxX, wherein "X" denotes an LNA unit and "x" denotes a non-LNA unit. In yet another embodiment, the oligonucleotide according to the present invention has the following substitution pattern, which is repeated from nucleotide eleven, counting from the 3' end, to the 5' end: XxxxXxxx, xXxxxXxx, xxXxxxXx or xxxXxxxX, wherein "X" denotes an LNA unit and "x" denotes a non-LNA unit.

The specific substitution pattern for the nucleotides from position 11, counting from the 3' end, to the 5' end depends on the number of nucleotides in the oligonucleotides according to the present invention. In a preferred embodiment, the oligonucleotide according to the present invention contains 12 nucleotides and the substitution pattern for positions 11 to 12, counting from the 3' end, is selected from the group consisting of xX and Xx, wherein "X" denotes an LNA unit and "x" denotes a non-LNA unit. In a more preferred embodiment thereof, the substitution pattern for positions 11 to 12, counting from the 3' end, is xX, wherein "X" denotes an LNA unit and "x" denotes a non-LNA unit. Alternatively, no LNA units are present in positions 11 to 12, counting from the 3' end, i.e. the substitution pattern is xx.

In another preferred embodiment, the oligonucleotide according to the present invention contains 13 nucleotides and the substitution pattern for positions 11 to 13, counting from the 3' end, is selected from the group consisting of Xxx, xXx, xxX, XXx, XxX, xXX and XXX, wherein "X" denotes an LNA unit and "x" denotes a non-LNA unit. In a more preferred embodiment thereof, the substitution pattern for positions 11 to 13, counting from the 3' end, is selected from the group consisting of xXx, xxX and xXX, wherein "X" denotes an LNA unit and "x" denotes a non-LNA unit. In a most preferred embodiment thereof, the substitution pattern for positions 11 to 13, counting from the 3' end, is xxX, wherein "X" denotes an LNA unit and "x" denotes a non-LNA unit. Alternatively, no LNA units are present in positions 11 to 13, counting from the 3' end, i.e. the substitution pattern is xxx.

In yet another preferred embodiment, the oligonucleotide according to the present invention contains 14 nucleotides and the substitution pattern for positions 11 to 14, counting from the 3' end, is selected from the group consisting of Xxxx, xXxx, xxXx, xxxX, XXxx, XxXx, XxxX, xXXx, xXxX and xxXX, wherein "X" denotes an LNA unit and "x" denotes a non-LNA unit. In a preferred embodiment thereof, the substitution pattern for positions 11 to 14, counting from the 3' end, is selected from the group consisting of xXxx, xxXx, xxxX, xXxX and xxXX, wherein "X" denotes an LNA unit and "x" denotes a non-LNA unit. In a more preferred embodiment thereof, the substitution pattern for positions 11 to 14, counting from the 3' end, is xXxX, wherein "X" denotes an LNA unit and "x" denotes a non-LNA unit. Alternatively, no LNA units are present in positions 11 to 14, counting from the 3' end, i.e. the substitution pattern is xxxx

In a further preferred embodiment, the oligonucleotide according to the present invention contains 15 nucleotides and the substitution pattern for positions 11 to 15, counting from the 3' end, is selected from the group consisting of Xxxxx, xXxxx, xxXxx, xxxXx, xxxxX, XXxxx, XxXxx, XxxXx, XxxxX, xXXxx, xXxXx, xXxxX, xxXXx, xxXxX, xxxXX and XxXxX, wherein "X" denotes an LNA unit and "x" denotes a non-LNA unit. In a preferred embodiment thereof, the substitution pattern for positions 11 to 15, counting from the 3' end, is selected from the group consisting of xxXxx, XxXxx, XxxXx, xXxXx, xXxxX and xxXxX, wherein "X" denotes an LNA unit and "x" denotes a non-LNA unit. In a more preferred embodiment thereof, the substitution pattern for positions 11 to 15, counting from the 3' end, is selected from the group consisting of xxXxx, xXxXx, xXxxX and xxXxX, wherein "X" denotes an LNA unit and "x" denotes a non-LNA unit. In an even more preferred embodiment thereof, the substitution pattern for positions 11 to 15, counting from the 3' end, is selected from the group consisting of xXxxX and xxXxX, wherein "X" denotes an LNA unit and "x" denotes a non-LNA unit. In a most preferred embodiment, the substitution pattern for positions 11 to 15, counting from the 3' end, is xxXxX, wherein "X" denotes an LNA unit and "x" denotes a non-LNA unit. Alternatively, no LNA units are present in positions 11 to 15, counting from the 3' end, i.e. the substitution pattern is xxxxx

In yet a further preferred embodiment, the oligonucleotide according to the present invention contains 16 nucleotides and the substitution pattern for positions 11 to 16, counting from the 3' end, is selected from the group consisting of Xxxxxx, xXxxxx, xxXxxx, xxxXxx, xxxxXx, xxxxxX, XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX, xxxxXX, XXXxxx, XXxXxx, XXxxXx, XXxxxX, XxXXxx, XxXxXx, XxXxxX, XxxXXx, XxxXxX, XxxxXX, xXXXxx, xXXxXx, xXXxxX, xXxXXx, xXxXxX, xXxxXX, xxXXXx, xxXXxX, xxXxXX and xxxXXX, wherein "X" denotes an LNA unit and "x" denotes a non-LNA unit. In a preferred embodiment thereof, the substitution pattern for positions 11 to 16, counting from the 3' end, is selected from the group consisting of XxxXxx, xXxXxx, xXxxXx, xxXxXx, xxXxxX, XxXxXx, XxXxxX, XxxXxX, xXxXxX, xXxxXX and xxXxXX, wherein "X" denotes an LNA unit and "x" denotes a non-LNA unit. In a more preferred embodiment thereof, the substitution pattern for positions 11 to 16, counting from the 3' end, is selected from the group consisting of xXxXxx, xXxxXx, xxXxXx, xxXxxX, xXxXxX, xXxxXX and xxXxXX, wherein "X" denotes an LNA unit and "x" denotes a non-LNA unit. In an even more preferred embodiment thereof, the substitution pattern for positions 11 to 16, counting from the 3' end, is selected from the group consisting of xxXxxX, xXxXxX, xXxxXX and xxXxXX, wherein "X" denotes an LNA unit and "x" denotes a non-LNA unit. In a still more preferred embodiment thereof, the substitution pattern for positions 11 to 16, counting from the 3' end, is selected from the group consisting of xxXxxX and xXxXxX, wherein "X" denotes an LNA unit and "x" denotes a non-LNA unit. In a most preferred embodiment thereof, the substitution pattern for positions 11 to 16, counting from the 3' end, is xxXxxX, wherein "X" denotes an LNA unit and "x" denotes a non-LNA unit. Alternatively, no LNA units are present in positions 11 to 16, counting from the 3' end, i.e. the substitution pattern is xxxxxx

In a preferred embodiment of the invention, the oligonucleotide according to the present invention contains an LNA unit at the 5' end. In another preferred embodiment, the oligonucleotide according to the present invention contains an LNA unit at the first two positions, counting from the 5' end.

In a particularly preferred embodiment, the oligonucleotide according to the present invention contains 13 nucleotides and the substitution pattern, starting from the 3' end, is XXxXxXxxXXxxX, wherein "X" denotes an LNA unit and "x" denotes a non-LNA unit. The preferred sequence for this embodiment, starting from the 3' end, is CCtCaCacTGttA, wherein a capital letter denotes a nitrogenous base in an LNA-unit and a small letter denotes a nitrogenous base in a non-LNA unit.

In another particularly preferred embodiment, the oligonucleotide according to the present invention contains 15 nucleotides and the substitution pattern, starting from the 3' end, is XXxXxXxxXXxxXxX, wherein "X" denotes an LNA unit and "x" denotes a non-LNA unit. The preferred sequence for this embodiment, starting from the 3' end, is CCtCaCacTGttAcC, wherein a capital letter denotes a nitrogenous base in an LNA-unit and a small letter denotes a nitrogenous base in a non-LNA unit.

### Modification of the internucleoside linkage group

The oligonucleotide of the invention is modified in its internucleoside linkage group structure, i.e. the modified oligonucleotide comprises an internucleoside linkage group which differs from phosphate
Specific examples of internucleoside linkage groups which differ from phosphate (-O-P(O)₂-O-) include -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, - O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, -NR^{H}-CO-NR^{H}-, -O-CO-O-, -O-CO-NR^{H}-, -NR^{H}-CO-CH₂-, -O-CH₂-CO-NR^{H}-, - O-CH₂-CH₂-NR^{H}-, -CO-NR^{H}-CH₂-, -CH₂-NR^{H}-CO-, -O-CH₂-CH₂-S-, -S-CH₂-CH₂-O-, -S-CH₂-CH₂-S-, -CH₂-SO₂-CH₂-, -CH₂-CO-NR^{H}-, -O-CH₂-CH₂-NR^{H}-CO-, -CH₂-NCH₃-O-CH₂-, where R^{H} is hydrogen or C₁₋₄-alkyl.

The internucleoside linkage group of the single stranded oligonucleotides of the invention are phosphorothioate groups (-O-P(O,S)-O-). All internucleoside linkage groups of the oligonucleotides according to the present invention are phosphorothioate.

### The LNA unit

In a preferred embodiment, the LNA unit has the general chemical structure shown in Scheme 1 below: 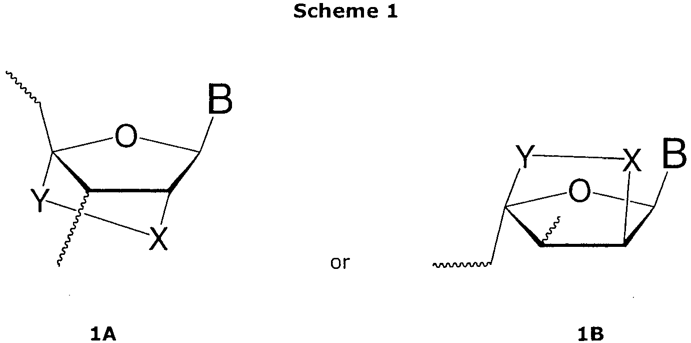 wherein
X is selected from the group consisting of O, S and NR^{H}, where R^{H} is H or C₁₋₄-alkyl;
Y is (-CH₂)ᵣ, where r is an integer of 1-4; and
B is a nitrogenous base.

In a preferred embodiment of the invention, r is 1 or 2, in particular 1, i.e. a preferred LNA unit has the chemical structure shown in Scheme 2 below: 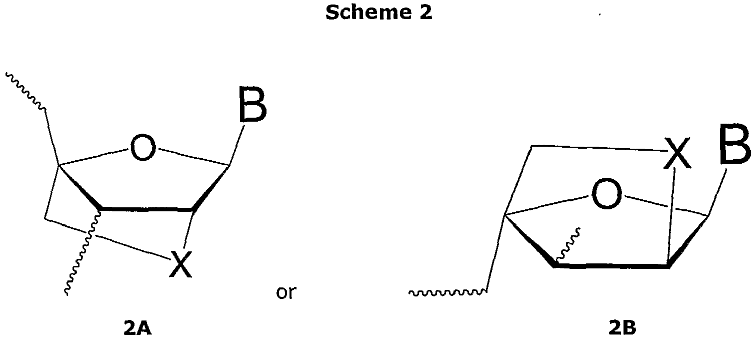 wherein X and B are as defined above.

In an interesting embodiment, the LNA units incorporated in the oligonucleotides of the invention are independently selected from the group consisting of thio-LNA units, amino-LNA units and oxy-LNA units.

Thus, the thio-LNA unit may have the chemical structure shown in Scheme 3 below: 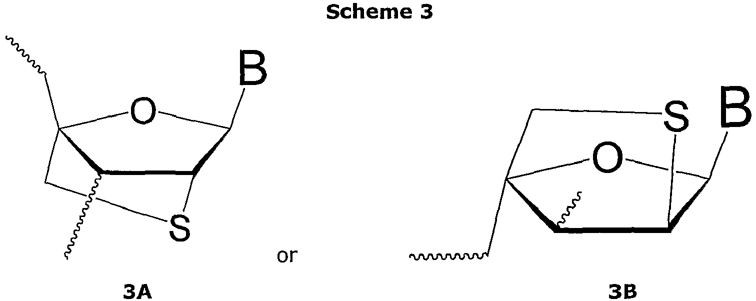 wherein B is as defined above.
Preferably, the thio-LNA unit is in its beta-D-form, i.e. having the structure shown in **3A** above.
likewise, the amino-LNA unit may have the chemical structure shown in Scheme 4 below: 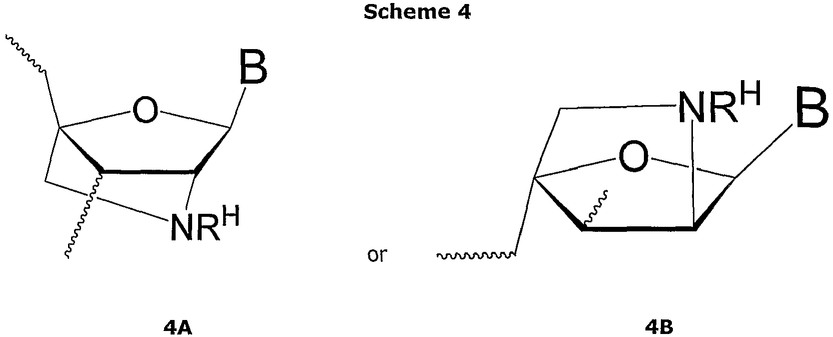 wherein B and R^{H} are as defined above.

Preferably, the amino-LNA unit is in its beta-D-form, i.e. having the structure shown in **4A** above.
The oxy-LNA unit may have the chemical structure shown in Scheme 5 below: 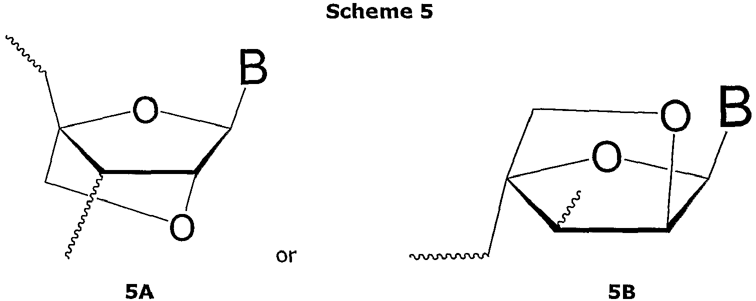 wherein B is as defined above.

Preferably, the oxy-LNA unit is in its beta-D-form, i.e. having the structure shown in **5A** above.

As indicated above, B is a nitrogenous base which may be of natural or non-natural origin. Specific examples of nitrogenous bases include adenine (A), cytosine (C), 5-methylcytosine (^{Me}C), isocytosine, pseudoisocytosine, guanine (G), thymine (T), uracil (U), 5-bromouracil, 5-propynyluracil, 5-propyny-6, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine.

### Terminal groups

Specific examples of terminal groups include terminal groups selected from the group consisting of hydrogen, azido, halogen, cyano, nitro, hydroxy, Prot-O-, mercapto, Prot-S-, C₁₋₆-alkylthio, amino, Prot-N(R^{H})-, mono- or di(C₁₋₆-alkyl)amino, optionally substituted C₁₋₆-alkoxy, optionally substituted C₁₋₆-alkyl, optionally substituted C₂₋₆-alkenyl, optionally substituted C₂₋₆-alkenyloxy, optionally substituted C₂₋₆-alkynyl, optionally substituted C₂₋₆-alkynyloxy, monophosphate including protected monophosphate, monothiophosphate including protected monothiophosphate, diphosphate including protected diphosphate, dithiophosphate including protected dithiophosphate, triphosphate including protected triphosphate, trithiophosphate including protected trithiophosphate, where Prot is a protection group for -OH, -SH and -NH(R^{H}), and R^{H} is hydrogen or C₁₋₆-alkyl.

Examples of phosphate protection groups include S-acetylthioethyl (SATE) and S-pivaloylthioethyl (t-butyl-SATE).

Still further examples of terminal groups include DNA intercalators, photochemically active groups, thermochemically active groups, chelating groups, reporter groups, ligands, carboxy, sulphono, hydroxymethyl, Prot-O-CH₂-, Act-O-CH₂-, aminomethyl, Prot-N(R^{H})-CH₂-, Act-N(R^{H})-CH₂-, carboxymethyl, sulphonomethyl, where Prot is a protection group for -OH, -SH and -NH(R^{H}), and Act is an activation group for -OH, -SH, and -NH(R^{H}), and R^{H} is hydrogen or C₁₋₆-alkyl.

Examples of protection groups for -OH and -SH groups include substituted trityl, such as 4,4'-dimethoxytrityloxy (DMT), 4-monomethoxytrityloxy (MMT); trityloxy, optionally substituted 9-(9-phenyl)xanthenyloxy (pixyl), optionally substituted methoxytetrahydro-pyranyloxy (mthp); silyloxy, such as trimethylsilyloxy (TMS), triisopropylsilyloxy (TIPS), tert-butyldimethylsilyloxy (TBDMS), triethylsilyloxy, phenyldimethylsilyloxy; *tert-*butylethers; acetals (including two hydroxy groups); acyloxy, such as acetyl or halogen-substituted acetyls, *e.g.* chloroacetyloxy or fluoroacetyloxy, isobutyryloxy, pivaloyloxy, benzoyloxy and substituted benzoyls, methoxymethyloxy (MOM), benzyl ethers or substituted benzyl ethers such as 2,6-dichlorobenzyloxy (2,6-Cl₂Bzl). Moreover, when Z or Z* is hydroxyl they may be protected by attachment to a solid support, optionally through a linker.

Examples of amine protection groups include fluorenylmethoxycarbonylamino (Fmoc), *tert-*butyloxycarbonylamino (BOC), trifluoroacetylamino, allyloxycarbonylamino (alloc, AOC), Z-benzyloxycarbonylamino (Cbz), substituted benzyloxycarbonylamino, such as 2-chloro benzyloxycarbonylamino (2-CIZ), monomethoxytritylamino (MMT), dimethoxytritylamino (DMT), phthaloylamino, and 9-(9-phenyl)xanthenylamino (pixyl).

In the present context, the term "phosphoramidite" means a group of the formula -P(OR^{x})-N(R^{y})₂, wherein R^{x} designates an optionally substituted alkyl group, e.g. methyl, 2-cyanoethyl, or benzyl, and each of R^{y} designates optionally substituted alkyl groups, *e.g.* ethyl or isopropyl, or the group -N(R^{y})₂ forms a morpholino group (-N(CH₂CH₂)₂O). R^{x} preferably designates 2-cyanoethyl and the two R^{y} are preferably identical and designates isopropyl. Accordingly, a particularly preferred phosphoramidite is N,N-diisopropyl-*O*-(2-cyanoethyl)phosphoramidite.

The most preferred terminal groups are hydroxy, mercapto and amino, in particular hydroxy.

### Designs for specific microRNAs

The following table provides examples of oligonucleotide according to the present invention, such as those used in pharmaceutical compositions, as compared to prior art type of molecules.

| **target: hsa-miR-122a MIMAT0000421** | | **SEQ ID** |
|---|---|---|
| **uggagugugacaaugguguuugu** | | **SEQ ID NO 535** |
| screened in HUH-7 cell line expressing miR-122 | | |

| **Oligo #, target microRNA, oligo sequence** | **Design** | |
|---|---|---|
| 3962: miR-1225'-ACAAacaccattgtcacacTCCA-3' | Full complement, gap | SEQ ID NO 536 |
| 3965: miR-122 5'-acaaacACCATTGTcacactcca-3' | Full complement, block | SEQ ID NO 537 |
| 3972: miR-1225'-acAaaCacCatTgtCacActCca-3' | Full complement, LNA_3 | SEQ ID NO 538 |
| 3549 (3649):miR-122 5'-CcAttGTcaCaCtCC-3' | New design | SEQ ID NO 539 |
| 3975: miR-122 5'-CcAtTGTcaCACtCC-3' | Enhanced new design | SEQ ID NO 540 |
| 3975': miR-122 5'-ATTGTcACACtCC-3' | ED - 13mer | SEQ ID NO 541 |
| 3975": miR-122 5'-TGTcACACtCC-3' | ED - 11mer | SEQ ID NO 542 |
| 3549' (3649):miR-122 5' CC^{M}AT^{M}T^{M}GTC^{M}A^{M}CA^{M}CT^{M}CC-3' | New design - 2'MOE | SEQ ID NO 543 |
| 3549" (3649):miR-122 5' CC^{F}AT^{F}T^{F}GTC^{F}A^{F}CA^{F}CT^{F}CC-3' | New design - 2'Fluoro | SEQ ID NO 544 |
| | | |

| **target: hsa-miR-19b MIMAT0000074** | | |
|---|---|---|
| **ugugcaaauccaugcaaaacuga** | | **SEQ ID NO 545** |
| screened HeLa cell line expressing miR-19b | | |

| **Oligo #, target microRNA, oligo sequence** | **Design** | |
|---|---|---|
| 3963: miR-19b 5'-TCAGttttgcatggatttgCACA-3' | Full complement, gap | SEQ ID NO 546 |
| 3967: miR-19b 5'-tcagttTTGCATGGatttgcaca-3' | Full complement, block | SEQ ID NO 547 |
| 3973: miR-19b 5'-tcAgtTttGcaTggAttTgcAca-3' | Full complement, LNA_3 | SEQ ID NO 548 |
| 3560: miR-19b 5'-TgCatGGatTtGcAC-3' | New design | SEQ ID NO 549 |
| 3976: miR-19b 5'-TgCaTGGatTTGcAC-3' | Enhanced new design | SEQ ID NO 550 |
| 3976': miR-19b 5'-CaTGGaTTTGcAC-3' | ED - 13mer | SEQ ID NO 551 |
| 3976": miR-19b 5'-TGGaTTTGcAC-3' | ED - 11mer | SEQ ID NO 552 |
| 3560': miR-19b 5'TG^{M}CA^{M}T^{M}GGA^{M}T^{M}TT^{M}GC^{M}AC-3' | New design - 2'MOE | SEQ ID NO 553 |
| 3560": miR-19b 5'-TG^{F}CA^{F}T^{F}GGA^{F}T^{F}TT^{F}GC^{F}AC-3' | New design - 2'MOE | SEQ ID NO 554 |
| | | |

| **target: hsa-miR-155 MIMAT0000646** | | |
|---|---|---|
| **uuaaugcuaaucgugauagggg** | | **SEQ ID NO 555** |
| screen in 518A2 cell line expressing miR-155 | | |

| **Oligo #, target microRNA, oligo sequence** | **Design** | |
|---|---|---|
| 3964: miR-155 5'-CCCCtatcacgattagcaTTAA-3' | Full complement, gap | SEQ ID NO 556 |
| 3968: miR-155 5'-cccctaTCACGATTagcattaa-3' | Full complement, block | SEQ ID NO 557 |
| 3974: miR-1555'-cCccTatCacGatTagCatTaa-3' | Full complement, LNA_3 | SEQ ID NO 558 |
| 3758: miR-155 5'-TcAcgATtaGcAtTA-3' | New design | SEQ ID NO 559 |
| 3818: miR-155 5'-TcAcGATtaGCAtTA-3' | Enhanced new design | SEQ ID NO 560 |
| 3818': miR-155 5'-ACGATtAGCAtTA-3' | ED - 13mer | SEQ ID NO 561 |
| 3818": miR-155 5'-GATtAGCaTTA-3' | ED - 11mer | SEQ ID NO 562 |
| 3758': miR-155 5'-TC^{M}AC^{M}G^{M}ATTA^{M}GC^{M}AT^{M}TA-3' | New design - 2'MOE | SEQ ID NO 563 |
| 3758": miR-155 5'-TC^{F}AC^{F}G^{F}ATT^{F}A^{F}GC^{F}AT^{F}TA-3' | New design - 2'Fluoro | SEQ ID NO 564 |
| | | |

| **target: hsa-miR-21** MIMAT0000076 | | |
|---|---|---|
| **uagcuuaucagacugauguuga** | | **SEQ ID NO 565** |
| miR-21 5'-TCAAcatcagtctgataaGCTA -3' | Full complement, gap | SEQ ID NO 566 |
| miR-21 5'-tcaacaTCAGTCTGataagcta -3' | Full complement, block | SEQ ID NO 567 |
| miR-21 5'- tcAtcAtcAgtCtgAtaAGcTta -3' | Full complement, LNA_3 | SEQ ID NO 568 |
| miR-21 5' - TcAgtCTgaTaAgCT -3' | New design | SEQ ID NO 569 |
| miR-21 5'- TcAgTCTgaTAAgCT -3'- | Enhanced new design | SEQ ID NO 570 |
| miR-21 5'- AGTCTgATAAgCT -3'- | ED - 13mer | SEQ ID NO 571 |
| miR-21 5'- TCTgAtAAGCT -3'- | ED - 11mer | SEQ ID NO 572 |
| miR-21 5'- TC^{M}AG^{M}T^{M}CTG^{M}A^{M}TA^{M}AG^{M}CT - 3' | New design - 2'MOE | SEQ ID NO 573 |
| miR-21 5'-TC^{F}AG^{F}T^{F}CTG^{F}A^{F}TA^{F}AG^{F}CT-3' | New design - 2'Fluoro | SEQ ID NO 574 |
| | | |

| **target: hsa-miR-375 MIMAT0000728** | | |
|---|---|---|
| **uuuguucguucggcucgcguga** | | **SEQ ID NO 575** |
| miR- 375 5'- TCTCgcgtgccgttcgttCTTT -3' | Full complement, gap | SEQ ID NO 576 |
| miR- 375 5'- tctcgcGTGCCGTTcgttcttt -3' | Full complement, block | SEQ ID NO 577 |
| miR- 375 5'- tcTcgCgtGccGttCgtTctTt -3' | Full complement, LNA_3 | SEQ ID NO 578 |
| miR- 375 5'- GtGccGTtcGtTcTT 3' | New design | SEQ ID NO 579 |
| miR- 375 5'- GtGcC6TtcGTTcTT 3' | Enhanced new design | SEQ ID NO 580 |
| miR- 375 5'- GCCGTtCgTTCTT 3' | ED - 13mer | SEQ ID NO 581 |
| miR- 375 5'- CGTTcGTTCTT 3' | ED - 11mer | SEQ ID NO 582 |
| miR- 375 5'- GT^{M}GC^{M}C^{M}GTT^{M}C^{M}GT^{M}TC^{M}TT 3' | New design - 2'MOE | SEQ ID NO 583 |
| miR- 375 5'-GT^{F}GC^{F}C^{F}GTT^{F}C^{F}GT^{F}TC^{F}TT 3' | New design - 2'Fluoro | SEQ ID NO 584 |

Captal Letters without a superscript M or F, refer to LNA units. Lower case = DNA, except for lower case in bold = RNA. The LNA cytosines may optionally be methylated). Capital letters followed by a superscript M refer to 2'OME RNA units, Capital letters followed by a superscript F refer to 2'fluoro DNA units, lowercase letter refer to DNA. The above oligos may in one embodiment be entirely phosphorothioate, but other nucleobase linkages as herein described bay be used. In one embodiment the nucleobase linkages are all phosphodiester. It is considered that for use within the brain/spinal cord it is preferable to use phosphodiester linkages, for example for the use of antimiRs targeting miR21.

Table 2 below provides non-limiting examples of oligonucleotide designs against known human microRNA sequences in miRBase microRNA database version 8.1.

The oligonucleotides according to the invention, such as those disclosed in table 2 may, in one embodiment, have a sequence of nucleobases 5' - 3' selected form the group consisting of:
LdLddLLddLdLdLL (New design)
LdLdLLLddLLLdLL (Enhanced new design)
LMLMMLLMMLMLMLL (New design - 2'MOE)
LMLMLLLMMLLLMLL (Enhanced new design- 2'MOE)
LFLFFLLFFLFLFLL (New design - 2' Fluoro)
LFLFLLLFFLLLFLL (Enhanced new design- 2' Fluoro)
LddLddLddL(d)(d)(L)(d)(d)(L)(d) 'Every third'
dLddLddLdd(L)(d)(d)(L)(d)(d)(L) 'Every third'
ddLddLddLd(d)(L)(d)(d)(L)(d)(d) 'Every third'
LMMLMMLMML(M)(M)(L)(M)(M)(L)(M) 'Every third'
MLMMLMMLMM(L)(M)(M)(L)(M)(M)(L) 'Every third'
MMLMMLMMLM(M)(L)(M)(M)(L)(M)(M) 'Every third'
LFFLFFLFFL(F)(F)(L)(F)(F)(L)(F) 'Every third'
FLFFLFFLFF(L)(F)(F)(L)(F)(F)(L) 'Every third'
FFLFFLFFLF(F)(L)(F)(F)(L)(F)(F) 'Every third'
dLdLdLdLdL(d)(L)(d)(L)(d)(L)(d) 'Every second'
LdLdLdLdL(d)(L)(d)(L)(d)(L)(d)(L) 'Every second'
MLMLMLMLML(M)(L)(M)(L)(M)(L)(M) 'Every second'
LMLMLMLML(M)(L)(M)(L)(M)(L)(M)(L) 'Every second'
FLFLFLFLFL(F)(L)(F)(L)(F)(L)(F) 'Every second'
LFLFLFLFL(F)(L)(F)(L)(F)(L)(F)(L) 'Every second'
Wherein L = LNA unit, d= DNA units, M = 2'MOE RNA, F = 2'Fluoro and residues in brackets are optional

### Conjugates

The invention also provides for conjugates comprising the oligonucleotide according ot the invention.

In one embodiment of the invention the oligomeric compound is linked to ligands/conjugates, which may be used, e.g. to increase the cellular uptake of antisense oligonucleotides. This conjugation can take place at the terminal positions 5'/3'-OH but the ligands may also take place at the sugars and/or the bases. In particular, the growth factor to which the antisense oligonucleotide may be conjugated, may comprise transferrin or folate. Transferrin-polylysine-oligonucleotide complexes or folate-polylysine-oligonucleotide complexes may be prepared for uptake by cells expressing high levels of transferrin or folate receptor. Other examples of conjugates/ligands are cholesterol moieties, duplex intercalators such as acridine, poly-L-lysine, "end-capping" with one or more nuclease-resistant linkage groups such as phosphoromonothioate, and the like. The invention also provides for a conjugate comprising the compound according to the invention as herein described, and at least one non-nucleotide or non-polynucleotide moiety covalently attached to said compound. Therefore, in one embodiment where the compound of the invention consists of s specified nucleic acid, as herein disclosed, the compound may also comprise at least one non-nucleotide or non-polynucleotide moiety (e.g. not comprising one or more nucleotides or nucleotide analogues) covalently attached to said compound. The non-nucleobase moiety may for instance be or comprise a sterol such as cholesterol.

Therefore, it will be recognised that the oligonucleotide of the invention, such as the oligonucleotide used in pharmaceutical (therapeutic) formulations may comprise further non-nucleobase components, such as the conjugates herein defined.

### Therapy and pharmaceutical compositions

As explained initially, the oligonucleotides of the invention will constitute suitable drugs with improved properties. The design of a potent and safe drug requires the fine-tuning of various parameters such as affinity/specificity, stability in biological fluids, cellular uptake, mode of action, pharmacokinetic properties and toxicity.

Accordingly, in a further aspect the present invention relates to a pharmaceutical composition comprising an oligonucleotide according to the invention and a pharmaceutically acceptable diluent, carrier or adjuvant. Preferably said carrier is saline of buffered saline.

In a still further aspect the present invention relates to an oligonucleotide according to the present invention for use as a medicament.

As will be understood, dosing is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual oligonucleotides. Generally it can be estimated based on EC50s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

### Pharmaceutical compositions

As indicated above, the invention also relates to a pharmaceutical composition, which comprises at least one oligonucleotide of the invention as an active ingredient. It should be understood that the pharmaceutical composition according to the invention optionally comprises a pharmaceutical carrier, and that the pharmaceutical composition optionally comprises further compounds, such as chemotherapeutic compounds, anti-inflammatory compounds, antiviral compounds and/or immuno-modulating compounds.

The oligonucleotides of the invention can be used "as is" or in form of a variety of pharmaceutically acceptable salts. As used herein, the term "pharmaceutically acceptable salts" refers to salts that retain the desired biological activity of the herein-identified oligonucleotides and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, *N*,*N*-dibenzylethylene-diamine, *D*-glucosamine, tetraethylammonium, or ethylenediamine.

In one embodiment of the invention, the oligonucleotide may be in the form of a pro-drug. Oligonucleotides are by virtue negatively charged ions. Due to the lipophilic nature of cell membranes the cellular uptake of oligonucleotides are reduced compared to neutral or lipophilic equivalents. This polarity "hindrance" can be avoided by using the pro-drug approach (see e.g. Crooke, R. M. (1998) in Crooke, S. T. Antisense research and Application. Springer-Verlag, Berlin, Germany, vol. 131, pp. 103-140). Pharmaceutically acceptable binding agents and adjuvants may comprise part of the formulated drug.

Examples of delivery methods for delivery of the therapeutic agents described herein, as well as details of pharmaceutical formulations, salts, may are well described elsewhere for example in US provisional application 60/838,710 and 60/788,995, , and Danish applications, PA 2006 00615.

Pharmaceutical compositions of the present invention include, but are not limited to, solutions, emulsions, and liposome-containing formulations. These compositions may be generated from a variety of components that include, but are not limited to, preformed liquids, self- emulsifying solids and self-emulsifying semisolids. Delivery of drug to tumour tissue may be enhanced by carrier-mediated delivery including, but not limited to, cationic liposomes, cyclodextrins, porphyrin derivatives, branched chain dendrimers, polyethylen-imine polymers, nanoparticles and microspheres (Dass CR. J Pharm Pharmacol 2002; 54(1):3-27). The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product. The compositions of the present invention may be formulated into any of many possible dosage forms such as, but not limited to, tablets, capsules, gel capsules, liquid syrups, soft gels and suppositories. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers. The compounds of the invention may also be conjugated to active drug substances, for example, aspirin, ibuprofen, a sulfa drug, an antidiabetic, an antibacterial or an antibiotic.

In another embodiment, compositions of the invention may contain one or more oligonucleotide compounds, targeted to a first microRNA and one or more additional oligonucleotide compounds targeted to a second microRNA target. Two or more combined compounds may be used together or sequentially.

The compounds disclosed herein are useful for a number of therapeutic applications as indicated above. In general, therapeutic methods of the invention include administration of a therapeutically effective amount of an oligonucleotide to a mammal, particularly a human. In a certain embodiment, the present invention provides pharmaceutical compositions containing (a) one or more compounds of the invention, and (b) one or more chemotherapeutic agents. When used with the compounds of the invention, such chemotherapeutic agents may be used individually, sequentially, or in combination with one or more other such chemotherapeutic agents or in combination with radiotherapy. All chemotherapeutic agents known to a person skilled in the art are here incorporated as combination treatments with compound according to the invention. Other active agents, such as anti-inflammatory drugs, including but not limited to nonsteroidal anti-inflammatory drugs and corticosteroids, antiviral drugs, and immuno-modulating drugs may also be combined in compositions of the invention. Two or more combined compounds may be used together or sequentially.

Examples of therapeutic indications which may be treated by the pharmaceutical compositions of the invention:

| **microRNA** | **Possible medical indications** |
|---|---|
| miR-21 | Glioblastoma, breast cancer |
| miR-122 | hypercholesterolemia, hepatitis C, hemochromatosis |
| miR-19b | lymphoma and other tumour types |
| miR-155 | lymphoma, breast and lung cancer |
| miR-375 | diabetes, metabolic disorders |
| miR-181 | myoblast differentiation, auto immune disorders |

Tumor suppressor gene tropomysin 1 (TPM1) mRNA has been indicated as a target of miR-21. Myotrophin (mtpn) mRNA has been indicated as a target of miR 375.

In an even further aspect, the present invention relates to the use of an oligonucleotide according to the invention for the manufacture of a medicament for the treatment of a disease selected from the group consisting of: atherosclerosis, hypercholesterolemia and hyperlipidemia; cancer, glioblastoma, breast cancer, lymphoma, lung cancer; diabetes, metabolic disorders; myoblast differentiation; immune disorders.

The invention further refers to an oligonucleotides according to the invention for the use in the treatment of from a disease selected from the group consisting of: atherosclerosis, hypercholesterolemia and hyperlipidemia; cancer, glioblastoma, breast cancer, lymphoma, lung cancer; diabetes, metabolic disorders; myoblast differentiation; immune disorders.

Described herein is a method of treating a subject suffering from a disease or condition selected from from the group consisting of: atherosclerosis, hypercholesterolemia and hyperlipidemia; cancer, glioblastoma, breast cancer, lymphoma, lung cancer; diabetes, metabolic disorders; myoblast differentiation; immune disorders, the method comprising the step of administering an oligonucleotide or pharmaceutical composition of the invention to the subject in need thereof.

### Cancer

In an even further aspect, the present invention relates to the use of an oligonucleotide according to the invention for the manufacture of a medicament for the treatment of cancer. The present invention concerns a method for treatment of, or prophylaxis against, cancer, said method comprising administering an oligonucleotide of the invention or a pharmaceutical composition of the invention to a patient in need thereof.

Such cancers may include lymphoreticular neoplasia, lymphoblastic leukemia, brain tumors, gastric tumors, plasmacytomas, multiple myeloma, leukemia, connective tissue tumors, lymphomas, and solid tumors.

In the use of a compound of the invention for the manufacture of a medicament for the treatment of cancer, said cancer may suitably be in the form of a solid tumor. Analogously, in the method for treating cancer disclosed herein said cancer may suitably be in the form of a solid tumor.

Furthermore, said cancer is also suitably a carcinoma. The carcinoma is typically selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumors. More typically, said carcinoma is selected from the group consisting of malignant melanoma, non-small cell lung cancer, breast carcinoma, colon carcinoma and renal cell carcinoma. The malignant melanoma is typically selected from the group consisting of superficial spreading melanoma, nodular melanoma, lentigo maligna melanoma, acral melagnoma, amelanotic melanoma and desmoplastic melanoma.

Alternatively, the cancer may suitably be a sarcoma. The sarcoma is typically in the form selected from the group consisting of osteosarcoma, Ewing's sarcoma, chondrosarcoma, malignant fibrous histiocytoma, fibrosarcoma and Kaposi's sarcoma.

Alternatively, the cancer may suitably be a glioma.

A further embodiment is directed to the use of an oligonucleotide according to the invention for the manufacture of a medicament for the treatment of cancer, wherein said medicament further comprises a chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, the further chemotherapeutic agent is selected from taxanes such as Taxol, Paclitaxel or Docetaxel.

Similarly, the invention is further directed to the use of an oligonucleotide according to the invention for the manufacture of a medicament for the treatment of cancer, wherein said treatment further comprises the administration of a further chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, said treatment further comprises the administration of a further chemotherapeutic agent selected from taxanes, such as Taxol, Paclitaxel or Docetaxel.

Alternatively stated, described herein is a method for treating cancer, said method comprising administering an oligonucleotide of the invention or a pharmaceutical composition according to the invention to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

### Infectious diseases

It is contemplated that the compounds of the invention may be broadly applicable to a broad range of infectious diseases, such as diphtheria, tetanus, pertussis, polio, hepatitis B, hepatitis C, hemophilus influenza, measles, mumps, and rubella.

Hsa-miR122 is indicated in hapatitus C infection and as such oligonucleotides according to the invention which target miR-122 may be used to treat Hepatitus C infection.

Accordingly, in yet another aspect the present invention relates the use of an oligonucleotide according to the invention for the manufacture of a medicament for the treatment of an infectious disease. Also described herein is a method for treating an infectious disease, said method comprising administering an oligonucleotide according to the invention or a pharmaceutical composition according to the invention to a patient in need thereof.

### Inflammatory diseases

The inflammatory response is an essential mechanism of defense of the organism against the attack of infectious agents, and it is also implicated in the pathogenesis of many acute and chronic diseases, including autoimmune disorders. In spite of being needed to fight pathogens, the effects of an inflammatory burst can be devastating. It is therefore often necessary to restrict the symptomatology of inflammation with the use of anti-inflammatory drugs. Inflammation is a complex process normally triggered by tissue injury that includes activation of a large array of enzymes, the increase in vascular permeability and extravasation of blood fluids, cell migration and release of chemical mediators, all aimed to both destroy and repair the injured tissue.

In yet another aspect, the present invention relates to the use of an oligonucleotide according to the invention for the manufacture of a medicament for the treatment of an inflammatory disease. Also described herein is a method for treating an inflammatory disease, said method comprising administering an oligonucleotide according to the invention or a pharmaceutical composition according to the invention to a patient in need thereof.

In one preferred embodiment of the invention, the inflammatory disease is a rheumatic disease and/or a connective tissue diseases, such as rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris and Sjorgren's syndrome, in particular inflammatory bowel disease and Crohn's disease.

Alternatively, the inflammatory disease may be a non-rheumatic inflammation, like bursitis, synovitis, capsulitis, tendinitis and/or other inflammatory lesions of traumatic and/or sportive origin.

### Metabolic diseases

A metabolic disease is a disorder caused by the accumulation of chemicals produced naturally in the body. These diseases are usually serious, some even life threatening. Others may slow physical development or cause mental retardation. Most infants with these disorders, at first, show no obvious signs of disease. Proper screening at birth can often discover these problems. With early diagnosis and treatment, metabolic diseases can often be managed effectively.
In yet another aspect, the present invention relates to the use of an oligonucleotide according to the invention or a conjugate thereof for the manufacture of a medicament for the treatment of a metabolic disease. Also described herein is a method for treating a metabolic disease, said method comprising administering an oligonucleotide according to the invention or a conjugate thereof, or a pharmaceutical composition according to the invention to a patient in need thereof.
In one preferred embodiment of the invention, the metabolic disease is selected from the group consisting of Amyloidosis, Biotinidase, OMIM (Online Mendelian Inheritance in Man), Crigler Najjar Syndrome, Diabetes, Fabry Support & Information Group, Fatty acid Oxidation Disorders, Galactosemia, Glucose-6-Phosphate Dehydrogenase (G6PD) deficiency, Glutaric aciduria, International Organization of Glutaric Acidemia, Glutaric Acidemia Type I, Glutaric Acidemia, Type II, Glutaric Acidemia Type I, Glutaric Acidemia Type-II, F-HYPDRR - Familial Hypophosphatemia, Vitamin D Resistant Rickets, Krabbe Disease, Long chain 3 hydroxyacyl CoA dehydrogenase deficiency (LCHAD), Mannosidosis Group, Maple Syrup Urine Disease, Mitochondrial disorders, Mucopolysaccharidosis Syndromes: Niemann Pick, Organic acidemias, PKU, Pompe disease, Porphyria, Metabolic Syndrome, Hyperlipidemia and inherited lipid disorders, Trimethylaminuria: the fish malodor syndrome, and Urea cycle disorders.

### Liver disorders

In yet another aspect, the present invention relates to the use of an oligonucleotide according to the invention or a conjugate thereof for the manufacture of a medicament for the treatment of a liver disorder. Also described herein is a method for treating a liver disorder, said method comprising administering an oligonucleotide according to the invention or a conjugate thereof, or a pharmaceutical composition according to the invention to a patient in need thereof.

In one preferred embodiment of the invention, the liver disorder is selected from the group consisting of Biliary Atresia, Alagille Syndrome, Alpha-1 Antitrypsin, Tyrosinemia, Neonatal Hepatitis, and Wilson Disease.

### Other uses

The oligonucleotides of the present invention can be utilized for as research reagents for diagnostics, therapeutics and prophylaxis. In research, the oligonucleotide may be used to specifically inhibit the synthesis of target genes in cells and experimental animals thereby facilitating functional analysis of the target or an appraisal of its usefulness as a target for therapeutic intervention. In diagnostics the oligonucleotides may be used to detect and quantitate target expression in cell and tissues by Northern blotting, *in-situ* hybridisation or similar techniques. For therapeutics, an animal or a human, suspected of having a disease or disorder, which can be treated by modulating the expression of target is treated by administering the oligonucleotide compounds in accordance with this invention. Also described herein are methods of treating an animal particular mouse and rat and treating a human, suspected of having or being prone to a disease or condition, associated with expression of target by administering a therapeutically or prophylactically effective amount of one or more of the oligonucleotide compounds or compositions of the invention.

### Therapeutic use of oligonucleotides targeting miR-122a

In the examples section, it is demonstrated that a LNA-antimiR™, such as SPC3372, targeting miR-122a reduces plasma cholesterol levels. Therefore, another aspect of the invention is use of the above described oligonucleotides targeting miR-122a as medicine. Still another aspect of the invention is use of the above described oligonucleotides targeting miR-122a for the preparation of a medicament for treatment of increased plasma cholesterol levels. The skilled man will appreciate that increased plasma cholesterol levels is undesireable as it increases the risk of various conditions, e.g. atherosclerosis. Still another aspect of the invention is use of the above described oligonucleotides targeting miR-122a for upregulating the mRNA levels of Nrdg3, Aldo A, Bckdk or CD320.

### Further Teachings

The following teachings may be combined with the other embodiments as described herein:
1. An oligonucleotide having a length of from 12 to 26 nucleotides, wherein
   i) the first nucleotide, counting from the 3' end, is a locked nucleic acid (LNA) unit;
   ii) the second nucleotide, counting from the 3' end, is an LNA unit; and
   iii) the ninth and/or the tenth nucleotide, counting from the 3' end, is an LNA unit.
2. The oligonucleotide according to paragraph 1, wherein the ninth nucleotide, counting from the 3' end, is an LNA unit.
3. The oligonucleotide according to paragraph 1, wherein the tenth nucleotide, counting from the 3' end, is an LNA unit.
4. The oligonucleotide according to paragraph 1, wherein both the ninth and the tenth nucleotide, calculated from the 3' end, are LNA units.
5. The oligonucleotide according to any of paragraphs 1-4, wherein said oligonucleotide comprises at least one LNA unit in positions three to eight, counting from the 3' end.
6. The oligonucleotide according to paragraph 5, wherein said oligonucleotide comprises one LNA unit in positions three to eight, counting from the 3' end.
7. The oligonucleotide according to paragraph 6, wherein the substitution pattern for the nucleotides in positions three to eight, counting from the 3' end, is selected from the group consisting of Xxxxxx, xXxxxx, xxXxxx, xxxXxx, xxxxXx and xxxxxX, wherein "X" denotes an LNA unit and "x" denotes a non-LNA unit.
8. The oligonucleotide according to paragraph 5, wherein said oligonucleotide comprises at least two LNA units in positions three to eight, counting from the 3' end.
9. The oligonucleotide according to paragraph 8, wherein said oligonucleotide comprises two LNA units in positions three to eight, counting from the 3' end.
10. The oligonucleotide according to paragraph 9, wherein the substitution pattern for the nucleotides in positions three to eight, counting from the 3' end, is selected from the group consisting of XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX and xxxxXX, wherein "X" denotes an LNA unit and "x" denotes a non-LNA unit.
11. The oligonucleotide according to paragraph 10, wherein the substitution pattern for the nucleotides in positions three to eight, counting from the 3' end, is selected from the group consisting of XxXxxx, XxxXxx, XxxxXx, XxxxxX, xXxXxx, xXxxXx, xXxxxX, xxXxXx, xxXxxX and xxxXxX, wherein "X" denotes an LNA unit and "x" denotes a non-LNA unit.
12. The oligonucleotide according to paragraph 11, wherein the substitution pattern for the nucleotides in positions three to eight, counting from the 3' end, is selected from the group consisting of xXxXxx, xXxxXx, xXxxxX, xxXxXx, xxXxxX and xxxXxX, wherein "X" denotes an LNA unit and "x" denotes a non-LNA unit.
13. The oligonucleotide according to paragraph 12, wherein the substitution pattern for the nucleotides in positions three to eight, counting from the 3' end, is selected from the group consisting of xXxXxx, xXxxXx and xxXxXx, wherein "X" denotes an LNA unit and "x" denotes a non-LNA unit.
14. The oligonucleotide according to paragraph 13, wherein the substitution pattern for the nucleotides in positions three to eight, counting from the 3' end, is xXxXxx, wherein "X" denotes an LNA unit and "x" denotes a non-LNA unit.
15. The oligonucleotide according to paragraph 5, wherein said oligonucleotide comprises at least three LNA units in positions three to eight, counting from the 3' end.
16. The oligonucleotide according to paragraph 15, wherein said oligonucleotide comprises three LNA units in positions three to eight, counting from the 3' end.
17. The oligonucleotide according to paragraph 16, wherein the substitution pattern for the nucleotides in positions three to eight, counting from the 3' end, is selected from the group consisting of XXXxxx, xXXXxx, xxXXXx, xxxXXX, XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit.
18. The oligonucleotide according to paragraph 17, wherein the substitution pattern for the nucleotides in positions three to eight, counting from the 3' end, is selected from the group consisting of XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit.
19. The oligonucleotide according to paragraph 18, wherein the substitution pattern for the nucleotides in positions three to eight, counting from the 3' end, is selected from the group consisting of xXXxXx, xXXxxX, xxXXxX, xXxXXx, xXxxXX, xxXxXX and xXxXxX, wherein "X" denotes an LNA unit and "x" denotes a non-LNA unit.
20. The oligonucleotide according to paragraph 18, wherein the substitution pattern for the nucleotides in positions three to eight, counting from the 3' end, is xXxXxX or XxXxXx, wherein "X" denotes an LNA unit and "x" denotes a non-LNA unit.
21. The oligonucleotide according to paragraph 20, wherein the substitution pattern for the nucleotides in positions three to eight, counting from the 3' end, is xXxXxX, wherein "X" denotes an LNA unit and "x" denotes a non-LNA unit.
22. The oligonucleotide according to any of paragraph 7-21, wherein said non-LNA unit is a DNA unit.
23. The oligonucleotide according to any of the preceding paragraphs , wherein said nucleotide has a length of from 12 to 24 nucleotides, such as a length of from 12 to 22 nucleotides, preferably a length of from 12 to 20 nucleotides, such as a length of from 12 to 19 nucleotides, more preferably a length of from 12 to 18 nucleotides, such as a length of from 12 to 17 nucleotides, even more preferably a length of from 12 to 16 nucleotides.
24. The oligonucleotide according to any of the preceding paragraphs , wherein said oligonucleotide comprises at least one LNA unit, such as one LNA unit, from position 11, counting from the 3' end, to the 5' end.
25. The oligonucleotide according to any of the preceding paragraphs , wherein said oligonucleotide comprises at least two LNA units, such as two LNA units, from position 11, counting from the 3' end, to the 5' end.
26. The oligonucleotide according to paragraph 24 or 25, wherein said oligonucleotide comprises 12 nucleotides and the substitution pattern for positions 11 to 12, counting from the 3' end, is selected from the group consisting of xX and Xx, wherein "X" denotes an LNA unit and "x" denotes a non-LNA unit.
27. The oligonucleotide according to paragraph 26, wherein the substitution pattern for positions 11 to 12, counting from the 3' end, is xX, wherein "X" denotes an LNA unit and "x" denotes a non-LNA unit.
28. The oligonucleotide according to paragraph 24 or 25, wherein said oligonucleotide comprises 13 nucleotides and the substitution pattern for positions 11 to 13, counting from the 3' end, is selected from the group consisting of Xxx, xXx, xxX, XXx, XxX, xXX and XXX, wherein "X" denotes an LNA unit and "x" denotes a non-LNA unit.
29. The oligonucleotide according to paragraph 28, wherein the substitution pattern for positions 11 to 13, counting from the 3' end, is xxX, wherein "X" denotes an LNA unit and "x" denotes a non-LNA unit.
30. The oligonucleotide according to paragraph 24 or 25, wherein said oligonucleotide comprises 14 nucleotides and the substitution pattern for positions 11 to 14, counting from the 3' end, is selected from the group consisting of Xxxx, xXxx, xxXx, xxxX, XXxx, XxXx, XxxX, xXXx, xXxX and xxXX, wherein "X" denotes an LNA unit and "x" denotes a non-LNA unit.
31. The oligonucleotide according to paragraph 30, wherein the substitution pattern for positions 11 to 14, counting from the 3' end, is xXxX, wherein "X" denotes an LNA unit and "x" denotes a non-LNA unit.
32. The oligonucleotide according to paragraph 24 or 25, wherein said oligonucleotide comprises 15 nucleotides and the substitution pattern for positions 11 to 15, counting from the 3' end, is selected from the group consisting of Xxxxx, xXxxx, xxXxx, xxxXx, xxxxX, XXxxx, XxXxx, XxxXx, XxxxX, xXXxx, xXxXx, xXxxX, xxXXx, xxXxX and xxxXX, wherein "X" denotes an LNA unit and "x" denotes a non-LNA unit.
33. The oligonucleotide according to paragraph 32, wherein the substitution pattern for positions 11 to 15, counting from the 3' end, is xxXxX, wherein "X" denotes an LNA unit and "x" denotes a non-LNA unit.
34. The oligonucleotide according to paragraph 24 or 25, wherein said oligonucleotide comprises 16 nucleotides and the substitution pattern for positions 11 to 16, counting from the 3' end, is selected from the group consisting of Xxxxxx, xXxxxx, xxXxxx, xxxXxx, xxxxXx, xxxxxX, XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX, xxxxXX, XXXxxx, XXxXxx, XXxxXx, XXxxxX, XxXXxx, XxXxXx, XxXxxX, XxxXXx, XxxXxX, XxxxXX, xXXXxx, xXXxXx, xXXxxX, xXxXXx, xXxXxX, xXxxXX, xxXXXx, xxXXxX, xxXxXX and xxxXXX, wherein "X" denotes an LNA unit and "x" denotes a non-LNA unit.
35. The oligonucleotide according to paragraph 34, wherein the substitution pattern for positions 11 to 16, counting from the 3' end, is xxXxxX, wherein "X" denotes an LNA unit and "x" denotes a non-LNA unit.
36. The oligonucleotide according to paragraph 24 or 25, wherein said oligonucleotide comprises an LNA unit at the 5' end.
37. The oligonucleotide according to paragraph 36 containing an LNA unit at the first two positions, counting from the 5' end.
38. The oligonucleotide according to any of the preceding paragraphs , wherein the oligonucleotide comprises at least one internucleoside linkage group which differs from phosphate.
39. The oligonucleotide according to paragraph 38, wherein said internucleoside linkage group, which differs from phosphate, is phosphorothioate.
40. The oligonucleotide according to paragraph 39, wherein all internucleoside linkage groups are phosphorothioate.
41. The oligonucleotide according to any of the preceding paragraphs, wherein said LNA units are independently selected from the group consisting of thio-LNA units, amino-LNA units and oxy-LNA units.
42. The oligonucleotide according to paragraph 41, wherein said LNA units are in the beta-D-form.
43. The oligonucleotide according to paragraph 41, wherein said LNA units are oxy-LNA units in the beta-D-form.
44. The oligonucleotide according to any of the preceding paragraphs for use as a medicament.
45. A pharmaceutical composition comprising an oligonucleotide according to any of paragraphs 1-43 and a pharmaceutically acceptable carrier.
46. The composition according to paragraph 45, wherein said carrier is saline or buffered saline.
47. Use of an oligonucleotide according to any of paragraphs 1-43 for the manufacture of a medicament for the treatment of cancer.
48. A method for the treatment of cancer, comprising the step of administering an oligonucleotide according to any of paragraphs 1-43 or a composition according to paragraph 45.

### References

Abelson, J.F. etal. 2005. Science 310: 317-20.
Bartel, D.P. 2004. Cell 116: 281-297.
Boehm, M., Slack, F. 2005. Science. 310:1954-7.
Brennecke, J. et al. 2003 Cell 113: 25-36.
Calin, G.A. et al. 2002. Proc. Natl. Acad. Sci. USA 99: 15524- 15529.
Calin, G. A. et al. 2004. Proc. Natl. Acad. Sci.U.S.A. 101: 2999-3004.
Calin, G.A. et al. 2005. N. Engl. J. Med. 353:1793-801
Chan, J.A.et al. 2005. Cancer Res. 65:6029-33.
Chen, C.Z., et al. 2004. Science 303: 83-86.
Chen, J.F., et al. 2005. Nat Genet. Dec 25, advance online publication.
Eis, P.S. et al. 2005. Proc Natl Acad Sci USA. 102: 3627-32.
Giraldez, A.J. et al. 2005. Science 308: 833-838.
Griffiths-Jones, S. et al. 2004. Nucleic Acids Res. 32: D109-D111.
Griffiths-Jones, S., et al. 2006. Nucleic Acids Res. 34: D140-4
He, L. et al. 2005. Nature 435: 828- 833.
Hornstein, E. et al. 2005. Nature 438: 671-4.
Hutvagner, G. et al 2001. Science 293: 834-838.
Hutvágner, G. et al. 2004. PLoS Biology 2: 1-11.
Iorio, M.V. et al. 2005. Cancer Res. 65: 7065-70.
Jin, P. et al. 2004. Nat Cell Biol. 6: 1048-53.
Johnson, S.M. et al. 2005. Cell 120: 635-647.
Jopling, C.L. et al. 2005. Science 309:1577-81.
Ketting, R.F. et al. 2001. Genes Dev. 15: 2654-2659.
Kwon, C. et al. 2005. Proc Natl Acad Sci USA. 102: 18986-91.
Landthaler, M. et al. 2004. Curr. Biol. 14: 2162-2167.
Leaman, D. et al. 2005. Cell 121: 1097-108.
Lee, Y., et al. 2003. Nature 425: 415-419.
Li, X. and Carthew, R.W. 2005. Cell 123: 1267-77.
Lu. J. et al. 2005. Nature 435: 834-838.
Michael, M.Z.et al. 2003. Mol. Cancer Res. 1: 882- 891.
Nelson, P. et al. 2003. TIBS 28: 534-540.
Paushkin, S., et al. 2002.Curr.Opin.Cell Biol. 14: 305-312.
Poy, M.N. et al. 2004. Nature 432: 226-230.
Wienholds, E. et al. 2005. Science 309: 310-311.
Yekta, S. et al. 2004. Science 304: 594-596.
Zhao, Y. et al. 2005. Nature 436: 214-220.

### EXPERIMENTAL

### Example 1: Monomer synthesis

The LNA monomer building blocks and derivatives thereof were prepared following published procedures and references cited therein, see, e.g. WO 03/095467 A1 and D. S. Pedersen, C. Rosenbohm, T. Koch (2002) Preparation of LNA Phosphoramidites, Synthesis 6, 802-808.

### Example 2: Oligonucleotide synthesis

Oligonucleotides were synthesized using the phosphoramidite approach on an Expedite 8900/MOSS synthesizer (Multiple Oligonucleotide Synthesis System) at 1 µmol or 15 µmol scale. For larger scale synthesis an Äkta Oligo Pilot (GE Healthcare) was used. At the end of the synthesis (DMT-on), the oligonucleotides were cleaved from the solid support using aqueous ammonia for 1-2 hours at room temperature, and further deprotected for 4 hours at 65°C. The oligonucleotides were purified by reverse phase HPLC (RP-HPLC). After the removal of the DMT-group, the oligonucleotides were characterized by AE-HPLC, RP-HPLC, and CGE and the molecular mass was further confirmed by ESI-MS. See below for more details.

### Preparation of the LNA-solid support:

### Preparation of the LNA succinyl hemiester

5'-O-Dmt-3'-hydroxy-LNA monomer (500 mg), succinic anhydride (1.2 eq.) and DMAP (1.2 eq.) were dissolved in DCM (35 mL). The reaction was stirred at room temperature overnight. After extractions with NaH₂PO₄ 0.1 M pH 5.5 (2x) and brine (1x), the organic layer was further dried with anhydrous Na₂SO₄ filtered and evaporated. The hemiester derivative was obtained in 95% yield and was used without any further purification.

### Preparation of the LNA-support

The above prepared hemiester derivative (90 µmol) was dissolved in a minimum amount of DMF, DIEA and pyBOP (90 µmol) were added and mixed together for 1 min. This pre-activated mixture was combined with LCAA-CPG (500 Å, 80-120 mesh size, 300 mg) in a manual synthesizer and stirred. After 1.5 hours at room temperature, the support was filtered off and washed with DMF, DCM and MeOH. After drying, the loading was determined to be 57 µmol/g (see Tom Brown, Dorcas J.S.Brown. Modern machine-aided methods of oligodeoxyribonucleotide synthesis. In: F.Eckstein, editor. Oligonucleotides and Analogues A Practical Approach. Oxford: IRL Press, 1991: 13-14).

### Elongation of the oligonucleotide

The coupling of phosphoramidites (A(bz), G(ibu), 5-methyl-C(bz)) or T-β-cyanoethyl-phosphoramidite) is performed by using a solution of 0.1 M of the 5'-O-DMT-protected amidite in acetonitrile and DCI (4,5-dicyanoimidazole) in acetonitrile (0.25 M) as activator. The thiolation is carried out by using xanthane chloride (0.01 M in acetonitrile:pyridine 10%). The rest of the reagents are the ones typically used for oligonucleotide synthesis.

### Purification by RP-HPLC:

| | |
|---|---|
| Column: | Xterra RP₁₈ |
| Flow rate: | 3 mL/min |
| Buffers: | 0.1 M ammonium acetate pH 8 and acetonitrile |

### Abbreviations:

- DMT:: Dimethoxytrityl
- DCI:: 4,5-Dicyanoimidazole
- DMAP:: 4-Dimethylaminopyridine
- DCM:: Dichloromethane
- DMF:: Dimethylformamide
- THF:: Tetrahydrofurane
- DIEA:: *N*,*N*-diisopropylethylamine
- PyBOP:: Benzotriazole-1-yl-oxy-tris-pyrrolidino-phosphonium hexafluorophosphate
- Bz:: Benzoyl
- Ibu:: Isobutyryl

### Example 3: Design of the LNA anti-miR oligonucleotides and melting temperatures

### Target microRNA:

| | | |
|---|---|---|
| miR-122a: | 5'-uggagugugacaaugguguuugu-3' | SEQ ID NO: 535 |
| miR-122a 3' to 5': orientation) | 3'-uguuugugguaacagugugaggu-5' | (SEQ ID NO: 535 reverse |

**Table 1 LNA anti-miR oligonucleotide sequences and Tₘ:**

| SEQ ID NO: | Oligo ID | | SED ID | Sequence: | | Tm (°C) |
|---|---|---|---|---|---|---|
| 2 | SPC3370 | XxxX design | SEQ ID 585 | 5'-cCatTgtCacActCca-3' | PS backbone | 75 |
| 3 | SPC3372 | XxxX design | SEQ ID 586 | 5'-ccAttGtcAcaCtcCa-3' | PS backbone | 69 |
| 4 | SPC3375 | Gapmer | SEQ ID 587 | 5'-CCAttgtcacacTCCa-3' | PS backbone | 69 |
| 5 | SPC3549 | 15-mer | SEQ ID 588 | 5'-CcAttGTcaCaCtCC-3' | PS backbone | 78 |
| 6 | SPC3550 | mismatch control | SEQ ID 589 | 5'-CcAttCTgaCcCtAC-3' | PS backbone | 32 |
| 7 | SPC3373 | mismatch control | SEQ ID 590 | 5'-ccAttGtcTcaAtcCa-3' | PS backbone | 46 |
| 8 | SPC3548 | 13-mer | SEQ ID 591 | 5'-AttGTcaCaCtCC-3' | PS backbone | |

| | | | | | | |
|---|---|---|---|---|---|---|
| lower case: DNA, uppercase: LNA (all LNA C were methylated), underlined: mismatch | | | | | | |

The melting temperatures were assessed towards the mature miR-122a sequence, using a synthetic miR-122a RNA oligonucleotide with phosphorothioate linkaged.

The LNA anti-miR/miR-122a oligo duplex was diluted to 3 µM in 500 µl RNase free H₂0, which was then mixed with 500 µl 2x dimerization buffer (final oligo/duplex conc. 1,5 µM, 2x Tm buffer: 200 mM NaCl, 0,2 mM EDTA, 20 mM NaP, pH 7,0, DEPC treated to remove RNases). The mix was first heated to 95 degrees for 3 minutes, then allowed to cool at room temperature (RT) for 30 minutes.

Following RT incubation Tₘ was measured on Lambda 40 UV/VIS Spectrophotometer with peltier temperature progammer PTP6 using PE Templab software (Perkin Elmer). The Temperature was ramped up from 20°C to 95°C and then down again to 20°C, continuously recording absorption at 260 nm. First derivative and local maximums of both the melting and annealing was used to assess melting/annealing point (Tₘ), both should give similar/same Tₘ values. For the first derivative 91 points was used to calculate the slope.

By substituting the antimir oligonucleotide and the complementary RNA molecule, the abaove assay can be used to determine the Tₘ of other oligonucleotides such as the oligonucleotides according to the invention.

However, in one embodiment the Tₘ may be made with a complementary DNA (phosphorothioate linkages) molecule. Typically the Tₘ measured against a DNA complementary molecule is abot 10°C lower than the Tₘ with an equivalent RNA complement. The Tₘ measured using the DNA complement may therefore be used in cases where the duplex has a very high Tₘ.

### Melting temperature (Tₘ) measurements:

| oligo to miR-122 RNA complement | Tₘ |
|---|---|
| SPC3372 + miR-122a, RNA | 69°C |
| SPC3648 + miR-122a, RNA | 74°C |
| SPC3649 + miR-122a, RNA | 79°C |

| oligo to DNA complement | Tₘ |
|---|---|
| SPC3372 + 122R, DNA | 57 °C |
| SPC3649 + 122R, DNA | 66 °C |

It is recognised that for oligonucleotides with very high Tₘ, the above Tₘ assays may be insufficient to determine the Tₘ. In such an instance the use of a phosphorothioated DNA complementary molecule may further lower the Tₘ.

The use of formamide is routine in the analysis of oligonucleotide hybridisation (see Hutton 1977, NAR 4 (10) 3537-3555). In the above assay the inclusion of 15% formamide typically lowers the Tₘ by about 9°C, and the inclusion of 50% formamide typically lowers the Tₘ by about 30°C. Using these ratios, it is therefore possible to determine the comparative Tₘ of an oligonucleotide against its complementary RNA (phosphodiester) molecule, even when the Tₘ of the duplex is, for example higher than 95°C (in the absence of formamide).

For oligonucleotides with a very high Tₘ, an alternative method of determining the Tₘ, is to make titrations and run it out on a gel to see single strand versus duplex and by those concentrations and ratios determine Kd (the dissociation constant) which is related to deltaG and also Tₘ.

### Example 4: Stability of LNA oligonucletides in human or rat plasma

LNA oligonucleotide stability was tested in plasma from human or rats (it could also be mouse, monkey or dog plasma). In 45 µl plasma, 5 µl LNA oligonucleotide is added (at a final concentration of 20 µM). The LNA oligonucleotides are incubated in plasma for times ranging from 0 to 96 hours at 37 °C (the plasma is tested for nuclease activity up to 96 hours and shows no difference in nuclease cleavage-pattern).

At the indicated time the sample were snap frozen in liquid nitrogen. 2 µL (equals 40 pmol) LNA oligonucleotide in plasma was diluted by adding 15 µL of water and 3 µL 6x loading dye (Invitrogen). As marker a 10 bp ladder (Invitrogen, USA 10821-015) is used. To 1 µl ladder, 1 µl 6x loading and 4 µl water is added. The samples are mixed, heated to 65 °C for 10 min and loaded to a pre-run gel (16% acrylamide, 7 M UREA, 1x TBE, pre-run at 50 Watt for 1 h) and run at 50-60 Watt for 2½ hours. Subsequently, the gel is stained with 1x SyBR gold (molecular probes) in 1x TBE for 15 min. The bands were visualised using a phosphoimager from BioRad.

### Example 5: In vitro model: Cell culture

The effect of LNA oligonucleotides on target nucleic acid expression (amount) can be tested in any of a variety of cell types provided that the target nucleic acid is present at measurable levels. Target can be expressed endogenously or by transient or stable transfection of a nucleic acid encoding said nucleic acid.

The expression level of target nucleic acid can be routinely determined using, for example, Northern blot analysis (including microRNA northern), Quantitative PCR (including microRNA qPCR), Ribonuclease protection assays. The following cell types are provided for illustrative purposes, but other cell types can be routinely used, provided that the target is expressed in the cell type chosen.

Cells were cultured in the appropriate medium as described below and maintained at 37°C at 95-98% humidity and 5% CO₂. Cells were routinely passaged 2-3 times weekly.

15PC3: The human prostate cancer cell line 15PC3 was kindly donated by Dr. F. Baas, Neurozintuigen Laboratory, AMC, The Netherlands and was cultured in DMEM (Sigma) + 10% fetal bovine serum (FBS) + Glutamax I + gentamicin.

PC3: The human prostate cancer cell line PC3 was purchased from ATCC and was cultured in F12 Coon's with glutamine (Gibco) + 10% FBS + gentamicin.

518A2: The human melanoma cancer cell line 518A2 was kindly donated by Dr. B. Jansen, Section of experimental Oncology, Molecular Pharmacology, Department of Clinical Pharmacology, University of Vienna and was cultured in DMEM (Sigma) + 10% fetal bovine serum (FBS) + Glutamax I + gentamicin.

HeLa: The cervical carcinoma cell line HeLa was cultured in MEM (Sigma) containing 10% fetal bovine serum gentamicin at 37°C, 95% humidity and 5% CO₂.

MPC-11: The murine multiple myeloma cell line MPC-11 was purchased from ATCC and maintained in DMEM with 4mM Glutamax+ 10% Horse Serum.

DU-145: The human prostate cancer cell line DU-145 was purchased from ATCC and maintained in RPMI with Glutamax + 10% FBS.

RCC-4 +/- VHL: The human renal cancer cell line RCC4 stably transfected with plasmid expressing VHL or empty plasmid was purchased from ECACC and maintained according to manufacturers instructions.

786-0: The human renal cell carcinoma cell line 786-0 was purchased from ATCC and maintained according to manufacturers instructions

HUVEC: The human umbilical vein endothelial cell line HUVEC was purchased from Camcrex and maintained in EGM-2 medium.

K562: The human chronic myelogenous leukaemia cell line K562 was purchased from ECACC and maintained in RPMI with Glutamax + 10% FBS. U87MG: The human glioblastoma cell line U87MG was purchased from ATCC and maintained according to the manufacturers instructions.

B16: The murine melanoma cell line B16 was purchased from ATCC and maintained according to the manufacturers instructions.

LNCap: The human prostate cancer cell line LNCap was purchased from ATCC and maintained in RPMI with Glutamax + 10% FBS

Huh-7: Human liver, epithelial like cultivated in Eagles MEM with 10 % FBS, 2mM Glutamax I, 1x non-essential amino acids, Gentamicin 25 µg/ml

L428: (Deutsche Sammlung für Mikroorganismen (DSM, Braunschwieg, Germany)): Human B cell lymphoma maintained in RPMI 1640 supplemented with 10% FCS, L-glutamine and antibiotics.

L1236: (Deutsche Sammlung für Mikroorganismen (DSM, Braunschwieg, Germany)): Human B cell lymphoma maintained in RPMI 1640 supplemented with 10% FCS, L-glutamine and antibiotics.

### Example 6: In vitro model: Treatment with LNA anti-miR antisense oligonucleotide

The miR-122a expressing cell line Huh-7 was transfected with LNA anti-miRs at 1 and 100 nM concentrations according to optimized lipofectamine 2000 (LF2000, Invitrogen) protocol (as follows).

Huh-7 cells were cultivated in Eagles MEM with 10 % FBS, 2mM Glutamax I, 1x non-essential amino acids, Gentamicin 25 µg/ml. The cells were seeded in 6-well plates (300000 cells per well), in a total vol. of 2,5 ml the day before transfection. At the day of transfection a solution containing LF2000 diluted in Optimem (Invitrogen) was prepared (1,2 ml optimem + 3,75 µl LF2000 per well, final 2,5 µg LF2000/ml, final tot vol 1,5 ml).

LNA Oligonucleotides (LNA anti-miRs) were also diluted in optimem. 285 µl optimem + 15 µl LNA oligonuclotide (10 µM oligonucleotide stock for final concentration 100 nM and 0,1 µM for final concentration 1 nM) Cells were washed once in optimem then the 1,2 ml optimem/LF2000 mix were added to each well. Cells were incubated 7 min at room temperature in the LF2000 mix where after the 300 µl oligonucleotide optimem solution was added.

Cell were further incubated for four hours with oligonucleotide and lipofectamine2000 (in regular cell incubator at 37 °C, 5% CO2). After these four hours the medium/mix was removed and regular complete medium was added. Cells were allowed to grow for another 20 hours. Cells were harvested in Trizol (Invitrogen) 24 hours after transfection. RNA was extracted according to a standard Trizol protocol according to the manufacturer's instructions (Invitrogen), especially to retain the microRNA in the total RNA extraction.

### Example 7: In vitro and in vivo model: Analysis of Oligonucleotide Inhibition of miR expression by microRNA specific quantitative PCR

miR-122a levels in the RNA samples were assessed on an ABI 7500 Fast real-time PCR instrument (Applied Biosystems, USA) using a miR-122a specific qRT-PCR kit, mirVana (Ambion, USA) and miR-122a primers (Ambion, USA). The procedure was conducted according to the manufacturers protocol.

### Results:

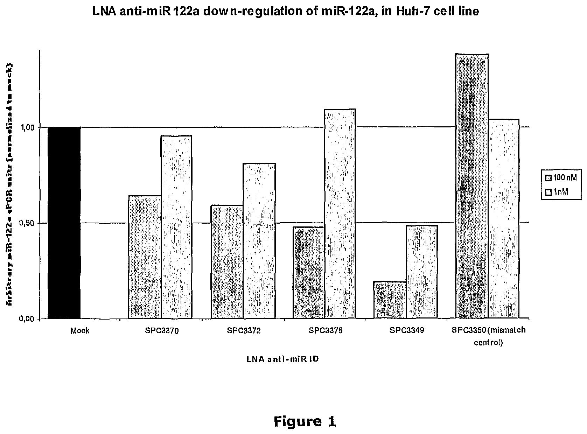

The miR-122a -specific new LNA anti-miR oligonucleotide design (ie SPC3349 (also referred to as SPC 3549)), was more efficient in inhibiting miR-122a at 1 nM compared to previous design models, including "every-third" and "gap-mer" (SPG3370, SPC3372, SPC3375) motifs were at 100 nM. The mismatch control was not found to inhibit miR-122a (SPC3350). Results are shown in figure 1.

### Example 8: Assessment of LNA antago-mir knock-down specificity using miRNA microarray expression profiling.

### A) RNA labeling for miRNA microarray profiling

Total RNA was extracted using Trizol reagent (Invitrogen) and 3'end labeled using T4 RNA ligase and Cy3- or Cy5-labeled RNA linker (5'-PO4-rUrUrU-Cy3/dT-3' or 5'-PO4-rUrUrU-Cy5/dT-3'). The labeling reactions contained 2-5 µg total RNA, 15 µM RNA linker, 50 mM Tris-HCl (pH 7.8), 10 mM MgCl2, 10 mM DTT, 1 mM ATP, 16% polyethylene glycol and 5 unit T4 RNA ligase (Ambion, USA) and were incubated at 30 °C for 2 hours followed by heat inactivation of the T4 RNA ligase at 80° C for 5 minutes.

### B) Microarray hybridization and post-hybridization washes

LNA-modified oligonucleotide capture probes comprising probes for all annotated miRNAs annotated from mouse (Mus musculus) and human (Homo sapiens) in the miRBase MicroRNA database Release 7.1 including a set of positive and negative control probes were purchased from Exiqon (Exiqon, Denmark) and used to print the microarrays for miRNA profiling. The capture probes contain a 5'-terminal C6-amino modified linker and were designed to have a Tm of 72° C against complementary target miRNAs by adjustment of the LNA content and length of the capture probes. The capture probes were diluted to a final concentration of 10 µM in 150 mM sodium phosphate buffer (pH 8.5) and spotted in quadruplicate onto Codelink slides (Amersham Biosciences) using the MicroGrid II arrayer from BioRobotics at 45% humidity and at room temperature. Spotted slides were post-processed as recommended by the manufacturer.
Labeled RNA was hybridized to the LNA microarrays overnight at 65° C in a hybridization mixture containing 4x SSC, 0.1% SDS, 1 µg/µl Herring Sperm DNA and 38% formamide. The hybridized slides were washed three times in 2x SSC, 0.025% SDS at 65°C, followed by three times in 0.08x SSC and finally three times in 0.4x SSC at room temperature.

### C) Array scanning, image analysis and data processing

The microarrays were scanned using the ArrayWorx scanner (Applied Precision, USA) according to the manufacturer's recommendations. The scanned images were imported into TIGR Spotfinder version 3.1 (Saeed et al., 2003) for the extraction of mean spot intensities and median local background intensities, excluding spots with intensities below median local background + 4x standard deviations. Background-correlated intensities were normalized using variance stabilizing normalization package version 1.8.0 (Huber et al., 2002) for R (www.r-project.org). Intensities of replicate spots were averaged using Microsoft Excel. Probes displaying a coefficient of variance > 100% were excluded from further data analysis.

### Example 9: Detection of microRNAs by in situ hybridizationDetection of microRNAs in formalin-fixed paraffin-embedded tissue sections by in situ hybridization.

### A) Preparation of the formalin-fixed, paraffin-embedded sections for in situ hybridization

Archival paraffin-embedded samples are retrieved and sectioned at 5 to 10 mm sections and mounted in positively-charged slides using floatation technique. Slides are stored at 4 °C until the in situ experiments are conducted.

### B) In situ hybridization

Sections on slides are deparaffinized in xylene and then rehydrated through an ethanol dilution series (from 100% to 25%). Slides are submerged in DEPC-treated water and subject to HCl and 0.2% Glycine treatment, re-fixed in 4% paraformaldehyde and treated with acetic anhydride/triethanolamine; slides are rinsed in several washes of 1X PBS in-between treatments. Slides are pre-hybridized in hyb solution (50% formamide, 5X SSC, 500 mg/mL yeast tRNA, 1X Denhardt) at 50 °C for 30 min. Then, 3 pmol of a FITC-labeled LNA probe (Exiqon, Denmark) complementary to each selected miRNA is added to the hyb. solution and hybridized for one hour at a temperature 20-25 °C below the predicted Tm of the probe (typically between 45-55 °C depending on the miRNA sequence). After washes in 0.1X and 0.5X SCC at 65 °C, a tyramide signal amplification reaction was carried out using the Genpoint Fluorescein (FITC) kit (DakoCytomation, Denmark) following the vendor's recommendations. Finally, slides are mounted with Prolong Gold solution. Fluorescence reaction is allowed to develop for 16-24 hr before documenting expression of the selected miRNA using an epifluorescence microscope.

### Detection of microRNAs by whole-mount in situ hybridization of zebrafish. Xenopus and mouse embryos.

All washing and incubation steps are performed in 2 ml eppendorf tubes. Embryos are fixed overnight at 4 oC in 4% paraformaldehyde in PBS and subsequently transferred through a graded series (25% MeOH in PBST (PBS containing 0.1% Tween-20), 50% MeOH in PBST, 75% MeOH in PBST) to 100% methanol and stored at -20 oC up to several months. At the first day of the in situ hybridization embryos are rehydrated by successive incubations for 5 min in 75% MeOH in PBST, 50% MeOH in PBST, 25% MeOH in PBST and 100% PBST (4 x 5 min).

Fish, mouse and Xenopus embryos are treated with proteinaseK (10 µg/ml in PBST) for 45 min at 37 oC, refixed for 20 min in 4% paraformaldehyde in PBS and washed 3 x 5 min with PBST. After a short wash in water, endogenous alkaline phosphatase activity is blocked by incubation of the embryos in 0.1 M tri-ethanolamine and 2.5% acetic anhydride for 10 min, followed by a short wash in water and 5 x 5 min washing in PBST. The embryos are then transferred to hybridization buffer (50% Formamide, 5x SSC, 0.1% Tween, 9.2 mM citric acid, 50 ug/ml heparin, 500 ug/ml yeast RNA) for 2-3 hour at the hybridization temperature. Hybridization is performed in fresh pre-heated hybridization buffer containing 10 nM of 3' DIG-labeled LNA probe (Roche Diagnostics) complementary to each selected miRNA. Post-hybridization washes are done at the hybridization temperature by successive incubations for 15 min in HM- (hybridization buffer without heparin and yeast RNA), 75% HM-/25% 2x SSCT (SSC containing 0.1% Tween-20), 50% HM-/50% 2x SSCT, 25% HM-/75% 2x SSCT, 100% 2x SSCT and 2 x 30 min in 0.2x SSCT.

Subsequently, embryos are transferred to PBST through successive incubations for 10 min in 75% 0.2x SSCT/25% PBST, 50% 0.2x SSCT/50% PBST, 25% 0.2x SSCT/75% PBST and 100% PBST. After blocking for 1 hour in blocking buffer (2% sheep serum/2mg:ml BSA in PBST), the embryos are incubated overnight at 4 °C in blocking buffer containing anti-DIG-AP FAB fragments (Roche, 1/2000). The next day, zebrafish embryos are washed 6 x 15 min in PBST, mouse and X. tropicalis embryos are washed 6 x 1 hour in TBST containing 2 mM levamisole and then for 2 days at 4oC with regular refreshment of the wash buffer.

After the post-antibody washes, the embryos are washed 3 x 5 min in staining buffer (100 mM tris HCl pH9.5, 50 mM MgCl2, 100 mM NaCl, 0.1% tween 20). Staining was done in buffer supplied with 4.5 µl/ml NBT (Roche, 50 mg/ml stock) and 3.5 µl/ml BCIP (Roche, 50 mg/ml stock). The reaction is stopped with 1 mM EDTA in PBST and the embryos are stored at 4°C. The embryos are mounted in Murray's solution (2:1 benzylbenzoate:benzylalcohol) via an increasing methanol series (25% MeOH in PBST, 50% MeOH in PBST, 75% MeOH in PBST, 100% MeOH) prior to imaging.

### Example 10: In vitro model: Isolation and analysis of mRNA expression (total RNA isolation and cDNA synthesis for mRNA analysis)

Total RNA was isolated either using RNeasy mini kit (Qiagen) or using the Trizol reagent (Invitrogen). For total RNA isolation using RNeasy mini kit (Qiagen), cells were washed with PBS, and Cell Lysis Buffer (RTL, Qiagen) supplemented with 1% mercaptoethanol was added directly to the wells. After a few minutes, the samples were processed according to manufacturer's instructions.

For in vivo analysis of mRNA expression tissue samples were first homogenised using a Retsch 300MM homogeniser and total RNA was isolated using the Trizol reagent or the RNeasy mini kit as described by the manufacturer.

First strand synthesis (cDNA from mRNA) was performed using either OmniScript Reverse Transcriptase kit or M-MLV Reverse transcriptase (essentially described by manufacturer (Ambion)) according to the manufacturer's instructions (Qiagen). When using OmniScript Reverse Transcriptase 0.5 µg total RNA each sample, was adjusted to 12 µl and mixed with 0.2 µl poly (dT)₁₂₋₁₈ (0.5 µg/µl) (Life Technologies), 2 µl dNTP mix (5 mM each), 2 µl 10x RT buffer, 0.5 µl RNAguard™ RNase Inhibitor (33 units/ml, Amersham) and 1 µl OmniScript Reverse Transcriptase followed by incubation at 37°C for 60 min. and heat inactivation at 93°C for 5 min.

When first strand synthesis was performed using random decamers and M-MLV-Reverse Transcriptase (essentially as described by manufacturer (Ambion)) 0.25 µg total RNA of each sample was adjusted to 10.8 µl in H₂O. 2 µl decamers and 2 µl dNTP mix (2.5 mM each) was added. Samples were heated to 70°C for 3 min. and cooled immediately in ice water and added 3.25 µl of a mix containing (2 µl 10x RT buffer; 1 µl M-MLV Reverse Transcriptase; 0.25 µl RNAase inhibitor). cDNA is synthesized at 42°C for 60 min followed by heating inactivation step at 95°°C for 10 min and finally cooled to 4°C. The cDNA can further be used for mRNA quantification by for example Real-time quantitative PCR.

mRNA expression can be assayed in a variety of ways known in the art. For example, mRNA levels can be quantitated by, e.g., Northern blot analysis, competitive polymerase chain reaction (PCR), Ribonuclease protection assay (RPA) or real-time PCR. Real-time quantitative PCR is presently preferred. RNA analysis can be performed on total cellular RNA or mRNA.

Methods of RNA isolation and RNA analysis such as Northern blot analysis are routine in the art and is taught in, for example, Current Protocols in Molecular Biology, John Wiley and Sons.

Real-time quantitative (PCR) can be conveniently accomplished using the commercially available iQ Multi-Color Real Time PCR Detection System available from BioRAD. Real-time Quantitative PCR is a technique well-known in the art and is taught in for example Heid et al. Real time quantitative PCR, Genome Research (1996), 6: 986-994.

### Example 11: LNA oligonucleotide uptake and efficacy in vivo.

In vivo study: Six groups of animals (5 mice per group) were treated in the following manner. Group 1 animals were injected with 0.2ml saline by i.v. on 3 successive days, Group 2 received 2.5mg/kg SPC3372, Group 3 received 6.25 mg/kg, Group 4 received 12.5 mg/kg and Group 5 received 25 mg/kg, while Group 6 received 25 mg/kg SPC 3373 (mismatch LNA-antimiR™ oligonucleotide), all in the same manner. All doses were calculated from the Day 0 body weights of each animal.

Before dosing (Day 0) and 24 hour after last dose (Day 3), retro-orbital blood was collected in tubes containing EDTA and the plasma fraction harvested and stored frozen-80°C for cholesterol analysis. At sacrifice livers were dissected and one portion was cut into 5 mm cubes and immersed in 5 volumes of ice-cold RNAlater. A second portion was snap frozen in liquid nitrogen and stored for cryo-sectioning.

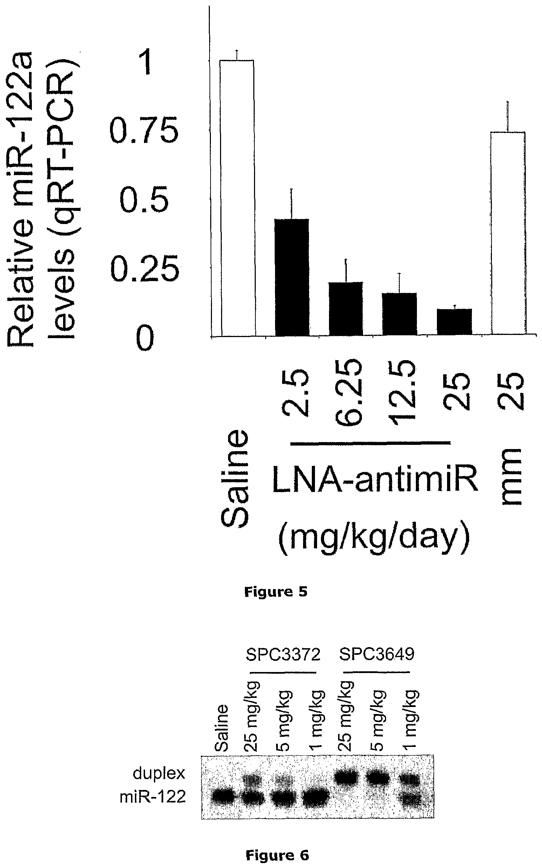

Total RNA was extracted from liver samples as described above and analysed for miR-122a levels by microRNA specific QPCR. Figure 5 demonstrates a clear dose-response obtained with SPC3372 with an IC50 at ca 3-5 mg/kg, whereas no miR-122a inhibition was detected using the mismatch LNA antago-mir SPC 3373 for miR-122a.

### Example 12: LNA-antimiR-122a dose-response in vivo in C57/BL/J female mice.

In vivo study: Ten groups of animals (female C57/BL6; 3 mice per group) were treated in the following manner. Group 1 animals were injected with 0.2ml saline by i.p. on day 0, day 2 and day 4. Groups 2-10 were dosed by i.p. with three different conc. (25 mg/kg, 5mg/kg and 1mg/kg) of either LNA antimiR-122a/SPC3372 (group 2-4), LNA antimir-122a/SPC3548 (group 5-7) or LNA antimir-122a/SPC3549 (group 8-10); the LNA antimir-122a sequences are given in the Table 1. All three LNA antimiR-122a oligonucleotides target the liver-specific miR-122a. The doses were calculated from the Day 0 body weights of each animal.

The animals were sacrificed 48 hours after last dose (Day 6), retro-orbital blood was collected in tubes containing EDTA and the plasma fraction harvested and stored frozen - 80°C for cholesterol analysis. At sacrifice livers were dissected and one portion was cut into 5 mm cubes and immersed in 5 volumes of ice-cold RNAlater. A second portion was snap frozen in liquid nitrogen and stored for cryo-sectioning.

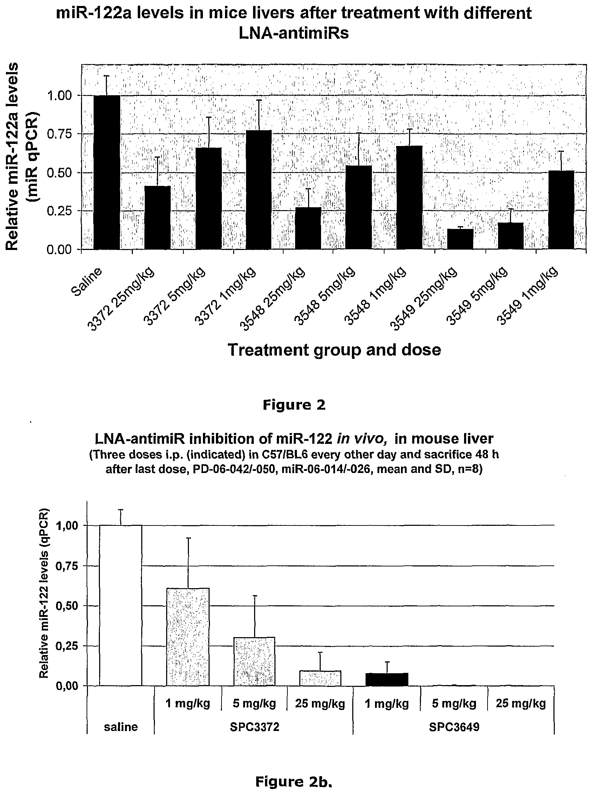

Total RNA was extracted from liver samples using Trizol reagent according to the manufacturer's recommendations (Invitrogen, USA) and analysed for miR-122a levels by microRNA-specific QPCR according to the manufacturer's recommendations (Ambion, USA). Figure 2 demonstrates a clear dose-response obtained with all three LNA antimir-122a molecules (SPC3372, SPC3548, SPC3549). Both SPC3548 and SPC3549 show significantly improved efficacy in vivo in miR-122a silencing (as seen from the reduced miR-122a levels) compared to SPC3372, with SPC3549 being most potent (IC₅₀ ca 1 mg/kg).

The above example was repeated using SPC3372 and SPC 3649 using 5 mice per group and the data combined (total of eight mice per group) is shown in Figure. 2b.

### Example 12a: Northern Blot.

MicroRNA specific northern blot showing enhanced miR-122 blocking by SPC3649 compared to SPC3372 in LNA-antimiR treated mouse livers.

Oligos used in this example:

| | |
|---|---|
| SPC3649: 5'-CcAttGTcaCaCtCC-3'(SEQ ID 539) | New design |
| SPC3372: 5'-CcAttGtcAcaCtcCa-3' (SEQ ID 586) | Old design |

We decided to assess the effect of SPC3649 on miR-122 miRNA levels in the livers of SPC3649-treated mice. The LNA-antimiRs SPC3649 and SPC3372 were administered into mice by three i.p. injections on every second day over a six-day-period at indicated doses followed by sacrificing the animals 48 hours after the last dose. Total RNA was extracted from the livers. miR-122 levels were assessed by microRNA specific northern blot (figure 6)

Treatment of normal mice with SPC3649 resulted in dramatically improved, dose-dependent reduction of miR-122. MicroRNA specific northern blot comparing SPC3649 with SPC3372 was performed (figure 6). SPC3649 completely blocked miR-122 at both 5 and 25 mg/kg as seen by the absence of mature single stranded miR-122 and only the presence of the duplex band between the LNA-antimiR and miR-122. Comparing duplex versus mature band on the northern blot SPC3649 seem equally efficient at 1 mg/kg as SPC3372 at 25 mg/kg.

### Example 13: Assessment of cholesterol levels in plasma in LNA anti-miR122 treated mice

Total cholesterol level was measured in plasma using a colometric assay Cholesterol CP from ABX Pentra. Cholesterol was measured following enzymatic hydrolysis and oxidation (2,3). 21.5µl water was added to 1.5µl plasma. 250µl reagent was added and within 5 min the cholesterol content measured at a wavelength of 540 nM. Measurements on each animal were made in duplicate. The sensitivity and linearity was tested with 2-fold diluted control compound (ABX Pentra N control). The cholesterol level was determined by subtraction of the background and presented relative to the cholesterol levels in plasma of saline treated mice.

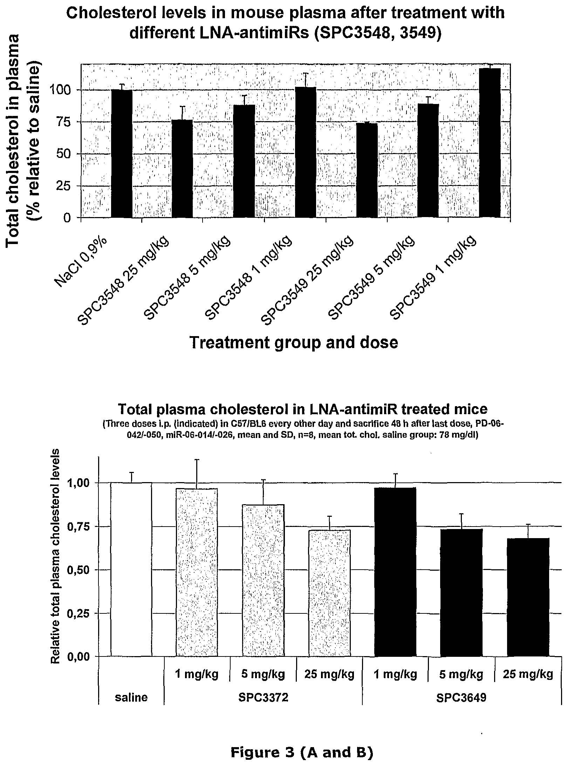

Figure 3 demonstrates a markedly lowered level of plasma cholesterol in the mice that received SPC3548 and SPC3549 compared to the saline control at Day 6.

### Example 14: Assessment of miR-122a target mRNA levels in LNA antimiR-122a treated mice

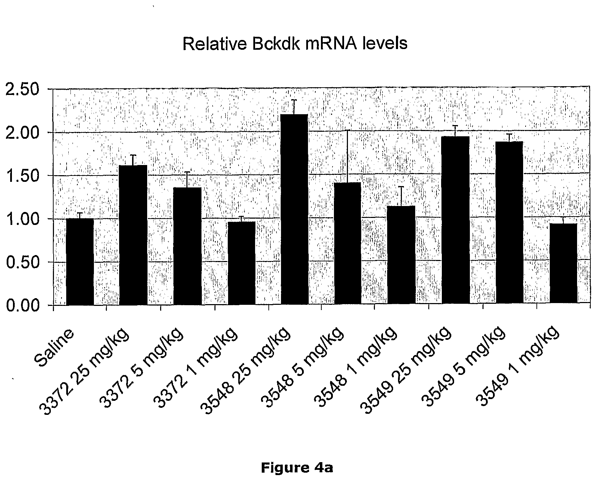

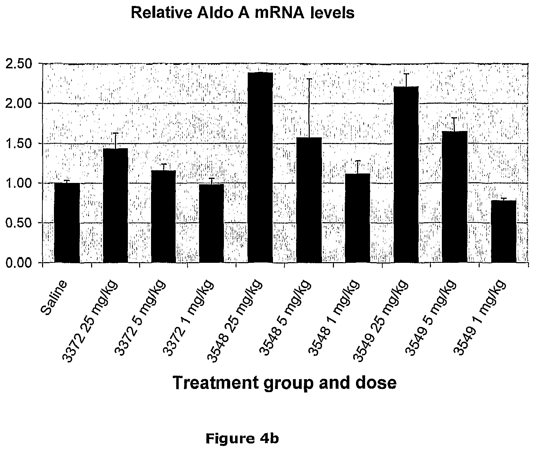

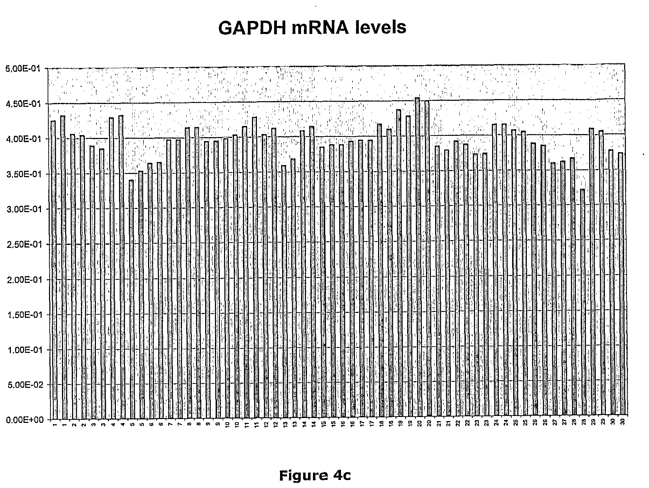

The saline control and different LNA-antimiR-122a treated animals were sacrificed 48 hours after last dose (Day 6), and total RNA was extracted from liver samples as using Trizol reagent according to the manufacturer's recommendations (Invitrogen, USA). The mRNA levels were assessed by real-time quantitative RT-PCR for two miR-122a target genes, **Bckdk** (branched chain ketoacid dehydrogenase kinase, ENSMUSG00000030802) and aldolase A (**aldoA,** ENSMUSG00000030695), respectively, as well as for GAPDH as control, using Taqman assays according to the manufacturer's instructions (Applied biosystems, USA). Figure 4a and 4b demonstrate a clear dose-dependent upregulation of the two miR-122a target genes, **Bckdk** and **AldoA,** respectively, as a response to treatment with all three LNA antimiR-122a molecules (SPC3372, SPC3548, SPC3549). In contrast, the qPCR assays for GAPDH control did not reveal any differences in the GAPD mRNA levels in the LNA-antimiR-122a treated mice compared to the saline control animals (Fig. 4c). The Bckdk and AldoA mRNA levels were significantly higher in the SPC3548 and SPC3549 treated mice compared to the SPC3372 treated mice (Fig. 4a and 4b), thereby demonstrating their improved in vivo efficacy.

### Example 15: LNA oligonucleotide duration of action in vivo.

In vivo study: Two groups of animals (21 mice per group) were treated in the following manner. Group 1 animals were injected with 0.2ml saline by i.v. on 3 successive days, Group 2 received 25mg/kg SPC3372 in the same manner. All doses were calculated from the Day 0 body weights of each animal.

After last dose (Day 3), 7 animals from each group were sacrificed on Day 9, Day 16 and Day 23, respectively. Prior to this, on each day, retro-orbital blood was collected in tubes containing EDTA and the plasma fraction harvested and stored frozen -80°C for cholesterol analysis from each day. At sacrifice livers were dissected and one portion was cut into 5 mm cubes and immersed in 5 volumes of ice-cold RNAlater. A second portion was snap frozen in liquid nitrogen and stored for cryo-sectioning.

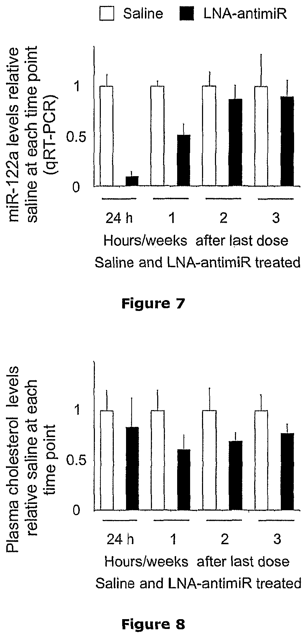

Total RNA was extracted from liver samples as described above and analysed for miR-122a levels by microRNA specific QPCR. Figure 7 (Sacrifice day 9, 16 or 23 correspond to sacrifice 1, 2 or 3 weeks after last dose) demonstrates a two-fold inhibition in the mice that received SPC3372 compared to the saline control, and this inhibition could still be detected at Day 16, while by Day 23 the mi122a levels approached those of the saline group.

### Example 16: LNA oligonucleotide duration of action in vivo.

In vivo study: Two groups of animals (21 mice per group) were treated in the following manner. Group 1 animals were injected with 0.2ml saline by i.v. on 3 successive days, Group 2 received 25mg/kg SPC3372 in the same manner. All doses were calculated from the Day 0 body weights of each animal.

After last dose (Day 3), 7 animals from each group were sacrificed on Day 9, Day 16 and Day 23, respectively. Prior to this, on each day, retro-orbital blood was collected in tubes containing EDTA and the plasma fraction harvested and stored frozen -80°C for cholesterol analysis from each day. At sacrifice livers were dissected and one portion was cut into 5 mm cubes and immersed in 5 volumes of ice-cold RNAlater. A second portion was snap frozen in liquid nitrogen and stored for cryo-sectioning.

Total RNA was extracted from liver samples as described above and analysed for miR-122a levels by microRNA specific QPCR. Figure 8 demonstrates a two-foldinhibition in the mice that received SPC3372 compared to the saline control, and this inhibition could still be detected at Day 16, while by Day23 the miR-122a levels approachied those of the saline group.

### As to examples 17-22, the following procedures apply:

NMRI mice were administered intravenously with SPC3372 using daily doses ranging from 2.5 to 25 mg/kg for three consecutive days. Animals were sacrificed 24 hours, 1, 2 or 3 weeks after last dose. Livers were harvested divided into pieces and submerged in RNAlater (Ambion) or snap-frozen. RNA was extracted with Trizol reagent according to the manufacturer's instructions (Invitrogen) from the RNAlater tissue, except that the precipitated RNA was washed in 80% ethanol and not vortexed. The RNA was used for mRNA TaqMan qPCR according to manufacturer (Applied biosystems) or northern blot (see below).The snap-frozen pieces were cryo-sectioned for in situ hybridizations.

Further, as to figures 9-14, SPC3372 is designated LNA-antimiR and SPC3373 (the mismatch control) is designated "mm" instead of using the SPC number.

### Example 17: Dose dependent miR-122a target mRNA induction by SPC3372 inhibition of miR-122a

Mice were treated with different SPC3372 doses for three consecutive days, as described above and sacrificed 24 hours after last dose. Total RNA extracted from liver was subjected to qPCR. Genes with predicted miR-122 target site and observed to be upregulated by microarray analysis were investigated for dose-dependent induction by increasing SPC3372 doses using qPCR. Total liver RNA from 2 to 3 mice per group sacrificed 24 hours after last dose were subjected to qPCR for the indicated genes. Shown in figure 9 is mRNA levels relative to Saline group, n=2-3 (2.5 - 12.5 mg/kg/day: n=2, no SD). Shown is also the mismatch control (mm, SPC3373).

Assayed genes: Nrdg3 Aldo A, Bckdk, CD320 with predicted miR-122 target site. Aldo B and Gapdh do not have a predicted miR-122a target site.

A clear dose-dependent induction was seen of the miR-122a target genes after treatment with different doses of SPC3372.

### Example 18: Transient induction of miR-122a target mRNAs following SPC3372 treatment

NMRI female mice were treated with 25 mg/kg/day SPC3372 along with saline control for three consecutive days and sacrificed 1, 2 or 3 weeks after last dose, respectively. RNA was extracted from livers and mRNA levels of predicted miR-122a target mRNAs, selected by microarray data were investigated by qPCR. Three animals from each group were analysed.

Assayed genes: Nrdg3 Aldo A, Bckdk, CD320 with predicted miR-122 target site. Gapdh does not have a predicted miR-122a target site.
A transient induction followed by a restoration of normal expression levels in analogy with the restoration of normal miR-122a levels was seen (figure 10).

mRNA levels are normalized to the individual GAPDH levels and to the mean of the Saline treated group at each individual time point. Included are also the values from the animals sacrificed 24 hours after last dose. Shown is mean and standard deviation, n=3 (24h n=3)

### Example 19: Induction of Vldlr in liver by SPC3372 treatment

The same liver RNA samples as in previous example were investigated for Vldlr induction.

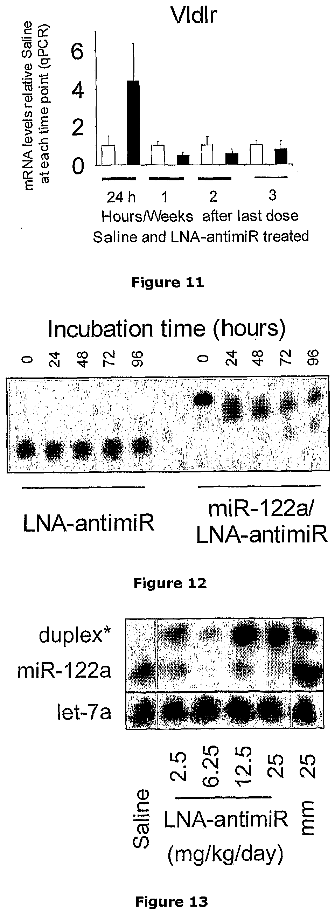

A transient up-regulation was seen after SPC3372 treatment, as with the other predicted miR-122a target mRNAs (figure 11)

### Example 20: Stability of miR-122a/ SPC3372 duplex in mouse plasma

Stability of SPC3372 and SPC3372/miR-122a duplex were tested in mouse plasma at 37°C over 96 hours. Shown in figure 12 is a SYBR-Gold stained PAGE.

SPC3372 was completely stable over 96 hours. The SPC3372/miR-122a duplex was immediately truncated (degradation of the single stranded miR-122a region not covered by SPC3372) but thereafter almost completely stable over 96 hours.

The fact that a preformed SPC3372/miR-122 duplex showed stability in serum over 96 hours together with the high thermal duplex stability of SPC3372 molecule supported our notion that inhibition of miR-122a by SPC3372 was due to stable duplex formation between the two molecules, which has also been reported in cell culture (Naguibneva et al. 2006).

### Example 21: Sequestering of mature miR-122a by SPC3372 leads to duplex formation

The liver RNA was also subjected to microRNA Northern blot. Shown in figure 13 is a membrane probed with a miR-122a specific probe (upper panel) and re-probed with a Let-7 specific probe (lower panel). With the miR-122 probe, two bands could be detected, one corresponding to mature miR-122 and one corresponding to a duplex between SPC3372 and miR-122.

To confirm silencing of miR-122, liver RNA samples were subjected to small RNA northern blot analysis, which showed significantly reduced levels of detectable mature miR-122, in accordance with our real-time RT-PCR results. By comparison, the levels of the let-7a control were not altered. Interestingly, we observed dose-dependent accumulation of a shifted miR-122/ SPC3372 heteroduplex band, suggesting that SPC3372 does not target miR-122 for degradation, but rather binds to the microRNA, thereby sterically hindering its function.

Northern blot analysis was performed as follows:
Preparation of northern membranes was done as described in Sempere et al. 2002, except for the following changes: Total RNA, 10 µg per lane, in formamide loading buffer (47.5% formamide, 9 mM EDTA, 0.0125% Bromophenol Blue, 0.0125% Xylene Cyanol, 0.0125% SDS) was loaded onto a 15% denaturing Novex TBE-Urea polyacrylamide gel (Invitrogen) without preheating the RNA. The RNA was electrophoretically transferred to a GeneScreen plus Hybridization Transfer Membrane (PerkinElmer) at 200 mA for 35 min. Membranes were probed with 32P-labelled LNA-modified oligonucleotides complimentary to the mature microRNAs*. The LNA oligonucleotides were labelled and hybridized to the membrane as described in (Válóczi et al. 2004) except for the following changes: The prehybridization and hybridization solutions contained 50% formamide, 0.5% SDS, 5x SSC, 5x Denhardt's solution and 20 µg/ml sheared denatured herring sperm DNA. Hybridizations were performed at 45°C. The blots were visualized by scanning in a Storm 860 scanner. The signal of the background membrane was subtracted from the radioactive signals originating from the miRNA bands. The values of the miR-122 signals were corrected for loading differences based on the let-7a signal. To determine the size of the radioactive signals the Decade Marker System (Ambion) was used according to the suppliers' recommendations.

### Example 22: miR-122a sequestering by SPC3372 along with SPC3372 distribution assessed by in situ hybridization of liver sections

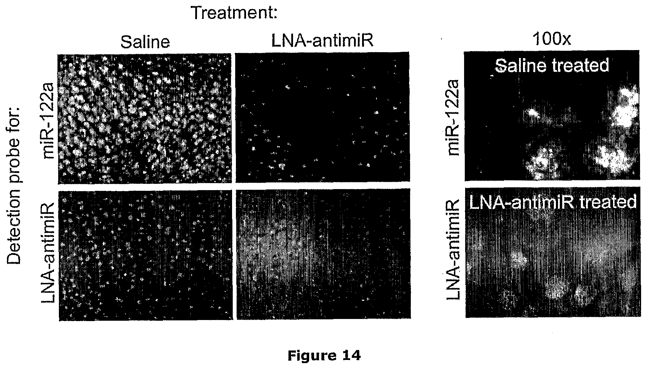

Liver cryo-sections from treated animals were subjected to in situ hybridizations for detection and localization of miR-122 and SPC3372 (figure 14). A probe complementary to miR-122 could detect miR-122a. A second probe was complementary to SPC3372. Shown in figure 14 is an overlay, in green is distribution and apparent amounts of miR-122a and SPC3372 and blue is DAPI nuclear stain, at 10x magnification. 100x magnifications reveal the intracellular distribution of miR-122a and SPC3372 inside the mouse liver cells. The liver sections from saline control animals showed a strong miR-122 staining pattern over the entire liver section, whereas the sections from SPC3372 treated mice showed a significantly reduced patchy staining pattern. In contrast, SPC3372 molecule was readily detected in SPC3372 treated liver, but not in the untreated saline control liver. Higher magnification localized miR-122a to the cytoplasm in the hepatocytes, where the miR-122 in situ pattern was clearly compartmentalized, while SPC3372 molecule was evenly distributed in the entire cytoplasm.

### Example 23: Micro Array Analysis

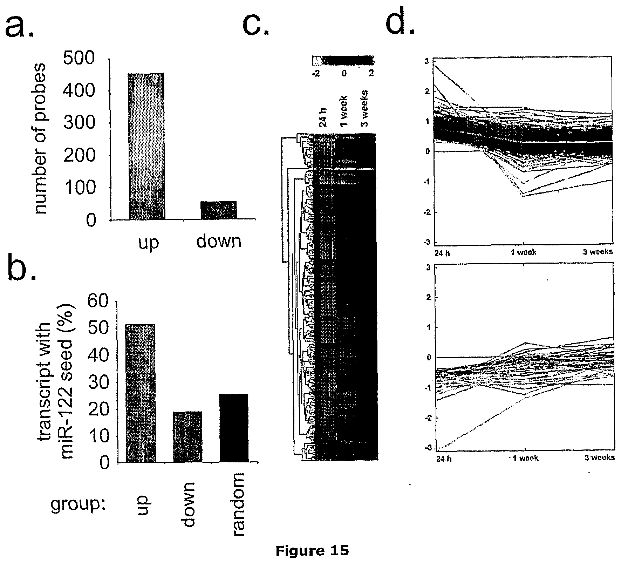

We carried out genome-wide expression profiling of total RNA samples from saline LNA-antimiR-122 treated and LNA mismatch control treated mice livers 24 hours after the last dose using Affymetrix Mouse Genome 430 2.0 arrays. Analysis of the array data revealed 455 transcripts that were upregulated in the LNA-antimiR treated mice livers compared to saline and LNA mismatch controls, while 54 transcripts were downregulated (**Fig. 15a**). A total of 415 of the upregulated and 53 downregulated transcripts could be identified in the Ensembl database. We subsequently examined the 3' untranslated regions (UTRs) of the differentially expressed mRNAs for the presence of the 6 nt sequence CACTCC, corresponding to the reverse complement of the nucleotide 2-7 seed region in mature miR-122. The number of transcripts having at least one miR-122 recognition sequence was 213 (51 %) among the upregulated transcripts, and 10 (19 %) within the downregulated transcripts, while the frequency in a random sequence population was 25 %, implying that a significant pool of the upregulated mRNAs represent direct miR-122 targets in the liver (Fig. 15b).
The LNA-antimiR treatment showed maximal reduction of miR-122 levels at 24 hours, 50% reduction at one week and matched saline controls at three weeks after last LNA dose (Example 12 "old design"). This coincided with a markedly reduced number of differentially expressed genes between the two mice groups at the later time points. Compared to the 509 mRNAs 24 hours after the last LNA dose we identified 251 differentially expressed genes after one week, but only 18 genes after three weeks post treatment (**Fig. 15c and 15d**). In general genes upregulated 24 hours after LNA-antimiR treatment then reverted towards control levels over the next two weeks (Fig. 15d).

In conclusion, a large portion of up-regulated/de-repressed genes after LNA-antimiR treatement are miR-122 targets, indicating a very specific effect for blocking miR-122. Also genes up-regulated/de-repressed approach normal levels 3 weeks after end of treatment, suggest a relative long therapeutic effect, but however not cause a permanent alteration, ie the effect is reversible.

### METHODS:

Gene expression profiling of LNA-antimiR treated mice.
Expression profiles of livers of saline and LNA-antimiR treated mice were compared. NMRI female mice were treated with 25 mg/kg/day of LNA-antimiR along with saline control for three consecutive days and sacrificed 24 h, 1, 2 or 3 weeks after last dose. Additionally, expression profiles of livers of mice treated with the mismatch LNA control oligonucleotide 24 h after last dose were obtained. Three mice from each group were analyzed, yielding a total of 21 expression profiles. RNA quality and concentration was measured using an Agilent 2100 Bioanalyzer and Nanodrop ND-1000, respectively. Total RNA was processed following the GeneChip Expression 3'-Amplification Reagents One-cycle cDNA synthesis kit instructions (Affymetrix Inc, Santa Clara, CA, USA) to produce double-stranded cDNA. This was used as a template to generate biotin-labeled cRNA following manufacturer's specifications. Fifteen micrograms of biotin-labeled cRNA was fragmented to strands between 35 and 200 bases in length, of which 10 micrograms were hybridised onto Affymetrix Mouse Genome 430 2.0 arrays overnight in the GeneChip Hybridisation oven 6400 using standard procedures. The arrays were washed and stained in a GeneChip Fluidics Station 450. Scanning was carried out using the GeneChip Scanner 3000 and image analysis was performed using GeneChip Operating Software. Normalization and statistical analysis were done using the LIMMA software package for the R programming environment 27. Probes reported as absent by GCOS software in all hybridizations were removed from the dataset. Additionally, an intensity filter was applied to the dataset to remove probes displaying background-corrected intensities below 16. Data were normalized using quantile normalization28. Differential expression was assessed using a linear model method. P values were adjusted for multiple testing using the Benjamini and Hochberg. Tests were considered to be significant if the adjusted p values were p<0.05. Clustering and visualization of Affymetrix array data were done using the MultiExperiment Viewer software29.

### Target site prediction

Transcripts with annotated 3' UTRs were extracted from the Ensembl database (Release 41) using the EnsMart data mining tool30 and searched for the presence of the CACTCC sequence which is the reverse complement of the nucleotide 2-7 seed in the mature miR-122 sequence. As a background control, a set of 1000 sequences with a length of 1200 nt, corresponding to the mean 3' UTR length of the up- and downregulated transcripts at 24 h after last LNA-antimiR dose, were searched for the 6 nucleotide miR-122 seed matches. This was carried out 500 times and the mean count was used for comparison

### Example 24. Dose-dependent inhibition of miR-122 in mouse liver by LNA-antimiR is enhanced as compared to antagomir inhibition of miR-122.

NMRI female mice were treated with indicated doses of LNA-antimiR (SPC3372) along with a mismatch control (mm, SPC3373), saline and antagomir (SPC3595) for three consecutive days and sacrificed 24 hours after last dose **(as in example 11 "old design", n=5).** miR-122 levels were analyzed by qPCR and normalized to the saline treated group. Genes with predicted miR-122 target site and up regulated in the expression profiling (AldoA, Nrdg3, Bckdk and CD320) showed dose-dependent de-repression by increasing LNA-antimiR doses measured by qPCR.

The de-repression was consistently higher on all tested miR-122 target mRNAs (AldoA, Bckdk, CD320 and Nrdg3 **figure 17****,** **18****,** **19****,** **20****)** in LNA-antimiR treated mice compared to antagomir treated mice. This was also indicated when analysing the inhibition of miR-122 by miR-122 specific qPCR **(****figure 16****).** Hence LNA-antimiRs give a more potent functional inhibition of miR-122 than corresponding dose antagomir.

### Example 25. Inhibition of miR-122 by LNA-antimiR in hypercholesterolemic mice along with cholesterol reduction and miR-122 target mRNA de-repression.

C57BL/6J female mice were fed on high fat diet for 13 weeks before the initiation of the SPC3649 treatment. This resulted in increased weight to 30-35 g compared to the weight of normal mice, which was just under 20 g, as weighed at the start of the LNA-antimiR treatment. The high fat diet mice lead to significantly increased total plasma cholesterol level of about 130 mg/dl, thus rendering the mice hypercholesterolemic compared to the normal level of about 70 mg/dl. Both hypercholesterolemic and normal mice were treated i.p. twice weekly with 5 mg/kg SPC3649 and the corresponding mismatch control SPC3744 for a study period of 5 1/2 weeks. Blood samples were collected weekly and total plasma cholesterol was measured during the entire course of the study. Upon sacrificing the mice, liver and blood samples were prepared for total RNA extraction, miRNA and mRNA quantification, assessment of the serum transaminase levels, and liver histology.

Treatment of hypercholesterolemic mice with SPC3649 resulted in reduction of total plasma cholesterol of about 30 % compared to saline control mice already after 10 days and sustained at this level during the entire study (Figure 21). The effect was not as pronounced in the normal diet mice. By contrast, the mismatch control SPC3744 did not affect the plasma cholesterol levels in neither hypercholesterolemic nor normal mice.

Quantification of miR-122 inhibition and miR-122 target gene mRNA de-repression (AldoA and Bckdk) after the long-term treatment with SPC3649 revealed a comparable profile in both hypercholesterolemic and normal mice (Figure 22, 23, 24), thereby demonstrating the potency of SPC3649 in miR-122 antagonism in both animal groups. The miR-122 qPCR assay indicated that also the mismatch control SPC3744 had an effect on miR-122 levels in the treated mice livers, albeit to a lesser extent compared to SPC3649. This might be a reduction associated with the stem-loop qPCR. Consistent with this notion, treatment of mice with the mismatch control SPC3744 did not result in any functional de-repression of the direct miR-122 target genes (Figure 23 and 24) nor reduction of plasma cholesterol (Figure 21), implying that SPC3649-mediated antagonism of miR-122 is highly specific *in vivo.*

Liver enzymes in hypercholesterolemic and normal mice livers were assessed after long term SPC3649 treatment. No changes in the alanine and aspartate aminotransferase (ALT and AST) levels were detected in the SPC3649 treated hypercholesterolemic mice compared to saline control mice (Figure 25 and 26). A possibly elevated ALT level was observed in the normal mice after long-term treatment with SPC3649 (Figure 26).

### Exmaple 26 Methods for performing the LNA-antimiR/hypercholesterolemic experiment and analysis:

### Mice and dosing.

C57BL/6J female mice (Taconic M&B Laboratory Animals, Ejby, Denmark) were used. All substances were formulated in physiological saline (0.9 % NaCl) to final concentration allowing the mice to receive an intraperitoneal injection volume of 10 ml/kg.
In the diet induced obesity study, the mice received a high fat (60EN%) diet (D12492, Research Diets) for 13 weeks to increase their blood cholesterol level before the dosing started. The dose regimen was stretched out to 5 1/2 weeks of 5 mg/kg LNA-antimiR™ twice weekly. Blood plasma was collected once a week during the entire dosing period. After completion of the experiment the mice were sacrificed and RNA extracted from the livers for further analysis. Serum was also collected for analysis of liver enzymes.

### Total RNA extraction.

The dissected livers from sacrificed mice were immediately stored in RNA later (Ambion). Total RNA was extracted with Trizol reagent according to the manufacturer's instructions (Invitrogen), except that the precipitated RNA pellet was washed in 80% ethanol and not vortexed.

### MicroRNA-specific quantitative RT-PCR.

The miR-122 and let-7a microRNA levels were quantified with TaqMan microRNA Assay (Applied Biosystems) following the manufacturer's instructions. The RT reaction was diluted ten times in water and subsequently used for real time PCR amplification according to the manufacturer's instructions. A two-fold cDNA dilution series from liver total RNA of a saline-treated animal or mock transfected cells cDNA reaction (using 2.5 times more total RNA than in samples) served as standard to ensure a linear range (Ct versus relative copy number) of the amplification. Applied Biosystems 7500 or 7900 real-time PCR instrument was used for amplification.

### Quantitative RT-PCR

mRNA quantification of selected genes was done using standard TaqMan assays (Applied Biosystems). The reverse transcription reaction was carried out with random decamers, 0.5 µg total RNA, and the M-MLV RT enzyme from Ambion according to a standard protocol. First strand cDNA was subsequently diluted 10 times in nuclease-free water before addition to the RT-PCR reaction mixture. A two-fold cDNA dilution series from liver total RNA of a saline-treated animal or mock transfected cells cDNA reaction (using 2.5 times more total RNA than in samples) served as standard to ensure a linear range (Ct versus relative copy number) of the amplification. Applied Biosystems 7500 or 7900 real-time PCR instrument was used for amplification.

### Metabolic measurements.

Immediately before sacrifice retro-orbital sinus blood was collected in EDTA-coated tubes followed by isolation of the plasma fraction. Total plasma cholesterol was analysed using ABX Pentra Cholesterol CP (Horiba Group, Horiba ABX Diagnostics) according to the manufacturer's instructions.

### Liver enzymes (ALT and AST) measurement

Serum from each individual mouse was prepared as follows: Blood samples were stored at room temperature for 2 h before centrifugation (10 min, 3000 rpm at room temperature). After centrifugation, serum was harvested and frozen at -20 °C.

ALT and AST measurement was performed in 96-well plates using ALT and AST reagents from ABX Pentra according to the manufacturer's instructions. In short, serum samples were diluted 2.5 fold with H₂O and each sample was assayed in duplicate. After addition of 50 µl diluted sample or standard (multical from ABX Pentra) to each well, 200 µl of 37 °C AST or ALT reagent mix was added to each well. Kinetic measurements were performed for 5 min with an interval of 30s at 340 nm and 37 °C using a spectrophotometer.

### Example 27 Modulation of Hepatitis C replication by LNA-antimiR (SPC3649)

Oligos used in this example (uppercase: LNA, lowercase DNA, LNA Cs are methyl - **^{m}c,** and LNAs are preferably B-D-oxy (o subscript after LNA residue e.g. **cₛ^{o}**):

| |
|---|
| **SPC3649** (LNA-antimiR targeting miR-122, |
| was in the initial small scale synthesis designated SPC3549) SEQ ID 558 |
| 5'-**^{m}Cₛ^{o}**cₛ**Aₛ^{o}**tₛtₛ**Gₛ^{o}Tₛ^{o}**cₛaₛ**^{m}Cₛ^{o}**aₛ**^{m}Cₛ^{o}**tₛ**^{m}Cₛ^{om}C^{o}**-3' |
| **SPC3648** (LNA-antimiR targeting miR-122, |
| was in the initial small scale synthesis designated SPC3548) |
| 5'-**Aₛ^{o}**tₛtₛ**Gₛ^{o}Tₛ^{o}**cₛaₛ**^{m}Cₛ^{o}**aₛ**^{m}Cₛ^{o}**tₛ**^{m}Cₛ^{om}C^{o}**-3' |
| **SPC3550** (4 nt mismatch control to SPC3649) SEQ ID 592 |
| 5'-**^{m}Cₛ^{o}**cₛ**Aₛ^{o}**tₛtₛ**^{m}Cₛ^{o}Tₛ^{o}**gₛaₛ**^{m}Cₛ^{o}**cₛ**^{m}**cₛ**^{o}**tₛ**Aₛ^{om}C^{o}**-3' |
| **2'OMe anti-122:** full length (23 nt) 2'OMe modified oligo complementary to miR-122 |
| **2'OMe Ctrl:** scrambled 2'OMe modified control |

Hepatitis C (HCV) replication has been shown to be facilitated by miR-122 and consequently, antagonizing miR-122 has been demonstrated to affect HCV replication in a hepatoma cell model in vitro. We assess the efficacy of SPC3649 reducing HCV replication in the Huh-7 based cell model.. The different LNA-antimiR molecules along with a 2' OMe antisense and scramble oligonucleotide are transfected into Huh-7 cells, HCV is allowed to replicate for 48 hours. Total RNA samples extracted from the Huh-7 cells are subjected to Northern blot analysis.

A significant reduction of HCV RNA was observed in cells treated with SPC3649 as compared to the mock and SPC3550 mismatch control. The inhibition was clearly dose-dependent with both SPC3649 and SPC3648. Interestingly, using a 2'OMe oligonucleotide fully complementary to miR-122 at 50 nM was much less efficient than SPC3649 at the same final concentration. Notably, the 13 nt SPC3648 LNA-antimiR showed comparable efficacy with SPC3649.

### Example 28 Enhanced LNA-antimiR™ antisense oligonucleotide targeting miR-21

### Mature miR-21 sequence from Sanger Institute miRBase:

>hsa-miR-21 MIMAT0000076
UAGCUUAUCAGACUGAUGUUGA (SEQ ID NO 565)
>mmu-miR-21 MIMAT0000530
UAGCUUAUCAGACUGAUGUUGA (SEQ ID NO 593)

### Sequence of Compounds:

| | |
|---|---|
| SPC3521 | miR-215'-FAM TCAgtctgataaGCTa-3' (gap-mer design) - (SEQ ID NO 594) |
| SPC3870 | miR-21(mm) 5'-FAM TCCgtcttagaaGATa-3' - (SEQ ID NO 595) |
| SPC3825 | miR-21 5'-FAM TcTgtCAgaTaCgAT-3' (new design) (SEQ ID NO 596) |
| SPC3826 | miR-21(mm) 5'-FAM TcAgtCTgaTaAgCT-3'- (SEQ ID NO 597) |
| SPC3827 | miR-215'-FAM TcAGtCTGaTaAgCT-3' (new, enhanced design) - (SEQ ID NO 598) |

All compounds preferably have a fully or almost fully thiolated backbone (preferably fully) and have here also a FAM label in the 5' end (optional).

miR-21 has been show to be up-regulated in both glioblastoma (Chan et al. Cancer Research 2005, 65 (14), p6029) and breast cancer (Iorio et al. Cancer Research 2005, 65 (16), p7065) and hence has been considered a potential 'oncogenic' microRNA. Chan *et al.* also show induction of apoptosis in glioblastoma cells by antagonising miR-21 with 2'OMe or LNA modified antisense oligonucleotides. Hence, agents antagonising miR-21 have the potential to become therapeutics for treatment of glioblastoma and other solid tumours, such as breast cancer. We present an enhanced LNA modified oligonucleotide targeting miR-21, an LNA-antimiR™, with surprisingly good properties to inhibit miR-21 suited for the abovementioned therapeutic purposes.

Suitable therapeutic administration routes are, for example, intracranial injections in glioblastomas, intratumoural injections in glioblastoma and breast cancer, as well as systemic delivery in breast cancer

### Inhibition of miR-21 in U373 glioblastoma cell line and MCF-7 breast cancer cell line.

Efficacy of current LNA-anitmiR™ is assessed by transfection at different concentrations, along with control oligonucleotides, into U373 and MCF-7 cell lines known to express miR-21 (or others miR-21 expressing cell lines as well). Transfection is performed using standard Lipofectamine2000 protocol (Invitrogen). 24 hours post transfection, the cells are harvested and total RNA extracted using the Trizol protocol (Invitrogen). Assessment of miR-21 levels, depending on treatment and concentration used is done by miR-21 specific, stem-loop real-time RT-PCR (Applied Biosystems), or alternatively by miR-21 specific non-radioactive northern blot analyses. The detected miR-21 levels compared to vehicle control reflects the inhibitory potential of the LNA-antimiR™.

### Functional inhibition of miR-21 by assessment of miR-21 target gene up-regulation.

The effect of miR-21 antagonism is investigated through cloning of the perfect match miR-21 target sequence behind a standard *Renilla* luciferase reporter system (between coding sequence and 3' UTR, psiCHECK-2, Promega) - see Example 29. The reporter construct and LNA-antimiR™ will be co-transfected into miR-21 expressing cell lines (f. ex. U373, MCF-7). The cells are harvested 24 hours post transfection in passive lysis buffer and the luciferase activity is measured according to a standard protocol (Promega, Dual Luciferase Reporter Assay System). The induction of luciferase activity is used to demonstrate the functional effect of LNA-antimiR™ antagonising miR-21.

### Example 29: Luciferase reporter assay for assessing functional inhibition of microRNA by LNA-antimiRs and other microRNA targeting oligos: Generalisation of new and enhanced new design as preferred design for blocking microRNA function

Oligos used in this example (uppercase: LNA, lowercase: DNA) to assess LNA-antimiR de-repressing effect on luciferase reporter with microRNA target sequence cloned by blocking respective microRNA :

| **target: hsa-miR-122a MIMAT0000421** | |
|---|---|
| **uggagugugacaaugguguuugu** | |
| screened in HUH-7 cell line expressing miR-122 | |

| **Oligo #, target microRNA, oligo sequence** | **Design** |
|---|---|
| 3962: miR-122 5'-ACAAacaccattgtcacacTCCA-3' | Full complement, gap |
| 3965: miR-122 5'-acaaacACCATTGTcacactcca-3' | Full complement, block |
| 3972: miR-122 5'-acAaaCacCatTgtCacActCca-3' | Full complement, LNA_3 |
| 3549 (3649):miR-122 5'-CcAttGTcaCaCtCC-3' | New design |
| 3975: miR-122 5'-CcAtTGTcaCACtCC-3' | Enhanced new design |
| | |

| **target: hsa-miR-19b MIMAT0000074** | |
|---|---|
| **ugugcaaauccaugcaaaacuga** | |
| screened HeLa cell line expressing miR-19b | |

| **Oligo #, target microRNA, oligo sequence** | **Design** |
|---|---|
| 3963: miR-19b 5'-TCAGttttgcatggatttgCACA-3' | Full complement, gap |
| 3967: miR-19b 5'-tcagttTTGCATGGatttgcaca-3' | Full complement, block |
| 3973: miR-19b 5'-tcAgtTttGcaTggAttTgcAca-3' | Full complement, LNA_3 |
| 3560: miR-19b 5'-TgCatGGatTtGcAC-3' | New design |
| 3976: miR-19b 5'-TgCaTGGatTTGcAC-3' | Enhanced new design |
| | |

| **target: hsa-miR-155 MIMAT0000646** | |
|---|---|
| **uuaaugcuaaucgugauagggg** | |
| screen in 518A2 cell line expressing miR-155 | |

| **Oligo #, target microRNA, oligo sequence** | **Design** |
|---|---|
| 3964: miR-155 5'-CCCCtatcacgattagcaTTAA-3' | Full complement, gap |
| 3968: miR-155 5'-cccctaTCACGATTagcattaa-3' | Full complement, block |
| 3974: miR-155 5'-cCccTatCacGatTagCatTaa-3' | Full complement, LNA_3 |
| 3758: miR-155 5'-TcAcgATtaGcAtTA-3' | New design |
| 3818: miR-155 5'-TcAcGATtaGCAtTA-3' | Enhanced new design |

| | |
|---|---|
| SEQ ID NOs as before. | |

A reporter plasmid (psiCheck-2 Promega) encoding both the Renilla and the Firefly variants of luciferase was engineered so that the 3'UTR of the Renilla luciferase includes a single copy of a sequence fully complementary to the miRNA under investigation.
Cells endogenously expressing the investigated miRNAs (HuH-7 for miR-122a, HeLa for miR-19b, 518A2 for miR-155) were co-transfected with LNA-antimiRs or other miR binding oligonucleotides (the full complementary ie full length) and the corresponding microRNA target reporter plasmid using Lipofectamine 2000 (Invitrogen). The transfection and measurement of luciferase activity were carried out according to the manufacturer's instructions (Invitorgen Lipofectamine 2000/Promega Dual-luciferase kit) using 150 000 to 300 000 cells per well in 6-well plates. To compensate for varying cell densities and transfection efficiencies the Renilla luciferase signal was normalized with the Firefly luciferase signal. All experiments were done in triplicate.

Surprisingly, new design and new enhanced design were the best functional inhibitors for all three microRNA targets, miR-155, miR-19b and miR-122 (figure 27, 28, 29). The results are summarized in following table 3.

### Result summary:

**Table 3. Degree of de-repression of endogenous miR-155, miR-19b and miR-122a function by various designs of LNA-antimiR's.**

| **Design** | **miR-155** | **miR-19b** | **miR-122a** |
|---|---|---|---|
| **New enhanced design** | *** | *** | no data |
| **New design** | *** | *** | *** |
| Full complement, LNA_3 | ** | *** | ** |
| Full complement, block | ** | ** | ** |
| Full complement, gap | * | not signif. | not signif. |

### Example 30: Design of a LNA antimiR library for all human microRNA sequences in miRBase microRNA database version 8.1, Griffiths-Jones, S., Grocock, R.J., van Dongen, S., Bateman, A., Enright, A.J. 2006. miRBase: microRNA sequences, targets and gene nomenclature. Nucleic Acids Res. 34: D140-4 (http://microrna.sanger.ac.uk/sequences/index.shtml).

LNA nucleotides are shown in uppercase letters, DNA nucleotides in lowercase letters, LNA C nucleotides denote LNA methyl-C (mC). The LNA-antimiR oligonucleotides can be conjugated with a variety of haptens or fluorochromes for monitoring uptake into cells and tissues using standard methods.

**Table 2 (SEQ ID refers to Example antimiR)**

| **microRNA** | **Accession nr.** | **SEQ ID NO** | **Example LNA antimiR 5'-3'** |
|---|---|---|---|
| hsa-let-7a | MIMAT0000062 | SEQ ID NO 1 | AcAacCTacTaCcTC |
| hsa-let-7b | MIMAT0000063 | SEQ ID NO 2 | AcAacCTacTaCcTC |
| hsa-let-7c | MIMAT0000064 | SEQ ID NO 3 | AcAacCTacTaCcTC |
| hsa-let-7d | MIMAT0000065 | SEQ ID NO 4 | GcAacCTacTaCcTC |
| hsa-let-7e | MIMAT0000066 | SEQ ID NO 5 | AcAacCTccTaCcTC |
| hsa-let-7f | MIMAT0000067 | SEQ ID NO 6 | AcAatCTacTaCcTC |
| hsa-miR-15a | MIMAT0000068 | SEQ ID NO 7 | CcAttATgtGcTgCT |
| hsa-miR-16 | MIMAT0000069 | SEQ ID NO 8 | TaTttACgtGcTgCT |
| hsa-miR-17-5p | MIMAT0000070 | SEQ ID NO 9 | CaCtgTAagCaCtTT |
| hsa-miR-17-3p | MIMAT0000071 | SEQ ID NO 10 | GtGccTTcaCtGcAG |
| hsa-miR-18a | MIMAT0000072 | SEQ ID NO 11 | CaCtaGAtgCaCcTT |
| hsa-miR-19a | MIMAT0000073 | SEQ ID NO 12 | TgCatAGatTtGcAC |
| hsa-miR-19b | MIMAT0000074 | SEQ ID NO 13 | TgCatGGatTtGcAC |
| hsa-miR-20a | MIMAT0000075 | SEQ ID NO 14 | CaCtaTAagCaCtTT |
| hsa-miR-21 | MIMAT0000076 | SEQ ID NO 15 | TcAgtCTgaTaAgCT |
| hsa-miR-22 | MIMAT0000077 | SEQ ID NO 16 | CtTcaACtgGcAgCT |
| hsa-miR-23a | MIMAT0000078 | SEQ ID NO 17 | TcCctGGcaAtGtGA |
| hsa-miR-189 | MIMAT0000079 | SEQ ID NO 18 | TcAgcTCagTaGgCA |
| hsa-miR-24 | MIMAT0000080 | SEQ ID NO 19 | CtGctGAacTgAgCC |
| hsa-miR-25 | MIMAT0000081 | SEQ ID NO 20 | CgAgaCAagTgCaAT |
| hsa-miR-26a | MIMAT0000082 | SEQ ID NO 21 | TcCtgGAttAcTtGA |
| hsa-miR-26b | MIMAT0000083 | SEQ ID NO 22 | TcCtgAAttAcTtGA |
| hsa-miR-27a | MIMAT0000084 | SEQ ID NO 23 | AcTtaGCcaCtGtGA |
| hsa-miR-28 | MIMAT0000085 | SEQ ID NO 24 | AgActGTgaGcTcCT |
| hsa-miR-29a | MIMAT0000086 | SEQ ID NO 25 | AtTtcAGatGgTgCT |
| hsa-miR-30a-5p | MIMAT0000087 | SEQ ID NO 26 | GtCgaGGatGtTtAC |
| hsa-miR-30a-3p | MIMAT0000088 | SEQ ID NO 27 | AaCatCCgaCtGaAA |
| hsa-miR-31 | MIMAT0000089 | SEQ ID NO 28 | AtGccAGcaTcTtGC |
| hsa-miR-32 | MIMAT0000090 | SEQ ID NO 29 | TtAgtAAtgTgCaAT |
| hsa-miR-33 | MIMAT0000091 | SEQ ID NO 30 | TgCaaCTacAaTgCA |
| hsa-miR-92 | MIMAT0000092 | SEQ ID NO 31 | CgGgaCAagTgCaAT |
| hsa-miR-93 | MIMAT0000093 | SEQ ID NO 32 | GcAcgAAcaGcAcTT |
| hsa-miR-95 | MIMAT0000094 | SEQ ID NO 33 | AtAaaTAccCgTtGA |
| hsa-miR-96 | MIMAT0000095 | SEQ ID NO 34 | AtGtgCTagTgCcAA |
| hsa-miR-98 | MIMAT0000096 | SEQ ID NO 35 | AcAacTTacTaCcTC |
| hsa-miR-99a | MIMAT0000097 | SEQ ID NO 36 | AtCggATctAcGgGT |
| hsa-miR-100 | MIMAT0000098 | SEQ ID NO 37 | TtCggATctAcGgGT |
| hsa-miR-101 | MIMAT0000099 | SEQ ID NO 38 | TtAtcACagTaCtGT |
| hsa-miR-29b | MIMAT0000100 | SEQ ID NO 39 | AtTtcAAatGgTgCT |
| hsa-miR-103 | MIMAT0000101 | SEQ ID NO 40 | CcTgtACaaTgCtGC |
| hsa-miR-105 | MIMAT0000102 | SEQ ID NO 41 | GaGtcTGagCaTtTG |
| hsa-miR-106a | MIMAT0000103 | SEQ ID NO 42 | CaCtgTAagCaCtTT |
| hsa-miR-107 | MIMAT0000104 | SEQ ID NO 43 | CcTgtACaaTgCtGC |
| hsa-miR-192 | MIMAT0000222 | SEQ ID NO 44 | TcAatTCatAgGtCA |
| hsa-miR-196a | MIMAT0000226 | SEQ ID NO 45 | AaCatGAaaCtAcCT |
| hsa-miR-197 | MIMAT0000227 | SEQ ID NO 46 | TgGagAAggTgGtGA |
| hsa-miR-198 | MIMAT0000228 | SEQ ID NO 47 | AtCtcCCctCtGgAC |
| hsa-miR-199a | MIMAT0000231 | SEQ ID NO 48 | TaGtcTGaaCaCtGG |
| hsa-miR-199a* | MIMAT0000232 | SEQ ID NO 49 | TgTgcAGacTaCtGT |
| hsa-miR-208 | MIMAT0000241 | SEQ ID NO 50 | TtTttGCtcGtCtTA |
| hsa-miR-129 | MIMAT0000242 | SEQ ID NO 51 | CcCagACcgCaAaAA |
| hsa-miR-148a | MIMAT0000243 | SEQ ID NO 52 | TtCtgTAgtGcAcTG |
| hsa-miR-30c | MIMAT0000244 | SEQ ID NO 53 | GtGtaGGatGtTtAC |
| hsa-miR-30d | MIMAT0000245 | SEQ ID NO 54 | GtCggGGatGtTtAC |
| hsa-miR-139 | MIMAT0000250 | SEQ ID NO 55 | AcAcgTGcaCtGtAG |
| hsa-miR-147 | MIMAT0000251 | SEQ ID NO 56 | AaGcaTTtcCaCaCA |
| hsa-miR-7 | MIMAT0000252 | SEQ ID NO 57 | AaTcaCTagTcTtCC |
| hsa-miR-10a | MIMAT0000253 | SEQ ID NO 58 | TcGgaTCtaCaGgGT |
| hsa-miR-10b | MIMAT0000254 | SEQ ID NO 59 | TcGgtTCtaCaGgGT |
| hsa-miR-34a | MIMAT0000255 | SEQ ID NO 60 | AgCtaAGacAcTgCC |
| hsa-miR-181a | MIMAT0000256 | SEQ ID NO 61 | GaCagCGttGaAtGT |
| hsa-miR-181b | MIMAT0000257 | SEQ ID NO 62 | GaCagCAatGaAtGT |
| hsa-miR-181c | MIMAT0000258 | SEQ ID NO 63 | CgAcaGGttGaAtGT |
| hsa-miR-182 | MIMAT0000259 | SEQ ID NO 64 | TtCtaCCatTgCcAA |
| hsa-miR-182* | MIMAT0000260 | SEQ ID NO 65 | GgCaaGTctAgAaCC |
| hsa-miR-183 | MIMAT0000261 | SEQ ID NO 66 | TtCtaCCagTgCcAT |
| hsa-miR-187 | MIMAT0000262 | SEQ ID NO 67 | GcAacACaaGaCaCG |
| hsa-miR-199b | MIMAT0000263 | SEQ ID NO 68 | TaGtcTAaaCaCtGG |
| hsa-miR-203 | MIMAT0000264 | SEQ ID NO 69 | GtCctAAacAtTtCA |
| hsa-miR-204 | MIMAT0000265 | SEQ ID NO 70 | AgGatGAcaAaGgGA |
| hsa-miR-205 | MIMAT0000266 | SEQ ID NO 71 | CcGgtGGaaTgAaGG |
| hsa-miR-210 | MIMAT0000267 | SEQ ID NO 72 | GcTgtCAcaCgCaCA |
| hsa-miR-211 | MIMAT0000268 | SEQ ID NO 73 | AgGatGAcaAaGgGA |
| hsa-miR-212 | MIMAT0000269 | SEQ ID NO 74 | TgActGGagAcTgTT |
| hsa-miR-181a* | MIMAT0000270 | SEQ ID NO 75 | AtCaaCGgtCgAtGG |
| hsa-miR-214 | MIMAT0000271 | SEQ ID NO 76 | TgTctGTgcCtGcTG |
| hsa-miR-215 | MIMAT0000272 | SEQ ID NO 77 | TcAatTCatAgGtCA |
| hsa-miR-216 | MIMAT0000273 | SEQ ID NO 78 | TtGccAGctGaGaTT |
| hsa-miR-217 | MIMAT0000274 | SEQ ID NO 79 | AgTtcCTgaTgCaGT |
| hsa-miR-218 | MIMAT0000275 | SEQ ID NO 80 | GtTagATcaAgCaCA |
| hsa-miR-219 | MIMAT0000276 | SEQ ID NO 81 | TgCgtTTggAcAaTC |
| hsa-miR-220 | MIMAT0000277 | SEQ ID NO 82 | GtCagATacGgTgTG |
| hsa-miR-221 | MIMAT0000278 | SEQ ID NO 83 | AgCagACaaTgTaGC |
| hsa-miR-222 | MIMAT0000279 | SEQ ID NO 84 | GtAgcCAgaTgTaGC |
| hsa-miR-223 | MIMAT0000280 | SEQ ID NO 85 | AtTtgACaaAcTgAC |
| hsa-miR-224 | MIMAT0000281 | SEQ ID NO 86 | AaCcaCTagTgAcTT |
| hsa-miR-200b | MIMAT0000318 | SEQ ID NO 87 | TtAccAGgcAgTaTT |
| hsa-let-7g | MIMAT0000414 | SEQ ID NO 88 | AcAaaCTacTaCcTC |
| hsa-let-7i | MIMAT0000415 | SEQ ID NO 89 | AcAaaCTacTaCcTC |
| hsa-miR-1 | MIMAT0000416 | SEQ ID NO 90 | AcTtcTTtaCaTtCC |
| hsa-miR-15b | MIMAT0000417 | SEQ ID NO 91 | CcAtgATgtGcTgCT |
| hsa-miR-23b | MIMAT0000418 | SEQ ID NO 92 | TcCctGGcaAtGtGA |
| hsa-miR-27b | MIMAT0000419 | SEQ ID NO 93 | AcTtaGCcaCtGtGA |
| hsa-miR-30b | MIMAT0000420 | SEQ ID NO 94 | GtGtaGGatGtTtAC |
| hsa-miR-122a | MIMAT0000421 | SEQ ID NO 95 | CcAttGTcaCaCtCC |
| hsa-miR-124a | MIMAT0000422 | SEQ ID NO 96 | TcAccGCgtGcCtTA |
| hsa-miR-125b | MIMAT0000423 | SEQ ID NO 97 | GtTagGGtcTcAgGG |
| hsa-miR-128a | MIMAT0000424 | SEQ ID NO 98 | GaCcgGTtcAcTgTG |
| hsa-miR-130a | MIMAT0000425 | SEQ ID NO 99 | TtTtaACatTgCaCT |
| hsa-miR-132 | MIMAT0000426 | SEQ ID NO 100 | TgGctGTagAcTgTT |
| hsa-miR-133a | MIMAT0000427 | SEQ ID NO 101 | GgTtgAAggGgAcCA |
| hsa-miR-135a | MIMAT0000428 | SEQ ID NO 102 | GgAatAAaaAgCcAT |
| hsa-miR-137 | MIMAT0000429 | SEQ ID NO 103 | GtAttCTtaAgCaAT |
| hsa-miR-138 | MIMAT0000430 | SEQ ID NO 104 | AtTcaCAacAcCaGC |
| hsa-miR-140 | MIMAT0000431 | SEQ ID NO 105 | AtAggGTaaAaCcAC |
| hsa-miR-141 | MIMAT0000432 | SEQ ID NO 106 | TtAccAGacAgTgTT |
| hsa-miR-142-5p | MIMAT0000433 | SEQ ID NO 107 | TgCttTCtaCtTtAT |
| hsa-miR-142-3p | MIMAT0000434 | SEQ ID NO 108 | AgTagGAaaCaCtAC |
| hsa-miR-143 | MIMAT0000435 | SEQ ID NO 109 | AcAgtGCttCaTcTC |
| hsa-miR-144 | MIMAT0000436 | SEQ ID NO 110 | CaTcaTCtaTaCtGT |
| hsa-miR-145 | MIMAT0000437 | SEQ ID NO 111 | CcTggGAaaAcTgGA |
| hsa-miR-152 | MIMAT0000438 | SEQ ID NO 112 | TtCtgTCatGcAcTG |
| hsa-miR-153 | MIMAT0000439 | SEQ ID NO 113 | TtTtgTGacTaTgCA |
| hsa-miR-191 | MIMAT0000440 | SEQ ID NO 114 | TtTtgGGatTcCgTT |
| hsa-miR-9 | MIMAT0000441 | SEQ ID NO 115 | GcTagATaaCcAaAG |
| hsa-miR-9* | MIMAT0000442 | SEQ ID NO 116 | CgGttATctAgCtTT |
| hsa-miR-125a | MIMAT0000443 | SEQ ID NO 117 | TaAagGGtcTcAgGG |
| hsa-miR-126* | MIMAT0000444 | SEQ ID NO 118 | AcCaaAAgtAaTaAT |
| hsa-miR-126 | MIMAT0000445 | SEQ ID NO 119 | AtTacTCacGgTaCG |
| hsa-miR-127 | MIMAT0000446 | SEQ ID NO 120 | GcTcaGAcgGaTcCG |
| hsa-miR-134 | MIMAT0000447 | SEQ ID NO 121 | TgGtcAAccAgTcAC |
| hsa-miR-136 | MIMAT0000448 | SEQ ID NO 122 | TcAaaACaaAtGgAG |
| hsa-miR-146a | MIMAT0000449 | SEQ ID NO 123 | TgGaaTTcaGtTcTC |
| hsa-miR-149 | MIMAT0000450 | SEQ ID NO 124 | AaGacACggAgCcAG |
| hsa-miR-150 | MIMAT0000451 | SEQ ID NO 125 | TaCaaGGgtTgGgAG |
| hsa-miR-154 | MIMAT0000452 | SEQ ID NO 126 | CaAcaCGgaTaAcCT |
| hsa-miR-154* | MIMAT0000453 | SEQ ID NO 127 | TcAacCGtgTaTgAT |
| hsa-miR-184 | MIMAT0000454 | SEQ ID NO 128 | AtCagTTctCcGtCC |
| hsa-miR-185 | MIMAT0000455 | SEQ ID NO 129 | AcTgcCTttCtCtCC |
| hsa-miR-186 | MIMAT0000456 | SEQ ID NO 130 | AaAggAGaaTtCtTT |
| hsa-miR-188 | MIMAT0000457 | SEQ ID NO 131 | CaCcaTGcaAgGgAT |
| hsa-miR-190 | MIMAT0000458 | SEQ ID NO 132 | TaTatCAaaCaTaTC |
| hsa-miR-193a | MIMAT0000459 | SEQ ID NO 133 | AcTttGTagGcCaGT |
| hsa-miR-194 | MIMAT0000460 | SEQ ID NO 134 | TgGagTTgcTgTtAC |
| hsa-miR-195 | MIMAT0000461 | SEQ ID NO 135 | TaTttCTgtGcTgCT |
| hsa-miR-206 | MIMAT0000462 | SEQ ID NO 136 | AcTtcCTtaCaTtCC |
| hsa-miR-320 | MIMAT0000510 | SEQ ID NO 137 | TcTcaACccAgCtTT |
| hsa-miR-200c | MIMAT0000617 | SEQ ID NO 138 | TtAccCGgcAgTaTT |
| hsa-miR-155 | MIMAT0000646 | SEQ ID NO 139 | TcAcgATtaGcAtTA |
| hsa-miR-128b | MIMAT0000676 | SEQ ID NO 140 | GaCcgGTtcAcTgTG |
| hsa-miR-106b | MIMAT0000680 | SEQ ID NO 141 | CaCtgTCagCaCtTT |
| hsa-miR-29c | MIMAT0000681 | SEQ ID NO 142 | AtTtcAAatGgTgCT |
| hsa-miR-200a | MIMAT0000682 | SEQ ID NO 143 | TtAccAGacAgTgTT |
| hsa-miR-302a* | MIMAT0000683 | SEQ ID NO 144 | AgTacATccAcGtTT |
| hsa-miR-302a | MIMAT0000684 | SEQ ID NO 145 | AaCatGGaaGcAcTT |
| hsa-miR-34b | MIMAT0000685 | SEQ ID NO 146 | CtAatGAcaCtGcCT |
| hsa-miR-34c | MIMAT0000686 | SEQ ID NO 147 | GcTaaCTacAcTgCC |
| hsa-miR-299-3p | MIMAT0000687 | SEQ ID NO 148 | TtTacCAtcCcAcAT |
| hsa-miR-301 | MIMAT0000688 | SEQ ID NO 149 | CaAtaCTatTgCaCT |
| hsa-miR-99b | MIMAT0000689 | SEQ ID NO 150 | GtCggTTctAcGgGT |
| hsa-miR-296 | MIMAT0000690 | SEQ ID NO 151 | AtTgaGGggGgGcCC |
| hsa-miR-130b | MIMAT0000691 | SEQ ID NO 152 | TtTcaTCatTgCaCT |
| hsa-miR-30e-5p | MIMAT0000692 | SEQ ID NO 153 | GtCaaGGatGtTtAC |
| hsa-miR-30e-3p | MIMAT0000693 | SEQ ID NO 154 | AaCatCCgaCtGaAA |
| hsa-miR-361 | MIMAT0000703 | SEQ ID NO 155 | CtGgaGAttCtGaTA |
| hsa-miR-362 | MIMAT0000705 | SEQ ID NO 156 | CtAggTTccAaGgAT |
| hsa-miR-363 | MIMAT0000707 | SEQ ID NO 157 | TgGatACcgTgCaAT |
| hsa-miR-365 | MIMAT0000710 | SEQ ID NO 158 | AtTttTAggGgCaTT |
| hsa-miR-302b* | MIMAT0000714 | SEQ ID NO 159 | AcTtcCAtgTtAaAG |
| hsa-miR-302b | MIMAT0000715 | SEQ ID NO 160 | AaCatGGaaGcAcTT |
| hsa-miR-302c* | MIMAT0000716 | SEQ ID NO 161 | GtAccCCcaTgTtAA |
| hsa-miR-302c | MIMAT0000717 | SEQ ID NO 162 | AaCatGGaaGcAcTT |
| hsa-miR-302d | MIMAT0000718 | SEQ ID NO 163 | AaCatGGaaGcAcTT |
| hsa-miR-367 | MIMAT0000719 | SEQ ID NO 164 | TtGctAAagTgCaAT |
| hsa-miR-368 | MIMAT0000720 | SEQ ID NO 165 | GgAatTTccTcTaTG |
| hsa-miR-369-3p | MIMAT0000721 | SEQ ID NO 166 | TcAacCAtgTaTtAT |
| hsa-miR-370 | MIMAT0000722 | SEQ ID NO 167 | TtCcaCCccAgCaGG |
| hsa-miR-371 | MIMAT0000723 | SEQ ID NO 168 | CaAaaGAtgGcGgCA |
| hsa-miR-372 | MIMAT0000724 | SEQ ID NO 169 | AaTgtCGcaGcAcTT |
| hsa-miR-373* | MIMAT0000725 | SEQ ID NO 170 | CgCccCCatTtTgAG |
| hsa-miR-373 | MIMAT0000726 | SEQ ID NO 171 | AaAatCGaaGcAcTT |
| hsa-miR-374 | MIMAT0000727 | SEQ ID NO 172 | TcAggTTgtAtTaTA |
| hsa-miR-375 | MIMAT0000728 | SEQ ID NO 173 | GaGccGAacGaAcAA |
| hsa-miR-376a | MIMAT0000729 | SEQ ID NO 174 | GaTttTCctCtAtGA |
| hsa-miR-377 | MIMAT0000730 | SEQ ID NO 175 | GtTgcCTttGtGtGA |
| hsa-miR-378 | MIMAT0000731 | SEQ ID NO 176 | GaCctGGagTcAgGA |
| hsa-miR-422b | MIMAT0000732 | SEQ ID NO 177 | CtGacTCcaAgTcCA |
| hsa-miR-379 | MIMAT0000733 | SEQ ID NO 178 | GtTccATagTcTaCC |
| hsa-miR-380-5p | MIMAT0000734 | SEQ ID NO 179 | GtTctATggTcAaCC |
| hsa-miR-380-3p | MIMAT0000735 | SEQ ID NO 180 | TgGacCAtaTtAcAT |
| hsa-miR-381 | MIMAT0000736 | SEQ ID NO 181 | AgCttGCccTtGtAT |
| hsa-miR-382 | MIMAT0000737 | SEQ ID NO 182 | CaCcaCGaaCaAcTT |
| hsa-miR-383 | MIMAT0000738 | SEQ ID NO 183 | AaTcaCCttCtGaTC |
| hsa-miR-340 | MIMAT0000750 | SEQ ID NO 184 | AaGtaACtgAgAcGG |
| hsa-miR-330 | MIMAT0000751 | SEQ ID NO 185 | AgGccGTgtGcTtTG |
| hsa-miR-328 | MIMAT0000752 | SEQ ID NO 186 | GgGcaGAgaGgGcCA |
| hsa-miR-342 | MIMAT0000753 | SEQ ID NO 187 | CgAttTCtgTgTgAG |
| hsa-miR-337 | MIMAT0000754 | SEQ ID NO 188 | TcAtaTAggAgCtGG |
| hsa-miR-323 | MIMAT0000755 | SEQ ID NO 189 | CgAccGTgtAaTgTG |
| hsa-miR-326 | MIMAT0000756 | SEQ ID NO 190 | AgGaaGGgcCcAgAG |
| hsa-miR-151 | MIMAT0000757 | SEQ ID NO 191 | GgAgcTTcaGtCtAG |
| hsa-miR-135b | MIMAT0000758 | SEQ ID NO 192 | GgAatGAaaAgCcAT |
| hsa-miR-148b | MIMAT0000759 | SEQ ID NO 193 | TtCtgTGatGcAcTG |
| hsa-miR-331 | MIMAT0000760 | SEQ ID NO 194 | GgAtaGGccCaGgGG |
| hsa-miR-324-5p | MIMAT0000761 | SEQ ID NO 195 | TgCccTAggGgAtGC |
| hsa-miR-324-3p | MIMAT0000762 | SEQ ID NO 196 | GcAccTGggGcAgTG |
| hsa-miR-338 | MIMAT0000763 | SEQ ID NO 197 | AaTcaCTgaTgCtGG |
| hsa-miR-339 | MIMAT0000764 | SEQ ID NO 198 | TcCtgGAggAcAgGG |
| hsa-miR-335 | MIMAT0000765 | SEQ ID NO 199 | TcGttATtgCtCtTG |
| hsa-miR-133b | MIMAT0000770 | SEQ ID NO 200 | GgTtgAAggGgAcCA |
| hsa-miR-325 | MIMAT0000771 | SEQ ID NO 201 | CtGgaCAccTaCtAG |
| hsa-miR-345 | MIMAT0000772 | SEQ ID NO 202 | GgActAGgaGtCaGC |
| hsa-miR-346 | MIMAT0000773 | SEQ ID NO 203 | GgCatGCggGcAgAC |
| ebv-miR-BHRF1-1 | MIMAT0000995 | SEQ ID NO 204 | GgGgcTGatCaGgTT |
| ebv-miR-BHRF1-2* | MIMAT0000996 | SEQ ID NO 205 | TgCtgCAacAgAaTT |
| ebv-miR-BHRF1-2 | MIMAT0000997 | SEQ ID NO 206 | TcTgcCGcaAaAgAT |
| ebv-miR-BHRF1-3 | MIMAT0000998 | SEQ ID NO 207 | TaCacACttCcCgTT |
| ebv-miR-BART1-5p | MIMAT0000999 | SEQ ID NO 208 | GtCacTTccAcTaAG |
| ebv-miR-BART2 | MIMAT0001000 | SEQ ID NO 209 | GcGaaTGcaGaAaAT |
| hsa-miR-384 | MIMAT0001075 | SEQ ID NO 210 | AaCaaTTtcTaGgAA |
| hsa-miR-196b | MIMAT0001080 | SEQ ID NO 211 | AaCagGAaaCtAcCT |
| hsa-miR-422a | MIMAT0001339 | SEQ ID NO 212 | CtGacCCtaAgTcCA |
| hsa-miR-423 | MIMAT0001340 | SEQ ID NO 213 | GgCctCAgaCcGaGC |
| hsa-miR-424 | MIMAT0001341 | SEQ ID NO 214 | AcAtgAAttGcTgCT |
| hsa-miR-425-3p | MIMAT0001343 | SEQ ID NO 215 | AcAcgACatTcCcGA |
| hsa-miR-18b | MIMAT0001412 | SEQ ID NO 216 | CaCtaGAtgCaCcTT |
| hsa-miR-20b | MIMAT0001413 | SEQ ID NO 217 | CaCtaTGagCaCtTT |
| hsa-miR-448 | MIMAT0001532 | SEQ ID NO 218 | CaTccTAcaTaTgCA |
| hsa-miR-429 | MIMAT0001536 | SEQ ID NO 219 | TtAccAGacAgTaTT |
| hsa-miR-449 | MIMAT0001541 | SEQ ID NO 220 | TaAcaATacAcTgCC |
| hsa-miR-450 | MIMAT0001545 | SEQ ID NO 221 | GaAcaCAtcGcAaAA |
| hcmv-miR-UL22A | MIMAT0001574 | SEQ ID NO 222 | AcGggAAggCtAgTT |
| hcmv-miR-UL22A* | MIMAT0001575 | SEQ ID NO 223 | AcTagCAttCtGgTG |
| hcmv-miR-UL36 | MIMAT0001576 | SEQ ID NO 224 | CaGgtGTctTcAaCG |
| hcmv-miR-UL112 | MIMAT0001577 | SEQ ID NO 225 | GaTctCAccGtCaCT |
| hcmv-miR-UL148D | MIMAT0001578 | SEQ ID NO 226 | AaGaaGGggAgGaCG |
| hcmv-miR-US5-1 | MIMAT0001579 | SEQ ID NO 227 | CtCgtCAggCtTgTC |
| hcmv-miR-US5-2 | MIMAT0001580 | SEQ ID NO 228 | GtCacACctAtCaTA |
| hcmv-miR-US25-1 | MIMAT0001581 | SEQ ID NO 229 | GaGccACtgAgCgGT |
| hcmv-miR-US25-2-5p | MIMAT0001582 | SEQ ID NO 230 | AcCtgAAcaGaCcGC |
| hcmv-miR-US25-2-3p | MIMAT0001583 | SEQ ID NO 231 | AgCtcTCcaAgTgGA |
| hcmv-miR-US33 | MIMAT0001584 | SEQ ID NO 232 | CgGtcCGggCaCaAT |
| hsa-miR-191* | MIMAT0001618 | SEQ ID NO 233 | GaAatCCaaGcGcAG |
| hsa-miR-200a* | MIMAT0001620 | SEQ ID NO 234 | AcTgtCCggTaAgAT |
| hsa-miR-369-5p | MIMAT0001621 | SEQ ID NO 235 | AtAacACggTcGaTC |
| hsa-miR-431 | MIMAT0001625 | SEQ ID NO 236 | GaCggCCtgCaAgAC |
| hsa-miR-433 | MIMAT0001627 | SEQ ID NO 237 | AgGagCCcaTcAtGA |
| hsa-miR-329 | MIMAT0001629 | SEQ ID NO 238 | GtTaaCCagGtGtGT |
| hsa-miR-453 | MIMAT0001630 | SEQ ID NO 239 | CaCcaCGgaCaAcCT |
| hsa-miR-451 | MIMAT0001631 | SEQ ID NO 240 | GtAatGGtaAcGgTT |
| hsa-miR-452 | MIMAT0001635 | SEQ ID NO 241 | GtTtcCTctGcAaAC |
| hsa-miR-452* | MIMAT0001636 | SEQ ID NO 242 | TtGcaGAtgAgAcTG |
| hsa-miR-409-5p | MIMAT0001638 | SEQ ID NO 243 | GtTgcTCggGtAaCC |
| hsa-miR-409-3p | MIMAT0001639 | SEQ ID NO 244 | CaCcgAGcaAcAtTC |
| hsa-miR-412 | MIMAT0002170 | SEQ ID NO 245 | GtGgaCCagGtGaAG |
| hsa-miR-410 | MIMAT0002171 | SEQ ID NO 246 | CcAtcTGtgTtAtAT |
| hsa-miR-376b | MIMAT0002172 | SEQ ID NO 247 | GaTttTCctCtAtGA |
| hsa-miR-483 | MIMAT0002173 | SEQ ID NO 248 | GgGagGAgaGgAgTG |
| hsa-miR-484 | MIMAT0002174 | SEQ ID NO 249 | AgGggACtgAgCcTG |
| hsa-miR-485-5p | MIMAT0002175 | SEQ ID NO 250 | AtCacGGccAgCcTC |
| hsa-miR-485-3p | MIMAT0002176 | SEQ ID NO 251 | GaGagCCgtGtAtGA |
| hsa-miR-486 | MIMAT0002177 | SEQ ID NO 252 | GcAgcTCagTaCaGG |
| hsa-miR-487a | MIMAT0002178 | SEQ ID NO 253 | AtGtcCCtgTaTgAT |
| kshv-miR-K12-10a | MIMAT0002179 | SEQ ID NO 254 | CgGggGGacAaCaCT |
| kshv-miR-K12-10b | MIMAT0002180 | SEQ ID NO 255 | CgGggGGacAaCaCC |
| kshv-miR-K12-11 | MIMAT0002181 | SEQ ID NO 256 | AcAggCTaaGcAtTA |
| kshv-miR-K12-1 | MIMAT0002182 | SEQ ID NO 257 | CcCagTTtcCtGtAA |
| kshv-miR-K12-2 | MIMAT0002183 | SEQ ID NO 258 | GaCccGGacTaCaGT |
| kshv-miR-K12-9* | MIMAT0002184 | SEQ ID NO 259 | GtTtaCGcaGcTgGG |
| kshv-miR-K12-9 | MIMAT0002185 | SEQ ID NO 260 | AgCtgCGtaTaCcCA |
| kshv-miR-K12-8 | MIMAT0002186 | SEQ ID NO 261 | CtCtcAGtcGcGcCT |
| kshv-miR-K12-7 | MIMAT0002187 | SEQ ID NO 262 | CaGcaACatGgGaTC |
| kshv-miR-K12-6-5p | MIMAT0002188 | SEQ ID NO 263 | GaTtaGGtgCtGcTG |
| kshv-miR-K12-6-3p | MIMAT0002189 | SEQ ID NO 264 | AgCccGAaaAcCaTC |
| kshv-miR-K12-5 | MIMAT0002190 | SEQ ID NO 265 | AgTtcCAggCaTcCT |
| kshv-miR-K12-4-5p | MIMAT0002191 | SEQ ID NO 266 | GtActGCggTtTaGC |
| kshv-miR-K12-4-3p | MIMAT0002192 | SEQ ID NO 267 | AgGccTCagTaTtCT |
| kshv-miR-K12-3 | MIMAT0002193 | SEQ ID NO 268 | CgTccTCagAaTgTG |
| kshv-miR-K12-3* | MIMAT0002194 | SEQ ID NO 269 | CaTtcTGtgAcCgCG |
| hsa-miR-488 | MIMAT0002804 | SEQ ID NO 270 | AgTgcCAttAtCtGG |
| hsa-miR-489 | MIMAT0002805 | SEQ ID NO 271 | TaTatGTgaTgTcAC |
| hsa-miR-490 | MIMAT0002806 | SEQ ID NO 272 | GgAgtCCtcCaGgTT |
| hsa-miR-491 | MIMAT0002807 | SEQ ID NO 273 | GgAagGGttCcCcAC |
| hsa-miR-511 | MIMAT0002808 | SEQ ID NO 274 | GcAgaGCaaAaGaCA |
| hsa-miR-146b | MIMAT0002809 | SEQ ID NO 275 | TgGaaTTcaGtTcTC |
| hsa-miR-202* | MIMAT0002810 | SEQ ID NO 276 | GtAtaTGcaTaGgAA |
| hsa-miR-202 | MIMAT0002811 | SEQ ID NO 277 | CaTgcCCtaTaCcTC |
| hsa-miR-492 | MIMAT0002812 | SEQ ID NO 278 | TtGtcCCgcAgGtCC |
| hsa-miR-493-5p | MIMAT0002813 | SEQ ID NO 279 | AgCctACcaTgTaCA |
| hsa-miR-432 | MIMAT0002814 | SEQ ID NO 280 | AtGacCTacTcCaAG |
| hsa-miR-432* | MIMAT0002815 | SEQ ID NO 281 | TgGagGAgcCaTcCA |
| hsa-miR-494 | MIMAT0002816 | SEQ ID NO 282 | TcCcgTGtaTgTtTC |
| hsa-miR-495 | MIMAT0002817 | SEQ ID NO 283 | TgCacCAtgTtTgTT |
| hsa-miR-496 | MIMAT0002818 | SEQ ID NO 284 | AgAttGGccAtGtAA |
| hsa-miR-193b | MIMAT0002819 | SEQ ID NO 285 | AcTttGAggGcCaGT |
| hsa-miR-497 | MIMAT0002820 | SEQ ID NO 286 | CcAcaGTgtGcTgCT |
| hsa-miR-181d | MIMAT0002821 | SEQ ID NO 287 | GaCaaCAatGaAtGT |
| hsa-miR-512-5p | MIMAT0002822 | SEQ ID NO 288 | CcCtcAAggCtGaGT |
| hsa-miR-512-3p | MIMAT0002823 | SEQ ID NO 289 | AgCtaTGacAgCaCT |
| hsa-miR-498 | MIMAT0002824 | SEQ ID NO 290 | GcCccCTggCtTgAA |
| hsa-miR-520e | MIMAT0002825 | SEQ ID NO 291 | AaAaaGGaaGcAcTT |
| hsa-miR-515-5p | MIMAT0002826 | SEQ ID NO 292 | GcTttCTttTgGaGA |
| hsa-miR-515-3p | MIMAT0002827 | SEQ ID NO 293 | CcAaaAGaaGgCaCT |
| hsa-miR-519e* | MIMAT0002828 | SEQ ID NO 294 | GcTccCTttTgGaGA |
| hsa-miR-519e | MIMAT0002829 | SEQ ID NO 295 | TaAaaGGagGcAcTT |
| hsa-miR-520f | MIMAT0002830 | SEQ ID NO 296 | CtAaaAGgaAgCaCT |
| hsa-miR-526c | MIMAT0002831 | SEQ ID NO 297 | GcGctTCccTcTaGA |
| hsa-miR-519c | MIMAT0002832 | SEQ ID NO 298 | TaAaaAGatGcAcTT |
| hsa-miR-520a* | MIMAT0002833 | SEQ ID NO 299 | GtActTCccTcTgGA |
| hsa-miR-520a | MIMAT0002834 | SEQ ID NO 300 | CaAagGGaaGcAcTT |
| hsa-miR-526b | MIMAT0002835 | SEQ ID NO 301 | GtGctTCccTcAaGA |
| hsa-miR-526b* | MIMAT0002836 | SEQ ID NO 302 | TaAaaGGaaGcAcTT |
| hsa-miR-519b | MIMAT0002837 | SEQ ID NO 303 | TaAaaGGatGcAcTT |
| hsa-miR-525 | MIMAT0002838 | SEQ ID NO 304 | GtGcaTCccTcTgGA |
| hsa-miR-525* | MIMAT0002839 | SEQ ID NO 305 | AaAggGAagCgCcTT |
| hsa-miR-523 | MIMAT0002840 | SEQ ID NO 306 | TaTagGGaaGcGcGT |
| hsa-miR-518f* | MIMAT0002841 | SEQ ID NO 307 | GtGctTCccTcTaGA |
| hsa-miR-518f | MIMAT0002842 | SEQ ID NO 308 | TaAagAGaaGcGcTT |
| hsa-miR-520b | MIMAT0002843 | SEQ ID NO 309 | TaAaaGGaaGcAcTT |
| hsa-miR-518b | MIMAT0002844 | SEQ ID NO 310 | AaAggGGagCgCtTT |
| hsa-miR-526a | MIMAT0002845 | SEQ ID NO 311 | GtGctTCccTcTaGA |
| hsa-miR-520c | MIMAT0002846 | SEQ ID NO 312 | TaAaaGGaaGcAcTT |
| hsa-miR-518c* | MIMAT0002847 | SEQ ID NO 313 | TgCttCCctCcAgAG |
| hsa-miR-518c | MIMAT0002848 | SEQ ID NO 314 | AaAgaGAagCgCtTT |
| hsa-miR-524* | MIMAT0002849 | SEQ ID NO 315 | GtGctTCccTtTgTA |
| hsa-miR-524 | MIMAT0002850 | SEQ ID NO 316 | AaAggGAagCgCcTT |
| hsa-miR-517* | MIMAT0002851 | SEQ ID NO 317 | TgCttCCatCtAgAG |
| hsa-miR-517a | MIMAT0002852 | SEQ ID NO 318 | TaAagGGatGcAcGA |
| hsa-miR-519d | MIMAT0002853 | SEQ ID NO 319 | AaAggGAggCaCtTT |
| hsa-miR-521 | MIMAT0002854 | SEQ ID NO 320 | TaAagGGaaGtGcGT |
| hsa-miR-520d* | MIMAT0002855 | SEQ ID NO 321 | GgCttCCctTtGtAG |
| hsa-miR-520d | MIMAT0002856 | SEQ ID NO 322 | CaAagAGaaGcAcTT |
| hsa-miR-517b | MIMAT0002857 | SEQ ID NO 323 | CtAaaGGgaTgCaCG |
| hsa-miR-520g | MIMAT0002858 | SEQ ID NO 324 | AaGggAAgcAcTtTG |
| hsa-miR-516-5p | MIMAT0002859 | SEQ ID NO 325 | TtCttACctCcAgAT |
| hsa-miR-516-3p | MIMAT0002860 | SEQ ID NO 326 | CcTctGAaaGgAaGC |
| hsa-miR-518e | MIMAT0002861 | SEQ ID NO 327 | TgAagGGaaGcGcTT |
| hsa-miR-527 | MIMAT0002862 | SEQ ID NO 328 | GgGctTCccTtTgCA |
| hsa-miR-518a | MIMAT0002863 | SEQ ID NO 329 | CaAagGGaaGcGcTT |
| hsa-miR-518d | MIMAT0002864 | SEQ ID NO 330 | AaAggGAagCgCtTT |
| hsa-miR-517c | MIMAT0002866 | SEQ ID NO 331 | TaAaaGGatGcAcGA |
| hsa-miR-520h | MIMAT0002867 | SEQ ID NO 332 | AaGggAAgcAcTtTG |
| hsa-miR-522 | MIMAT0002868 | SEQ ID NO 333 | TaAagGGaaCcAtTT |
| hsa-miR-519a | MIMAT0002869 | SEQ ID NO 334 | TaAaaGGatGcAcTT |
| hsa-miR-499 | MIMAT0002870 | SEQ ID NO 335 | TcActGCaaGtCtTA |
| hsa-miR-500 | MIMAT0002871 | SEQ ID NO 336 | CcTtgCCcaGgTgCA |
| hsa-miR-501 | MIMAT0002872 | SEQ ID NO 337 | CcAggGAcaAaGgAT |
| hsa-miR-502 | MIMAT0002873 | SEQ ID NO 338 | CcCagATagCaAgGA |
| hsa-miR-503 | MIMAT0002874 | SEQ ID NO 339 | AcTgtTCccGcTgCT |
| hsa-miR-504 | MIMAT0002875 | SEQ ID NO 340 | GtGcaGAccAgGgTC |
| hsa-miR-505 | MIMAT0002876 | SEQ ID NO 341 | AcCagCAagTgTtGA |
| hsa-miR-513 | MIMAT0002877 | SEQ ID NO 342 | GaCacCTccCtGtGA |
| hsa-miR-506 | MIMAT0002878 | SEQ ID NO 343 | TcAgaAGggTgCcTT |
| hsa-miR-507 | MIMAT0002879 | SEQ ID NO 344 | TcCaaAAggTgCaAA |
| hsa-miR-508 | MIMAT0002880 | SEQ ID NO 345 | CaAaaGGctAcAaTC |
| hsa-miR-509 | MIMAT0002881 | SEQ ID NO 346 | AcAgaCGtaCcAaTC |
| hsa-miR-510 | MIMAT0002882 | SEQ ID NO 347 | GcCacTCtcCtGaGT |
| hsa-miR-514 | MIMAT0002883 | SEQ ID NO 348 | TcAcaGAagTgTcAA |
| hsa-miR-532 | MIMAT0002888 | SEQ ID NO 349 | CtAcaCTcaAgGcAT |
| hsa-miR-299-5p | MIMAT0002890 | SEQ ID NO 350 | GtGggACggTaAaCC |
| hsa-miR-18a* | MIMAT0002891 | SEQ ID NO 351 | GaGcaCTtaGgGcAG |
| hsa-miR-455 | MIMAT0003150 | SEQ ID NO 352 | AgTccAAagGcAcAT |
| hsa-miR-493-3p | MIMAT0003161 | SEQ ID NO 353 | AcAcaGTagAcCtTC |
| hsa-miR-539 | MIMAT0003163 | SEQ ID NO 354 | CaAggATaaTtTcTC |
| hsa-miR-544 | MIMAT0003164 | SEQ ID NO 355 | GcTaaAAatGcAgAA |
| hsa-miR-545 | MIMAT0003165 | SEQ ID NO 356 | AtAaaTGttTgCtGA |
| hsa-miR-487b | MIMAT0003180 | SEQ ID NO 357 | AtGacCCtgTaCgAT |
| hsa-miR-551a | MIMAT0003214 | SEQ ID NO 358 | AcCaaGAgtGgGtCG |
| hsa-miR-552 | MIMAT0003215 | SEQ ID NO 359 | TaAccAGtcAcCtGT |
| hsa-miR-553 | MIMAT0003216 | SEQ ID NO 360 | AaAatCTcaCcGtTT |
| hsa-miR-554 | MIMAT0003217 | SEQ ID NO 361 | CtGagTCagGaCtAG |
| hsa-miR-92b | MIMAT0003218 | SEQ ID NO 362 | CgGgaCGagTgCaAT |
| hsa-miR-555 | MIMAT0003219 | SEQ ID NO 363 | AgGttCAgcTtAcCC |
| hsa-miR-556 | MIMAT0003220 | SEQ ID NO 364 | TtAcaATgaGcTcAT |
| hsa-miR-557 | MIMAT0003221 | SEQ ID NO 365 | GcCcaCCcgTgCaAA |
| hsa-miR-558 | MIMAT0003222 | SEQ ID NO 366 | TtGgtACagCaGcTC |
| hsa-miR-559 | MIMAT0003223 | SEQ ID NO 367 | GtGcaTAttTaCtTT |
| hsa-miR-560 | MIMAT0003224 | SEQ ID NO 368 | GcCggCCggCgCaCG |
| hsa-miR-561 | MIMAT0003225 | SEQ ID NO 369 | AgGatCTtaAaCtTT |
| hsa-miR-562 | MIMAT0003226 | SEQ ID NO 370 | AtGgtACagCtAcTT |
| hsa-miR-563 | MIMAT0003227 | SEQ ID NO 371 | AaAcgTAtgTcAaCC |
| hsa-miR-564 | MIMAT0003228 | SEQ ID NO 372 | TgCtgACacCgTgCC |
| hsa-miR-565 | MIMAT0003229 | SEQ ID NO 373 | AcAtcGCgaGcCaGC |
| hsa-miR-566 | MIMAT0003230 | SEQ ID NO 374 | GgGatCAcaGgCgCC |
| hsa-miR-567 | MIMAT0003231 | SEQ ID NO 375 | CcTggAAgaAcAtAC |
| hsa-miR-568 | MIMAT0003232 | SEQ ID NO 376 | GtAtaCAttTaTaCA |
| hsa-miR-551b | MIMAT0003233 | SEQ ID NO 377 | AcCaaGTatGgGtCG |
| hsa-miR-569 | MIMAT0003234 | SEQ ID NO 378 | CcAggATtcAtTaAC |
| hsa-miR-570 | MIMAT0003235 | SEQ ID NO 379 | GgTaaTTgcTgTtTT |
| hsa-miR-571 | MIMAT0003236 | SEQ ID NO 380 | TcAgaTGgcCaAcTC |
| hsa-miR-572 | MIMAT0003237 | SEQ ID NO 381 | CcAccGCcgAgCgGA |
| hsa-miR-573 | MIMAT0003238 | SEQ ID NO 382 | TtAcaCAtcAcTtCA |
| hsa-miR-574 | MIMAT0003239 | SEQ ID NO 383 | TgTgtGCatGaGcGT |
| hsa-miR-575 | MIMAT0003240 | SEQ ID NO 384 | CcTgtCCaaCtGgCT |
| hsa-miR-576 | MIMAT0003241 | SEQ ID NO 385 | GtGgaGAaaTtAgAA |
| hsa-miR-577 | MIMAT0003242 | SEQ ID NO 386 | AcCaaTAttTtAtCT |
| hsa-miR-578 | MIMAT0003243 | SEQ ID NO 387 | CcTagAGcaCaAgAA |
| hsa-miR-579 | MIMAT0003244 | SEQ ID NO 388 | TtTatACcaAaTgAA |
| hsa-miR-580 | MIMAT0003245 | SEQ ID NO 389 | GaTtcATcaTtCtCA |
| hsa-miR-581 | MIMAT0003246 | SEQ ID NO 390 | TcTagAGaaCaCaAG |
| hsa-miR-582 | MIMAT0003247 | SEQ ID NO 391 | GgTtgAAcaAcTgTA |
| hsa-miR-583 | MIMAT0003248 | SEQ ID NO 392 | GgGacCTtcCtCtTT |
| hsa-miR-584 | MIMAT0003249 | SEQ ID NO 393 | CcCagGCaaAcCaTA |
| hsa-miR-585 | MIMAT0003250 | SEQ ID NO 394 | CaTacAGatAcGcCC |
| hsa-miR-548a | MIMAT0003251 | SEQ ID NO 395 | GtAatTGccAgTtTT |
| hsa-miR-586 | MIMAT0003252 | SEQ ID NO 396 | AaAaaTAcaAtGcAT |
| hsa-miR-587 | MIMAT0003253 | SEQ ID NO 397 | TcAtcACctAtGgAA |
| hsa-miR-548b | MIMAT0003254 | SEQ ID NO 398 | GcAacTGagGtTcTT |
| hsa-miR-588 | MIMAT0003255 | SEQ ID NO 399 | AaCccATtgTgGcCA |
| hsa-miR-589 | MIMAT0003256 | SEQ ID NO 400 | CcGgcATttGtTcTG |
| hsa-miR-550 | MIMAT0003257 | SEQ ID NO 401 | CtGagGGagTaAgAC |
| hsa-miR-590 | MIMAT0003258 | SEQ ID NO 402 | TtTtaTGaaTaAgCT |
| hsa-miR-591 | MIMAT0003259 | SEQ ID NO 403 | TgAgaACccAtGgTC |
| hsa-miR-592 | MIMAT0003260 | SEQ ID NO 404 | TcGcaTAttGaCaCA |
| hsa-miR-593 | MIMAT0003261 | SEQ ID NO 405 | TgCctGGctGgTgCC |
| hsa-miR-595 | MIMAT0003263 | SEQ ID NO 406 | CaCcaCGgcAcAcTT |
| hsa-miR-596 | MIMAT0003264 | SEQ ID NO 407 | GgAgcCGggCaGgCT |
| hsa-miR-597 | MIMAT0003265 | SEQ ID NO 408 | GtCatCGagTgAcAC |
| hsa-miR-598 | MIMAT0003266 | SEQ ID NO 409 | TgAcaACgaTgAcGT |
| hsa-miR-599 | MIMAT0003267 | SEQ ID NO 410 | GaTaaACtgAcAcAA |
| hsa-miR-600 | MIMAT0003268 | SEQ ID NO 411 | GcTctTGtcTgTaAG |
| hsa-miR-601 | MIMAT0003269 | SEQ ID NO 412 | CaAcaATccTaGaCC |
| hsa-miR-602 | MIMAT0003270 | SEQ ID NO 413 | AgCtgTCgcCcGtGT |
| hsa-miR-603 | MIMAT0003271 | SEQ ID NO 414 | GtAatTGcaGtGtGT |
| hsa-miR-604 | MIMAT0003272 | SEQ ID NO 415 | CtGaaTTccGcAgCC |
| hsa-miR-605 | MIMAT0003273 | SEQ ID NO 416 | GgCacCAtgGgAtTT |
| hsa-miR-606 | MIMAT0003274 | SEQ ID NO 417 | TgAttTTcaGtAgTT |
| hsa-miR-607 | MIMAT0003275 | SEQ ID NO 418 | AgAtcTGgaTtTgAA |
| hsa-miR-608 | MIMAT0003276 | SEQ ID NO 419 | TcCcaACacCaCcCC |
| hsa-miR-609 | MIMAT0003277 | SEQ ID NO 420 | AtGagAGaaAcAcCC |
| hsa-miR-610 | MIMAT0003278 | SEQ ID NO 421 | GcAcaCAttTaGcTC |
| hsa-miR-611 | MIMAT0003279 | SEQ ID NO 422 | CcCgaGGggTcCtCG |
| hsa-miR-612 | MIMAT0003280 | SEQ ID NO 423 | AgAagCCctGcCcAG |
| hsa-miR-613 | MIMAT0003281 | SEQ ID NO 424 | AaGaaGGaaCaTtCC |
| hsa⁻miR-614 | MIMAT0003282 | SEQ ID NO 425 | GcAagAAcaGgCgTT |
| hsa-miR-615 | MIMAT0003283 | SEQ ID NO 426 | GaGacCCagGcTcGG |
| hsa-miR-616 | MIMAT0003284 | SEQ ID NO 427 | CtGaaGGgtTtTgAG |
| hsa-miR-548c | MIMAT0003285 | SEQ ID NO 428 | GtAatTGagAtTtTT |
| hsa-miR-617 | MIMAT0003286 | SEQ ID NO 429 | TtCaaATggGaAgTC |
| hsa-miR-618 | MIMAT0003287 | SEQ ID NO 430 | AgGacAAgtAgAgTT |
| hsa-miR-619 | MIMAT0003288 | SEQ ID NO 431 | CaAacATgtCcAgGT |
| hsa-miR-620 | MIMAT0003289 | SEQ ID NO 432 | CtAtaTCtaTcTcCA |
| hsa-miR-621 | MIMAT0003290 | SEQ ID NO 433 | AgCgcTGttGcTaGC |
| hsa-miR-622 | MIMAT0003291 | SEQ ID NO 434 | AaCctCAgcAgAcTG |
| hsa-miR-623 | MIMAT0003292 | SEQ ID NO 435 | AgCccCTgcAaGgGA |
| hsa-miR-624 | MIMAT0003293 | SEQ ID NO 436 | CaAggTActGgTaCT |
| hsa-miR-625 | MIMAT0003294 | SEQ ID NO 437 | AtAgaACttTcCcCC |
| hsa-miR-626 | MIMAT0003295 | SEQ ID NO 438 | AcAttTTcaGaCaGC |
| hsa-miR-627 | MIMAT0003296 | SEQ ID NO 439 | TtTctTAgaGaCtCA |
| hsa-miR-628 | MIMAT0003297 | SEQ ID NO 440 | TgCcaCTctTaCtAG |
| hsa-miR-629 | MIMAT0003298 | SEQ ID NO 441 | CtTacGTtgGgAgAA |
| hsa-miR-630 | MIMAT0003299 | SEQ ID NO 442 | CcTggTAcaGaAtAC |
| hsa-miR-631 | MIMAT0003300 | SEQ ID NO 443 | GgTctGGgcCaGgTC |
| hsa-miR-33b | MIMAT0003301 | SEQ ID NO 444 | TgCaaCAgcAaTgCA |
| hsa-miR-632 | MIMAT0003302 | SEQ ID NO 445 | CaCagGAagCaGaCA |
| hsa-miR-633 | MIMAT0003303 | SEQ ID NO 446 | TgGtaGAtaCtAtTA |
| hsa-miR-634 | MIMAT0003304 | SEQ ID NO 447 | AgTtgGGgtGcTgGT |
| hsa-miR-635 | MIMAT0003305 | SEQ ID NO 448 | GtTtcAGtgCcCaAG |
| hsa-miR-636 | MIMAT0003306 | SEQ ID NO 449 | GgGacGAgcAaGcAC |
| hsa-miR-637 | MIMAT0003307 | SEQ ID NO 450 | CcCgaAAgcCcCcAG |
| hsa-miR-638 | MIMAT0003308 | SEQ ID NO 451 | CcCgcCCgcGaTcCC |
| hsa-miR-639 | MIMAT0003309 | SEQ ID NO 452 | TcGcaACcgCaGcGA |
| hsa-miR-640 | MIMAT0003310 | SEQ ID NO 453 | CaGgtTCctGgAtCA |
| hsa-miR-641 | MIMAT0003311 | SEQ ID NO 454 | TcTatCCtaTgTcTT |
| hsa-miR-642 | MIMAT0003312 | SEQ ID NO 455 | AcAttTGgaGaGgGA |
| hsa-miR-643 | MIMAT0003313 | SEQ ID NO 456 | GaGctAGcaTaCaAG |
| hsa-miR-644 | MIMAT0003314 | SEQ ID NO 457 | CtAagAAagCcAcAC |
| hsa-miR-645 | MIMAT0003315 | SEQ ID NO 458 | GcAgtACcaGcCtAG |
| hsa-miR-646 | MIMAT0003316 | SEQ ID NO 459 | TcAgaGGcaGcTgCT |
| hsa-miR-647 | MIMAT0003317 | SEQ ID NO 460 | AaGtgAGtgCaGcCA |
| hsa-miR-648 | MIMAT0003318 | SEQ ID NO 461 | AgTgcCCtgCaCaCT |
| hsa-miR-649 | MIMAT0003319 | SEQ ID NO 462 | TgAacAAcaCaGgTT |
| hsa-miR-650 | MIMAT0003320 | SEQ ID NO 463 | GaGagCGctGcCtCC |
| hsa-miR-651 | MIMAT0003321 | SEQ ID NO 464 | TcAagCTtaTcCtAA |
| hsa-miR-652 | MIMAT0003322 | SEQ ID NO 465 | CcCtaGTggCgCcAT |
| hsa-miR-548d | MIMAT0003323 | SEQ ID NO 466 | GaAacTGtgGtTtTT |
| hsa-miR-661 | MIMAT0003324 | SEQ ID NO 467 | GcCagAGacCcAgGC |
| hsa-miR-662 | MIMAT0003325 | SEQ ID NO 468 | GgGccACaaCgTgGG |
| hsa-miR-663 | MIMAT0003326 | SEQ ID NO 469 | CcGcgGCgcCcCgCC |
| hsa-miR-449b | MIMAT0003327 | SEQ ID NO 470 | TaAcaATacAcTgCC |
| hsa-miR-653 | MIMAT0003328 | SEQ ID NO 471 | GtAgaGAttGtTtCA |
| hsa-miR-411 | MIMAT0003329 | SEQ ID NO 472 | GcTatACggTcTaCT |
| hsa-miR-654 | MIMAT0003330 | SEQ ID NO 473 | GtTctGCggCcCaCC |
| hsa-miR-655 | MIMAT0003331 | SEQ ID NO 474 | GtTaaCCatGtAtTA |
| hsa-miR-656 | MIMAT0003332 | SEQ ID NO 475 | TtGacTGtaTaAtAT |
| hsa-miR-549 | MIMAT0003333 | SEQ ID NO 476 | TcAtcCAtaGtTgTC |
| hsa-miR-657 | MIMAT0003335 | SEQ ID NO 477 | AgGgtGAgaAcCtGC |
| hsa-miR-658 | MIMAT0003336 | SEQ ID NO 478 | CcTacTTccCtCcGC |
| hsa-miR-659 | MIMAT0003337 | SEQ ID NO 479 | CcCtcCCtgAaCcAA |
| hsa-miR-660 | MIMAT0003338 | SEQ ID NO 480 | CgAtaTGcaAtGgGT |
| hsa-miR-421 | MIMAT0003339 | SEQ ID NO 481 | AtTaaTGtcTgTtGA |
| hsa-miR-542-5p | MIMAT0003340 | SEQ ID NO 482 | AcAtgATgaTcCcCG |
| hcmv-miR-US4 | MIMAT0003341 | SEQ ID NO 483 | CtGcaCGtcCaTgTC |
| hcmv-miR-UL70-5p | MIMAT0003342 | SEQ ID NO 484 | AcGagGCcgAgAcGC |
| hcmv-miR-UL70-3p | MIMAT0003343 | SEQ ID NO 485 | GcGccAGccCaTcCC |
| hsa-miR-363* | MIMAT0003385 | SEQ ID NO 486 | CaTcgTGatCcAcCC |
| hsa-miR-376a* | MIMAT0003386 | SEQ ID NO 487 | AgAagGAgaAtCtAC |
| hsa-miR-542-3p | MIMAT0003389 | SEQ ID NO 488 | TtAtcAAtcTgTcAC |
| ebv-miR-BART1-3p | MIMAT0003390 | SEQ ID NO 489 | GtGgaTAgcGgTgCT |
| hsa-miR-425-5p | MIMAT0003393 | SEQ ID NO 490 | GaGtgATcgTgTcAT |
| ebv-miR-BART3-5p | MIMAT0003410 | SEQ ID NO 491 | AcActAAcaCtAgGT |
| ebv-miR-BART3-3p | MIMAT0003411 | SEQ ID NO 492 | GgTgaCTagTgGtGC |
| ebv-miR-BART4 | MIMAT0003412 | SEQ ID NO 493 | CcAgcAGcaTcAgGT |
| ebv-miR-BART5 | MIMAT0003413 | SEQ ID NO 494 | AgCtaTAttCaCcTT |
| ebv-miR-BART6-5p | MIMAT0003414 | SEQ ID NO 495 | AtGgaTTggAcCaAC |
| ebv-miR-BART6-3p | MIMAT0003415 | SEQ ID NO 496 | GcTagTCcgAtCcCC |
| ebv-miR-BART7 | MIMAT0003416 | SEQ ID NO 497 | AcActGGacTaTgAT |
| ebv-miR-BART8-5p | MIMAT0003417 | SEQ ID NO 498 | AaTctAGgaAaCcGT |
| ebv-miR-BART8-3p | MIMAT0003418 | SEQ ID NO 499 | CcCcaTAgaTtGtGA |
| ebv-miR-BART9 | MIMAT0003419 | SEQ ID NO 500 | GaCccATgaAgTgTT |
| ebv-miR-BART10 | MIMAT0003420 | SEQ ID NO 501 | AaCtcCAtgGtTaTG |
| ebv-miR-BART11-5p | MIMAT0003421 | SEQ ID NO 502 | AgCgcACcaAaCtGT |
| ebv-miR-BART11-3p | MIMAT0003422 | SEQ ID NO 503 | TcAgcCTggTgTgCG |
| ebv-miR-BART12 | MIMAT0003423 | SEQ ID NO 504 | AcCaaACacCaCaGG |
| ebv-miR-BART13 | MIMAT0003424 | SEQ ID NO 505 | TcCctGGcaAgTtAC |
| ebv-miR-BART14-5p | MIMAT0003425 | SEQ ID NO 506 | TcGgcAGcgTaGgGT |
| ebv-miR-BART14-3p | MIMAT0003426 | SEQ ID NO 507 | AcTacTGcaGcAtTT |
| kshv-miR-K12-12 | MIMAT0003712 | SEQ ID NO 508 | GgAatGGtgGcCtGG |
| ebv-miR-BART15 | MIMAT0003713 | SEQ ID NO 509 | AgGaaACaaAaCcAC |
| ebv-miR-BART16 | MIMAT0003714 | SEQ ID NO 510 | CaCacACccAcTcTA |
| ebv-miR-BART17-5p | MIMAT0003715 | SEQ ID NO 511 | AtGccTGcgTcCtCT |
| ebv-miR-BART17-3p | MIMAT0003716 | SEQ ID NO 512 | GaCacCAggCaTaCA |
| ebv-miR-BART18 | MIMAT0003717 | SEQ ID NO 513 | AgGaaGTgcGaAcTT |
| ebv-miR-BART19 | MIMAT0003718 | SEQ ID NO 514 | CcAagCAaaCaAaAC |
| ebv-miR-BART20-5p | MIMAT0003719 | SEQ ID NO 515 | AaGacATgcCtGcTA |
| ebv-miR-BART20-3p | MIMAT0003720 | SEQ ID NO 516 | AgGctGTgcCtTcAT |
| hsv1-miR-H1 | MIMAT0003744 | SEQ ID NO 517 | AcTtcCCgtCcTtCC |
| hsa-miR-758 | MIMAT0003879 | SEQ ID NO 518 | TgGacCAggTcAcAA |
| hsa-miR-671 | MIMAT0003880 | SEQ ID NO 519 | CcCtcCAggGcTtCC |
| hsa-miR-668 | MIMAT0003881 | SEQ ID NO 520 | GcCgaGCcgAgTgAC |
| hsa-miR-767-5p | MIMAT0003882 | SEQ ID NO 521 | AgAcaACcaTgGtGC |
| hsa-miR-767-3p | MIMAT0003883 | SEQ ID NO 522 | AtGggGTatGaGcAG |
| hsa-miR-454-5p | MIMAT0003884 | SEQ ID NO 523 | AcAatATtgAtAgGG |
| hsa-miR-454-3p | MIMAT0003885 | SEQ ID NO 524 | AaGcaATatTgCaCT |
| hsa-miR-769-5p | MIMAT0003886 | SEQ ID NO 525 | GaAccCAgaGgTcTC |
| hsa-miR-769-3p | MIMAT0003887 | SEQ ID NO 526 | AcCccGGagAtCcCA |
| hsa-miR-766 | MIMAT0003888 | SEQ ID NO 527 | GcTgtGGggCtGgAG |
| hsa-miR-765 | MIMAT0003945 | SEQ ID NO 528 | CcTtcCTtcTcCtCC |
| hsa-miR-768-5p | MIMAT0003946 | SEQ ID NO 529 | AcTttCAtcCtCcAA |
| hsa-miR-768-3p | MIMAT0003947 | SEQ ID NO 530 | AgTgtCAgcAtTgTG |
| hsa-miR-770-5p | MIMAT0003948 | SEQ ID NO 531 | GaCacGTggTaCtGG |
| hsa-miR-802 | MIMAT0004185 | SEQ ID NO 532 | TgAatCTttGtTaCT |
| hsa-miR-801 | MIMAT0004209 | SEQ ID NO 533 | CgCacGCagAgCaAT |
| hsa-miR-675 | MIMAT0004284 | SEQ ID NO 534 | GgCccTCtcCgCaCC |

### TEACHINGS

1. A pharmaceutical composition comprising a single stranded oligonucleotide, or a conjugate of said oligonucleotide, having a length of between 8 and 17 nucleobase units, and a pharmaceutically acceptable diluent, carrier, or adjuvant; wherein at least one of the nucleobase units of the single stranded oligonucleotide is a Locked Nucleic Acid (LNA) nucleobase unit, and wherein the single stranded oligonucleotide is complementary to a human microRNA sequence.
2. The pharmaceutical composition according to paragraph 1 wherein the single stranded oligonucleotide comprises a region of contiguous nucleobase sequence which is 100% complementary to the human microRNA seed region.
3. The pharmaceutical composition according to paragraph 1 or 2 wherein the single stranded oligonucleotide is complementary to the mature human microRNA sequence.
4. The pharmaceutical composition according to any one of paragraphs 1 - 3, wherein the single stranded oligonucleotide is complementary to a microRNA sequence selected from the group consisting of: hsa-let-7a, hsa-let-7b, hsa-let-7c, hsa-let-7d, hsa-let-7e, hsa-let-7f, hsa-miR-15a, hsa-miR-16, hsa-miR-17-5p, hsa-miR-17-3p, hsa-miR-18a, hsa-miR-19a, hsa-miR-19b, hsa-miR-20a, hsa-miR-21, hsa-miR-22, hsa-miR-23a, hsa-miR-189, hsa-miR-24, hsa-miR-25, hsa-miR-26a, hsa-miR-26b, hsa-miR-27a, hsa-miR-28, hsa-miR-29a, hsa-miR-30a-5p, hsa-miR-30a-3p, hsa-miR-31, hsa-miR-32, hsa-miR-33, hsa-miR-92, hsa-miR-93, hsa-miR-95, hsa-miR-96, hsa-miR-98, hsa-miR-99a, hsa-miR-100, hsa-miR-101, hsa-miR-29b, hsa-miR-103, hsa-miR-105, hsa-miR-106a, hsa-miR-107, hsa-miR-192, hsa-miR-196a, hsa-miR-197, hsa-miR-198, hsa-miR-199a, hsa-miR-199a*, hsa-miR-208, hsa-miR-129, hsa-miR-148a, hsa-miR-30c, hsa-miR-30d, hsa-miR-139, hsa-miR-147, hsa-miR-7, hsa-miR-10a, hsa-miR-10b, hsa-miR-34a, hsa-miR-181a, hsa-miR-181b, hsa-miR-181c, hsa-miR-182, hsa-miR-182*, hsa-miR-183, hsa-miR-187, hsa-miR-199b, hsa-miR-203, hsa-miR-204, hsa-miR-205, hsa-miR-210, hsa-miR-211, hsa-miR-212, hsa-miR-181a*, hsa-miR-214, hsa-miR-215, hsa-miR-216, hsa-miR-217, hsa-miR-218, hsa-miR-219, hsa-miR-220, hsa-miR-221, hsa-miR-222, hsa-miR-223, hsa-miR-224, hsa-miR-200b, hsa-let-7g, hsa-let-7i, hsa-miR-1, hsa-miR-15b, hsa-miR-23b, hsa-miR-27b, hsa-miR-30b, hsa-miR-122a, hsa-miR-124a, hsa-miR-125b, hsa-miR-128a, hsa-miR-130a, hsa-miR-132, hsa-miR-133a, hsa-miR-135a, hsa-miR-137, hsa-miR-138, hsa-miR-140, hsa-miR-141, hsa-miR-142-5p, hsa-miR-142-3p, hsa-miR-143, hsa-miR-144, hsa-miR-145, hsa-miR-152, hsa-miR-153, hsa-miR-191, hsa-miR-9, hsa-miR-9*, hsa-miR-125a, hsa-miR-126*, hsa-miR-126, hsa-miR-127, hsa-miR-134, hsa-miR-136, hsa-miR-146a, hsa-miR-149, hsa-miR-150, hsa-miR-154, hsa-miR-154*, hsa-miR-184, hsa-miR-185, hsa-miR-186, hsa-miR-188, hsa-miR-190, hsa-miR-193a, hsa-miR-194, hsa-miR-195, hsa-miR-206, hsa-miR-320, hsa-miR-200c, hsa-miR-155, hsa-miR-128b, hsa-miR-106b, hsa-miR-29c, hsa-miR-200a, hsa-miR-302a*, hsa-miR-302a, hsa-miR-34b, hsa-miR-34c, hsa-miR-299-3p, hsa-miR-301, hsa-miR-99b, hsa-miR-296, hsa-miR-130b, hsa-miR-30e-5p, hsa-miR-30e-3p, hsa-miR-361, hsa-miR-362, hsa-miR-363, hsa-miR-365, hsa-miR-302b*, hsa-miR-302b, hsa-miR-302c*, hsa-miR-302c, hsa-miR-302d, hsa-miR-367, hsa-miR-368, hsa-miR-369-3p, hsa-miR-370, hsa-miR-371, hsa-miR-372, hsa-miR-373*, hsa-miR-373, hsa-miR-374, hsa-miR-375, hsa-miR-376a, hsa-miR-377, hsa-miR-378, hsa-miR-422b, hsa-miR-379, hsa-miR-380-5p, hsa-miR-380-3p, hsa-miR-381, hsa-miR-382, hsa-miR-383, hsa-miR-340, hsa-miR-330, hsa-miR-328, hsa-miR-342, hsa-miR-337, hsa-miR-323, hsa-miR-326, hsa-miR-151, hsa-miR-135b, hsa-miR-148b, hsa-miR-331, hsa-miR-324-5p, hsa-miR-324-3p, hsa-miR-338, hsa-miR-339, hsa-miR-335, hsa-miR-133b, hsa-miR-325, hsa-miR-345, hsa-miR-346, ebv-miR-BHRF1-1, ebv-miR-BHRF1-2*, ebv-miR-BHRF1-2, ebv-miR-BHRF1-3, ebv-miR-BART1-5p, ebv-miR-BART2, hsa-miR-384, hsa-miR-196b, hsa-miR-422a, hsa-miR-423, hsa-miR-424, hsa-miR-425-3p, hsa-miR-18b, hsa-miR-20b, hsa-miR-448, hsa-miR-429, hsa-miR-449, hsa-miR-450, hcmv-miR-UL22A, hcmv-miR-UL22A*, hcmv-miR-UL36, hcmv-miR-UL112, hcmv-miR-UL148D, hcmv-miR-US5-1, hcmv-miR-US5-2, hcmv-miR-US25-1, hcmv-miR-US25-2-5p, hcmv-miR-US25-2-3p, hcmv-miR-US33, hsa-miR-191*, hsa-miR-200a*, hsa-miR-369-5p, hsa-miR-431, hsa-miR-433, hsa-miR-329, hsa-miR-453, hsa-miR-451, hsa-miR-452, hsa-miR-452*, hsa-miR-409-5p, hsa-miR-409-3p, hsa-miR-412, hsa-miR-410, hsa-miR-376b, hsa-miR-483, hsa-miR-484, hsa-miR-485-5p, hsa-miR-485-3p, hsa-miR-486, hsa-miR-487a, kshv-miR-K12-10a, kshv-miR-K12-10b, kshv-miR-K12-11, kshv-miR-K12-1, kshv-miR-K12-2, kshv-miR-K12-9*, kshv-miR-K12-9, kshv-miR-K12-8, kshv-miR-K12-7, kshv-miR-K12-6-5p, kshv-miR-K12-6-3p, kshv-miR-K12-5, kshv-miR-K12-4-5p, kshv-miR-K12-4-3p, kshv-miR-K12-3, kshv-miR-K12-3*, hsa-miR-488, hsa-miR-489, hsa-miR-490, hsa-miR-491, hsa-miR-511, hsa-miR-146b, hsa-miR-202*, hsa-miR-202, hsa-miR-492, hsa-miR-493-5p, hsa-miR-432, hsa-miR-432*, hsa-miR-494, hsa-miR-495, hsa-miR-496, hsa-miR-193b, hsa-miR-497, hsa-miR-181d, hsa-miR-512-5p, hsa-miR-512-3p, hsa-miR-498, hsa-miR-520e, hsa-miR-515-5p, hsa-miR-515-3p, hsa-miR-519e*, hsa-miR-519e, hsa-miR-520f, hsa-miR-526c, hsa-miR-519c, hsa-miR-520a*, hsa-miR-520a, hsa-miR-526b, hsa-miR-526b*, hsa-miR-519b, hsa-miR-525, hsa-miR-525*, hsa-miR-523, hsa-miR-518f*, hsa-miR-518f, hsa-miR-520b, hsa-miR-518b, hsa-miR-526a, hsa-miR-520c, hsa-miR-518c*, hsa-miR-518c, hsa-miR-524*, hsa-miR-524, hsa-miR-517*, hsa-miR-517a, hsa-miR-519d, hsa-miR-521, hsa-miR-520d*, hsa-miR-520d, hsa-miR-517b, hsa-miR-520g, hsa-miR-516-5p, hsa-miR-516-3p, hsa-miR-518e, hsa-miR-527, hsa-miR-518a, hsa-miR-518d, hsa-miR-517c, hsa-miR-520h, hsa-miR-522, hsa-miR-519a, hsa-miR-499, hsa-miR-500, hsa-miR-501, hsa-miR-502, hsa-miR-503, hsa-miR-504, hsa-miR-505, hsa-miR-513, hsa-miR-506, hsa-miR-507, hsa-miR-508, hsa-miR-509, hsa-miR-510, hsa-miR-514, hsa-miR-532, hsa-miR-299-5p, hsa-miR-18a*, hsa-miR-455, hsa-miR-493-3p, hsa-miR-539, hsa-miR-544, hsa-miR-545, hsa-miR-487b, hsa-miR-551a, hsa-miR-552, hsa-miR-553, hsa-miR-554, hsa-miR-92b, hsa-miR-555, hsa-miR-556, hsa-miR-557, hsa-miR-558, hsa-miR-559, hsa-miR-560, hsa-miR-561, hsa-miR-562, hsa-miR-563, hsa-miR-564, hsa-miR-565, hsa-miR-566, hsa-miR-567, hsa-miR-568, hsa-miR-551b, hsa-miR-569, hsa-miR-570, hsa-miR-571, hsa-miR-572, hsa-miR-573, hsa-miR-574, hsa-miR-575, hsa-miR-576, hsa-miR-577, hsa-miR-578, hsa-miR-579, hsa-miR-580, hsa-miR-581, hsa-miR-582, hsa-miR-583, hsa-miR-584, hsa-miR-585, hsa-miR-548a, hsa-miR-586, hsa-miR-587, hsa-miR-548b, hsa-miR-588, hsa-miR-589, hsa-miR-550, hsa-miR-590, hsa-miR-591, hsa-miR-592, hsa-miR-593, hsa-miR-595, hsa-miR-596, hsa-miR-597, hsa-miR-598, hsa-miR-599, hsa-miR-600, hsa-miR-601, hsa-miR-602, hsa-miR-603, hsa-miR-604, hsa-miR-605, hsa-miR-606, hsa-miR-607, hsa-miR-608, hsa-miR-609, hsa-miR-610, hsa-miR-611, hsa-miR-612, hsa-miR-613, hsa-miR-614, hsa-miR-615, hsa-miR-616, hsa-miR-548c, hsa-miR-617, hsa-miR-618, hsa-miR-619, hsa-miR-620, hsa-miR-621, hsa-miR-622, hsa-miR-623, hsa-miR-624, hsa-miR-625, hsa-miR-626, hsa-miR-627, hsa-miR-628, hsa-miR-629, hsa-miR-630, hsa-miR-631, hsa-miR-33b, hsa-miR-632, hsa-miR-633, hsa-miR-634, hsa-miR-635, hsa-miR-636, hsa-miR-637, hsa-miR-638, hsa-miR-639, hsa-miR-640, hsa-miR-641, hsa-miR-642, hsa-miR-643, hsa-miR-644, hsa-miR-645, hsa-miR-646, hsa-miR-647, hsa-miR-648, hsa-miR-649, hsa-miR-650, hsa-miR-651, hsa-miR-652, hsa-miR-548d, hsa-miR-661, hsa-miR-662, hsa-miR-663, hsa-miR-449b, hsa-miR-653, hsa-miR-411, hsa-miR-654, hsa-miR-655, hsa-miR-656, hsa-miR-549, hsa-miR-657, hsa-miR-658, hsa-miR-659, hsa-miR-660, hsa-miR-421, hsa-miR-542-5p, hcmv-miR-US4, hcmv-miR-UL70-5p, hcmv-miR-UL70-3p, hsa-miR-363*, hsa-miR-376a*, hsa-miR-542-3p, ebv-miR-BART1-3p, hsa-miR-425-5p, ebv-miR-BART3-5p, ebv-miR-BART3-3p, ebv-miR-BART4, ebv-miR-BART5, ebv-miR-BART6-5p, ebv-miR-BART6-3p, ebv-miR-BART7, ebv-miR-BART8-5p, ebv-miR-BART8-3p, ebv-miR-BART9, ebv-miR-BART10, ebv-miR-BART11-5p, ebv-miR-BART11-3p, ebv-miR-BART12, ebv-miR-BART13, ebv-miR-BART14-5p, ebv-miR-BART14-3p, kshv-miR-K12-12, ebv-miR-BART15, ebv-miR-BART16, ebv-miR-BART17-5p, ebv-miR-BART17-3p, ebv-miR-BART18, ebv-miR-BART19, ebv-miR-BART20-5p, ebv-miR-BART20-3p, hsv1-miR-H1, hsa-miR-758, hsa-miR-671, hsa-miR-668, hsa-miR-767-5p, hsa-miR-767-3p, hsa-miR-454-5p, hsa-miR-454-3p, hsa-miR-769-5p, hsa-miR-769-3p, hsa-miR-766, hsa-miR-765, hsa-miR-768-5p, hsa-miR-768-3p, hsa-miR-770-5p, hsa-miR-802, hsa-miR-801, hsa-miR-675.
5. The pharmaceutical composition according to any one of paragraphs 1 - 4 wherein the first nucleobase of the single stranded oligonucleotide, counting from the 3' end, is a nucleotide analogue, such as an LNA unit.
6. The pharmaceutical composition according to any one of paragraphs 1 - 5, wherein the second nucleobase of the single stranded oligonucleotide, counting from the 3' end, is a nucleotide analogue, such as an LNA unit.
7. The pharmaceutical composition according to any one of paragraphs 1 -6, wherein the ninth and/or the tenth nucleotide of the single stranded oligonucleotide, counting from the 3' end, is a nucleotide analogue, such as an LNA unit.
8. The pharmaceutical composition according to paragraph 7 wherein the ninth nucleobase of the single stranded oligonucleotide, counting from the 3' end is a nucleotide analogue, such as an LNA unit.
9. The pharmaceutical composition according to paragraph 7 wherein the tenth nucleobase of the single stranded oligonucleotide, counting from the 3' end is a nucleotide analogue, such as an LNA unit.
10. The pharmaceutical composition according to paragraph 7 wherein both the ninth and the tenth nucleobase of the single stranded oligonucleotide, calculated from the 3' end is a nucleotide analogue, such as an LNA unit.
11. The pharmaceutical composition according to any one of paragraphs 1 - 10, wherein the single stranded oligonucleotide does not comprise a region of more than 5 consecutive DNA nucleotide units.
12. The pharmaceutical composition according to any one of paragraphs 1 - 11, wherein the single stranded oligonucleotide comprises of at least one region of at least two consecutive nucleotide analogue units, such as at least two consecutive LNA units.
13. The pharmaceutical composition according to any one of paragraphs 1 - 12, wherein the single stranded oligonucleotide does not comprise a region of more than 7 consecutive nucleotide analogue units, such as LNA units.
14. The pharmaceutical composition according to paragraph 13, wherein the single stranded oligonucleotide does not comprise a region of more than 3 consecutive nucleotide analogue units, such as LNA units.
15. The pharmaceutical composition according to any one of paragraphs 1 - 14 wherein the first or second 3' nucleobase of the single stranded oligonucleotide corresponds to the second 5' nucleotide of the microRNA sequence.
16. The pharmaceutical composition according to paragraph 15 wherein nucleobase units 1 to 6 (inclusive) of the single stranded oligonucleotide as measured from the 3' end the region of the single stranded oligonucleotide are complementary to the microRNA seed region sequence.
17. The pharmaceutical composition according to paragraph 15 wherein nucleobase units 1 to 7 (inclusive) of the single stranded oligonucleotide as measured from the 3' end the region of the single stranded oligonucleotide are complementary to the microRNA seed region sequence.
18. The pharmaceutical composition according to paragraph 15 wherein nucleobase units 2 to 7 (inclusive) of the single stranded oligonucleotide as measured from the 3' end the region of the single stranded oligonucleotide are complementary to the microRNA seed region sequence.
19. The pharmaceutical composition according to any one of paragraphs 1 - 18, wherein the single stranded oligonucleotide comprises at least one nucleotide analogue unit, such as at least one LNA unit, in a position which is within the region complementary to the miRNA seed region.
20. The pharmaceutical composition according to paragraph 19, wherein the nucleobase sequence of the single stranded oligonucleotide which is complementary to the sequence of the microRNA seed region, is selected from the group consisting of (X)Xxxxxx, (X)xXxxxx, (X)xxXxxx, (X)xxxXxx, (X)xxxxXx and (X)xxxxxX, as read in a 3' - 5'direction, wherein "X" denotes a nucleotide analogue, (X) denotes an optional nucleotide analogue, such as an LNA unit, and "x" denotes a DNA or RNA nucleotide unit,
21. The pharmaceutical composition according to paragraph 19, wherein the single stranded oligonucleotide comprises at least two nucleotide analogue units, such as at least two LNA units, in positions which are complementary to the miRNA seed region.
22. The pharmaceutical composition according to paragraph 21, wherein the nucleobase sequence of the single stranded oligonucleotide which is complementary to the sequence of the microRNA seed region, is selected from the group consisting of (X)XXxxxx, (X)XxXxxx, (X)XxxXxx, (X)XxxxXx, (X)XxxxxX, (X)xXXxxx, (X)xXxXxx, (X)xXxxXx, (X)xXxxxX, (X)xxXXxx, (X)xxXxXx, (X)xxXxxX, (X)xxxXXx, (X)xxxXxX and (X)xxxxXX, wherein "X" denotes a nucleotide analogue, such as an LNA unit, (X) denotes an optional nucleotide analogue, such as an LNA unit, and "x" denotes a DNA or RNA nucleotide unit.
23. The pharmaceutical composition according to paragraph 19, wherein the single stranded oligonucleotide comprises at least three nucleotide analogue units, such as at least three LNA units, in positions which are complementary to the miRNA seed region.
24. The pharmaceutical composition according to paragraph 21, wherein the nucleobase sequence of the single stranded oligonucleotide which is complementary to the sequence of the microRNA seed region, is selected from the group consisting of (X)XXXxxx, (X)xXXXxx, (X)xxXXXx, (X)xxxXXX, (X)XXxXxx, (X)XXxxXx, (X)XXxxxX, (X)xXXxXx, (X)xXXxxX, (X)xxXXxX, (X)XxXXxx, (X)XxxXXx, (X)XxxxXX, (X)xXxXXx, (X)xXxxXX, (X)xxXxXX, (X)xXxXxX and (X)XxXxXx, wherein "X" denotes a nucleotide analogue, such as an LNA unit, (X) denotes an optional nucleotide analogue, such as an LNA unit, and "x" denotes a DNA or RNA nucleotide unit.
25. The pharmaceutical composition according to paragraph 19, wherein the single stranded oligonucleotide comprises at least four nucleotide analogue units, such as at least four LNA units, in positions which are complementary to the miRNA seed region.
26. The pharmaceutical composition according to paragraph 25, wherein the nucleobase sequence of the single stranded oligonucleotide which is complementary to the sequence of the microRNA seed region, is selected from the group consisting of (X)xxXXX, (X)xXxXXX, (X)xXXxXX, (X)xXXXxX, (X)xXXXXx, (X)XxxXXXX, (X)XxXxXX, (X)XxXXxX, (X)XxXXx, (X)XXxxXX, (X)XXxXxX, (X)XXxXXx, (X)XXXxxX, (X)XXXxXx, and (X)XXXXxx, wherein "X" denotes a nucleotide analogue, such as an LNA unit, (X) denotes an optional nucleotide analogue, such as an LNA unit, and "x" denotes a DNA or RNA nucleotide unit.
27. The pharmaceutical composition according to paragraph 19, wherein the single stranded oligonucleotide comprises at least five nucleotide analogue units, such as at least five LNA units, in positions which are complementary to the miRNA seed region.
28. The pharmaceutical composition according to paragraph 27, wherein the nucleobase sequence of the single stranded oligonucleotide which is complementary to the sequence of the microRNA seed region, is selected from the group consisting of (X)xXXXXX, (X)XxXXXX, (X)XXxXXX, (X)XXXxXX, (X)XXXXxX and (X)XXXXXx, wherein "X" denotes a nucleotide analogue, such as an LNA unit, (X) denotes an optional nucleotide analogue, such as an LNA unit, and "x" denotes a DNA or RNA nucleotide unit.
29. The pharmaceutical composition according to paragraph 19, wherein the single stranded oligonucleotide comprises six or seven nucleotide analogue units, such as six or seven LNA units, in positions which are complementary to the miRNA seed region.
30. The pharmaceutical composition according to paragraph 29, wherein the nucleobase sequence of the single stranded oligonucleotide which is complementary to the sequence of the microRNA seed region, is selected from the group consisting of XXXXXX, XxXXXXX, XXxXXXX, XXXxXXX, XXXXxXX, XXXXXxX and XXXXXXx, wherein "X" denotes a nucleotide analogue, such as an LNA unit, such as an LNA unit, and "x" denotes a DNA or RNA nucleotide unit.
31. The pharmaceutical composition according to any one of paragraphs 1 - 30, wherein the two nucleobase motif at position 7 to 8, counting from the 3' end of the single stranded oligonucleotide is selected from the group consisting of xx, XX, xX and Xx, wherein "X" denotes a nucleotide analogue, such as an LNA unit, such as an LNA unit, and "x" denotes a DNA or RNA nucleotide unit.
32. The pharmaceutical composition according to paragraph 31, wherein the two nucleobase motif at position 7 to 8, counting from the 3' end of the single stranded oligonucleotide is selected from the group consisting of XX, xX and Xx, wherein "X" denotes a nucleotide analogue, such as an LNA unit, such as an LNA unit, and "x" denotes a DNA or RNA nucleotide unit.
33. The pharmaceutical composition according to any one of paragraphs 1 - 32, wherein the single stranded oligonucleotide comprises at least 12 nucleobases and wherein the two nucleobase motif at position 11 to 12, counting from the 3' end of the single stranded oligonucleotide is selected from the group consisting of xx, XX, xX and Xx, wherein "X" denotes a nucleotide analogue, such as an LNA unit, such as an LNA unit, and "x" denotes a DNA or RNA nucleotide unit.
34. The pharmaceutical composition according to paragraph 33, wherein the single stranded oligonucleotide comprises at least 12 nucleobases and wherein the two nucleobase motif at position 11 to 12, counting from the 3' end of the single stranded oligonucleotide is selected from the group consisting of XX, xX and Xx, wherein "X" denotes a nucleotide analogue, such as an LNA unit, such as an LNA unit, and "x" denotes a DNA or RNA nucleotide unit.
35. The pharmaceutical composition according to any one of paragraphs 1 - 34, wherein the single stranded oligonucleotide comprises at least 13 nucleobases and wherein the three nucleobase motif at position 11 to 13, counting from the 3' end, is selected from the group consisting of xxx, Xxx, xXx, xxX, XXx, XxX, xXX and XXX, wherein "X" denotes a nucleotide analogue, such as an LNA unit, such as an LNA unit, and "x" denotes a DNA or RNA nucleotide unit.
36. The pharmaceutical composition according to paragraph 35, wherein the three nucleobase motif at position 11 to 13, counting from the 3' end, is selected from the group consisting of Xxx, xXx, xxX, XXx, XxX, xXX and XXX, wherein "X" denotes a nucleotide analogue, such as an LNA unit, such as an LNA unit, and "x" denotes a DNA or RNA nucleotide unit.
37. The pharmaceutical composition according to any one of paragraphs 1 - 36, wherein the single stranded oligonucleotide comprises at least 14 nucleobases and wherein the four nucleobase motif at positions 11 to 14, counting from the 3' end, is selected from the group consisting of xxxx, Xxxx, xXxx, xxXx, xxxX, XXxx, XxXx, XxxX, xXXx, xXxX, xxXX, XXXx, XxXX, xXXX, XXxX and XXXX wherein "X" denotes a nucleotide analogue, such as an LNA unit, such as an LNA unit, and "x" denotes a DNA or RNA nucleotide unit.
38. The pharmaceutical composition according to paragraph 37, wherein the four nucleobase motif at position 11 to 14, counting from the 3' end, is selected from the group consisting of Xxxx, xXxx, xxXx, xxxX, XXxx, XxXx, XxxX, xXXx, xXxX, xxXX, XXXx, XxXX, xXXX, XXxX and XXXX, wherein "X" denotes a nucleotide analogue, such as an LNA unit, and "x" denotes a DNA or RNA nucleotide unit.
39. The pharmaceutical composition according to any one of paragraphs 1 - 38, wherein said oligonucleotide comprises 15 nucleobases and the five nucleobase motif at position 11 to 15, counting from the 3' end, is selected from the group consisting of Xxxxx, xXxxx, xxXxx, xxxXx, xxxxX, XXxxx, XxXxx, XxxXx, XxxxX, xXXxx, xXxXx, xXxxX, xxXXx, xxXxX, xxxXX, XXXxx, XXxxX, XxxXX, xXXXx, xxXXX, XXxXX, XxXxX, XXXXx, XXXxX, XXxXX, XxXXXX, xXXXX, and XXXXX wherein "X" denotes a nucleotide analogue, such as an LNA unit, such as an LNA unit, and "x" denotes a DNA or RNA nucleotide unit.
40. The pharmaceutical composition according to any one of paragraphs 1 - 39, wherein said oligonucleotide comprises 16 nucleobases and the six nucleobase motif at positions 11 to 16, counting from the 3' end, is selected from the group consisting of Xxxxxx, xXxxxx, xxXxxx, xxxXxx, xxxxXx, xxxxxX, XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX, xxxxXX, XXXxxx, XXxXxx, XXxxXx, XXxxxX, XxXXxx, XxXxXx, XxXxxX, XxxXXx, XxxXxX, XxxxXX, xXXXxx, xXXxXx, xXXxxX, xXxXXx, xXxXxX, xXxxXX, xxXXXx, xxXXxX, xxXxXX, xxxXXX, XXXXxx, XXXxxX, XXxxXX, XxxXXX, xxXXXX, xXxXXX, XxXxXX, XXxXxX, XXXxXx, xXXxXX, XxXXxX, XXxXXx, xXXXxX, XxXXXx, xXXXXx, xXXXXX, XxXXXX, XXxXXX, XXXxXX, XXXXxX, XXXXXx, and XXXXXX wherein "X" denotes a nucleotide analogue, such as an LNA unit, such as an LNA unit, and "x" denotes a DNA or RNA nucleotide unit.
41. The pharmaceutical composition according to paragraph 40, wherein the six nucleobase motif at positions 11 to 16 of the single stranded oligonucleotide, counting from the 3' end, is xxXxxX, wherein "X" denotes a nucleotide analogue, such as an LNA unit, such as an LNA unit, and "x" denotes a DNA or RNA nucleotide unit.
42. The pharmaceutical composition according to any one of paragraphs 1 - 41 wherein the nucleobase motif for the three 5' most nucleobases, is selected from the group consisting of Xxx, xXx, xxX, XXx, XxX, xXX and XXX, wherein "X" denotes a nucleotide analogue, such as an LNA unit, such as an LNA unit, and "x" denotes a DNA or RNA nucleotide unit.
43. The pharmaceutical composition according to any one or paragraphs 18 - 42, wherein "x" denotes a DNA unit.
44. The pharmaceutical composition according to any one of paragraphs 1 - 43, wherein the single stranded oligonucleotide comprises a nucleotide analogue unit, such as an LNA unit, at the 5' end.
45. The pharmaceutical composition according to any one of paragraphs 1 - 44, wherein the nucleotide analogue units, such as X, are independently selected form the group consisting of: 2'-O-alkyl-RNA unit, 2'-OMe-RNA unit, 2'-amino-DNA unit, 2'-fluoro-DNA unit, LNA unit, PNA unit, HNA unit, INA unit.
46. The pharmaceutical composition according to paragraph 45 wherein all nucleobases are nucleotide analogue units.
47. The pharmaceutical composition according to paragraph 45 or 46, wherein the nucleotide analogue units, such as X, are independently selected form the group consisting of: 2'-OMe-RNA units, 2'-fluoro-DNA units, and LNA units,
48. The pharmaceutical composition according to any one of paragraph 45 - 47 wherein the single stranded oligonucleotide comprises said at least one LNA analogue unit and at least one further nucleotide analogue unit other than LNA.
49. The pharmaceutical composition according to paragraph 48, wherein the non-LNA nucleotide analogue unit or units are independently selected from 2'-OMe RNA units and 2'-fluoro DNA units.
50. The pharmaceutical composition according to paragraph 49, wherein the single stranded oligonucleotide consists of at least one sequence XYX or YXY, wherein X is LNA and Y is either a 2'-OMe RNA unit and 2'-fluoro DNA unit.
51. The pharmaceutical composition according to paragraph 50, wherein the sequence of nucleobases of the single stranded oligonucleotide consists of alternative X and Y units.
52. The pharmaceutical composition according to any one of paragraphs 1 - 45, wherein the single stranded oligonucleotide comprises alternating LNA and DNA units (Xx) or (xX).
53. The pharmaceutical composition according to any one of paragraphs 1 - 45, wherein the single stranded oligonucleotide comprises a motif of alternating LNA followed by 2 DNA units (Xxx), xXx or xxX.
54. The pharmaceutical composition according to any one of paragraphs 51 - 53 wherein at least one of the DNA or non-LNA nucleotide analogue units are replaced with a LNA nucleobase in a position selected from the positions identified as LNA nucleobase units in any one of the preceding paragraphs.
55. The pharmaceutical composition according to any one of paragraphs paragraph 19 - 54 wherein "X" donates an LNA unit.
56. The pharmaceutical composition according to any one of paragraphs 1 - 55, wherein the single stranded oligonucleotide comprises at least 5 LNA units.
57. The pharmaceutical composition according to paragraph 57, wherein the single stranded oligonucleotide comprises at least 7 LNA units.
58. The pharmaceutical composition according to any one of paragraphs 1 - 57, wherein at least one of the LNA nucleobases is either cytosine or guanine.
59. The pharmaceutical composition according to paragraph 58, wherein at least three of the LNA nucleobases are independently selected from either cytosine or guanine.
60. The pharmaceutical composition according to any one of paragraphs 1 - 59, wherein the nucleotide analogues have a higher thermal duplex stability to a complementary RNA nucleotide than the thermal duplex stability of an equivalent DNA nucleotide to said complementary RNA nucleotide.
61. The pharmaceutical composition according to any one of paragraphs 1 - 60, wherein the nucleotide analogues confer enhanced serum stability to the single stranded oligonucleotide.
62. The pharmaceutical composition according to any one of paragraphs 1 - 61, wherein the single stranded oligonucleotide forms an A-helix conformation with a complementary single stranded RNA molecule.
63. The pharmaceutical composition according to any one of paragraphs 1 - 62 wherein the oligonucleotide does not mediate RNAseH based cleavage of a complementary single stranded RNA molecule.
64. The pharmaceutical composition according to any one of paragraphs 1- 63 wherein the single stranded oligonucleotide is capable of forming a duplex with a complementary single stranded RNA nucleic acid molecule with phosphodiester internucleoside linkages, wherein the duplex has a Tₘ of at least about 60°C.
65. The pharmaceutical composition according to paragraph 64 wherein the single stranded oligonucleotide is capable of forming a duplex with a complementary single stranded RNA nucleic acid molecule with phosphodiester internucleoside linkages, wherein the duplex has a Tₘ of between about 70°C to about 95°C
66. The pharmaceutical composition according to paragraph 64 wherein the single stranded oligonucleotide is capable of forming a duplex with a complementary single stranded RNA nucleic acid molecule with phosphodiester internucleoside linkages, wherein the duplex has a Tₘ of between about 70°C to about 90°C, such as between about 70°C and about 85°C.
67. The pharmaceutical composition according to any one of paragraphs 1 - 66 wherein the single stranded oligonucleotide is capable of forming a duplex with a complementary single stranded DNA nucleic acid molecule with phosphodiester internucleoside linkages, wherein the duplex has a Tₘ of between about 50°C to about 90°C
68. The pharmaceutical composition according to any one of paragraphs 1 - 67, wherein the single stranded oligonucleotide has a length of from between 10 to 16 nucleobases, such as a length of 10, 11, 12, 13,14, 15 or 16 nucleobases.
69. The pharmaceutical composition according to paragraph 68, wherein the single stranded oligonucleotide has a length of 15 or 16 nucleobases.
70. The pharmaceutical composition according to any one of paragraphs 1 - 69, wherein the LNA unit or units are independently selected from the group consisting of oxy-LNA, thio-LNA, and amino-LNA, in either of the D-β and L-α configurations or combinations thereof.
71. The pharmaceutical composition according to paragraph 70, wherein the LNA unit or units are beta D oxy-LNA.
72. The pharmaceutical composition according to paragraph 70, wherein the LNA units are in alpha-L amino LNA.
73. The pharmaceutical composition according to any one of paragraphs 1 - 72, wherein the single stranded oligonucleotide comprises between 3 and 17 LNA units.
74. The pharmaceutical composition according to any of one paragraphs 1 - 73, wherein the oligonucleotide comprises at least one internucleoside linkage group which differs from phosphate.
75. The pharmaceutical composition according to paragraph 74, wherein the oligonucleotide comprises at least one phosphorothioate internucleoside linkage.
76. The pharmaceutical composition according to paragraph 75, wherein the oligonucleotide comprises phosphodiester and phosphorothioate linkages.
77. The pharmaceutical composition according to paragraph 75, wherein all the internucleoside linkages are phosphorothioate linkages.
78. The pharmaceutical composition according to any one of paragraphs 1 - 76, wherein the oligonucleotide comprises at least one phosphodiester internucleoside linkage.
79. The pharmaceutical composition according to paragraph 78, wherein all the internucleoside linkages are phosphodiester linkages.
80. The pharmaceutical composition according to any one of paragraphs 1 - 79, wherein said carrier is saline or buffered saline.
81. A single stranded oligonucleotide as defined in any one of paragraphs 1 - 80, wherein said single stranded oligonucleotide comprises at least one phosphorothioate linkage and/or wherein at least the first 5'and/or last 3' nucleobase is an LNA nucleobase.
82. Use of an oligonucleotide as defined in any of paragraphs 1-81 for the manufacture of a medicament for the treatment of a disease or medical disorder associated with the presence or over-expression of the microRNA.
83. A method for the treatment of a disease or medical disorder associated with the presence or over-expression of the microRNA, comprising the step of administering a composition according to any one of paragraphs 1 - 81 to a person in need of treatment.
84. A method for reducing the effective amount of a miRNA target in a cell or an organism, comprising administering a composition or a single stranded oligonucleotide according to any one of paragraphs 1 - 81 to the cell or organism.
85. The method according to paragraph 84 wherein the reduction of the effective amount of the miRNA target occurs via in vivo antagonism.
86. A method for de-repression of a target mRNA of a miRNA in a cell or an organism, comprising administering a composition or a single stranded oligonucleotide according to any one of paragraphs 1 - 81 to the cell or organism.
87. A method for the synthesis of a single stranded oligonucleotide targeted against a human microRNA, such as a single stranded oligonucleotide according to any one of paragraphs 1 - 81 said method comprising the steps of:
   a. Optionally selecting a first nucleobase, counting from the 3' end, which is a LNA nucleobase.
   b. Optionally selecting a second nucleobase, counting from the 3' end, which is a LNA nucleobase.
   c. Selecting a region of the single stranded oligonucleotide which corresponds to the miRNA seed region, wherein said region is as defined in any one of paragraphs 16 - 30.
   d. Optionally selecting a seventh and eight nucleobase as according to paragraph 31 or 32.
   e. Optionally selecting a 5' region of the single stranded oligonucleotide according to any one of paragraphs 33 - 41.
   f. Optionally selecting a 5' terminal of the single stranded oligonucleotide according to paragraph 42.

   Wherein the synthesis is performed by sequential synthesis of the regions defined in steps a - f, wherein said synthesis may be performed in either the 3'-5' (a to f) or 5' - 3' (f to a)direction, and wherein said single stranded oligonucleotide is complementary to a sequence of the miRNA target.
88. The method according to paragraph 87, wherein the synthesis is performed in the 3' to 5' direction a - f.
89. The method according to paragraph 87 and 88, wherein the miRNA target is selected from the group consisting of the miRNA targets shown in table 2.
90. The use of a single stranded oligonucleotide of between 8 - 16 nucleobases in length, for the manufacture of a medicament for the treatment of a disease or medical disorder associated with the presence or over-expression of the microRNA.
91. The use of a single stranded oligonucleotide of between 8 - 16 nucleobases in length, for the manufacture of a medicament for the treatment of a disease or medical disorder associated with the presence or over-expression of the microRNA
92. The treatment of a disease or medical disorder associated with the presence or over-expression of the microRNA, comprising the step of administering a composition (such as the pharmaceutical composition) comprising a single stranded oligonucleotide of between 8 - 16 nucleobases in length to a person in need of treatment.
93. A method for reducing the effective amount of a miRNA target in a cell or an organism, comprising administering a composition (such as the pharmaceutical composition) comprising a single stranded oligonucleotide of between 8 - 16 nucleobases to the cell or the organism.
94. A method for de-repression of a target mRNA of a miRNA in a cell or an organism, comprising a single stranded oligonucleotide of between 8 - 16 nucleobases or (or a composition comprising said oligonucleotide) to the cell or the organism.
95. A pharmaceutical composition comprising a single stranded oligonucleotide of a length of between 8 and 16 nucleobase units, a pharmaceutically acceptable diluent, carrier, or adjuvant, wherein at least one of the nucleobase units of the single stranded oligonucleotide is a nucleotide analogue, and wherein the single stranded oligonucleotide is complementary to a human microRNA sequence.

## Claims

1. A single stranded oligonucleotide, 8-17 nucleotides in length, capable of reducing the effective amount of a mature human microRNA in a cell, wherein at least one of the nucleobase units of the oligonucleotide is a LNA nucleotide unit, and wherein all the internucleoside linkages are phosphorothioate linkages, wherein the oligonucleotide is complementary to the mature human microRNA sequence, wherein the single stranded oligonucleotide comprises a region of contiguous nucleobase sequence which is 100% complementary to the human microRNA seed region, and wherein the oligonucleotide comprises at least one LNA nucleotide unit in a position which is within the region complementary to the miRNA seed region, further wherein:
(a) nucleobase units 1 to 6 (inclusive) of the single stranded oligonucleotide as measured from the 3' end of the single stranded oligonucleotide are complementary to the microRNA seed region sequence; or
(b) nucleobase units 1 to 7 (inclusive) of the single stranded oligonucleotide as measured from the 3' end of the single stranded oligonucleotide are complementary to the microRNA seed region sequence; or
(c) nucleobase units 2 to 7 (inclusive) of the single stranded oligonucleotide as measured from the 3' end of the single stranded oligonucleotide are complementary to the microRNA seed region sequence.

2. A single stranded oligonucleotide, 8-17 nucleotides in length, capable of reducing the effective amount of a mature human microRNA in a cell, wherein at least one of the nucleobase units of the oligonucleotide is a LNA nucleotide unit, and wherein all the internucleoside linkages are phosphorothioate linkages, wherein the oligonucleotide is complementary to the mature human microRNA sequence, wherein the single stranded oligonucleotide comprises a region of contiguous nucleobase sequence which is 100% complementary to the human microRNA seed region, and wherein the oligonucleotide comprises at least one LNA nucleotide unit in a position which is within the region complementary to the miRNA seed region, wherein the oligonucleotide is for use as a medicament, further wherein:
(a) nucleobase units 1 to 6 (inclusive) of the single stranded oligonucleotide as measured from the 3' end of the single stranded oligonucleotide are complementary to the microRNA seed region sequence; or
(b) nucleobase units 1 to 7 (inclusive) of the single stranded oligonucleotide as measured from the 3' end of the single stranded oligonucleotide are complementary to the microRNA seed region sequence; or
(c) nucleobase units 2 to 7 (inclusive) of the single stranded oligonucleotide as measured from the 3' end of the single stranded oligonucleotide are complementary to the microRNA seed region sequence.

3. The oligonucleotide according to claim 1 or the oligonucleotide for use according to claim 2, wherein the first or second 3' nucleobase of the single stranded oligonucleotide corresponds to the second 5' nucleotide of the microRNA sequence.

4. The oligonucleotide of claim 1 or claim 3, or the oligonucleotide for use according to claim 2, wherein the oligonucleotide is capable of forming a duplex with a complementary single stranded RNA nucleic acid molecule with phosphodiester internucleoside linkages, wherein the duplex has a Tm of at least about 60°C.

5. The oligonucleotide of any one of claims 1, 3 or 4, or the oligonucleotide for use according to any one of claims 2-4, wherein the oligonucleotide is capable of forming a duplex with a complementary single stranded DNA nucleic acid molecule with phosphodiester internucleoside linkages, wherein the duplex has a Tm of 60 - 90°C.

6. The oligonucleotide of any one of claims 1 or 3-5, or the oligonucleotide for use according to any one of claims 2-5, which comprises at least one 3' terminal LNA nucleotide unit.

7. The oligonucleotide of any one of claims 1 or 3-6, or the oligonucleotide for use according to any one of claims 2-6, which comprises at least one 5' terminal LNA nucleotide unit.

8. The oligonucleotide of any one of claims 1 or 3-7, or the oligonucleotide for use according to any one of claims 2-7, wherein at least 30% of the nucleobases are LNA units.

9. The oligonucleotide of any one of claims 1 or 3-8, or the oligonucleotide for use according to any one of claims 2-8, wherein the oligonucleotide comprises at least one region of at least two consecutive LNA nucleotide units.

10. The oligonucleotide of any one of claims 1 or 3-9, or the oligonucleotide for use according to any one of claims 2-9, wherein the oligonucleotide does not comprise a region of more than 7 consecutive LNA nucleotide units.

11. The oligonucleotide of any one of claims 1 or 3-10, or the oligonucleotide for use according to any one of claims 2-10, wherein the oligonucleotide does not comprise a region of more than 6, or more than 5, or more than 4, or more than 3 or more than 2 consecutive LNA nucleotide units.

12. The oligonucleotide of any one of claims 1 or 3-11, or the oligonucleotide for use according to any one of claim 2-11, which does not comprise a region of more than 5 consecutive DNA nucleotide units.

13. The oligonucleotide of any one of claims 1 or 3-12, or the oligonucleotide for use according to any one of claims 2-12, wherein the oligonucleotide does not mediate RNAseH based cleavage of a complementary single stranded RNA molecule.

14. The oligonucleotide of any one of claims 1 or 3-13, or the oligonucleotide for use according to any one of claims 2-13, wherein the oligonucleotide has a length of between 8-16 nucleobases.

15. The oligonucleotide of any one of claims 1 or 3-13, or the oligonucleotide for use according to any one of claims 2-13, wherein the oligonucleotide has a length of between 12-17 nucleobases.

16. The oligonucleotide of any one of claims 1 or 3-13, or the oligonucleotide for use according to any one of claims 2-13, wherein the oligonucleotide has a length of between 14-17 nucleobases.

17. The oligonucleotide of any one of claims 1 or 3-14, or the oligonucleotide for use according to any one of claims 2-14, wherein the oligonucleotide has a length of 10, 11, 12, 13, 14, or 15 nucleobases.

18. The oligonucleotide of any one of claims 1 or 3-15, or the oligonucleotide for use according to any one of claims 2-15, wherein the oligonucleotide has a length of 14 nucleobases.

19. The oligonucleotide of any one of claims 1 or 3-15, or the oligonucleotide for use according to any one of claims 2-15, wherein the oligonucleotide has a length of 15 nucleobases.

20. The oligonucleotide of any one of claims 1 or 3-15, or the oligonucleotide for use according to any one of claims 2-15, wherein the oligonucleotide has a length of 16 nucleobases.

21. The oligonucleotide of any one of claims 1 or 3-15, or the oligonucleotide for use according to any one of claims 2-15, wherein the oligonucleotide has a length of 17 nucleobases.

22. The oligonucleotide of any one of claims 1 or 3-14, or the oligonucleotide for use according to any one of claims 2-14, wherein the oligonucleotide has a length of between 8-16 nucleobases and wherein the oligonucleotide does not comprise a region of more than 7 consecutive LNA nucleotide units.

23. The oligonucleotide of any one of claims 1 or 3-14, or the oligonucleotide for use according to any one of claims 2-14, wherein the oligonucleotide has a length of between 8-16 nucleobases and wherein nucleobase units 1 to 6 (inclusive) of the single stranded oligonucleotide as measured from the 3' end of the single stranded oligonucleotide are complementary to the microRNA seed region sequence.

24. The oligonucleotide of any one of claims 1 or 3-14, or the oligonucleotide for use according to any one of claims 2-14, wherein the oligonucleotide has a length of between 8-16 nucleobases and wherein the oligonucleotide does not comprise a region of more than 7 consecutive LNA nucleotide units, further wherein nucleobase units 1 to 6 (inclusive) of the single stranded oligonucleotide as measured from the 3' end of the single stranded oligonucleotide are complementary to the microRNA seed region sequence.

25. The oligonucleotide of any one of claims 22-24 or the oligonucleotide for use according to any one of claims 22-24, wherein the oligonucleotide has a length of 14 nucleobases.

26. The oligonucleotide of any one of claims 22-24 or the oligonucleotide for use according to any one of claims 22-24, wherein the oligonucleotide has a length of 15 nucleobases.

27. The oligonucleotide of any one of claims 22-24 or the oligonucleotide for use according to any one of claims 22-24, wherein the oligonucleotide has a length of 16 nucleobases.

28. The oligonucleotide of any one of claims 1 or 3-27, or the oligonucleotide for use according to any one of claims 2-27, wherein the oligonucleotide has at least two LNA units in positions which are complementary to the miRNA seed region.

29. The oligonucleotide of any one of claims 1 or 3-28, or the oligonucleotide for use according to any one of claims 2-28, wherein the oligonucleotide has at least three LNA units in positions which are complementary to the miRNA seed region.

30. The oligonucleotide of any one of claims 1 or 3-29, or the oligonucleotide for use according to any one of claims 2-29, wherein the oligonucleotide has at least four LNA units in positions which are complementary to the miRNA seed region.

31. A pharmaceutical composition comprising the single stranded oligonucleotide of any one of claims 1 or 3-30 and a pharmaceutically acceptable diluent, carrier, or adjuvant.

## Patentansprüche

1. Einzelstrang-Oligonukleotid mit einer Länge von 8-17 Nukleotiden, mit der Fähigkeit zur Verringerung der wirksamen Menge einer reifen menschlichen miRNA in einer Zelle, wobei es sich bei wenigstens einer der Nukleobaseneinheiten des Oligonukleotids um eine LNA-Nukleotideinheit handelt und wobei es sich bei allen Internukleosidverknüpfungen um Phosphorothioatverknüpfungen handelt, wobei das Oligonukleotid zur reifen menschlichen miRNA-Sequenz komplementär ist, wobei das Einzelstrang-Oligonukleotid einen Bereich zusammenhängender Nukleobasensequenz umfasst, der zur seed-Region menschlicher miRNA 100% komplementär ist, und wobei das Oligonukleotid wenigstens eine LNA-Nukleotideinheit in einer Position umfasst, die innerhalb des zur miRNA-seed-Region komplementären Bereichs liegt, ferner wobei:
(a) Nukleobaseneinheiten 1 bis 6 (inklusive) des Einzelstrang-Oligonukleotids, wie vom 3'-Ende des Einzelstrang-Oligonukleotids gemessen, zur miRNA-seed-Region-Sequenz komplementär sind; oder
(b) Nukleobaseneinheiten 1 bis 7 (inklusive) des Einzelstrang-Oligonukleotids, wie vom 3'-Ende des Einzelstrang-Oligonukleotids gemessen, zur miRNA-seed-Region-Sequenz komplementär sind; oder
(c) Nukleobaseneinheiten 2 bis 7 (inklusive) des Einzelstrang-Oligonukleotids, wie vom 3'-Ende des Einzelstrang-Oligonukleotids gemessen, zur miRNA-seed-Region-Sequenz komplementär sind.

2. Einzelstrang-Oligonukleotid mit einer Länge von 8-17 Nukleotiden, mit der Fähigkeit zur Verringerung der wirksamen Menge einer reifen menschlichen miRNA in einer Zelle, wobei es sich bei wenigstens einer der Nukleobaseneinheiten des Oligonukleotids um eine LNA-Nukleotideinheit handelt und wobei es sich bei allen Internukleosidverknüpfungen um Phosphorothioatverknüpfungen handelt, wobei das Oligonukleotid zur reifen menschlichen miRNA-Sequenz komplementär ist, wobei das Einzelstrang-Oligonukleotid einen Bereich zusammenhängender Nukleobasensequenz umfasst, der zur seed-Region menschlicher miRNA 100% komplementär ist, und wobei das Oligonukleotid wenigstens eine LNA-Nukleotideinheit in einer Position umfasst, die innerhalb des zur miRNA-seed-Region komplementären Bereichs liegt, wobei das Oligonukleotid zur Verwendung als Arzneimittel vorgesehen ist, ferner wobei:
(a) Nukleobaseneinheiten 1 bis 6 (inklusive) des Einzelstrang-Oligonukleotids, wie vom 3'-Ende des Einzelstrang-Oligonukleotids gemessen, zur miRNA-seed-Region-Sequenz komplementär sind; oder
(b) Nukleobaseneinheiten 1 bis 7 (inklusive) des Einzelstrang-Oligonukleotids, wie vom 3'-Ende des Einzelstrang-Oligonukleotids gemessen, zur miRNA-seed-Region-Sequenz komplementär sind; oder
(c) Nukleobaseneinheiten 2 bis 7 (inklusive) des Einzelstrang-Oligonukleotids, wie vom 3'-Ende des Einzelstrang-Oligonukleotids gemessen, zur miRNA-seed-Region-Sequenz komplementär sind.

3. Oligonukleotid gemäß Anspruch 1 oder Oligonukleotid zur Verwendung gemäß Anspruch 2, wobei die erste oder zweite 3'-Nukleobase des Einzelstrang-Oligonukleotids dem zweiten 5'-Nukleotid der miRNA-Sequenz entspricht.

4. Oligonukleotid nach Anspruch 1 oder Anspruch 3 oder Oligonukleotid zur Verwendung gemäß Anspruch 2, wobei das Oligonukleotid zur Bildung eines Duplex mit einem komplementären Einzelstrang-RNA-Nukleinsäuremolekül mit Phosphodiester-Internukleosidverknüpfungen fähig ist, wobei das Duplex eine Tm von wenigstens etwa 60°C aufweist.

5. Oligonukleotid nach einem der Ansprüche 1, 3 oder 4 oder Oligonukleotid zur Verwendung gemäß einem der Ansprüche 2-4, wobei das Oligonukleotid zur Bildung eines Duplex mit einem komplementären Einzelstrang-DNA-Nukleinsäuremolekül mit Phosphodiester-Internukleosidverknüpfungen fähig ist, wobei das Duplex eine Tm von 60 - 90°C aufweist.

6. Oligonukleotid nach einem der Ansprüche 1 oder 3-5 oder Oligonukleotid zur Verwendung gemäß einem der Ansprüche 2-5, das wenigstens eine 3'-terminale LNA-Nukleotideinheit umfasst.

7. Oligonukleotid nach einem der Ansprüche 1 oder 3-6 oder Oligonukleotid zur Verwendung gemäß einem der Ansprüche 2-6, das wenigstens eine 5'-terminale LNA-Nukleotideinheit umfasst.

8. Oligonukleotid nach einem der Ansprüche 1 oder 3-7 oder Oligonukleotid zur Verwendung gemäß einem der Ansprüche 2-7, wobei es sich bei wenigstens 30% der Nukleobasen um LNA-Einheiten handelt.

9. Oligonukleotid nach einem der Ansprüche 1 oder 3-8 oder Oligonukleotid zur Verwendung gemäß einem der Ansprüche 2-8, wobei das Oligonukleotid wenigstens einen Bereich von wenigstens zwei aufeinanderfolgenden LNA-Nukleotideinheiten umfasst.

10. Oligonukleotid nach einem der Ansprüche 1 oder 3-9 oder Oligonukleotid zur Verwendung gemäß einem der Ansprüche 2-9, wobei das Oligonukleotid keinen Bereich von mehr als 7 aufeinanderfolgenden LNA-Nukleotideinheiten umfasst.

11. Oligonukleotid nach einem der Ansprüche 1 oder 3-10 oder Oligonukleotid zur Verwendung gemäß einem der Ansprüche 2-10, wobei das Oligonukleotid keinen Bereich von mehr als 6 oder mehr als 5 oder mehr als 4 oder mehr als 3 oder mehr als 2 aufeinanderfolgenden LNA-Nukleotideinheiten umfasst.

12. Oligonukleotid nach einem der Ansprüche 1 oder 3-11 oder Oligonukleotid zur Verwendung gemäß einem der Ansprüche 2-11, das keinen Bereich von mehr als 5 aufeinanderfolgenden DNA-Nukleotideinheiten umfasst.

13. Oligonukleotid nach einem der Ansprüche 1 oder 3-12 oder Oligonukleotid zur Verwendung gemäß einem der Ansprüche 2-12, wobei das Oligonukleotid keine RNAseH-basierte Spaltung eines komplementären Einzelstrang-RNA-Moleküls vermittelt.

14. Oligonukleotid nach einem der Ansprüche 1 oder 3-13 oder Oligonukleotid zur Verwendung gemäß einem der Ansprüche 2-13, wobei das Oligonukleotid eine Länge zwischen 8-16 Nukleobasen aufweist.

15. Oligonukleotid nach einem der Ansprüche 1 oder 3-13 oder Oligonukleotid zur Verwendung gemäß einem der Ansprüche 2-13, wobei das Oligonukleotid eine Länge zwischen 12-17 Nukleobasen aufweist.

16. Oligonukleotid nach einem der Ansprüche 1 oder 3-13 oder Oligonukleotid zur Verwendung gemäß einem der Ansprüche 2-13, wobei das Oligonukleotid eine Länge zwischen 14-17 Nukleobasen aufweist.

17. Oligonukleotid nach einem der Ansprüche 1 oder 3-14 oder Oligonukleotid zur Verwendung gemäß einem der Ansprüche 2-14, wobei das Oligonukleotid eine Länge von 10, 11, 12, 13, 14 oder 15 Nukleobasen aufweist.

18. Oligonukleotid nach einem der Ansprüche 1 oder 3-15 oder Oligonukleotid zur Verwendung gemäß einem der Ansprüche 2-15, wobei das Oligonukleotid eine Länge von 14 Nukleobasen aufweist.

19. Oligonukleotid nach einem der Ansprüche 1 oder 3-15 oder Oligonukleotid zur Verwendung gemäß einem der Ansprüche 2-15, wobei das Oligonukleotid eine Länge von 15 Nukleobasen aufweist.

20. Oligonukleotid nach einem der Ansprüche 1 oder 3-15 oder Oligonukleotid zur Verwendung gemäß einem der Ansprüche 2-15, wobei das Oligonukleotid eine Länge von 16 Nukleobasen aufweist.

21. Oligonukleotid nach einem der Ansprüche 1 oder 3-15 oder Oligonukleotid zur Verwendung gemäß einem der Ansprüche 2-15, wobei das Oligonukleotid eine Länge von 17 Nukleobasen aufweist.

22. Oligonukleotid nach einem der Ansprüche 1 oder 3-14 oder Oligonukleotid zur Verwendung gemäß einem der Ansprüche 2-14, wobei das Oligonukleotid eine Länge zwischen 8-16 Nukleobasen aufweist und wobei das Oligonukleotid keinen Bereich von mehr als 7 aufeinanderfolgenden LNA-Nukleotideinheiten umfasst.

23. Oligonukleotid nach einem der Ansprüche 1 oder 3-14 oder Oligonukleotid zur Verwendung gemäß einem der Ansprüche 2-14, wobei das Oligonukleotid eine Länge zwischen 8-16 Nukleobasen aufweist und wobei Nukleobaseneinheiten 1 bis 6 (inklusive) des Einzelstrang-Oligonukleotids, wie vom 3'-Ende des Einzelstrang-Oligonukleotids gemessen, zur miRNA-seed-Region-Sequenz komplementär sind.

24. Oligonukleotid nach einem der Ansprüche 1 oder 3-14 oder Oligonukleotid zur Verwendung gemäß einem der Ansprüche 2-14, wobei das Oligonukleotid eine Länge zwischen 8-16 Nukleobasen aufweist und wobei das Oligonukleotid keinen Bereich von mehr als 7 aufeinanderfolgenden LNA-Nukleotideinheiten umfasst, ferner wobei Nukleobaseneinheiten 1 bis 6 (inklusive) des Einzelstrang-Oligonukleotids, wie vom 3'-Ende des Einzelstrang-Oligonukleotids gemessen, zur miRNA-seed-Region-Sequenz komplementär sind.

25. Oligonukleotid nach einem der Ansprüche 22-24 oder Oligonukleotid zur Verwendung gemäß einem der Ansprüche 22-24, wobei das Oligonukleotid eine Länge von 14 Nukleobasen aufweist.

26. Oligonukleotid nach einem der Ansprüche 22-24 oder Oligonukleotid zur Verwendung gemäß einem der Ansprüche 22-24, wobei das Oligonukleotid eine Länge von 15 Nukleobasen aufweist.

27. Oligonukleotid nach einem der Ansprüche 22-24 oder Oligonukleotid zur Verwendung gemäß einem der Ansprüche 22-24, wobei das Oligonukleotid eine Länge von 16 Nukleobasen aufweist.

28. Oligonukleotid nach einem der Ansprüche 1 oder 3-27 oder Oligonukleotid zur Verwendung gemäß einem der Ansprüche 2-27, wobei das Oligonukleotid wenigstens zwei LNA-Einheiten in Positionen aufweist, die zur miRNA-seed-Region komplementär sind.

29. Oligonukleotid nach einem der Ansprüche 1 oder 3-28 oder Oligonukleotid zur Verwendung gemäß einem der Ansprüche 2-28, wobei das Oligonukleotid wenigstens drei LNA-Einheiten in Positionen aufweist, die zur miRNA-seed-Region komplementär sind.

30. Oligonukleotid nach einem der Ansprüche 1 oder 3-29 oder Oligonukleotid zur Verwendung gemäß einem der Ansprüche 2-29, wobei das Oligonukleotid wenigstens vier LNA-Einheiten in Positionen aufweist, die zur miRNA-seed-Region komplementär sind.

31. Pharmazeutische Zusammensetzung, umfassend das Einzelstrang-Oligonukleotid nach einem der Ansprüche 1 oder 3-30 und ein pharmazeutisch unbedenkliches Verdünnungsmittel, Trägermittel oder Adjuvans.

## Revendications

1. Oligonucléotide simple brin, de 8 à 17 nucléotides de longueur, capable de réduire la quantité efficace d'un microARN humain mature dans une cellule, dans lequel au moins l'un des motifs de bases azotées de l'oligonucléotide est un motif de nucléotides LNA, et dans lequel toutes les liaisons internucléosidiques sont des liaisons phosphorothioate, l'oligonucléotide étant complémentaire de la séquence de microARN humain mature, l'oligonucléotide simple brin comprenant une région de séquence de bases azotées contiguës qui est 100 % complémentaire de la région de germe de microARN humain, et l'oligonucléotide comprenant au moins un motif de nucléotides LNA à une position qui est dans la région complémentaire de la région de germe de miARN, dans lequel, en outre :
(a) les motifs de bases azotées 1 à 6 (inclus) de l'oligonucléotide simple brin tels que mesurés à partir de l'extrémité 3' de l'oligonucléotide simple brin sont complémentaires de la région de microARN de séquence de germe ; ou
(b) les motifs de bases azotées 1 à 7 (inclus) de l'oligonucléotide simple brin tels que mesurés à partir de l'extrémité 3' de l'oligonucléotide simple brin sont complémentaires de la région de microARN de séquence de germe ; ou
(c) les motifs de bases azotées 2 à 7 (inclus) de l'oligonucléotide simple brin tels que mesurés à partir de l'extrémité 3' de l'oligonucléotide simple brin sont complémentaires de la région de microARN de séquence de germe.

2. Oligonucléotide simple brin, de 8 à 17 nucléotides de longueur, capable de réduire la quantité efficace d'un microARN humain mature dans une cellule, dans lequel au moins l'un des motifs de bases azotées de l'oligonucléotide est un motif de nucléotides LNA, et dans lequel toutes les liaisons internucléosidiques sont des liaisons phosphorothioate, l'oligonucléotide étant complémentaire de la séquence de microARN humain mature, l'oligonucléotide simple brin comprenant une région de séquence de bases azotées contiguës qui est 100 % complémentaire de la région de germe de microARN humain, et l'oligonucléotide comprenant au moins un motif de nucléotides LNA à une position qui est dans la région complémentaire de la région de germe de miARN, l'oligonucléotide étant pour utilisation en tant que médicament, dans lequel, en outre :
(a) les motifs de bases azotées 1 à 6 (inclus) de l'oligonucléotide simple brin tels que mesurés à partir de l'extrémité 3' de l'oligonucléotide simple brin sont complémentaires de la région de microARN de séquence de germe ; ou
(b) les motifs de bases azotées 1 à 7 (inclus) de l'oligonucléotide simple brin tels que mesurés à partir de l'extrémité 3' de l'oligonucléotide simple brin sont complémentaires de la région de microARN de séquence de germe ; ou
(c) les motifs de bases azotées 2 à 7 (inclus) de l'oligonucléotide simple brin tels que mesurés à partir de l'extrémité 3' de l'oligonucléotide simple brin sont complémentaires de la région de microARN de séquence de germe.

3. Oligonucléotide selon la revendication 1 ou oligonucléotide pour utilisation selon la revendication 2, dans lequel la première ou la deuxième base azotée en 3' de l'oligonucléotide simple brin correspond au deuxième nucléotide en 5' de la séquence de microARN.

4. Oligonucléotide selon la revendication 1 ou la revendication 3, ou oligonucléotide pour utilisation selon la revendication 2, l'oligonucléotide étant capable de former une double hélice avec une molécule d'acide nucléique d'ARN simple brin complémentaire avec des liaisons internucléosidiques phosphodiester, dans lequel la double hélice a un Tm d'au moins environ 60 °C.

5. Oligonucléotide selon l'une quelconque des revendications 1, 3 ou 4, ou oligonucléotide pour utilisation selon l'une quelconque des revendications 2 à 4, l'oligonucléotide étant capable de former une double hélice avec une molécule d'acide nucléique d'ADN simple brin complémentaire avec des liaisons internucléosidiques phosphodiester, dans lequel la double hélice a un Tm de 60 à 90 °C.

6. Oligonucléotide selon l'une quelconque des revendications 1 ou 3 à 5, ou oligonucléotide pour utilisation selon l'une quelconque des revendications 2 à 5, qui comprend au moins un motif de nucléotides LNA terminal en 3'.

7. Oligonucléotide selon l'une quelconque des revendications 1 ou 3 à 6, ou oligonucléotide pour utilisation selon l'une quelconque des revendications 2 à 6, qui comprend au moins un motif de nucléotides LNA terminal en 5'.

8. Oligonucléotide selon l'une quelconque des revendications 1 ou 3 à 7, ou oligonucléotide pour utilisation selon l'une quelconque des revendications 2 à 7, dans lequel au moins 30 % des bases azotées sont des motifs LNA.

9. Oligonucléotide selon l'une quelconque des revendications 1 ou 3 à 8, ou oligonucléotide pour utilisation selon l'une quelconque des revendications 2 à 8, l'oligonucléotide comprenant au moins une région d'au moins deux motifs de nucléotides LNA consécutifs.

10. Oligonucléotide selon l'une quelconque des revendications 1 ou 3 à 9, ou oligonucléotide pour utilisation selon l'une quelconque des revendications 2 à 9, l'oligonucléotide ne comprenant pas une région de plus de 7 motifs de nucléotides LNA consécutifs.

11. Oligonucléotide selon l'une quelconque des revendications 1 ou 3 à 10, ou oligonucléotide pour utilisation selon l'une quelconque des revendications 2 à 10, l'oligonucléotide ne comprenant pas une région de plus de 6, ou plus de 5, ou plus de 4, ou plus de 3 ou plus de 2 motifs de nucléotides LNA consécutifs.

12. Oligonucléotide selon l'une quelconque des revendications 1 ou 3 à 11, ou oligonucléotide pour utilisation selon l'une quelconque des revendications 2 à 11, qui ne comprend pas une région de plus de 5 motifs de nucléotides d'ADN consécutifs.

13. Oligonucléotide selon l'une quelconque des revendications 1 ou 3 à 12, ou oligonucléotide pour utilisation selon l'une quelconque des revendications 2 à 12, l'oligonucléotide ne médiant pas un clivage à base de RNAseH d'une molécule d'ARN simple brin complémentaire.

14. Oligonucléotide selon l'une quelconque des revendications 1 ou 3 à 13, ou oligonucléotide pour utilisation selon l'une quelconque des revendications 2 à 13, l'oligonucléotide ayant une longueur comprise entre 8 et 16 bases azotées.

15. Oligonucléotide selon l'une quelconque des revendications 1 ou 3 à 13, ou oligonucléotide pour utilisation selon l'une quelconque des revendications 2 à 13, l'oligonucléotide ayant une longueur comprise entre 12 et 17 bases azotées.

16. Oligonucléotide selon l'une quelconque des revendications 1 ou 3 à 13, ou oligonucléotide pour utilisation selon l'une quelconque des revendications 2 à 13, l'oligonucléotide ayant une longueur comprise entre 14 et 17 bases azotées.

17. Oligonucléotide selon l'une quelconque des revendications 1 ou 3 à 14, ou oligonucléotide pour utilisation selon l'une quelconque des revendications 2 à 14, l'oligonucléotide ayant une longueur de 10, 11, 12, 13, 14, ou 15 bases azotées.

18. Oligonucléotide selon l'une quelconque des revendications 1 ou 3 à 15, ou oligonucléotide pour utilisation selon l'une quelconque des revendications 2 à 15, l'oligonucléotide ayant une longueur de 14 bases azotées.

19. Oligonucléotide selon l'une quelconque des revendications 1 ou 3 à 15, ou oligonucléotide pour utilisation selon l'une quelconque des revendications 2 à 15, l'oligonucléotide ayant une longueur de 15 bases azotées.

20. Oligonucléotide selon l'une quelconque des revendications 1 ou 3 à 15, ou oligonucléotide pour utilisation selon l'une quelconque des revendications 2 à 15, l'oligonucléotide ayant une longueur de 16 bases azotées.

21. Oligonucléotide selon l'une quelconque des revendications 1 ou 3 à 15, ou oligonucléotide pour utilisation selon l'une quelconque des revendications 2 à 15, l'oligonucléotide ayant une longueur de 17 bases azotées.

22. Oligonucléotide selon l'une quelconque des revendications 1 ou 3 à 14, ou oligonucléotide pour utilisation selon l'une quelconque des revendications 2 à 14, l'oligonucléotide ayant une longueur comprise entre 8 et 16 bases azotées et l'oligonucléotide ne comprenant pas une région de plus de 7 motifs de nucléotides LNA consécutifs.

23. Oligonucléotide selon l'une quelconque des revendications 1 ou 3 à 14, ou oligonucléotide pour utilisation selon l'une quelconque des revendications 2 à 14, l'oligonucléotide ayant une longueur comprise entre 8 et 16 bases azotées et dans lequel les motifs de bases azotées 1 à 6 (inclus) de l'oligonucléotide simple brin tels que mesurés à partir de l'extrémité 3' de l'oligonucléotide simple brin sont complémentaires de la région de microARN de séquence de germe.

24. Oligonucléotide selon l'une quelconque des revendications 1 ou 3 à 14, ou oligonucléotide pour utilisation selon l'une quelconque des revendications 2 à 14, l'oligonucléotide ayant une longueur comprise entre 8 et 16 bases azotées et l'oligonucléotide ne comprenant pas une région de plus de 7 motifs de nucléotides LNA consécutifs, en outre, dans lequel les motifs de bases azotées 1 à 6 (inclus) de l'oligonucléotide simple brin tels que mesurés à partir de l'extrémité 3' de l'oligonucléotide simple brin sont complémentaires de la région de microARN de séquence de germe.

25. Oligonucléotide selon l'une quelconque des revendications 22 à 24 ou oligonucléotide pour utilisation selon l'une quelconque des revendications 22 à 24, l'oligonucléotide ayant une longueur de 14 bases azotées.

26. Oligonucléotide selon l'une quelconque des revendications 22 à 24 ou oligonucléotide pour utilisation selon l'une quelconque des revendications 22 à 24, l'oligonucléotide ayant une longueur de 15 bases azotées.

27. Oligonucléotide selon l'une quelconque des revendications 22 à 24 ou oligonucléotide pour utilisation selon l'une quelconque des revendications 22 à 24, l'oligonucléotide ayant une longueur de 16 bases azotées.

28. Oligonucléotide selon l'une quelconque des revendications 1 ou 3 à 27, ou oligonucléotide pour utilisation selon l'une quelconque des revendications 2 à 27, l'oligonucléotide comportant au moins deux motifs LNA à des positions qui sont complémentaires de la région de germe de miARN.

29. Oligonucléotide selon l'une quelconque des revendications 1 ou 3 à 28, ou oligonucléotide pour utilisation selon l'une quelconque des revendications 2 à 28, l'oligonucléotide comportant au moins trois motifs LNA à des positions qui sont complémentaires de la région de germe de miARN.

30. Oligonucléotide selon l'une quelconque des revendications 1 ou 3 à 29, ou oligonucléotide pour utilisation selon l'une quelconque des revendications 2 à 29, l'oligonucléotide comportant au moins quatre motifs LNA à des positions qui sont complémentaires de la région de germe de miARN.

31. Composition pharmaceutique comprenant l'oligonucléotide simple brin selon l'une quelconque des revendications 1 ou 3 à 30 et un diluant, véhicule ou adjuvant pharmaceutiquement acceptable.
